(19) **Europäisches Patentamt**
**European Patent Office**
**Office européen des brevets**

(11) **EP 3 445 391 B1**

(12) **EUROPEAN PATENT SPECIFICATION**

(45) Date of publication and mention
of the grant of the patent:
**02.07.2025 Bulletin 2025/27**

(21) Application number: **17721339.4**

(22) Date of filing: **12.04.2017**

(51) International Patent Classification (IPC):
***A61K 39/00*** *(2006.01)*

(52) Cooperative Patent Classification (CPC):
**A61P 35/02; A61K 40/11; A61K 40/421;
A61K 40/4211; A61K 40/4217; C12N 5/0636;
C12N 5/0637; C12N 5/0638;** A61K 2039/572;
A61K 2239/48

(86) International application number:
**PCT/EP2017/058891**

(87) International publication number:
**WO 2017/178572 (19.10.2017 Gazette 2017/42)**

(54) **EX VIVO BITE-ACTIVATED T CELLS**

EX VIVO VON BITE AKTIVIERTE T-ZELLEN

CÉLLULLES T ACTIVÉES PAR BITE EX VIVO

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.04.2016 US 201662321964 P**

(43) Date of publication of application:
**27.02.2019 Bulletin 2019/09**

(73) Proprietor: **Vivia Biotech, S.L.
28760 Madrid (ES)**

(72) Inventors:
• **BALLESTEROS NOBELL, Juan Antonio
28760 Madrid (ES)**
• **BENNET, Teresa Ann
28760 Madrid (ES)**
• **HERNÁNDEZ CAMPO, Pilar
28760 Madrid (ES)**
• **GÓMEZ GONZÁLEZ, Cristina
28760 Madrid (ES)**
• **GORROCHATEGUI GUILLÉN, Julián
28760 Madrid (ES)**
• **MARTÍNEZ LÓPEZ, Joaquín
28005 Madrid (ES)**
• **ROBLES MATEOS, Alicia
28760 Madrid (ES)**

• **PRIMO RAMOS, Daniel
28760 Madrid (ES)**

(74) Representative: **Manuel Illescas y Asociados, S.L.
C/ Príncipe de Vergara 197, Oficina 1°A
28002 Madrid (ES)**

(56) References cited:
**WO-A2-03/015705**

• **FORSLUND ANN ET AL: "Ex Vivo Activity Profile
of the CD123xCD3 Duobody (R) Antibody
JNJ-63709178 Against Primary Acute Myeloid
Leukemia Bone Marrow Samples", BLOOD, vol.
128, no. 22, 2 December 2016 (2016-12-02), &
58TH ANNUAL MEETING AND EXPOSITION OF
THE AMERICAN-SOCIETY-OF-HEMATOLOGY;
SAN DIEGO, CA, USA; DECEMBER 03 -06, 2016,
pages 2875, XP009500052, ISSN: 0006-4971**
• **WONG RYAN ET AL: "Blinatumomab induces
autologous T-cell killing of chronic lymphocytic
leukemia cells", HAEMATOLOGICA, vol. 98, no.
12, December 2013 (2013-12-01), pages 1930 -
1938, XP055358323, ISSN: 0390-6078**
• **BOHLEN H ET AL: "Cytolysis of leukemic B-cells
by T-cells activated via two bispecific
antibodies", CANCER RESEARCH, vol. 53, no.
18, 1993, pages 4310 - 4314, XP002916499, ISSN:
0008-5472**

EP 3 445 391 B1

- KUO SHU-RU ET AL: "Engineering a CD123xCD3 bispecific scFv immunofusion for the treatment of leukemia and elimination of leukemia stem cells.", PROTEIN ENGINEERING, DESIGN & SELECTION : PEDS OCT 2012, vol. 25, no. 10, October 2012 (2012-10-01), pages 561 - 569, XP002771673, ISSN: 1741-0134
- SOMANCHI SRINIVAS S ET AL: "Engineering lymph node homing of ex vivo-expanded human natural killer cells via trogocytosis of the chemokine receptor CCR7", BLOOD, vol. 119, no. 22, May 2012 (2012-05-01), pages 5164 - 5172, XP002771674, ISSN: 0006-4971
- WU KANGNI ET AL: "Ex Vivo-Expanded V gamma 9V delta 2 T Cells Can Efficiently Kill Human Acute Myeloid Leukemia Cells Via Trogocytosis", BLOOD, vol. 118, no. 21, November 2011 (2011-11-01), & 53RD ANNUAL MEETING AND EXPOSITION OF THE AMERICAN-SOCIETY-OF-HEMATOLOGY (ASH); SAN DIEGO, CA, USA; DECEMBER 10 -13, 2011, pages 267 - 268, XP009500061, ISSN: 0006-4971

**Description**

**Background of the Invention**

Adoptive cell therapy (ACT) is a process involving collection of immune cells from a patient, expansion of the cells, and reintroduction of the cells into the same patient or a different patient. For example, ACT of donor-derived, *ex-vivo* expanded human cytotoxic T lymphocytes (CTLs) has emerged as a promising approach to treat cancer. Examples of ACT include cultured tumor infiltrating lymphocytes (TILs), isolated and expanded T cell clones, and genetically engineered lymphocytes (e.g., T cells) that express conventional T cell receptors or chimeric antigen receptors. The genetically engineered lymphocytes are designed to eliminate cancer cells expressing specific antigen(s) and are expanded and delivered to a patient. Another example of an ACT is the isolation and use of T cells from a patient's blood after administration of a cancer vaccine. ACT can provide tumor specific lymphocytes (e.g., T cells) that lead to a reduction in tumor cells in a patient.

However, limitations with ACT can include, for example, off-target activation of T cells and/or concerns around choosing the antigens that are least likely to target non-cancer tissues. There remains a need for a method of selecting the appropriate subset of T cells (e.g., CTLs) to provide an effective therapy, while minimizing the adverse side effects.

Document by Wong et al. (Wong et al. (2013). Blinatumomab induces autologous T-cell killing of chronic lymphocytic leukemia cells. Haematologica 98(12):1930-8) discloses tests with blinatumomab on peripheral blood mononuclear cells from several patients. This document further discloses measures of T-cell activation and function and cytotoxicity against leukemic cells.

**Summary of the Invention**

The present disclosure features, at least in part, an optimized approach to generate an activated immune effector cell (e.g., an activated T cell) that selectively and effectively targets a diseased tissue (e.g., cancer).

In one embodiment, the activated immune cell (e.g., the activated T cell) induces killing of a target cell, e.g., a cancer cell. Activated T cells are generally referred to herein as "cancer-killing T cells", which term includes activated tumor antigen-specific T cells, including, but not limited to, effector memory T cells, cytotoxic T lymphocytes (CTLs), helper T cells, tumor infiltrating lymphocytes (TILs) and trogocytotic T cells. The disclosure is based, at least in part, on the surprising discovery that generation and identification of highly effective immune effector cell (e.g., a T cell) in terms of target cell-killing activity can be enhanced, e.g., by optimizing the proximity between a target (e.g., a cancer) cell and the immune effector cell.

The cancer-killing T cells described herein can provide highly effective therapies for diverse cancer types, e.g., solid cancers, hematological cancers, and metastatic forms thereof. Therapies using the cancer-killing T cells disclosed herein are also suited for treating cancers that typically do not elicit a strong immune response in a subject, e.g., a cancer other than a melanoma. In embodiments, the cancer therapies disclosed herein can be tailored or personalized to a given subject, e.g., by generating cancer-killing T cells (e.g., autologous cancer-killing T cells) that selectively and effectively target the subject's cancer.

Disclosed herein are methods of generating immune effector cells that have enhanced target cell killing activity (e.g., cancer-killing T cells); compositions comprising such immune cells; methods of using the cells (e.g., methods of treatment); methods of selecting optimal agents for enhancing the target cell killing activity, e.g., by enhancing the proximity, e.g., spatial proximity, between the target cell and the immune cell, e.g., T cell; methods of selecting an optimized (e.g., highest activity fractions/clones) immune cell, e.g., T cell; and methods of using this approach to evaluate patient responsiveness to other cancer therapies.

The invention is set out in the appended set of claims.

*Method of Producing a cancer-killing T cell*

In one aspect of the disclosure, a method of producing (e.g., making/providing) a cancer-killing T cell includes:

(a) providing a T cell from a subject having a cancer (e.g., a hematological cancer, a solid cancer, a metastatic cancer, or a combination thereof);
(b) providing a cancer cell, e.g., from the subject;
(c) forming an *ex vivo* reaction mixture comprising the T cell, the cancer cell, and a proximity immuno-coaching factor (pICF), e.g., under conditions (e.g., for a period of time) sufficient to activate the T cell, e.g., by allowing the T cell to acquire a cell surface marker from the cancer cell. In some aspects of the disclorure, the activated T cell acquires a cell surface marker from the cancer cell, e.g., becomes a trogocytotic T cell.

In some aspects of the disclosure, the cancer cell and the T cell are present in a sample, e.g., a sample from the subject having the cancer. In one embodiment, the cancer cell and the T cell are present in the same sample, e.g., a single sample (e.g., a sample as described herein). In other aspects of the disclosure, the cancer cell and the T cell are present in different samples, e.g., one or more samples, e.g., from the subject having the cancer (e.g., one or more samples as described herein).

In aspects of the disclosure, step (a) and step (b) of the method (e.g., of producing) comprise providing one sample comprising both the cancer cell and the T cell.

In aspects of the disclosure, step (a) and step (b) of the method (e.g., of producing) comprise providing two samples, wherein one of the two samples comprises the cancer cell, and the other sample comprises the T cell.

In aspects of the disclosure, the method further includes one, two, three or all of:

i) expanding the cancer-killing T cell from (c);
ii) selecting the cancer-killing T cell from (c);
iii) enriching for the cancer-killing T cell from (c); or
iv) purifying the cancer-killing T cell from (c).

In another aspect of the disclosure, also disclosed herein is a method of producing (e.g., making/providing) a cancer-killing T cell. The method includes:

(a) providing a sample comprising a cancer cell and a T cell ("a cancer and a T cell sample"), e.g., from a subject having a cancer (e.g., a hematological cancer, a solid cancer, a metastatic cancer, or a combination thereof);
(b) contacting the sample with a proximity immune-coaching factor (pICF), e.g., under conditions (e.g., for a period of time) sufficient to activate the T cell, e.g., by allowing the T cell to acquire a cell surface marker from the cancer cell (in embodiments, forming an *ex-vivo* reaction mixture that produces the cancer-killing T cell);
(c) enriching for the cancer-killing T cell, e.g., an activated T cell (e.g., a T cell that has acquired a cell surface marker from the cancer cell).

In yet another aspect of the disclosure, featured herein is a method of producing (e.g., making/providing) a cancer-killing T cell. The method includes:

(a) providing a cancer sample, e.g., a sample comprising a cancer cell (e.g., a cancer cell from: a hematological cancer, a solid cancer, a metastatic cancer (e.g., a circulating tumor cell (CTC), or a combination thereof), e.g., from a subject having a cancer;
(b) providing a T cell sample, e.g., a sample comprising a T cell (e.g., a blood sample (e.g., peripheral blood sample), a bone marrow sample, a lymph node sample, a spleen sample, a tumor sample comprising a cytotoxic T lymphocyte (CTL) and/or a Tumor Infiltrating Lymphocyte (TIL), or a combination thereof), e.g., from the subject having the cancer;
(c) contacting the sample from step (a) with the sample from step (b) and a proximity immune-coaching factor (pICF), e.g., under conditions (e.g., for a period of time) sufficient to activate the T cell, e.g., to allow the T cell to acquire a cell surface marker from the cancer cell (in embodiments, forming an *ex-vivo* reaction mixture that produces the cancer-killing T cell); and
(d) enriching for the cancer-killing T cell, e.g., the activated T cell (e.g., the T cell that has acquired a cell surface marker from the cancer cell).

In some aspects of the disclosure of any of the methods and compositions disclosed herein, the sample is a cancer sample chosen from a hematological cancer, a solid cancer, a metastatic cancer (e.g., a CTC, a primary, secondary or additional metastatic cancer), or a combination thereof.

In another aspect of the disclosure of any of the methods and compositions disclosed herein, the sample is a T cell sample chosen from a blood sample (e.g., peripheral blood sample), a bone marrow sample, a lymph node sample, a spleen sample, a tumor sample comprising a CTL, a TIL, or a combination thereof.

In aspects of the disclosure of any of the methods and compositions disclosed herein, substantially no components (e.g., cells) have been removed or isolated from the sample.

In aspects of the disclosure of any of the methods and compositions disclosed herein, the sample substantially maintains the microenvironment from the tissue of origin, e.g., substantially maintains the structure of the tumor or immune microenvironment.

In aspects of the disclosure of any of the methods and compositions disclosed herein, the sample comprises a tumor-antigen specific T cell (e.g., a CTL or a TIL). Without being bound by theory, tumor-antigen specific T cells can be immunosuppressed, e.g., when present in the tumor microenvironment. In one aspect of the disclosure, the immunosuppressed tumor-antigen specific T cell is activated under the conditions described herein, e.g., upon contact with the cancer cell and the pICF.

In some aspects of the disclosure of any of the methods and compositions disclosed herein, the sample or samples comprise the cancer cell and the T cell. For example, the sample may be from a hematological cancer (e.g., bone marrow, lymph-node derived cancer) that includes a T cell (e.g., a tumor-antigen specific CTL). The hematological sample may also comprise cancer cells, e.g., leukemic or lymphoma blast cells (e.g., a blast cell expressing one or more markers chosen from CD19, CD123, CD20 or others). In aspects of the disclosure, addition of the pICF to the sample promotes an interaction between the T cell and the cancer cell that activates the T cell (e.g., activates the tumor-antigen specific CTL). In some aspects of the disclosure, the activated T cell acquires a cell surface marker from the cancer cell, e.g., becomes a trogocytotic T cell.

In other aspects of the disclosure of any of the methods and compositions disclosed herein, the cancer is a solid tumor. The sample may comprise a tumor-antigen specific T cell (e.g., a CTL or a TIL) as described herein and a cancer cell. In aspects of the disclosure, addition of the pICF to the sample promotes an interaction between the T cell and the cancer cell that activates the T cell (e.g., activates the tumor-antigen specific CTL or TIL). In some embodiments, the activated T cell acquires a cell surface marker from the cancer cell, e.g., becomes a trogocytotic T cell.

In other aspects of the disclosure of any of the methods and compositions disclosed herein, the sample comprises a metastatic sample, e.g., a sample derived from a subject with a metastatic cancer. In one aspect of the disclosure, the metastatic sample comprises a CTC. In aspects of the disclosure, the CTC is a tumor cell found in the peripheral blood of a subject with a cancer, e.g., a solid tumor. An *ex vivo* reaction mixture can be formed comprising a metastatic cancer cell and a T cell. In aspects of the disclosure, the T cell can be obtained from the metastatic cancer sample (e.g., a primary tumor sample or a secondary tumor sample, or a combination thereof). In some aspects of the disclosure, the *ex vivo* reaction mixture comprises a tumor-antigen specific T cell (e.g., a CTL or a TIL) that targets the metastatic sample (e.g., that targets the CTC, the primary tumor sample or a secondary tumor sample, or a combination thereof). In aspects of the disclosure, the tumor-antigen specific T cell is activated in the presence of the pICF and the metastatic sample (e.g., the CTC, the primary tumor sample or the secondary tumor sample, or a combination thereof). For example, in a metastatic cancer, tumor growth may occur in tissues different from the primary tumor site, e.g., referred to herein as secondary tumors. Cancer cells from the primary tumor may be different from secondary or other metastatic sites. For example, bone marrow tumor infiltration may occur in a solid tumor. As another example, metastatic tumor cells from a solid cancer, e.g., pancreas or breast cancer, that grow in the bone marrow can be biologically different from the tumor cells in the primary tumor. In such aspects of the disclosure, activation of a T cell in the presence of the pICF can be repeated in every tissue affected by the tumor cells in the subject. In such aspects of the disclosure, the activated T cells (e.g., the activated tumor-antigen specific T cells) can be selective against the primary and secondary tumors present in the subject.

In one aspect of the disclosure, the sample comprises a CTC. An *ex vivo* reaction mixture can be formed with the CTC-containing sample with a sample from the primary and secondary tumors present in the subject, thereby producing activated T cells (e.g., the activated tumor-antigen specific T cells) selective against the CTCs, the primary and secondary tumors present in the subject.

*Composition and Reaction Mixtures*

For the purposes of the present specification, the term "composition" includes cancer-killing T cells, which term includes activated tumor antigen-specific T cells, including, but not limited to, effector memory T cells, cytotoxic T lymphocytes (CTLs), helper T cells, tumor infiltrating lymphocytes (TILs) and trogocytotic T cells, and pharmaceutical compositions thereof.

In an aspect of the disclosure, also featured herein is an *ex vivo* reaction mixture comprising a T cell, a cancer cell, and a proximity immuno-coaching factor (pICF), where the T cell and the cancer cell are in a sample, e.g., a blood sample (e.g., whole blood, peripheral blood); a sample from a hematological cancer; a sample from a bone marrow, a sample from a lymph node; or a sample from a spleen, a sample from a solid tumor; a sample from a metastatic cancer (e.g., a CTC); where substantially no components (e.g., cells) have been removed or isolated from the sample.

In aspects of the disclosure, the sample is from a subject having a cancer, e.g., a hematological cancer, a solid cancer or a metastatic cancer.

In aspects of the disclosure, the sample substantially maintains the microenvironment, e.g., substantially maintains the structure of the tumor microenvironment.

In aspects of the disclosure, the sample comprises a tumor-antigen specific T cell (e.g., a CTL or a TIL). Without being bound by theory, the tumor-antigen specific T cell can be immunosuppressed, e.g., when present in the tumor microenvironment. In one aspect of the disclosure, the immunosuppressed tumor-antigen specific T cell can be activated under the conditions described herein, e.g., upon contact with the cancer cell and the pICF.

Another aspect of the disclosure refers to a composition, e.g., a pharmaceutical composition, comprising a cancer-killing cell produced by a method described herein and a pharmaceutically acceptable carrier, e.g., a GMP-acceptable carrier.

In yet another aspect, the disclosure features a composition (e.g., a purified preparation). The composition includes:

(1) a cancer-killing cell, which: (i) has cytotoxic activity toward a cancer cell, and (ii) comprises a cell surface marker derived from the cancer cell in an amount of 90 - 500 copies of a cell surface marker (e.g., at least 90, 100, 200, 300, 400, or 500 copies) of e.g. one or more cancer cell surface markers; and
(optionally) (2) a proximity immuno-coaching factor (pICF), e.g., a detectable (e.g., trace) amount of pICF.

In aspects of the disclosure, the composition further comprises a pharmaceutically acceptable carrier, e.g., a GMP- acceptable carrier.

In one aspect of the disclosure, about 2 to 75% (e.g., about 2 to 70%, 2 to 60%, 2 to 50%, or 2 to 40%) of the total T cells in the reaction mixture express one or more cancer cell surface markers (e.g., one or more leukemic cell cancers).

In aspects of the disclosure, the cancer-killing cell is enriched or purified. In some aspects of the disclosure, the enriched or purified cancer-killing cell population comprises at least 80%, 90%, 95%, 99% or 100% cancer-killing cells, wherein the cancer-killing cells comprise one or more cancer cell surface markers.

*Method of Treatment*

In another aspect, the disclosure features a method of treating a subject having cancer (e.g., a hematological cancer, a solid cancer, or a metastatic cancer as described herein). The method includes providing a preparation comprising cancer-killing T cells made by a method described herein; and administering the preparation to the subject.

In an aspect of the disclosure, a method of treating a subject having cancer (e.g., a hematological cancer, a solid cancer, or a metastatic cancer as described herein) includes:

(a) providing a sample from the subject, wherein the sample comprises a T cell and a cancer cell (e.g., a sample as described herein);
(b) contacting the sample with a proximity immuno-coaching factor (pICF), under conditions such that cancer-killing T cells are produced (e.g., for a period of time);
(c) (optionally) enriching for, or expanding, the cancer-killing T cells that have acquired a cell surface marker from the cancer cell;
(d) administering the cancer-killing T cells to the subject, thereby treating the subject.

In yet another aspect of the disclosure, a method of treating a subject having cancer (e.g., a hematological cancer or a solid cancer or a metastatic cancer) comprises:

(a) providing a cancer sample as described herein, e.g., from the subject;
(b) providing a T cell sample as described herein, e.g., from the subject;
(c) contacting the cancer sample with the T cell sample and a proximity immuno-coaching factor (pICF), under conditions such that cancer-killing T cells are produced (e.g., for a period of time);
(d) (optionally) enriching for, or expanding, the cancer-killing T cells that have acquired a cell surface marker from the cancer cell;
(e) administering the cancer-killing T cells to the subject, thereby treating the subject.

In some aspects of the disclosure, the cancer-killing T cells are administered without substantial expansion. In other aspects of the disclosure, the cancer-killing T cells are administered after cell expansion, e.g., after expansion of individual cells.

In some aspects of any of the aforesaid methods, the number of activated (e.g., cancer-killing) T cells, e.g., in the sample, administered to the subject is at least 5-10,000 (e.g., 5, 10, 100, 1000, 10,000 or more).

*Assays and Methods for Evaluating Subjects*

In another aspect of the disclosure, a method of, or assay for, evaluating a subject's ability to generate cancer-killing T cells (e.g., activated T cells, including trogocytotic T cells) includes:

(a) providing a T cell from a subject having a cancer (e.g., a T cell as described herein);
(b) providing a cancer cell from the subject (e.g., a cancer cell as described herein);
(c) forming an *ex vivo* reaction mixture comprising the T cell, the cancer cell, and a pICF, e.g., where the *ex vivo* reaction mixture is formed under conditions, e.g., for a period of time, sufficient to activate the T cell, e.g., to allow the T cell to acquire a cell surface marker from the cancer cell, thereby forming cancer-killing T cells (e.g., trogocytotic T cells);
(d) optionally, identifying the cancer-killing T cells (e.g., trogocytotic T cells) in the *ex vivo* reaction mixture; and
(e) determining, e.g., quantifying, the level or activity of the cancer-killing T cells (e.g., trogocytotic T cells), in the *ex vivo* reaction mixture.

In another aspect of the disclosure, a method of evaluating a subject, e.g., identifying or selecting a subject that is responsive to a therapy, e.g., an immunomodulatory agent and/or cancer therapy comprises:

(a) providing a T cell from a subject having a cancer (e.g., a T cell or T cell sample as described herein);
(b) providing a cancer cell, e.g., from the subject (e.g., a cancer cell or cancer cell sample as described herein);
(c) forming an *ex vivo* reaction mixture comprising the T cell, the cancer cell, and the immunomodulatory agent and/or the cancer therapy;
(d) determining the cell killing activity of the reaction mixture of step (c),
wherein an increase in cell killing activity of the reaction mixture in the presence of the therapy, relative to a reference value (e.g., absence of the therapy) is indicative of a higher responsiveness to the therapy.

In aspects of the disclosure, step (c) comprises forming *ex vivo* mixtures of the cancer-killing T cell or the preparation thereof with target cells, e.g., cancer cells. In embodiments, the cancer cell is a cell chosen from a hematological cancer, a solid cancer, a metastatic cancer, a CTC, or a combination thereof. In aspects of the disclosure, the cancer cell is a leukemic or lymphoma blast cell (e.g., a blast cell expressing one or more markers chosen from CD19, CD123, CD20 or others). In an aspect of the disclosure, the T cell is a cell chosen from a blood sample (e.g., peripheral blood sample), a bone marrow sample, a lymph node sample, a tumor sample comprising a CTL and/or a TIL, or a combination thereof). In aspects of the disclosure, the T cell expresses CD8 and/or CD25 (e.g., it is a CD8+CD25+ T cell). In aspects of the disclosure, the T cell expresses CD4 and/or CD25 (e.g., it is a CD4+CD25+ T cell).

In aspects of the disclosure, step (e) is performed using an automated platform, e.g., an automated flow cytometry fluorescence-based platform, e.g., the ExviTech platform described herein. In such aspects, the level of detectably (fluorescent-) labeled cancer-killing T cells is evaluated.

In one aspect of the disclosure, the level and/or presence of a tumor antigen on the cancer-killing T cell is detected (e.g., the number or activity of trogocytotic T cells is detected).

In aspects of the disclosure, the level of cancer-killing T cells is determined by detecting the number or activity of CTLs or TILs. In other embodiments, the number or activity of T cells expressing CD8 and/or CD25 (e.g., a CD8+CD25+ T cell) is detected. In other aspects of the disclosure, the number or activity of T cells expressing CD4 and/or CD25 (e.g., a CD4+CD25+ T cell) is detected.

In aspects of the disclosure, an elevated level of cancer-killing T cells (e.g., trogocytotic T cells) in the *ex vivo* reaction mixture (e.g., compared to a reference level) indicates that the subject is capable of generating (e.g., is a high producer of) cancer-killing T cells. In aspects of the disclosure, said subject is identified as being likely to respond to the methods and compositions disclosed herein.

In other aspects of the disclosure, a low level of cancer-killing T cells (e.g., trogocytotic T cells) in the *ex vivo* reaction mixture (e.g., compared to a reference level depending on the specific effector to target ratios in each sample) indicates that the subject is less capable of generating cancer-killing T cells. In aspects of the disclosure, said subject is identified as being less likely to respond to the methods and compositions disclosed herein.

In aspects of the disclosure, the method, or assay, further comprises identifying or selecting a subject based on the ability to generate cancer-killing T cells, e.g., identifying the subject being a high or low producer of cancer-killing T cells.

In aspects of the disclosure, the reference level comprises the number or activity of activated T cells (e.g., cancer-killing T cells (e.g., trogocytotic T cells)) generated in a similar *ex vivo* reaction mixture in the absence of one or more of a T cell, a cancer cell, or a pICF and/or immunomodulatory agent. In one aspect of the disclosure, the reference level is determined by detecting the level and/or presence of a tumor antigen on the cancer-killing T cell. In other aspects of the disclosure, the reference level is determined by detecting the number or activity of CTLs or TILs. In one aspect of the disclosure, the reference level comprises the number or activity of T cells expressing CD8 and/or CD25 (e.g., a CD8+CD25+ T cell). In one aspect of the disclosure, the reference level comprises the number or activity of T cells expressing CD4 and/or CD25 (e.g., a CD4+CD25+ T cell).

In other aspects of the disclosure, the activity of the cancer-killing T cells (e.g., trogocytotic T cells) produced in the *ex vivo* reaction mixture is evaluated. In some aspects of the disclosure, the *ex vivo* reaction mixture from step (c) is incubated in the presence of cancer cells (e.g., a blast cell expressing one or more markers chosen from CD19, CD123, CD20 or others) under conditions such that cancer cell killing occurs. Detection of the level or amount of cancer cell killing in the presence of the *ex vivo* reaction mixture is indicative of the activity of the cancer-killing T cells in the reaction mixture. In aspects of the disclosure, a decrease in the level or amount of cancer cells, e.g., relative to a reference level, is indicative of increased cancer cell killing. In other aspects of the disclosure, a small change or no substantial change in the level or amount of cancer cells, e.g., relative to a reference level, is indicative of decreased cancer cell killing.

In aspects of the disclosure, the method further comprises administering the cancer-killing T cells to a subject, e.g., a subject with a cancer as described herein. In embodiments, the subject is identified as being likely to be responsive to the methods and compositions disclosed herein, e.g., shows a higher number of cancer-killing T cells (e.g., trogocytotic T cells) in the reaction mixture (e.g., compared to a reference level).

*Assays and Methods for Evaluating E:T Ratios*

Another aspect of the disclosure refers to a method of, or assay for, evaluating the potency of a cancer-killing T cell or preparation thereof. The method, or assay, includes:

(a) providing a cancer-killing T cell or a preparation thereof, e.g., produced according to a method described herein, e.g., from a subject (e.g., a subject with a cancer as described herein);

(b) providing a target cell (e.g., a cancer cell), e.g., wherein the cancer cell is from the subject;

(c) contacting the cancer-killing T cell or the preparation thereof with the target cell (e.g., cancer cells), under conditions (e.g., for a period of time) sufficient to allow the cancer-killing T cell to kill the cancer cell (in embodiments, the contacting step further comprises addition of a pICF and/or an immunomodulatory agent as described herein, e.g., at different doses (e.g., increasing dosages);

(d) determining the level, e.g., number, of target cells that have been eliminated after step (c) (e.g. relative to a sample without adding a pICF or immunomodulatory agent, e.g., a sample from the same subject without adding a pICF), and (optionally) determining the level, e.g., number, of cancer-killing T cells produced (e.g., newly generated cells) after step (c) (e.g. relative to a sample without adding a pICF, e.g., a sample from the same subject without adding a pICF or immunomodulatory agent) (in embodiments, the level, e.g., number, of target cells and/or cancer-killing cells is determined at one or more time intervals after step (c)); and

(optionally) (e) determining the ratio of either target cell to cancer killing T cell, or cancer killing T cell to target cell, from step (d).

Yet another aspect of the disclosure refers to a method of, or assay for, evaluating a candidate pICF and/or immunomodulatory agent. The method includes:

(a) providing a cancer-killing T cell or a preparation thereof, e.g., produced according to a method described herein, e.g., from a subject (e.g., a subject with a cancer as described herein);

(b) providing a target cell (e.g., a cancer cell), e.g., wherein the cancer cell is from the subject;

(c) contacting the candidate pICF and/or immunomodulatory agent (e.g., one or more candidate pICFs and/or immunomodulatory agents) with the cancer-killing T cell or the preparation thereof and the target cell (e.g., cancer cells), under conditions (e.g., for a period of time and/or in the presence of a predetermined concentration of the candidate pICFs and/or immunomodulatory agents) sufficient to allow the cancer-killing T cell to kill the cancer cell;

(d) determining the level, e.g., the number, of target cells that have been eliminated after step (c) (e.g. relative to another sample without adding the pICF and/or immunomodulatory agent), and optionally determining the level, e.g., the number, of cancer-killing T cells newly generated after step (c) (e.g. relative to another sample without adding the pICF and/or immunomodulatory agent) (in embodiments, the number of target cells and/or cancer-killing cells is determined at one or more time intervals and at one or more concentrations of the candidate pICF and/or immunomodulatory agents after step (c)); and

(optionally) (e) determining the ratio of either target cell to cancer killing T cell, or cancer killing T cell to target cell, from step (d).

In some embodiments of any of the aforesaid methods or assays, a basal E:T ratio is obtained. In one embodiment, the basal E:T is the ratio between the cytotoxic T cells and the cancer cells before pICF and/or immunomodulatory agent exposure.

In some embodiments of any of the aforesaid methods or assays, an Effective E:T ratio is obtained. In one embodiment, the Effective E:T ratio is the ratio between the cancer-killing T cells generated and the cancer cells killed after bispecific or multispecific antibody and/or immunomodulatory agent exposure.

In some embodiments of any of the aforesaid methods or assays, the Effective E:T ratio can be calculated at one or more predetermined concentrations of the bispecific or multispecific antibody. In one embodiment, the predetermined concentration of the pICF is optimized for calculating the Effective E:T ratio. In one embodiment, the E:T ratio is calculated using the numbers of tumor and activated T cells when exposed to the maximum concentration of bispecific or multispecific antibody. In another embodiment, the E:T ratio is calculated using the numbers of tumor and activated T cells when exposed to the concentration of the bispecific or multispecific antibody that generate a maximum peak in the number of activated T cells. In a further embodiment, the E:T ratio is calculated using the numbers of tumor and activated T cells that correspond to the EC50 concentration of the respective dose response curves.

In some embodiments of any of the aforesaid methods or assays, the Effective E:T ratio can also be expressed as the Effective T:E ratio (e.g., ratio between cancer cells to the cancer-killing T cells).

In some embodiments, the cancer-killing T cell produced by a method described herein is provided. In some embodiments, the cancer-killing T cell is a trogocytic T cell. In other embodiments, the cancer-killing T cell is a CD8+CD25+ T cell. In other embodiments, the cancer-killing T cell is a CD4+CD25+ T cell. The trogocytic T cell is believed to be a more effective cancer cell killer, although the cytotoxic T cells, e.g., CD8+ T cells and activated CD4+ T cells also have cancer cell killing activity. Accordingly, all activated T cell types can be included in the Effective E:T ratio.

In some embodiments, the method or assay includes detecting, e.g., counting, the number of newly generated cancer-killing cytotoxic T cells, and the number of targets cells that have been killed under the same conditions, e.g., in the same well. The ratio of these values is the Effective E:T ratio.

In some embodiments, the ratio is a ratio between two subtractions, one subtraction is the number of targets after incubation relative to before incubation (i.e., to measure the number of target cells killed in such condition), and the other subtraction is the number of cancer killing T cells after relative to before incubation (i.e., to measure the number of cytotoxic t cells that kill the target cells in such condition). In some embodiments, there are zero (or no detectable) cancer killing T cells without the pICF, and thus the subtraction equals the total number for cancer killing T cells (e.g., total number of CD8+CD25+ T cells or total number of CD4+CD25+ T cells).

In embodiments of any of the aforesaid methods or assays, a decrease in the level or amount of cancer cells, e.g., relative to a reference level without adding a bispecific or multispecific antibody and/or immunomodulatory agent, is indicative of increased cancer cell killing. In other embodiments, a reduced change or no substantial change in the level or amount of cancer cells, e.g., relative to a reference level, is indicative of decreased cancer cell killing.

In embodiments of any of the aforesaid methods or assays, a high level of target cell killing relative to the newly generated target killing T cells (e.g., a high Effective ratio of target cell to cancer killing T cell) induced by the pICF and/or immunomodulatory agent indicates that the cancer-killing T cell or preparation thereof is an effective killer of cancer cells. In one embodiment, the target to T cell ratio is compared to a reference ratio. For example, a ratio of 1 (T cell) to 10, 20, 30, 40, 50, 75, 100, 500 or higher (target cells) is indicative of potent T cell killing activity. In a preferred embodiment, the ratio T cell: target cells ranges 1:500, or higher. A subject having T cells having potent cell killing activity can be identified as being a strong responder to the pICF and/or immunomodulatory agent.

In one embodiment, the reference ratios are the ratio between two subtractions:

- The number of target cells without bispecific or multispecific antibody minus the number of target cells adding bispecific or multispecific antibody ($\Delta T^{pICF}$), both sharing the same experimental incubation conditions,

    o Wherein this number is calculated by subtracting the number of target cells at the dose of bispecific or multispecific antibody that induces the highest target cell killing, or alternatively the highest dose of bispecific or multispecific antibody,
    o Wherein the maximum and minimum values are derived from mathematical fitting of the experimental values of a dose response curve of multiple doses of the bispecific or multispecific antibody.

- The number of effector cancer killing T cells adding the bispecific or multispecific antibody minus the number of effector cancer killing T cells without the bispecific or multispecific antibody ($\Delta E^{pICF}$), both sharing the same incubation conditions,

    o Wherein this number is calculated by subtracting the number of target cells at the dose of bispecific or multispecific antibody that induces the highest target cell killing, or alternatively the highest dose of bispecific or multispecific antibody,
    o Wherein the maximum and minimum values are derived from mathematical fitting of the experimental values of a dose response curve of multiple doses of the bispecific or multispecific antibody.

- The ratio between these two variables is defined as the Effective E:T Ratio and equals $\Delta T^{pICF} / \Delta E^{pICF}$.

- This Effective E:T ratio measures the number of target cells that have been killed by a single cancer killing T cell in such conditions. This ratio can be similar for the same sample and pICF in different incubation times, because it represents the activity of the same cancer killing T cell, generated at different times.

In some embodiments, the Effective E:T Ratio represents an estimate of the activity of the generated cancer killing T cell in killing cancer target cells. Without wishing to be bound by theory, it is equivalent to the activity of a drug in killing cancer cells, because the cancer killing T cell is indeed an active medicament for treating a subject, e.g., a cancer patient. The Effective E:T Ratio can rank the activity of cancer killing T cells from different patients thus stratifying those patients. This ranking or stratification can be very different than the ranking or stratification derived from the standard method of measuring the efficacy in killing cancer target cells. For example, a very efficacious cancer killing T cell with a 1:200 Effective E:T Ratio, that eliminates 200 target cells per cancer killing T cell, may not be able to kill all cancer cells if that patient has a very large density of cancer target cells. Leaving alive many cancer cells would normally be considered a sign of low activity for the cancer killing T cell in a standard chemotherapy activity measurement; in this case, it would miss the true high activity of the cancer killing T cell generated by the pICF, the problem being some cancer cells are immunosuppressed and resistant to the otherwise high activity cancer-killing T cells generated. Hence, the Effective E:T ratio can identify the most active cancer killing T cells, e.g., those cancer killing T cells better suited to be administered to the patient.

Alternatively, a low level of Effective E:T Ratio is indicative of a poor T cell killing activity. In one embodiment, a ratio cancer-killing T cells:target cells of 1:5 (e.g., 1, 3, or 5 ) is indicative of poor T cell killing activity. A subject having T cells having reduced cell killing activity can be identified as being a poor responder to the pICF and/or immunomodulatory agent.

There are alternative approaches to estimate the activity of cancer-killing T cells, besides the Effective E:T Ratios, basal E:T Ratios, EC50, Emax, kinetics, and association of these variables. More sophisticated mathematical calculations and different ways of fitting the experimental data, using different pharmacological operational models, can provide different ways to calculate how many cancer cells are killed by a cancer-killing T cells relative to the herein proposed Effective E:T Ratio. Those alternative approaches to calculate essentially the same concept to estimate the activity of the cancer-killing T cells are also considered covered by the definition of Effective E:T Ratios.

In aspects of any of the aforesaid methods or assays, the level of target cells and/or cancer-killing cells is determined at one or more time intervals after step (c). In exemplary aspects of the disclosure, the level of target cells and/or cancer-killing cells is determined at time 0, at time 1 - 168 hours (e.g., 1, 2, 4, 8, 16, 24, 48, 72, 96, 120, 144, or 168 hours) or several days or weeks after step (c).

In aspects of any of the aforesaid methods or assays, the contacting step further comprises addition of a pICF and/or immunomodulatory agent at different doses (e.g., increasing dosages) of the pICF and/or immunomodulatory agent, e.g., to generate a dose-response curve. In one aspect of the disclosure, the difference between the level of T cells or cancer cells at a dose zero or at control level (e.g., a threshold dose) and a saturated dose of the pICF and/or immunomodulatory agent is determined. In aspects of the disclosure, the difference in the level of T cells or cancer cells at the saturated dose vs. threshold dose is determined. In embodiments, the Effective E:T ratio as used herein is the ratio of the difference in the level of T cells relative to the difference in the level of cancer cells. In embodiments, the Effective E:T ratio as used herein is the ratio of the number of T cells and target cells at their respective EC50 concentration.

In aspects of any of the aforesaid methods or assays, the method is performed using an automated platform, e.g., an automated fluorescence-based platform, e.g., the ExviTech platform described herein.

In aspects of any of the aforesaid methods or assays, the activity of the pICF and/or immunomodulatory agent is determined using an *ex vivo/in vitro* assay to measure dose response curves, whose mathematical fitting enable quantitative parameters to estimate the activity, selected from at least one from EC50, Effective E:T ratio, Emax or kinetics. In aspects of any of the aforesaid methods or assays, the activity of the pICF and/or immunomodulatory agent assessed by step (e) is different from an activity assessment using a dose response of the pICF and/or immunomodulatory agent activity, e.g., compared to a standard depletion dose response curve.

In aspects of any of the aforesaid methods or assays, the reference ratio is a predetermined ratio, e.g., about 1:3 to 1:10, e.g., about 1:3, 1:4, 1:5, 1:6, 1:7, 1:8, 1:9, or 1:10. In aspects of the disclosure, the T cell to high target cell ratio from step (e) is about 1:4 - 1:500 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, 1:500, or higher).

In embodiments of any of the aforesaid methods or assays, step (c) comprises forming *ex vivo* mixtures of the cancer-killing T cell or the preparation thereof with target cells, e.g., cancer cells. In embodiments, the cancer cell is a cell chosen from a hematological cancer, a solid cancer, a metastatic cancer (e.g., a CTC, or a combination thereof). In embodiments, the cancer cell is a leukemic or lymphoma blast cell (e.g., a blast cell expressing one or more markers chosen from CD19, CD123, CD20 or others). In embodiments, the T cell is a cell chosen from a blood sample (e.g., peripheral blood sample), a bone marrow sample, a lymph node sample, a spleen sample, a tumor sample comprising a CTL and/or a TIL, or a combination thereof). In embodiments, the T cell expresses CD8 and/or CD25 (e.g., it is a CD8+CD25+ T cell). In other embodiments, the T cell expresses CD4 and/or CD25 (e.g. it is a CD4+CD25+ T cell).

In aspects of any of the aforesaid methods or assays, the cancer killing T cell or preparation thereof is produced using a method that comprises use of a pICF and/or immunomodulatory agent, e.g., a pICF and/or immunomodulatory agent described herein.

In embodiments of any of the aforesaid methods or assays, the cancer-killing T cell or preparation thereof comprises a T cell, e.g., CTL, that is CD8+ and CD25+, or a CD4+ and CD25+, or both.

In aspects of any of the aforesaid methods or assays, the candidate pICF and/or immunomodulatory agent is administered at different dosages (e.g., at increasing dosages).

In aspects of any of the aforesaid methods or assays, an increase in the cell killing activity of the T cells in the presence of the candidate pICF and/or immunomodulatory agent is indicative of high efficacy of the pICF and/or immunomodulatory agent. Alternatively, a small change or no substantial change in the cell killing activity of the T cells in the presence of the candidate pICF and/or immunomodulatory agent is indicative of low efficacy of the pICF and/or immunomodulatory agent.

*Screening Assays*

In another aspect of the disclosure, a method of, or assay for, identifying an effective pICF for use in producing a cancer-killing T cell from a subject includes:

(a) providing a candidate pICF (e.g., one or more candidate pICFs);
(b) providing a sample comprising a cancer cell and a T cell, e.g., one or more samples as described herein); and
(c) forming an *ex vivo* reaction mixture comprising the candidate pICF(s) and the sample, e.g., under conditions (e.g., for a period of time) sufficient to activate the T cell (e.g., allow the T cell to acquire a cell surface marker from the cancer cell), thereby producing a cancer-killing T cell.

In yet another aspect of the disclosure, a method of, or assay for, identifying an effective immunomodulator for use in producing a cancer-killing T cell from a subject includes:

(a) providing a candidate immunomodulator (e.g., one or more candidate immunomodulators described herein);
(b) providing a sample comprising a cancer cell and a T cell, *e.g.*, one or more samples as described herein); and
(c) forming an *ex vivo* reaction mixture comprising the candidate immunomodulator(s) and the sample, e.g., under conditions (e.g., for a period of time and/or in the presence of a predetermined concentration of the candidate immunomodulator(s)) sufficient to activate the T cell (e.g., allow the T cell to acquire a cell surface marker from the cancer cell), thereby producing a cancer-killing T cell.

In aspects of the disclosure, the method or assay further includes:
(d) determining one or more parameters chosen from: depletion of cancer cells, numbers or percentages of activated, e.g., trogocytotic, T cells, and proliferation of activated, e.g., trogocytotic, T cells, e.g., for each candidate pICF or candidate immunomodulator. In some embodiments, one or more of (i)-(iv) indicate that the candidate pICF or immunomodulator is an effective pICF or immunomodulator for use in producing a cancer-killing T cell from a subject:

(i) the pICF or immunomodulator leads to a decreased number of live cancer cells compared to a reference level, e.g., wherein the reference level is the number of live cancer cells in the reaction mixture or the number of live cancer cells after incubation with the cancer-killing T cells produced by step (c) in the absence of the pICF or immunomodulator;
(ii) the pICF or immunomodulator leads to a greater number/percentage of activated cancer killing T cells compared to a reference level, e.g., where the reference level is the number/percentage of activated T cells in a similar ex vivo reaction mixture lacking an pICF or immunomodulator, or the number/percentage of activated T cells in the ex vivo reaction mixture formed for a period of time insufficient to allow the T cell to acquire a cell surface marker from the cancer cell;
(iii) the pICF or immunomodulator leads to a greater proliferation of activated cancer killing T cells compared to a reference level, e.g., where the reference level is the proliferation of a T cell (e.g, CTL) that has not been exposed to a cancer cell (e.g., a T cell, e.g., CTL, from a non-cancerous subject) or a T cell, e.g., CTL (e.g., from a cancerous or non-cancerous subject) that was not exposed to a pICF or immunomodulator ex vivo; and/or
(iv) the pICF or immunomodulator leads to a greater Effective E:T Ratio compared to a different pICF in the same sample and incubating conditions.

In any of the aforesaid methods or assays described herein, said methods or assays can further include providing an immunomodulatory agent (e.g., one or more of an inhibitor of an immune checkpoint molecule, an agonist of an immune cell, or other immunomodulatory drug or cell therapy, e.g., as described herein). The immunomodulatory agent can be added such that an *ex vivo* reaction mixture is formed comprising the candidate pICF(s), the immunomodulatory agent and the sample. The activity of the *ex vivo* reaction mixture can be measured according to step (d).

In any of the aforesaid methods or assays described herein, said methods or assays can further include evaluating the level or activity of a suppressor immune cell, e.g., one or more of a myeloid-derived suppressor cell (MDSC) or regulatory T (Treg) cell. In some aspects of the disclosure, a change in the phenotype of the suppressor immune cell is evaluated. In one aspect of the disclosure, the level or expression of a Treg cell marker, e.g., $CD4^+CD25^{hi}FoxP3^+CD127-$ expressing cells, is detected. Alternatively, or in combination, the level or expression of an MDSC marker is determined by detecting one or more of CD33$^+$/CD11b$^+$/HLA-DR$^{low/neg}$ (e.g., in AML), IDOhiCD14$^+$ HLA-DR$^{low}$ (e.g., in CLL) or CD11b$^+$/CD14$^-$/HLA-DR$^{low/neg}$/CD33$^+$CD15$^+$ (e.g., in MM). The marker of the immune suppressor cell can be detected, *e.g.*, by automated, fluorescent cell sorting (e.g., using the ExviTech platform).

In some aspects of any of the aforesaid methods or assays described herein, the effect of the pICF, e.g., the candidate pICF, can be evaluated as a function of the level and/or activity of one, two, three or all of T cell (e.g., activated T cells), trogocytotic T cell, Treg or MDSC. In some aspects of the disclosure, the number and/or ratio of one or more of the aforesaid cells in the presence or absence of the pICF can be determined.

In some aspects of any of the aforesaid methods or assays described herein, the therapeutic efficacy of any compound (e.g., an immunomodulatory agent, e.g., a pICF), can be evaluated by detecting the level or activity of a cancer-killing T cell and/or the level of activity of the suppressor immune cell, e.g., the Treg cell or the MDSC. In one aspect of the disclosure, an increase in the level or activity of the cancer-killing T cell and/or a decrease in the level of activity of the suppressor immune cell is indicative of increase therapeutic efficacy of the compound. Alternatively, a decrease in the level or activity of the cancer-killing T cell and/or an increase in the level of activity of the suppressor immune cell is indicative of decrease therapeutic efficacy of the compound.

In another aspect of the disclosure, a method of or assay for, identifying a cancer-killing T cell likely to be effective in killing a cancer cell *in vivo* comprises:

(a) providing a population of cancer-killing T cells specific for the cancer cell, e.g., produced by a method described herein; and
(b) determining one or more parameters chosen from: cytotoxic activity of the cancer-killing T cells toward the cancer cell, viability of the cancer-killing T cells, proliferation of the cancer-killing T cells, and expression of cell surface markers on the cancer-killing T cells.

In some aspects of the disclosure, the effectiveness of the cancer-killing T cell *in vivo* is evaluated by one or more of the following:

(i) detecting the cytotoxic activity of the cancer-killing T cells toward the cancer cell, e.g., compared to a reference level, wherein higher cytotoxic activity of the cancer-killing T cells is indicative of increased effectiveness *in vivo* (in embodiments, the reference level is detected by the cytotoxic activity of the cancer-killing T cells toward a different type of cancer cell or the cytotoxic activity of the cancer-killing T cells toward a non-cancerous cell);
(ii) detecting the proliferation of the cancer-killing T cells, e.g., compared to a reference level, e.g., wherein higher proliferation is indicative of increased effectiveness *in vivo* (in embodiments, the reference level is the proliferation of a T cell, e.g, CTL, that has not been exposed to a cancer cell (e.g., a T cell, e.g., CTL, from a non-cancerous subject) or a T cell, e.g., CTL (*e.g.*, from a cancerous or non-cancerous subject) that was not exposed to a pICF *ex vivo*);
(iii) detecting the viability of the cancer-killing T cells, e.g., compared to a reference level, wherein increased viability of the cancer-killing T cells is indicative of increased effectiveness *in vivo* (in embodiments, the reference level is the viability of a T cell, e.g., CTL, that has not been exposed to a cancer cell (e.g., a T cell, e.g., CTL, from a non-cancerous subject) or a T cell, e.g., CTL (e.g., from a cancerous or non-cancerous subject) that was not exposed to a pICF *ex vivo*); or
(iv) detecting the level of expression of one or more cancer antigens, PD-1, or TIM-3 on the cancer-killing T cells, e.g., compared to a reference level, wherein higher level of expression is indicative of increased effectiveness *in vivo* (in embodiments, the reference level is the expression of the cancer antigen(s), PD-1, and/or TIM-3 on a T cell, e.g., CTL, that has not been exposed to a cancer cell (e.g., a T cell, e.g., CTL, from a non-cancerous subject)).

In yet another aspect of the disclosure, also featured herein is a method of, or assay for, identifying (e.g., and selecting) one or more clones of cancer-killing T cells likely to be effective in killing a cancer cell *in vivo,* comprising:

(a) providing a population comprising a plurality of clones of cancer-killing T cells specific for the cancer cell, e.g., produced by a method described herein;
(b) separating the population, e.g., into individual clones; and
(c) determining one or more parameters chosen from: cytotoxic activity of the cancer-killing T cells toward the cancer cell, viability of the cancer-killing T cells, proliferation of the cancer-killing T cells, or expression of cell surface markers on the cancer-killing T cells. In embodiments, the clones are evaluated individually.

In aspects of the disclosure, one or more of (i)-(v) indicates that the clone is likely to be effective *in vivo*:

(i) higher cytotoxic activity of the clone toward the cancer cell, for example as measured using the Effective E:T Ratio, e.g., compared to a reference level (e.g., wherein the reference level is the cytotoxic activity of the clone toward a different type of cancer cell or the cytotoxic activity of the clone toward a non-cancerous cell);

(ii) higher proliferation of the clone, e.g., compared to a reference level (e.g., wherein the reference level is the proliferation of a T cell, e.g, CTL, that has not been exposed to a cancer cell (e.g., a T cell, e.g., CTL, from a non-cancerous subject) or a T cell, e.g., CTL (e.g., from a cancerous or non-cancerous subject) that was not exposed to a pICF *ex vivo*);

(iii) higher viability of the clone, e.g., compared to a reference level (e.g., wherein the reference level is the viability of a T cell, e.g, CTL, that has not been exposed to a cancer cell (e.g., a T cell, e.g., CTL, from a non-cancerous subject) or a T cell, e.g., CTL (e.g., from a cancerous or non-cancerous subject) that was not exposed to a pICF *ex vivo*); and/or

(iv) higher expression of one or more cancer antigens, PD-1, or TIM-3, e.g., compared to a reference level (e.g., wherein the reference level is the expression of the cancer antigen(s), PD-1, and/or TIM-3 on a T cell, e.g., CTL, that has not been exposed to a cancer cell (e.g., a T cell, e.g., CTL, from a non-cancerous subject)).

In aspects of the disclosure, the separating step comprises one or more of: limiting dilution (e.g., to obtain a clonal cell population), magnetic bead-based enrichment, or fluorescence cell activated sorting. In embodiments, the separating step further comprises plating individual cells (e.g., individual clones) into separate wells of a container. The method further includes expanding each of the individual cells to produce populations of each clone. In some embodiments, the cells are administered without expansion. In other embodiments, the cells are administered after cell expansion, e.g., after expansion of individual cells.

In aspects of the disclosure, the method further comprises expanding the clone identified as likely to be effective *in vivo.*

In aspects of the disclosure, the method further comprises combining two or more clones that have been identified as likely to be effective *in vivo,* and (optionally) expanding the mixture of clones.

*Assays and Methods for identifying subjects responsive to a cancer therapy*

In another aspect of the disclosure, a method of, or assay for, evaluating a subject, e.g., identifying or selecting a subject that is responsive to a therapy, e.g., an immunomodulatory and/or cancer therapy includes:

(a) providing a T cell from a subject having a cancer (e.g., a T cell or T cell sample as described herein);
(b) providing a cancer cell, e.g., from the subject (e.g., a cancer cell or cancer cell sample as described herein);
(c) forming an *ex vivo* reaction mixture comprising the T cell, the cancer cell, a proximity immuno-coaching factor (pICF), and the therapy, e.g., the cancer therapy;
(d) determining the cell killing activity of the reaction mixture of step (c).

In some embodiments, an increase in cell killing activity of the reaction mixture in the presence of the therapy, relative to a reference value (e.g., absence of the therapy) is indicative of a higher responsiveness to the therapy.

In some aspects of the disclosure, step (d) comprises determining the level of target cells after step (c), and optionally determining the level of cancer-killing T cells after step (c) (in embodiments, the level of target cells and/or cancer-killing cells is determined at one or more time intervals after step (c)). In aspects of the disclosure, the method further comprises determining the Effective E:T ratio of either target cell to T cell, or T cell to target cell, e.g., as described herein.

In aspects of the disclosure, the pICF is a bispecific antibody molecule, e.g., a bispecific immunoglobulin (BsIgG), an immunoglobulin operatively linked to additional antigen-binding molecule, a bispecific antibody (BsAb) fragment, a bispecific fusion protein, or a BsAb conjugate.

In aspects of the disclosure, the pICF is a bi-specific antibody selected from the list consisting of BsMAb CD123/CD3, BsMAb CD19/CD3 and EpCAM/CD3.

In embodiments, the therapy is an immunomodulatory agent, e.g., an immunomodulatory agent as described herein. In one embodiment, the immunomodulatory agent is an inhibitor of an immune checkpoint inhibitor, e.g., an immune checkpoint inhibitor described herein, e.g., an inhibitor of one or more of: CTLA4, PD1, PDL1, PDL2, B7-H3, B7-H4, TIM3, LAG3, BTLA, CD80, CD86, or HVEM. In embodiments, the immunomodulatory agent is an agonist of T cells, e.g., an agonist of T cells described herein, e.g., agonistic antibody, e.g., an agonistic antibody or fragment thereof to CD137, GITR, or CD40. In one embodiment, the immunomodulatory agent is an inhibitor of MDSCs and/or Treg cells.

In one embodiment, the immunomodulatory agent is lenalidomide. In other embodiments, the immunomodulatory agent activates an immune response to a tumor specific antigen, e.g., it is a vaccine (e.g., a vaccine against targets such as gp100, MUC1 or MAGEA3. In other embodiments, the immunomodulatory agent is a cytokine, e.g., a recombinant cytokine chosen from one or more of GM-CSF, IL-7, IL-12, IL-15, IL-18 or IL-21. In other embodiments, the immunomodulatory agent is an autologous T cell, e.g., a tumor-targeted extracellular and intracellular tumor-specific antigen (e.g., a CAR T cell or a TCR T cell). In yet other embodiments, the immunomodulatory agent is a modulator of a component (e.g.,

enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Additional examples of immunomodulatory agents are further described in, e.g., Adams JL (2015) and Serafini (2008).

*Methods or assays for identifying an effective pICF and/or immunodulator for use in producing a cancer-killing T cell*

In an aspect of the disclosure, a method of, or assay for, identifying an effective pICF and/or immunomodulator for use in producing a cancer-killing T cell from a subject comprises:

(a) providing a candidate pICF and/or immunomodulator (e.g., one or more candidate pICFs and/or immunomodulators);
(b) providing a sample comprising a cancer cell and a T cell, e.g., one or more samples as described herein); and
(c) forming an *ex vivo* reaction mixture comprising the candidate pICF(s) and/or immunomodulator(s) and the sample, e.g., under conditions (e.g., for a period of time) sufficient to activate the T cell (e.g., allow the T cell to acquire a cell surface marker from the cancer cell), thereby producing a cancer-killing T cell.

In aspects of the disclosure, the method or assay further comprises:
(d) determining one or more parameters chosen from: depletion of cancer cells, numbers or percentages of activated, e.g., trogocytotic, T cells, and proliferation of activated, e.g., trogocytotic, T cells, e.g., for each candidate pICF and/or immunomodulator.
In aspects of the disclosure, one or more of (i)-(iii) indicate that the candidate pICF and/or immunomodulator is an effective pICF and/or immunomodulator for use in producing a cancer-killing T cell from a subject:

(i) the pICF and/or immunomodulator leads to a decreased number of live cancer cells compared to a reference level, e.g., wherein the reference level is the number of live cancer cells in the reaction mixture or the number of live cancer cells after incubation with the cancer-killing T cells produced by step (c);
(ii) the pICF and/or immunomodulator leads to a greater number/percentage of activated T cells compared to a reference level, e.g., where the reference level is the number/percentage of activated T cells in a similar *ex vivo* reaction mixture lacking an pICF and/or immunomodulator, or the number/percentage of activated T cells in the *ex vivo* reaction mixture formed for a period of time insufficient to allow the T cell to acquire a cell surface marker from the cancer cell; and/or
(iii) the pICF and/or immunomodulator leads to a greater proliferation of activated T cells compared to a reference level, e.g., where the reference level is the proliferation of a T cell (e.g., CTL) that has not been exposed to a cancer cell (e.g., a T cell, e.g., CTL, from a non-cancerous subject) or a T cell, e.g., CTL (e.g., from a cancerous or non-cancerous subject) that was not exposed to a pICF and/or immunomodulator *ex vivo.*

*Method of evaluating (e.g., screening for) a candidate pICF and/or immunomodulator*

In some aspects of the disclosure, a screening method comprises generating cancer-killing T cells using a method described herein, e.g., wherein a first sample from a patient is mixed with a pICF. In embodiments, the screening method comprises subsequently mixing a second sample from the patient with the cancer-killing T cells and one or more candidate pICFs and/or immunomodulators. In aspects of the disclosure, different ratios of T cells to target cells (E:T ratios) are combined with a fixed concentration of the pICF(s) and/or immunomodulator(s). In other embodiments, a fixed ratio of T cells to target cells (E:T ratio) is combined with various concentrations of the pICF(s) and/or immunomodulator(s), e.g., thereby permitting the ranking of pICF/immunomodulator by activity.
Accordingly, in an aspect of the disclosure, a method of evaluating a candidate pICF and/or immunomodulator comprises:

(a) providing a cancer-killing T cell or a preparation thereof, e.g., produced according to a method described herein, e.g., from a subject (e.g., a subject with a cancer as described herein);
(b) providing a sample, e.g., from said subject, wherein said sample comprises a target cell (e.g., a cancer cell);
(c) contacting the candidate pICF and/or immunomodulator (e.g., one or more candidate pICFs and/or immunomodulators) with the cancer-killing T cell or the preparation thereof of step (a) and the second sample of step (b), under conditions (e.g., for a period of time) sufficient to allow the cancer-killing T cell to kill the cancer cell;
(d) determining the level of target cells after step (c), and optionally determining the level of cancer-killing T cells after step (c) (in embodiments, the level of target cells and/or cancer-killing cells is determined at one or more time intervals after step (c)); and optionally, (e) determining the Effective ratio of either target cell to T cell, or T cell to target cell, from step (d).

In aspects of the disclosure, the cancer-killing T cell or a preparation thereof and the target cell (e.g., a cancer cell) are from the same sample.

In aspects of the disclosure, the cancer-killing T cell or a preparation thereof and the target cell (e.g., a cancer cell) are from different samples.

In aspects of the disclosure, step (a), (b) or both (a) and (b) are repeated, e.g., using one or more additional samples, e.g., from the same subject.

In aspects of the disclosure, step (e) comprises contacting different ratios of T cell to target cell with a fixed amount of the candidate, thereby identifying an effective candidate.

In aspects of the disclosure, step (e) comprises contacting a fixed T cell to target cell ratio with different concentrations of the candidate(s), thereby determining the activity of the candidate(s) and optionally ranking the candidate(s) based on activity.

In aspects of the disclosure, a decrease in the level or amount of cancer cells, e.g., relative to a reference level, is indicative of increased cancer cell killing.

In aspects of the disclosure, a reduced change or no substantial change in the level or amount of cancer cells, e.g., relative to a reference level, is indicative of decreased cancer cell killing.

In aspects of the disclosure, a high level of target cell relative to cancer-killing T cell (e.g., a high Effective ratio of target cell to cancer-killing T cell) indicates that the cancer-killing T cell or preparation thereof is an effective killer of cancer cells.

In aspects of the disclosure, the target to cancer-killing T cell ratio is compared to a reference ratio.

In aspects, an Effective ratio of 1 (cancer-killing T cell) to 10, 20, 30, 40, 50, 75, 100 or higher (target cells) is indicative of potent T cell killing activity. In a preferred aspect of the disclosure, the ratio T cell: target cells ranges 1:100, or higher. In aspects of the disclosure, an Effective ratio of target cell to cancer-killing T cell of 1:100 or higher determines that the cancer-killing T cells are super killer T cells.

In aspects of the disclosure, an increase in the cell killing activity of the T cells and/or a high Effective ratio of target cell to cancer-killing T cell in the presence of the candidate pICF is indicative of high efficacy of the pICF.

In aspects of the disclosure, a low level of target cell relative to cancer-killing T cell (e.g., a low Effective ratio of target cell to cancer-killing T cell) is indicative of a poor T cell killing activity.

In aspects of the disclosure, the target to cancer-killing T cell ratio is compared to a reference ratio.

In aspects of the disclosure, an Effective ratio of 1 (T cell) to 1, 3, or 5 (target cells) is indicative of poor T cell killing activity.

In aspects of the disclosure, a small change or no substantial change in the cell killing activity of the T cells and/or a low Effective ratio of target cell to T cell in the presence of the candidate pICF is indicative of low efficacy of the pICF.

In aspects of the disclosure, the level of target cells and/or cancer-killing cells is determined at one or more time intervals after step (c).

In aspects of the disclosure, the level of target cells and/or cancer-killing cells is determined at time 0, at time 1-75 hours (e.g., 1, 2, 4, 8, 16, 24, 36 or 72 hours), or several days after step (c).

In aspects of the disclosure, the contacting step further comprises addition of a pICF at different doses (e.g., increasing dosages) of the pICF, e.g., to generate a dose response curve.

In aspects of the disclosure, the method further comprises determining the difference between the level of T cells or cancer cells at a dose zero or at control level (e.g., a threshold dose) and a saturated dose of the pICF.

In aspects of the disclosure, the method comprises determining the difference in the level of T cells or cancer cells at the saturated dose versus the threshold dose.

In aspects of the disclosure, the Effective ratio of the target cell (e.g., cancer cell) to cancer-killing T cell (e.g., in step (e)) is the ratio of the difference in the level of cancer-killing T cells relative to the difference in the level of cancer cells.

In aspects of the disclosure, the method is performed using an automated platform, e.g., an automated fluorescence-based platform, e.g., an ExviTech platform described herein.

In aspects of the disclosure, the activity of the pICF and/or immunomodulatory agent is determined using an *ex vivo/in vitro* assay to measure dose response curves, whose mathematical fitting enable quantitative parameters to estimate the activity, selected from at least one from EC50, Effective E:T ratio, Emax or kinetics. In embodiments, the activity of the pICF assessed by step (e) is different from an activity assessment using a dose response of the pICF activity, e.g., compared to a standard depletion dose response curve.

In aspects of the disclosure, the reference ratio is a predetermined ratio, e.g., about 1:3 to 1:10, e.g., about 1:3, 1:4, 1:5, 1:6, 1:7, 1:8, 1:9, or 1:10.

In aspects of the disclosure, the high Effective ratio of target cell to cancer-killing T cell is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher). In aspects of the disclosure, an Effective ratio of target cell to cancer-killing T cell of 1:100 or higher determines that the cancer-killing T cells are super killer T cells.

In aspects of the disclosure, step (c) comprises forming *ex vivo* mixtures of the cancer-killing T cell or the preparation thereof with target cells, e.g., cancer cells.

In aspects of the disclosure, the cancer cell is a cell chosen from a hematological cancer, a solid cancer, a metastatic cancer (e.g., a CTC, or a combination thereof).

In aspects of the disclosure, the cancer cell is a leukemic or lymphoma blast cell (e.g., a tumor cell expressing one or more markers chosen from CD19, CD123, CD20 or others).

In aspects of the disclosure, the T cell is a cell chosen from a blood sample (e.g., peripheral blood sample), a bone marrow sample, a lymph node sample, a tumor sample comprising a CTL and/or a TIL, or a combination thereof).

In aspects of the disclosure, the T cell expresses CD8 and/or CD25 (e.g., it is a CD8+CD25+ T cell).

In aspects of the disclosure, the cancer killing T cell or preparation thereof is produced using a method that comprises use of a pICF, e.g., a pICF described herein.

In aspects of the disclosure, the cancer-killing T cell or preparation thereof comprises a T cell, e.g., CTL, that is CD8+ and CD25+.

In aspects of the disclosure, the candidate pICF is added at different dosages (e.g., at increasing dosages).

Additional features of any of the methods and compositions described herein include one or more of the following:

In aspects of any of the aforesaid methods or compositions, the cancer-killing cell: (i) has cytotoxic activity toward a cancer cell, and (ii) comprises a cell surface marker derived from the cancer cell, e.g., at least 90-500 copies of a cell surface marker (e.g., 90, 100, 200, 300, 400, or 500, e.g., one or more cancer cell surface markers); and

In embodiments of any of the aforesaid methods or compositions, about 2 to 75% (e.g., about 2 to 70%, 2 to 60%, 2 to 50%, or 2 to 40%) of the total T cells in the reaction mixture express one or more cancer cell surface markers (e.g., one or more leukemic cell cancers).

In aspects of the disclosure, the cancer-killing cell is enriched or purified. In some embodiments, the enriched or purified cancer-killing cell population comprises at least 80% of cancer-killing T cells (e.g., 80%, 90%, 95%, 99% or 100%), wherein the cancer-killing cells comprise one or more cancer cell surface markers.

In some embodiments of any of the methods and compositions described herein, the bispecific or multispecific antibody has a first element providing affinity (e.g., a first binding specificity) for the T cell and a second element having affinity (e.g., a second binding specificity) for the cancer cell. In embodiments, each of the first and second elements comprises one, two or three, four, five or six CDRs. In embodiments, each of the first and second elements is other than an Fc region.

In embodiments, each of the first and second element has a dissociation constant (Kd) for its target of 1 pM or less, e.g., about 1 pM or less.

In embodiments, the first element binds to a T cell and does not bind to a substantial number of cancer cells (e.g., the first element binds to T cells with at least 10-10000 times higher affinity than to cancer cells (e.g., 10, 100, 1000, 10000 times or more)); and the second element binds to a cancer cell and does not bind to a substantial number of T cells (e.g., the second element binds to cancer cells with at least 10-10000 times higher affinity than T cells (e.g., 10, 100, 1000, 10000 times (or more)).

In embodiments, the second element binds (e.g., specifically binds) to one or more of: CD20, CD30, CD33, CD52, EpCAM, CEA, gpA33, mucin, TAG-72, carbonic anhydrase IX, PSMA, folate binding protein, ganglioside (e.g., GD2, GD3, or GM2), Lewis-Y2, VEGF, VEGFR, $\alpha V\beta 3$, $\alpha 5\beta 1$, ErbB1/EGFR, ErbB2/HER2, ERbB3, c-MET, IGF1R, EphA3, TRAIL-R1, TRAIL-R2, RANKL, FAP, tenascin, CD123, C19, CD19, BCMA, or other cancer cell antigen.

In embodiments, the first element binds (e.g., specifically binds) to a T cell, e.g., a cytotoxic T cell, e.g., a T cell comprising one or more of the following: CD8, CD3, $\alpha/\beta$ T cell receptor (TCR), $\gamma\delta$ TCR, CD45RO, and/or CD45RA.

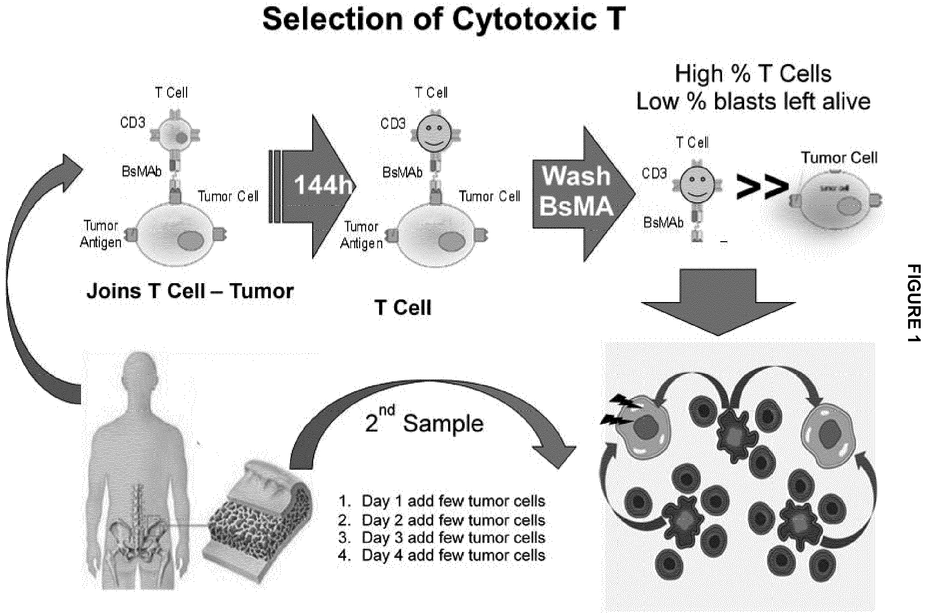

In embodiments, the bispecific or multispecific antibody comprises a bispecific antibody molecule, e.g., a bispecific immunoglobulin (BsIgG), an immunoglobulin operatively linked to additional antigen-binding molecule, a bispecific antibody (BsAb) fragment, a bispecific fusion protein, or a BsAb conjugate, e.g., as described by Spiess et al. (2015) Mol. Immunol. 67:95-106 (see for example, Fig. 1).

In embodiments, the BsAb fragment comprises two or more scFv fragments, e.g., operatively linked by a linker (e.g., a diabody), e.g., a dual-affinity retargeting (DART) antibody fragment or a Bispecific T-cell Engager (BiTE). In embodiments, the BsAb fragment comprises two or more single domain antibody fragments (dAbs), e.g., operatively linked by a linker. In embodiments, the BsAb fragment does not comprise a Fc region.

In embodiments, the bispecific antibody is selected from the list consisting of BsMAb CD123/CD3, BsMAb CD19/CD3 and EpCAM/CD3.

In embodiments, the bispecific or multispecific antibody comprises a CDR-grafted scaffold domain, e.g., a fibronectin scaffold domain.

In other embodiments, the antibody molecules are attached (e.g., conjugated) to a solid surface, e.g., a bead or a polymer.

In any of the aforesaid methods or assays described herein, said methods or assays can further include providing an immunomodulatory agent (e.g., one or more of an inhibitor of an immune checkpoint molecule, an agonist of an immune cell, or other immunomodulatory drug, e.g., as described herein). In some embodiments, the immunomodulatory agent can be added to an *ex vivo* reaction mixture as described herein, thereby forming an *ex vivo* reaction mixture comprising the candidate bispecific or multispecific antibody, the immunomodulatory agent and the sample. In other embodiments, the immunomodulatory agent is administered to a subject, e.g., a subject receiving a cancer-killing T cell as described herein, as part of a combination therapeutic protocol.

In one embodiment, the immunomodulatory agent is an inhibitor of an immune checkpoint inhibitor, e.g., an immune checkpoint inhibitor described herein, e.g., an inhibitor of one or more of: CTLA4, PD1, PDL1, PDL2, B7-H3, B7-H4, TIM3, LAG3, BTLA, CD80, CD86, or HVEM. In embodiments, the immunomodulatory agent is an agonist of T cells, e.g., an agonist of T cells described herein, e.g., agonistic antibody, e.g., an agonistic antibody or fragment thereof to CD137, GITR, or CD40. In one embodiment, the immunomodulatory agent is an inhibitor of MDSCs and/or Treg cells.

In one embodiment, the immunomodulatory agent is lenalidomide. In other embodiments, the immunomodulatory agent activates an immune response to a tumor specific antigen, e.g., it is a vaccine (e.g., a vaccine against targets such as gp100, MUC1 or MAGEA3. In other embodiments, the immunomodulatory agent is a cytokine, e.g., a recombinant cytokine chosen from one or more of GM-CSF, IL-7, IL-12, IL-15, IL-18 or IL-21. In other embodiments, the immunomodulatory agent is an autologous T cell, e.g., a tumor-targeted extracellular and intracellular tumor-specific antigen (e.g., a CAR T cell or a TCR T cell). In yet other embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Additional examples of immunomodulatory agents are further described in, *e.g.*, Adams JL (2015) and Serafini P (2008).

*Further embodiments of methods of producing cancer-killing T cells*

In embodiments, the method (e.g., of producing) further comprises producing a cancer-killing T cell preparation, e.g., a pharmaceutical preparation.

In embodiments, the method (e.g., of producing) further comprises detecting the presence of the cancer-killing T cell.

In embodiments, the method (e.g., of producing) further comprises purifying the cancer-killing T cell from the bispecific or multispecific antibody.

In embodiments, the bispecific or multispecific antibody is present, e.g., in the preparation, at a concentration of less than 10% by weight, e.g., less than 10%, 9%, 8%, 7%, 6%, 5%, 4%, 3%, 2%, 1%, 0.5%, 0.1%, 0.05%, 0.01%, 0.005 or less (e.g., but no less than 0.001%). In a preferred embodiment, the bispecific or multispecific antibody is present in the preparation at a concentration of 0.005% to 10% by weight.

In embodiments, the reaction mixture contains in volume a few nanoliters (e.g., less than 10 nl, about 1 to 5 nanoliters) of bispecific or multispecific antibody, which are added to over 50 microliters (e.g., about 60 microliters) of cell suspension.

In embodiments, the preparation comprises bispecific or multispecific antibody, e.g., a level of bispecific or multispecific antibody, detectable by immune assay.

In embodiments, the selecting and/or enriching step comprises using a fluorescently labeled molecule (e.g., a cell surface label, e.g., a fluorescently labeled antibody or fragment thereof, or a cell tracker dye) that diffuses into the cancer cell membrane or binds to i) one or more cancer antigens or ii) one or more markers of activated T cells, or both i) and ii). In embodiments, the selecting and/or enriching step comprises using fluorescence activated cell sorting (FACS).

In embodiments, the selecting and/or enriching comprises using a bead (e.g., magnetic bead) coated with an antibody or fragment thereof that binds to i) one or more cancer antigens or ii) one or more markers of activated T cells, or both i) and ii).

In embodiments, the selecting and/or enriching step comprises the sequential addition of a low, e.g., an insufficient, number of cancer cells. In embodiments, the methods of producing described above can generate different clones of cytotoxic T cells. In embodiments, selection of the cytotoxic T cell clones that are the most efficient or most potent at killing cancer cells can be achieved by sequentially adding low, e.g., insufficient, amounts of cancer cells. In embodiments, a low, or insufficient, number or amount of cancer cells that can be added to a reaction comprising cancer-killing T cells is 50% or less, e.g., 30%, 10%, 1%, 0.1%, or 0.01% or less, of the number of activated T cells. In one embodiment, the low, or insufficient, number of cancer cells can be added to cancer-killing T cells (e.g., a reaction comprising cancer cells, T cells, and/or a bispecific or multispecific antibody) one or more times, e.g., 2, 3, 4, 5, 6, 7, 8, 9 or 10, times. In one embodiment, the low, or insufficient, number of cancer cells is added every 6 hours, 12 hours, 24 hours, 36 hours, or 48 hours. In an embodiment, the low, or insufficient, number of cancer cells that are added are cancer cells from the patient. In an embodiment, the low, or insufficient, number of cancer cells that are added are not cancer cells from the patient. In an embodiment, the low, or insufficient, number of cancer cells that are added are cancer cells from a cancer cell line.

In embodiments, the cancer-killing T cells are expanded. In embodiments, the expansion of the cancer-killing T cells comprises increasing the number of cancer-killing T cells, e.g., in a preparation, e.g., by at least about 2-fold (e.g., at least about 3-, 4-, 5-, 6-, 7-, 8-, 9-, 10-, 15-, 20-, 50-, 100-, 1000-, $10^4$-, $10^5$-, $10^6$-fold, or more).

In other embodiments, the cancer-killing T cells are not substantially expanded.

In embodiments, the cancer-killing T cell preparation comprises a fluorescently labeled molecule (e.g., a cell surface label, e.g., a fluorescently labeled antibody or fragment thereof or a cell tracker dye) and/or the pICF, e.g., wherein the fluorescently labeled molecule and/or the pICF are present at trace amounts (e.g., less than 5% by weight, e.g., less than 5%, 4%, 3%, 2%, 1%, 0.5%, 0.25%, 0.1%, 0.05%, 0.01%, 0.005%, 0.001% by weight, or less).

In embodiments, the cancer-killing T cell preparation (prior to purification or expansion) comprises cancer-killing T cells at a concentration of 5% or less of the total number of cells in the preparation.

In embodiments, a purified or enriched cancer-killing T cell preparation comprises cancer-killing T cells at a concentration of at least 50% (e.g., at least 50%, 55%, 60%, 65%, 70%, 75%, 80%, 85%, 90%, 95%, 99%, or greater) of the total number of cells in the preparation.

In embodiments, a purified or enriched cancer-killing T cell preparation comprises activated cancer-killing T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, a purified or enriched cancer-killing T cell preparation comprises trogocytotic cancer-killing T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, the cancer-killing T cell or preparation comprises one or more CD8+ T cells. In embodiments, the cancer-killing T cell or preparation comprises one or more CD4+ T cells. In embodiments, the cancer-killing T cell or preparation comprises one or more CD25+ T cells. In embodiments, the cancer-killing T cell or preparation comprises one or more CD8+/CD25+ CTLs. In embodiments, the cancer-killing T cell or preparation comprises one or more CD4+/CD25+ T cells. In embodiments, the cancer-killing T cell or preparation comprises one or more cytotoxic T lymphocytes (CTLs), e.g., cancer antigen-specific CTLs. In embodiments, the cancer-killing T cell or preparation comprises one or more effector memory T cells. In embodiments, the cancer-killing T cell preparation does not comprise a substantial number of regulatory T cells (Tregs).

In embodiments, the method (e.g., of producing) further comprises reducing the number of Tregs in the cancer-killing T cell preparation. In one embodiment, the bispecific or multispecific antibody selectively expands the cancer-killing T cells, thus increasing the Effective E:T ratio of cancer-killing T cells:Tregs. In other embodiments, method further comprises removing (e.g., depleting) Tregs by physical separation, e.g., using a bead (e.g., a magnetic bead) attached to a Treg cell surface marker.

In embodiments, the cancer-killing T cell preparation comprises Tregs at a concentration of less than 10% (e.g., less than 9%, 8%, 7%, 6%, 5%, 4%, 3%, 2%, 1% or less) of the total number of cells in the preparation.

In embodiments, the cancer-killing T cell preparation does not comprise a substantial number of naïve T cells.

In embodiments, the cancer-killing T cell preparation comprises naïve T cells at a concentration of less than 10% (e.g., less than 9%, 8%, 7%, 6%, 5%, 4%, 3%, 2%, 1% or less) of the total number of cells in the preparation.

In embodiments, the naïve T cells express CD45RA, CD62L, CCR7, CD27, CD28 and/or CD57.

In embodiments, the cancer-killing T cell preparation comprises more than one clone of cancer-killing T cells.

In embodiments, the method (e.g., of producing) further comprises separating individual clones from the cancer-killing T cell preparation.

In embodiments, the separating step comprises clonal expansion of single cells (e.g., (i) separating the preparation of cancer-killing T cells into single cells (e.g., a single cell per well or container) and (ii) expanding the single cells to generate one or more preparations of cancer-killing T cells, wherein each preparation comprises a single clone).

In embodiments, the separating step comprises flow cytometry or limited dilution.

In aspects of the disclosure, the method (e.g., of producing) further comprises determining the cancer-killing activity of the cancer-killing T cell preparation, and optionally, selecting the preparation based on a parameter chosen from one or more of: increased cancer cell killing activity, reduced toxicity, reduced off-target effect, increased viability, increased proliferation, or Effective E:T ratio for cancer cell killing.

In embodiments, the cancer-killing T cell preparation comprises cells having high cancer-killing activity and/or low toxicity.

The cells comprised in the cancer-killing T cell preparation with low toxicity are cells which kill significantly less non-pathological cells, i.e. they kill more selectively. In embodiments, the cancer-killing T cell preparation comprises cells having low toxicity because they generate less cytokines in the supernatant and/or intracellularly. In embodiments, the cancer-killing T cell preparation comprises cells having both and simultaneously higher cancer-killing activity and low toxicity, because they generate less cytokines in the supernatant and/or intracellularly per unit of cancer cell killing, that is once the types and/or levels of cytokines released is normalized by the quantitative estimation of cancer cell killing activity such as Effective E:T Ratios, EC50, Emax, kinetics, or a combination of these factors.

In embodiments, the cancer-killing T cell preparation comprises cells that effectively kill cancer cells at a high target cell per T cell. In embodiments, a T cell to high target cell ratio is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In embodiments, the cancer-killing T cell preparation comprises a population of cells consisting of less than 10 clones of cancer-killing T cells. In embodiments, 10, 9, 8, 7, 6, 5, 4, 3, 2 or 1 clone of cancer-killing T cells is present in the preparation. In one embodiment, 2-4 clones are present in the preparation. In other embodiments, a single clone of cancer-killing T cells.

In embodiments, the T cell or T cell sample of the method (e.g., of producing) and the cancer cell or cancer cell sample of the method (e.g., of producing) are from the same subject.

In embodiments, the T cell or T cell sample of the method (e.g., of producing) and the cancer cell or cancer cell sample of the method (e.g., of producing) are from a different subject.

In embodiments, the cancer-killing T cell or preparation is administered to the subject, e.g., wherein the subject is the same subject as the subject from whom the T cells (and/or the cancer cells) were obtained. For example, the cancer-killing T cell or preparation is autologous.

In embodiments, the cancer-killing T cell or preparation is administered to the subject, e.g., wherein the subject is a different subject from the subject from whom the T cells (and/or the cancer cells) were obtained. For example, the cancer-killing T cell or preparation is allogeneic.

In embodiments, the method (e.g., of producing) comprises providing a sample comprising the T cell. In embodiments, method (e.g., of producing) comprises providing a sample comprising the cancer cell.

In embodiments, the T cell and the cancer cell the method (e.g., of producing) are from the same sample.

In embodiments, the T cell and the cancer cell of the method (e.g., of producing) are from different samples.

In embodiments, the sample is derived from a tissue with a microenvironment, e.g., a bone marrow, a lymph node, a primary tumor, or a metastasis.

In embodiments, the sample comprises blood (e.g., whole blood, peripheral blood, or bone marrow), a solid tumor (e.g., a sample resected from a primary tumor or a metastasis), a lymph node, or spleen of the subject. In embodiments, the sample is a blood sample e.g., whole blood, peripheral blood, or bone marrow, wherein substantially no components (e.g., cells or plasma) have been removed or isolated from the blood sample. In embodiments, the sample is diluted, e.g., with a physiologically compatible buffer or media, e.g., prior to and/or during step (c).

In embodiments, the method (e.g., of producing) comprises providing a T cell from a blood sample from the subject, e.g., where the T cell is not purified from other components, e.g., cells or plasma, in the blood sample. In embodiments, the blood sample is a bone marrow sample, a peripheral blood sample, or a whole blood sample.

In embodiments, the method (e.g., of producing) comprises providing a cancer cell from a blood sample from the subject, e.g., wherein the cancer cell is not purified from other components, e.g., cells or plasma, in the blood sample. In embodiments, the blood sample is a bone marrow sample, a whole blood sample, or a peripheral blood sample.

In embodiments, the cancer cell of the method (e.g., of producing) comprises a circulating cancer cell, e.g., from a blood sample, e.g., peripheral blood sample, of the subject.

In embodiments, the method (e.g., of producing) comprises providing a cancer cell from a tissue sample, e.g., a biopsy, e.g., of a tumor or metastasis, from the subject.

In embodiments, the method (e.g., of producing) comprise providing a sample, e.g., blood sample (e.g., bone marrow, peripheral blood, or whole blood sample), that comprises both the T cell and the cancer cell.

In embodiments, the subject is an adult or a pediatric subject.

In embodiments, the cancer is a hematological cancer, e.g., a B-cell or T cell malignancy.

In embodiments, the cancer is a Hodgkin's lymphoma, Non-Hodgkin's lymphoma (e.g., B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.

In embodiments, the cancer is a solid cancer, e.g., wherein the solid cancer comprises ovarian cancer, rectal cancer, stomach cancer, testicular cancer, cancer of the anal region, uterine cancer, colon cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not melanoma.

In embodiments, the method (e.g., of producing) does not comprise labelling the cancer cell (e.g., cancer cell membrane) with a fluorescent molecule prior to contacting the sample with the bispecific or multispecific antibody.

In embodiments, the subject:

(a) has not received a prior treatment for the cancer;
(b) has received one or more previous treatments for the cancer; or
(c) has minimal residual disease (MRD).

In embodiments, the period of time is 12 to 120 hours (e.g., 12-24 hours, 24-48 hours, 48-36 hours, 36-60 hours, 60-90 hours, or 90-120 hours) or 1-7 days (e.g., 1, 2, 3, 4, 5, 6, or 7 days).

In embodiments, the method described here further comprises repeating the sample or cell providing step, *ex vivo* reaction formation step and/or the enrichment step (e.g., steps (a)-(d) of the methods of producing) using a different sample of T cells and cancer cells, e.g., wherein each repeat of steps uses a different sample of T cells and cancer cells. In embodiments, the different sample of T cells and cancer cells comprises a sample derived from a tissue with a microenvironment, e.g., a bone marrow, a lymph node, a primary tumor, or a metastasis.

In embodiments, the cancer-killing T cell produced from each repeat of steps is pooled to a form a mixture of cancer-killing T cells.

In embodiments, the T cell comprises a CTC, and the T cell is from a sample (e.g., blood (e.g., whole blood, peripheral blood, or bone marrow), lymph node, primary tumor, or metastasis) from the subject. In embodiments, the T cell is enriched for the CTC. In embodiments, the T cell is purified, e.g., purified from other types of cells, e.g., from a blood sample from the subject (e.g., whole blood, peripheral blood, or bone marrow).

In embodiments, the method further comprises repeating the sample or cell providing step, *ex vivo* reaction formation step and/or the enrichment step (e.g., steps (a)-(d) of the methods of producing) using a different sample of T cells from the subject, e.g., wherein each repeat of steps uses a different sample of T cells from the subject.

In embodiments, the different sample of T cells comprises a sample derived from a cancer-containing tissue from the subject, e.g., a primary tumor, one or more metastases, a lymph node, a lymph sample, or a blood sample (e.g., whole blood, peripheral blood, or bone marrow).

In embodiments, the cancer-killing T cell produced from each repeat of the sample or cell providing step, *ex vivo* reaction formation step and/or the enrichment step (e.g., steps (a)-(d) of the methods of producing) is pooled to a form a mixture of cancer-killing T cells.

In embodiments, the method (e.g., of producing) further comprises evaluating the cancer-killing activity of the cancer-killing T cell. In embodiments, the evaluating comprises:

(a) contacting the cancer-killing T cells with target cells, wherein the target cells are derived from the cancer (e.g., wherein the target cells comprise a cell line derived from the cancer, e.g., wherein the target cells are not isolated from the subject) under conditions (e.g., for a period of time) sufficient to allow the cancer-killing T cells to kill the cancer cell;
(b) determining the number of target cells after step (a), and optionally determining the number of cancer-killing T cells after step (a);
where a decrease in the number of target cells compared to the number of target cells before the contacting step indicates that the cancer-killing T cells are effective in killing cancer cells. In embodiments, an increase in the number of cancer-killing T cells, e.g., compared to the number of cancer-killing T cells before the contacting step indicates that the cancer-killing T cells are effective in killing cancer cells.

In embodiments, the evaluating comprises:

(a) providing a cancer-killing T cell or a preparation thereof, e.g., produced according to a method described herein, e.g., from a subject (e.g., a subject with a cancer as described herein);
(b) providing a target cell (e.g., a cancer cell), e.g., wherein the cancer cell is from the subject;
(c) contacting the cancer-killing T cell or the preparation thereof with the target cell (e.g., cancer cells), under conditions (e.g., for a period of time) sufficient to allow the cancer-killing T cell to kill the cancer cell (in embodiments, the contacting step further comprises addition of a pICF, e.g., at different doses (e.g., increasing dosages);
(d) determining the level of target cells after step (c), and optionally determining the level of cancer-killing T cells after step (c) (in embodiments, the level of target cells and/or cancer-killing cells is determined at one or more time intervals after step (c)); and
(optionally) (e) determining the ratio of either target cell to T cell, or T cell to target cell, from step (d) (e.g., determining an Effective E:T ratio as described herein).

In embodiments, the evaluating comprises using a first patient sample, e.g., containing T cells and cancer cells, to generate a cancer-killing T cell, e.g., using a method described herein. In embodiments, the cancer-killing T cells are purified, sorted, enriched, expanded, and/or selected. In embodiments, the evaluating comprises subsequently mixing a second sample from the same patient with the cancer-killing T cells generated using the first patient sample. In embodiments, various concentrations of cancer-killing T cells can be mixed with the second sample, e.g., where the second sample is at a fixed concentration, e.g., to generate a dose response curve.

Accordingly, in embodiments, the evaluating comprises:

(a) producing a cancer-killing T cell or a preparation thereof, e.g., according to the method of claim 1, wherein the T cell and the cancer cell are present in a patient sample,
(b) optionally expanding, selecting, enriching, and/or purifying the cancer-killing T cell from (a),
(c) contacting the cancer-killing T cell from (a) or (b) with a second sample, e.g., from the same patient, wherein the second sample comprises one or more cancer cells, and wherein the cancer-killing T cell is contacted with the cancer cells, and
(d) determining a dose response and/or pharmacodynamic parameter as described herein.

In an embodiment, the level of activity of the cancer-killing T cells (e.g., trogocytotic cells) in the *ex vivo* mixture is measured by Effective E:T Ratios, EC50s, Emax, kinetics, or a combination of these factors.

In embodiments, step (c) comprises contacting the cancer cells with the cancer-killing T cells at a plurality of ratios, e.g., Effective E:T ratios.

In embodiments, step (c) comprises mixing different amounts of cancer-killing T cells with a fixed amount of cancer cells.

In one aspect of the disclosure, the Effective E:T is the ratio between the cancer-killing T cells and the cancer cells after pICF exposure.

In embodiments of any of the aforesaid methods, a decrease in the level or amount of cancer cells, e.g., relative to a reference level, is indicative of increased cancer cell killing. In other embodiments, a reduced change or no substantial change in the level or amount of cancer cells, e.g., relative to a reference level, is indicative of decreased cancer cell killing.

In embodiments of any of the aforesaid methods, a high level of target cell relative to T cell (e.g., a high Effective E:T ratio of target cell to cancer-killing T cell) indicates that the cancer-killing T cell or preparation thereof is an effective killer of cancer cells. In one embodiment, the target to T cell ratio is compared to a reference ratio. For example, an Effective E:T ratio of 1 (cancer-killing T cell) to 100 (e.g., 10, 20, 30, 40, 50, 75, 100 or higher) (target cells) is indicative of potent T cell killing activity. A subject having T cells having potent cell killing activity can be identified as being a strong responder to the bispecific or multispecific antibody.

Alternatively, a low level of target cell relative to T cell (e.g., a low Effective E:T ratio of target cell to cancer killing T cell) is indicative of a poor T cell killing activity. In one embodiment, the target to T cell ratio is compared to a reference ratio. In one embodiment, an Effective E:T ratio of 1 (cancer-killing T cell) to 5 (target cells) (e.g., 1, 3, or 5) is indicative of poor T cell killing activity. A subject having T cells having reduced cell killing activity can be identified as being a poor responder to the bispecific or multispecific antibody.

In embodiments of any of the aforesaid methods, the level of target cells and/or cancer-killing T cells is determined at one or more time intervals after step (c). In exemplary embodiments, the level of target cells and/or cancer-killing cells is determined at time 0, at time of 1-75 hours (e.g., 1, 2, 4, 8, 16, 24, 36 or 72 hours) or several days after step (c).

In embodiments of any of the aforesaid methods, the contacting step further comprises addition of a bispecific or multispecific antibody at different doses (e.g., increasing dosages) of the bispecific or multispecific antibody, e.g., to generate a dose response curve. In one embodiment, the difference between the level of cancer-killing T cells or cancer cells at a dose zero or at control level (e.g., a threshold dose) and a saturated dose of the bispecific or multispecific antibody is determined. In embodiments, the difference in the level of cancer-killing T cells or cancer cells at the saturated dose vs. threshold dose is determined. In embodiments, the Effective E:T ratio as used herein is the ratio of the difference in the level of cancer-killing T cells relative to the difference in the level of cancer cells.

In embodiments of any of the aforesaid methods, method is performed using an automated platform, e.g., an automated fluorescence-based platform, e.g., the ExviTech platform described herein.

In aspects of any of the aforesaid methods or assays, the activity of the pICF and/or immunomodulatory agent is determined using an *ex vivo/in vitro* assay to measure dose response curves, whose mathematical fitting enable quantitative parameters to estimate the activity, selected from at least one from EC50, Effective E:T ratio, Emax or kinetics. In embodiments of any of the aforesaid methods, the activity of the pICF assessed by step (e) is different from an activity assessment using a dose response of the pICF activity, e.g., compared to a standard depletion dose response curve.

In aspects of any of the aforesaid methods, the reference ratio is a predetermined ratio, e.g., about 1:3 to 1:10, e.g., about 1:3, 1:4, 1:5, 1:6, 1:7, 1:8, 1:9, or 1:10. In aspects, the high target cell to T cell ratio from step (e) is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In embodiments of any of the aforesaid methods, step (c) comprises forming *ex vivo* mixtures of the cancer-killing T cell or the preparation thereof with target cells, e.g., cancer cells. In embodiments, the cancer cell is a cell chosen from a hematological cancer, a solid cancer, a metastatic cancer (e.g., a CTC, or a combination thereof). In embodiments, the cancer cell is a leukemic or lymphoma blast cell (e.g., a blast cell expressing one or more markers chosen from CD19, CD123, CD20 or others). In embodiment, the T cell is a cell chosen from a blood sample (e.g., peripheral blood sample), a bone marrow sample, a lymph node sample, a tumor sample comprising a CTL and/or a TIL, or a combination thereof). In embodiments, the T cell expresses CD8 and/or CD25 (e.g., it is a CD8+CD25+ T cell).

In aspects of any of the aforesaid methods, the cancer killing T cell or preparation thereof is produced using a method that comprises use of a pICF, e.g., a pICF described herein.

In embodiments of any of the aforesaid methods, the cancer-killing T cell or preparation thereof comprises a T cell, e.g., CTL, that is CD8+ and CD25+. In some embodiments, the cancer-killing T cell is a trogocytotic T cell. In other embodiments, the cancer-killing T cell is a CD28+CD25+ T cell.

In embodiments of any of the aforesaid methods, the cancer-killing cell: (i) has cytotoxic activity toward a cancer cell, and (ii) comprises a cell surface marker derived from the cancer cell, e.g., at least 90-500 copies of a cell surface marker (e.g., 90, 100, 200, 300, 400, or 500 copies, e.g., one or more cancer cell surface markers).

In embodiments of any of the aforesaid methods, about 2 to 75% (e.g., about 2 to 70%, 2 to 60%, 2 to 50%, or 2 to 40%) of the total T cells in the reaction mixture express one or more cancer cell surface markers (e.g., one or more leukemic cell cancers).

In embodiments, the cancer-killing cell is enriched or purified. In some embodiments, the enriched or purified cancer-killing cell population comprises at least 80%-100% cancer-killing T cells (e.g., 80%, 90%, 95%, 99% or 100%), wherein the cancer-killing cells comprise one or more cancer cell surface markers.

In embodiments, the *ex-vivo* reaction mixture is prepared according to Good Manufacturing Practice (GMP). In embodiments, one or more of the expansion, selection and/or enrichment of the cancer-killing T cells is according to Good Manufacturing Practice (GMP). In embodiments, the method further comprises sending the produced cancer-killing T cell, e.g., to a hospital, a health care provider. In embodiments, the method further comprises receiving the T cell, the cancer cell, or both, e.g., from a hospital, a health care provider.

*Further embodiments of methods of treating*

In aspects of the disclosure, the method (e.g., of treating) further comprises administering a second therapeutic agent or procedure. In aspects of the disclosure, the second therapeutic agent or procedure is chosen from one or more of chemotherapy, a targeted anti-cancer therapy, an oncolytic drug, a cytotoxic agent, an immune-based therapy, a cytokine, a surgical procedure, a radiation procedure, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy.

In aspects of the disclosure, the second therapeutic agent is an agonist of T cells (e.g., an agonistic antibody or fragment thereof or an activator of a costimulatory molecule) or an immune checkpoint inhibitor.

In embodiments, the immune checkpoint inhibitor is an inhibitor of one or more of: CTLA4, PD1, PDL1, PDL2, B7-H3, B7-H4, TIM3, LAG3, BTLA, CD80, CD86, or HVEM.

In embodiments, the immune checkpoint inhibitor comprises one or more of: ipilimumab, tremelimumab, MDX-1106, MK3475, CT-011, AMP-224, MDX-1105, IMP321, or MGA271.

In embodiments, the agonist of T cells comprises an antibody or fragment thereof to CD137, CD40, and/or glucocorticoid-induced TNF receptor (GITR).

In one embodiment, the immunomodulatory agent is an inhibitor of MDSCs and/or Treg cells. In embodiments, the immunomodulatory agent comprises/is lenalidomide.

In aspects of the disclosure, the second therapeutic agent enhances and/or restores the immunocompetence of T cells.

In other embodiments, the immunomodulatory agent activates an immune response to a tumor specific antigen, e.g., it is a vaccine (e.g., a vaccine against targets such as gp100, MUC1 or MAGEA3). In other embodiments, the immunomodulatory agent is a cytokine, e.g., a recombinant cytokine chosen from one or more of GM-CSF, IL-7, IL-12, IL-15, IL-18 or IL-21. In other embodiments, the immunomodulatory agent is an autologous T cell, e.g., a tumor-targeted extracellular and intracellular tumor-specific antigen (e.g., a CAR T cell or a TCR T cell). In yet other embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Additional examples of immunomodulatory agents are described herein.

*Further embodiments of compositions and reaction mixtures*

In embodiments, the bispecific or multispecific antibody is an antibody molecule, e.g., a bispecific antibody or fragment thereof, e.g., a bispecific immunoglobulin (BsIgG), an immunoglobulin operatively linked to additional antigen-binding molecule, a bispecific antibody (BsAb) fragment, a bispecific fusion protein, or a BsAb conjugate.

In embodiments, the bispecific antibody is selected from the list consisting of BsMAb CD123/CD3, BsMAb CD19/CD3 and EpCAM/CD3.

In embodiments, the bispecific or multispecific antibody is present at a detectable amount, e.g., a concentration of less than 10% by weight, e.g., less than 10%, 9%, 8%, 7%, 6%, 5%, 4%, 3%, 2%, 1%, 0.5%, 0.1%, 0.05%, 0.01%, 0.001% or less (but no less than 0.0001%), e.g., in a composition described herein. In one embodiment, the bispecific or multispecific antibody is present at a level of less than 1%. In a preferred embodiment, the bispecific or multispecific antibody is present in the preparation at a concentration of 0.005% to 10% by weight.

In embodiments, the cancer-killing cell comprises an activated T cell.

In embodiments, the cancer-killing cell comprises a cell that has undergone trogocytosis, e.g., a cell that comprises a portion of a cell surface membrane from the cancer cell.

In embodiments, the cancer-killing cell is a T cell, e.g., a cytotoxic T lymphocyte, e.g., a CD8+ T cell.

In embodiments, the composition or preparation does not comprise a substantial number of cancer cells, e.g., comprising cancer cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of regulatory T cells (Tregs), e.g., comprising Tregs at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of naïve T cells, e.g., comprising naïve T cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of red blood cells, e.g., comprising red blood cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of non-immune cells, e.g., comprising non-immune cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation comprises activated T cells at a concentration of at least 30%, (e.g., at least 30%, 40%, 50%, 60%, 70%, 80%, 90%, 95%, 99%, or more) of the total number of cells in the composition or preparation.

In embodiments, the composition or preparation comprises trogocytic T cells at a concentration of at least 30%, (e.g., at least 30%, 40%, 50%, 60%, 70%, 80%, 90%, 95%, 99%, or more) of the total number of cells in the composition or preparation.

Also, provided herein is a preparation of cancer-killing T cells (e.g., made by a method of the invention) for use in (e.g., use in preparation of a medicament for) treating a cancer (e.g., a hematological cancer, a solid cancer or a metastatic cancer) in a subject.

Also, disclosed herein is a cancer-killing T cell for use in (e.g., use in preparation of a medicament for) treating a cancer (e.g., a hematological cancer, a solid cancer or a metastatic cancer) in a subject, where the cancer-killing T cell is produced by a method comprising:

(a) providing a sample from the subject, wherein the sample comprises a T cell and a cancer cell;
(b) contacting the sample with a proximity immuno-coaching factor (pICF), e.g., for a period of time; and
(c) enriching for cancer-killing T cells that have acquired a cell surface marker from the cancer cell.

Also, disclosed herein is a cancer-killing T cell for use in (e.g., use in preparation of a medicament for) treating a cancer (e.g., a hematological cancer or a solid cancer) in a subject, where the cancer-killing T cell is produced by a method comprising:

(a) providing a tumor sample from the subject;

(b) providing a blood sample (e.g., peripheral blood sample) from the subject, wherein the blood sample comprises a T cell;

(c) contacting the tumor sample with the blood sample and a proximity immuno-coaching factor (pICF), e.g., for a period of time; and

(d) enriching for cancer-killing T cells that have acquired a cell surface marker from the cancer cell.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. The materials, methods, and examples are illustrative only and are not intended to be limiting.

Other features and advantages of the invention will be apparent from the following detailed description and claims.

**Brief Description of the Drawings**

**Figure 1:** Scheme showing an exemplary process for selecting cytotoxic T cells by subsequent additions of a low number of tumor cells to the cytotoxic T cells previously generated by incubating a sample with a pICF.

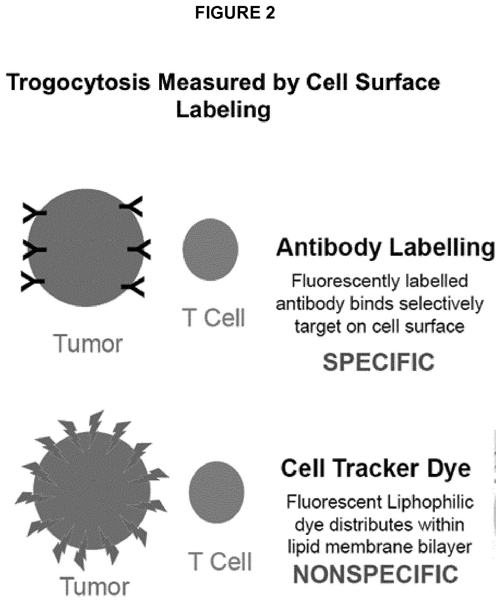

**Figure 2:** Scheme showing the mechanism of cell surface labeling used to measure trogocytosis. The top panel depicts cell surface labeling by a fluorescently labelled antibody that recognize specific cell surface markers on cancer cells. The lower panel shows nonspecific cell surface labeling of the cancer cell by cell tracker dye. The encounter between a labeled tumor cell with a T cell will lead to the formation of the immune synapse between the labeled tumor cell and the T cell, and trogocytosis in which the T cell incorporates portions of the tumor cell membrane such that the labeled antibody or the cell tracker dye is now present on the T cell surface.

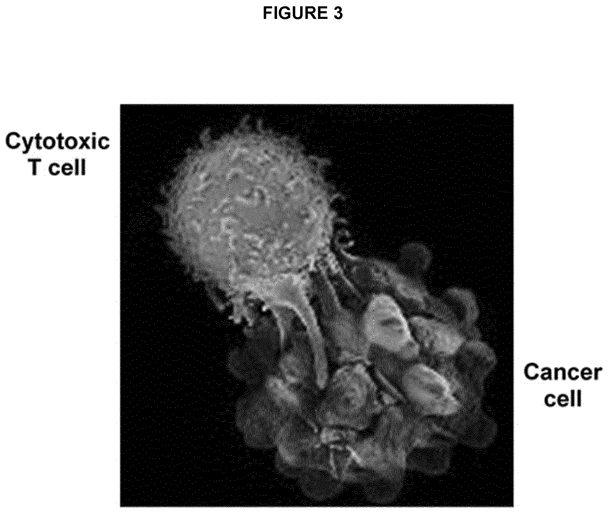

**Figure 3:** Picture showing the immune synapse created between a cancer-killing T cell (cytotoxic T cell) and a cancer cell, where the T cell extends its tentacles capturing the cancer cells. This contact involves deep overlap of the respective cell membranes involving patches of membrane across both cells.

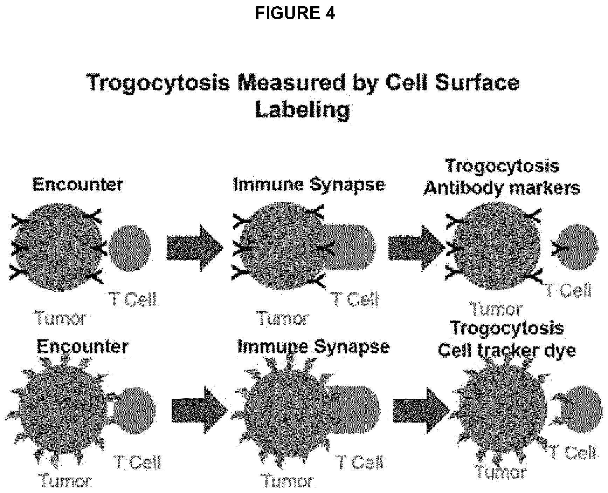

**Figure 4:** Scheme showing the measurement of trogocytosis by cell surface labeling using fluorescently-labeled antibodies that specifically bind to targets on the cell surface (top) and cell tracker dyes which are fluorescent lipophilic molecules that non-specifically distribute within the lipid membrane bilayer.

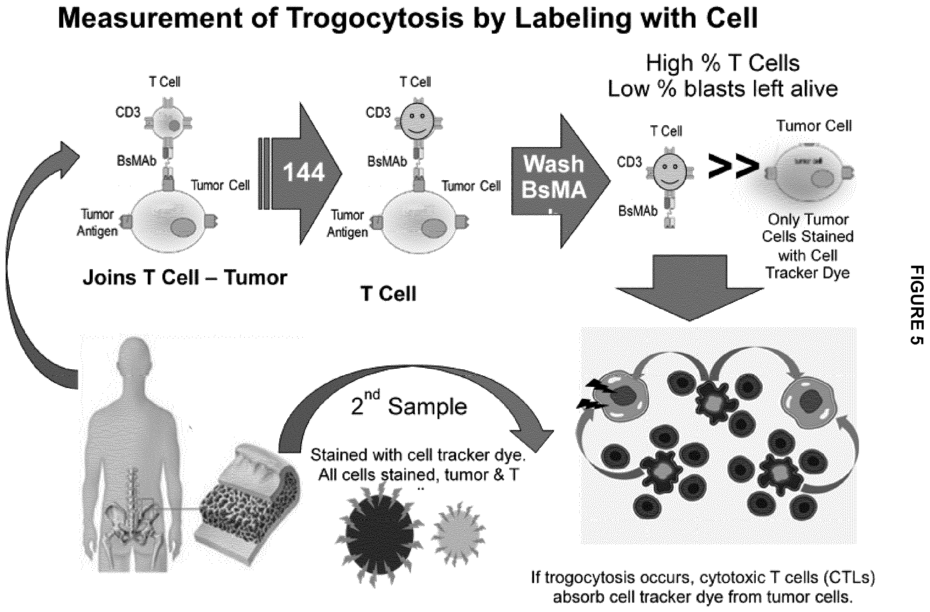

**Figure 5:** Scheme showing an exemplary process for measuring trogocytosis using a cell tracker dye in samples that contain both cancer cells and T cells. First, the cancer cells, T cells, and pICF are incubated together (e.g., for 144 hours). The pICF can be removed (e.g., by a wash step), and the cytotoxic T cells generated will kill the cancer cells such that there is a high percentage of cytotoxic T cells and a low percentage of cancer cells in the mixture. A second set of cancer cells labeled with cell tracker dye can be introduced, and trogocytosis can be measured by identifying the T cells that have incorporated labeled cell membrane from the second set of cancer cells.

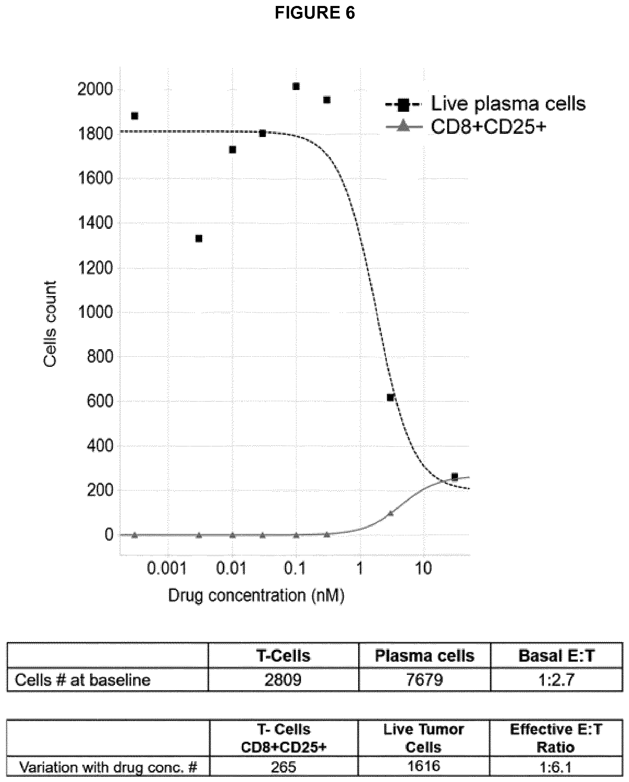

**Figure 6:** Graph showing the T lymphocyte cells count (CD8+ CD25+) and plasma cells count in a bone marrow sample from a Multiple Myeloma patient after incubation with various concentrations of a tumor target CD3 T-cell bispecific antibody.

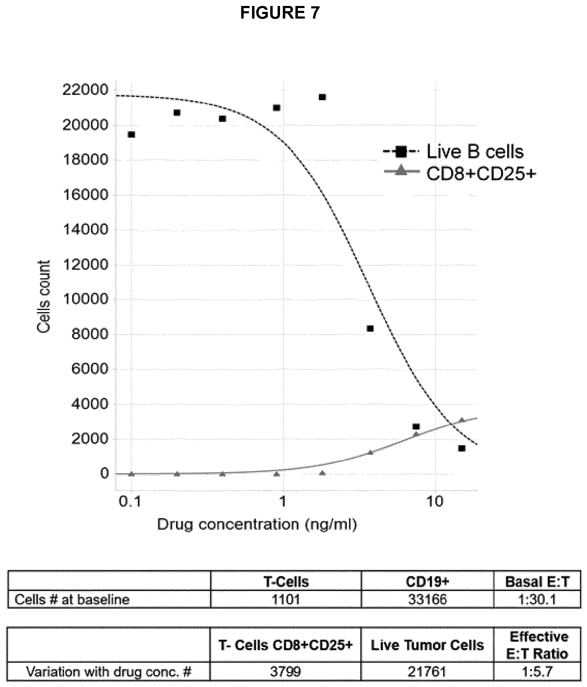

**Figure 7:** Graph showing the malignant B lymphocyte cell count and the T lymphoid cell count (CD8+CD25+) in a peripheral blood sample from a Chronic Lymphoid Leukemia patient after incubation with various concentration of a CD19 (tumor target)/CD3 (T-cell target) bispecific antibody.

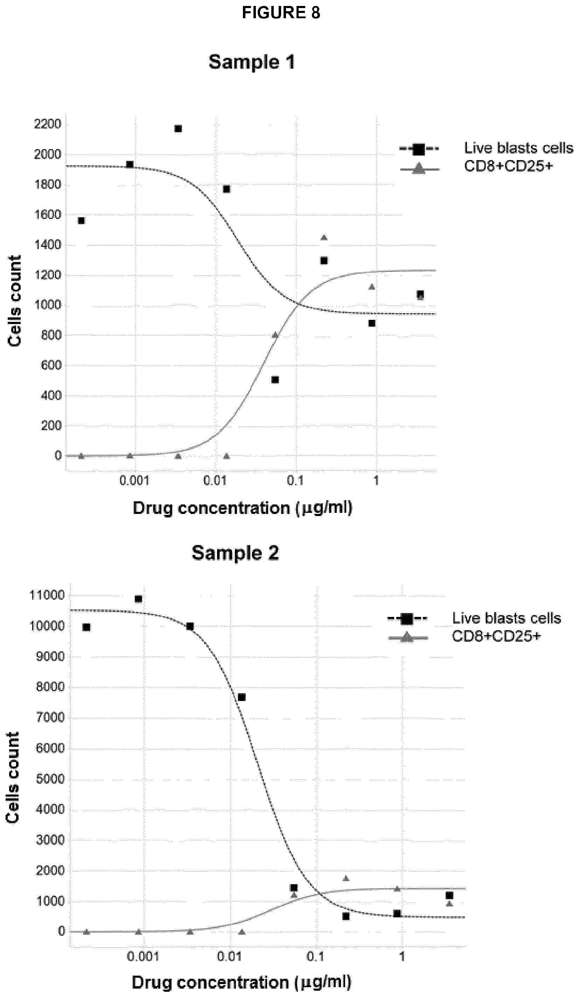

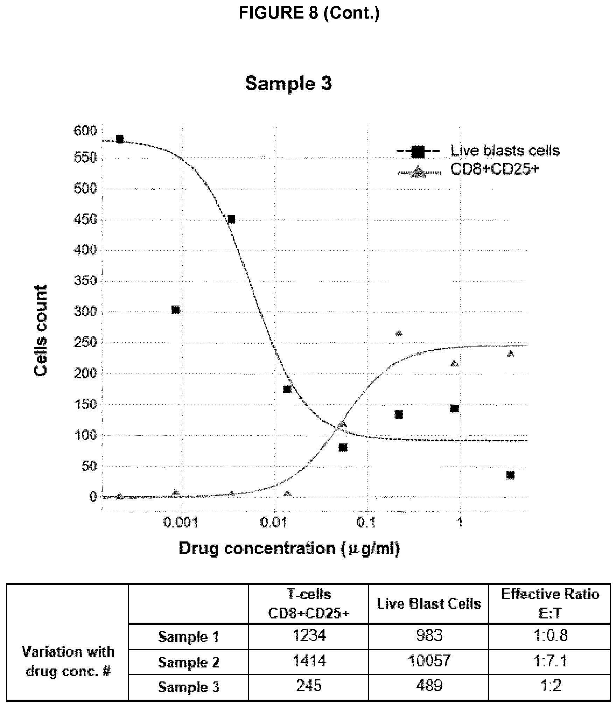

**Figure 8:** Set of graphs showing the blast cell count and T lymphoid cell count (CD8+CD25+) from bone marrow samples from three Acute Myeloid Leukemia patients (Sample 1, 2 and 3) after incubation with various concentrations of a CD123 (tumor target)/ CD3 (T-cell target) bispecific antibody.

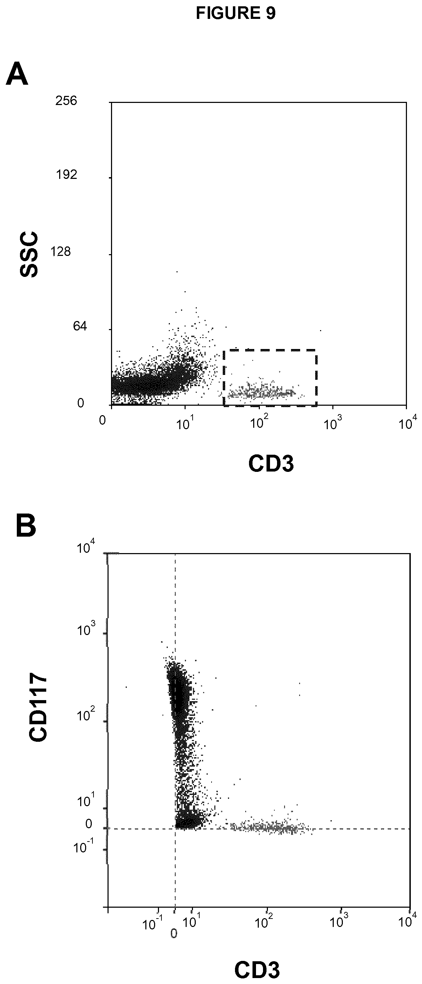

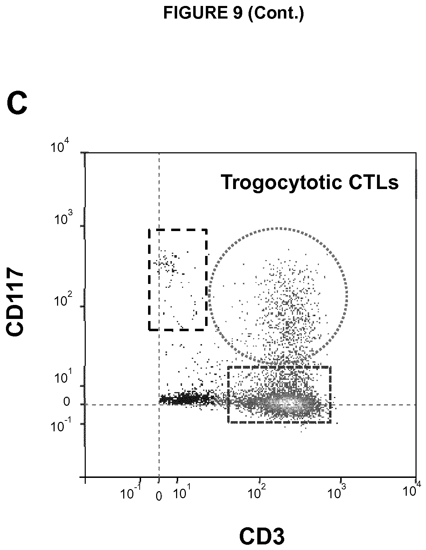

**Figure 9:** Graphs showing various types of cell populations in an AML patient bone marrow sample before (Figures 9A and 9B) and after (Figure 9C) incubation with a drug (a bispecific monoclonal antibody). The circle in Figure 9C shows the appearance of the trogocytotic population CD117+/CD3+.

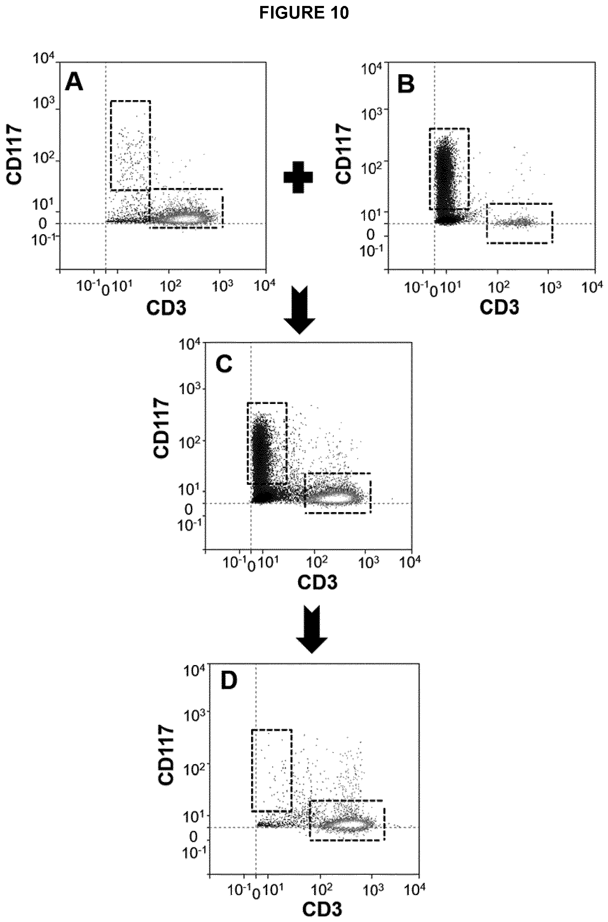

**Figure 10:** Flow cytometric dot plots (CD117vsCD3) of an experiment showing the killing capacity of a newly generated CTL population (trogocytotic cells) after exposure to a CD123/CD3 BsMAb for 144 hours. Figure 10A shows the dot plot from the first AML sample after the 144 hour BsMAb exposure, where the AML blast cells (CD117+/CD3-) have been eliminated leaving only the newly generated T cells (CD3+/CD117-). Figure 10B shows the dot plot of a new cryopreserved vial from the same patient with the presence of blast cells (CD117+/CD3-) and a low level of residual T cells (CD117-/CD3+). Figure 10C shows the dot plot of the mixture of the samples of Figure 10A and Figure 10B at 0-time point, where the presence of both T cells (CD3+/CD117-) and new AML blast cells (CD117+/CD3-) can be seen. Figure 10D is the dot plot after a 24-hour incubation, where clearly the AML blast cells (CD117+/CD3-) were depleted in the presence of the previously generated T-cell population. This was done in the absence of the CD123/CD3 BsMAb.

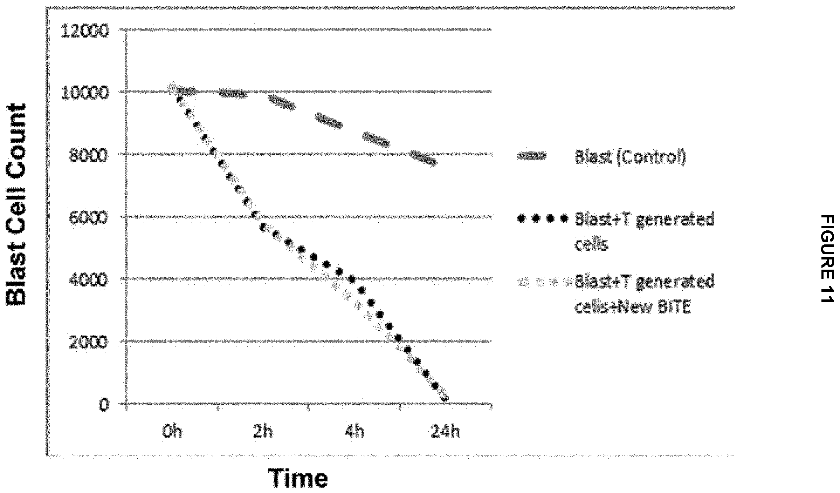

**Figure 11:** Graph showing the results over time of the capacity of newly generated trogocytotic T cells to kill AML blast cells both with and without the reintroduction of the drug (bispecific antibody).

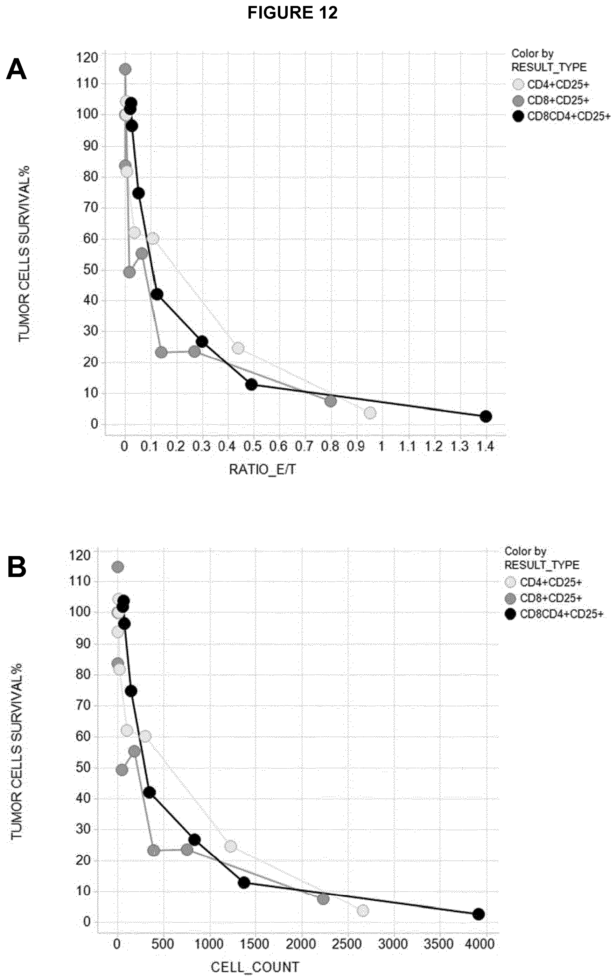

**Figure 12:** Set of representative plots of target cell lysis by sorted activated T-lymphocytes (CD4+CD25+, CD8+CD25+ and a mixture CD4+CD25+/CD8+CD25+) against blast cells from an AML sample (Tumor cell survival %), by E:T ratios (Figure 12A), or T cell numbers (Figure 12B). Individual data points joined by lines without model fitting.

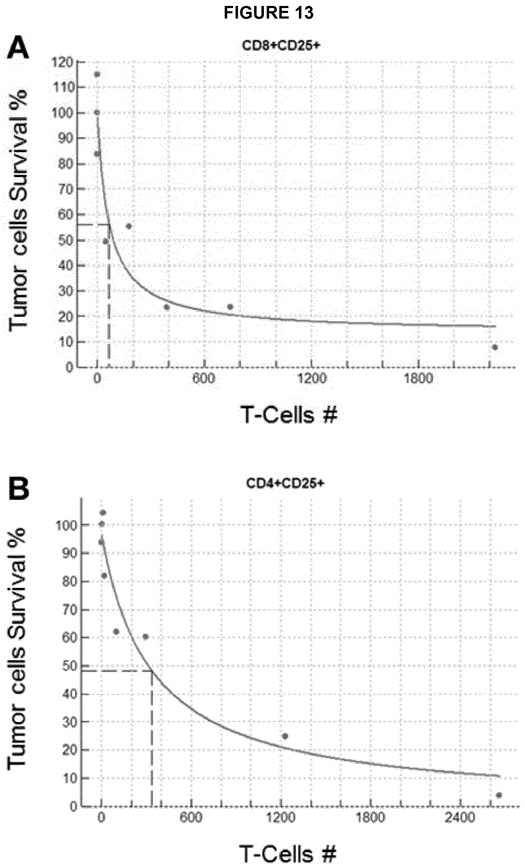

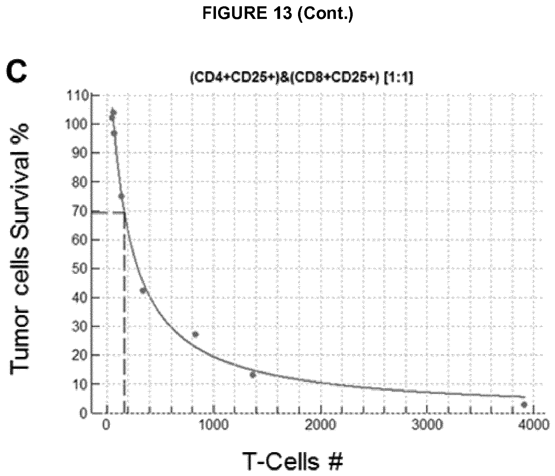

**Figure 13:** Set of graphs in which % tumor cell killing by activated cancer-killing T cells (Figure 13A: CD8+CD25+, Figure 13B: CD4+CD25+ and Figure 13C: mixture of CD4+CD25+/CD8+CD25+) fitted by a hyperbolic ligand-receptor model in an AML sample. Tumor cells survival % was estimated relative to the no-drug control. The dashed line corresponds to the IC50 value.

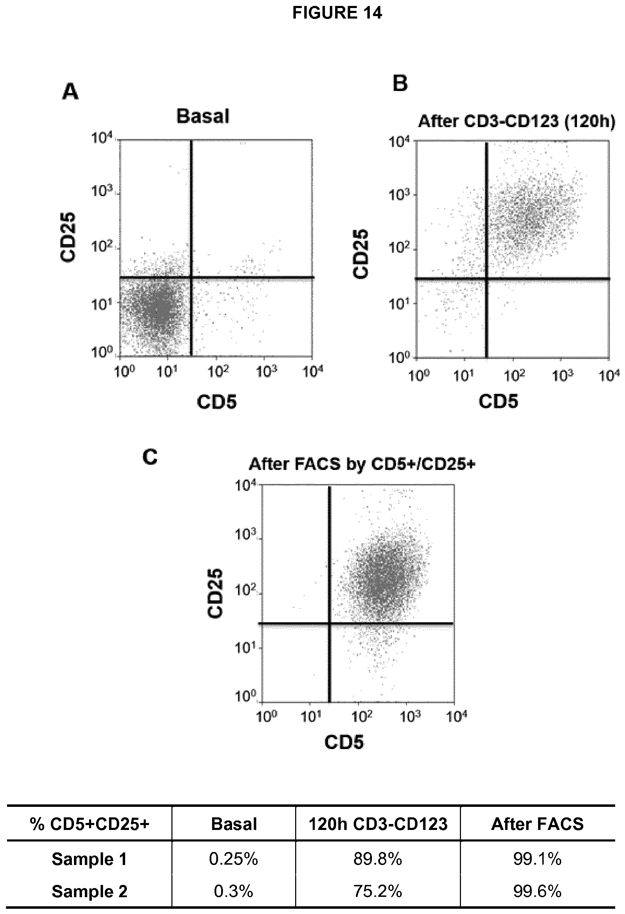

**Figure 14:** Flow cytometric dot plots (CD5 vs. CD25) showing how the CTL cell count can be enriched in two BM samples from an AML patient. Figure 14A shows the state of the sample upon receipt (CS5-CD25-). Figure 14B shows the sample after incubation with a CD123/CD3 BsMAb (CD5+CD25+). Figure 14C shows the sample after fluorescence activated cell sorting the CD5+CD25+ population. The table shows the percentage of CTLs present during each step of the process.

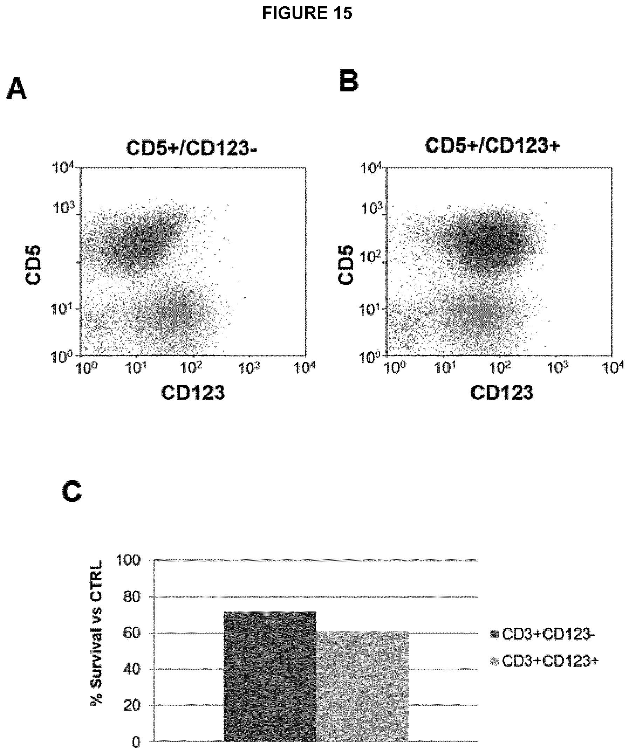

**Figure 15:** Flow cytometric dot plots (CD5 vs. CD123) showing how CTLs can be sorted into trogocytic (CD5+CD123+) and non-trogocytic (CD5+CD123-) CTL populations. Figure 15A shows the CD3+CD25+CD123- cell population. Figure 15B shows CD3+CD25+CD123+ cell population. Figure 15C shows how the ability of the CD123+ and CD123- populations to deplete AML blast cells.

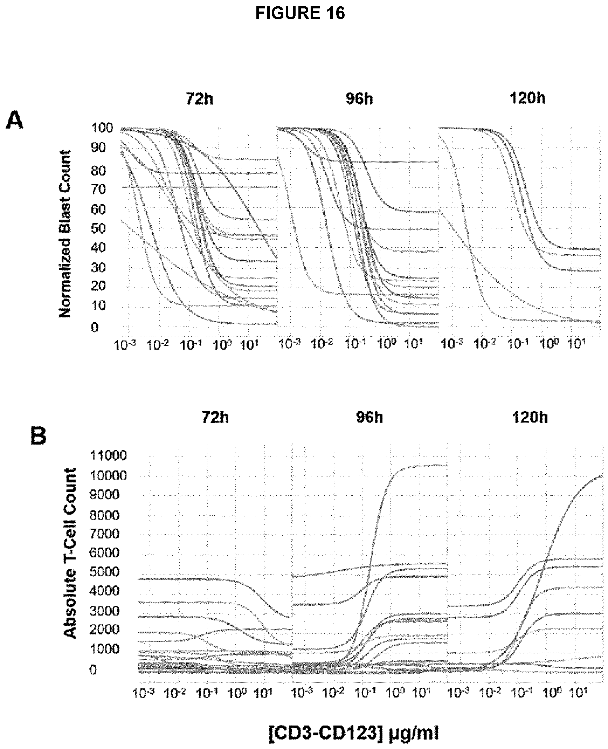

**Figure 16:** Set of graphs showing the effect of a CD123CD3 BsMAb on Blast Cell Counts (Figure 16A) and T-cell Counts (Figure 16B) in AML patient samples. There is a time- and concentration-dependent decrease in blast cell counts with a corresponding time- and concentration-dependent increase in absolute T-cell counts.

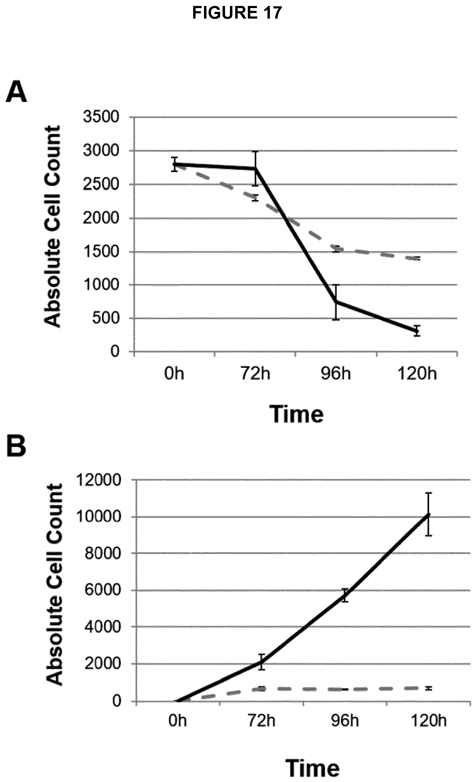

**Figure 17:** Effect of a CD123CD3 BsMAb in a sample from a patient with MRD AML. There is a time-dependent decrease in blast cell counts (Figure 17A) with a corresponding time-dependent increase in absolute T-cell counts (Figure 17B) even though the monocyte population of the sample contained less than 5% blast cells. The dashed line represents the control sample without BsMAb and the solid line represent the CD123/CD3 BsMAb data.

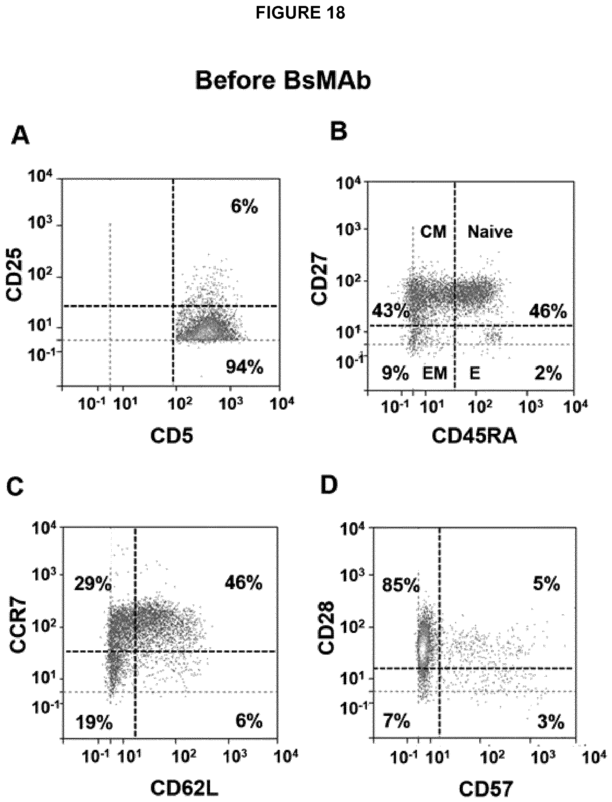

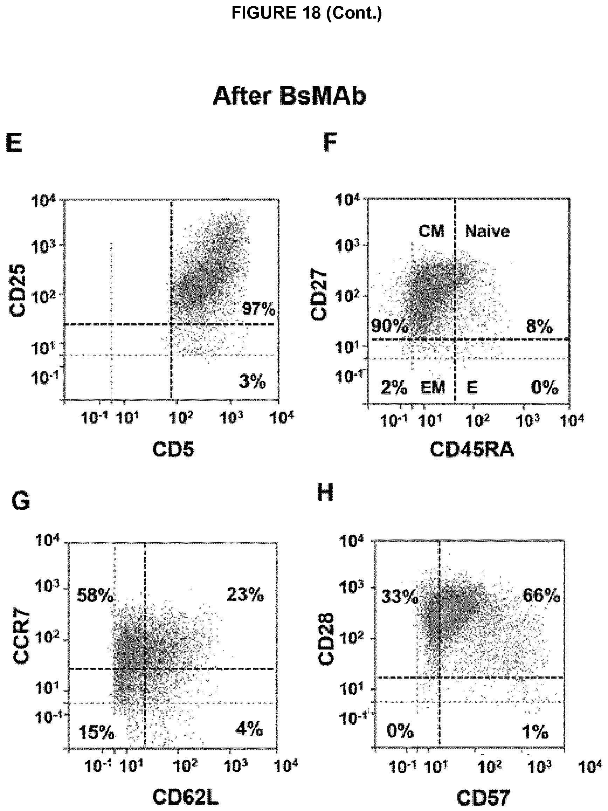

**Figure 18.** Flow cytometric dot plots showing the level of expressed markers before and after incubation of a BM sample from an AML patient with a CD123/CD3 BsMAb for 120 hours. Figures 18A - 18D represent the cells at the basal time point, prior to BsMAb, and Figures 18E -18H displays the status of the sample after incubation. Figures 18A and 18E show the level of expression of the activation marker CD25. Figures 18B and 18F show the distribution of the subpopulations indicated by phenotype: Naïve T-cells (Naive, CD45RA$^+$/CCR7$^+$/CD27$^+$), central memory T-cells (CM, CD45RA$^-$/CCR7$^+$/CD27$^+$), effector memory T-cells (EM, CD45RA$^-$/CCR7$^-$/CD27$^{+/-}$), and terminal effector memory T-cells (E, CD45RA$^+$/CCR7$^-$/CD27$^-$). Figures 18C and 18G show expression levels of markers CCR7 and CD62L and Figures 18D and 18H show markers CD28 and CD57. Data is from one AML sample that is representative of 4 AML samples tested.

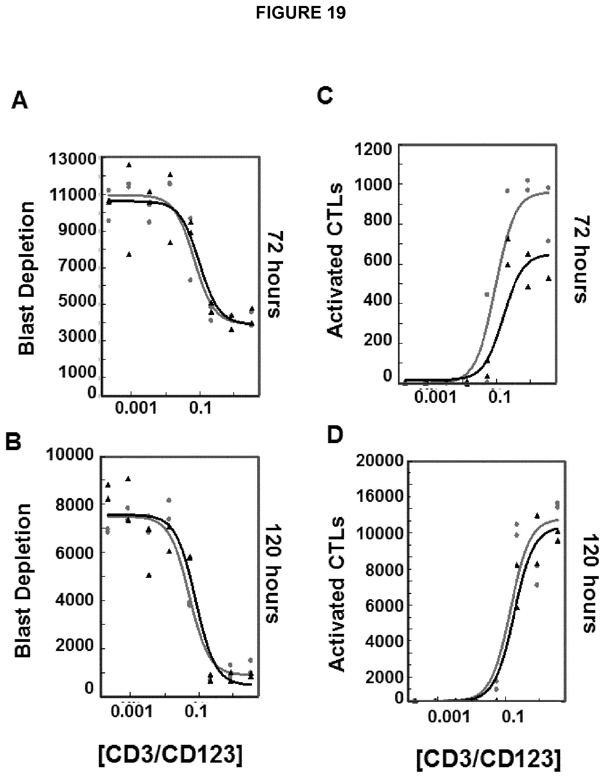

**Figure 19.** Results of incubating an AML BM sample with a CD123/CD3 BsMAb at two time points, 72 hours and 120 hours. Figure 19A shows blast cell depletion for a 72-hour incubation and in Figure 19B shows the blast cell depletion for a 120-hour incubation. Levels of activated CTLs were measured simultaneously, represented in Figure 19C for the 72h incubation and in Figure 19D for the 120h incubation.

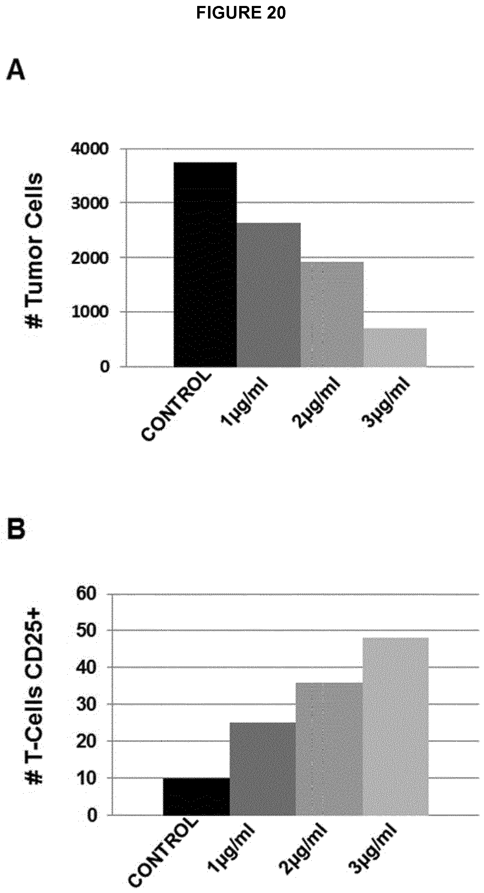

**Figure 20.** Effect of EpCAM/CD3 bispecific antibody in control and tumor cells. Figure 20A represent tumor cells killing in a dose-dependent manner. Figure 20B shows that the increase in tumor depletion is associated with an increase of the CD25 activation marker on the T cells.

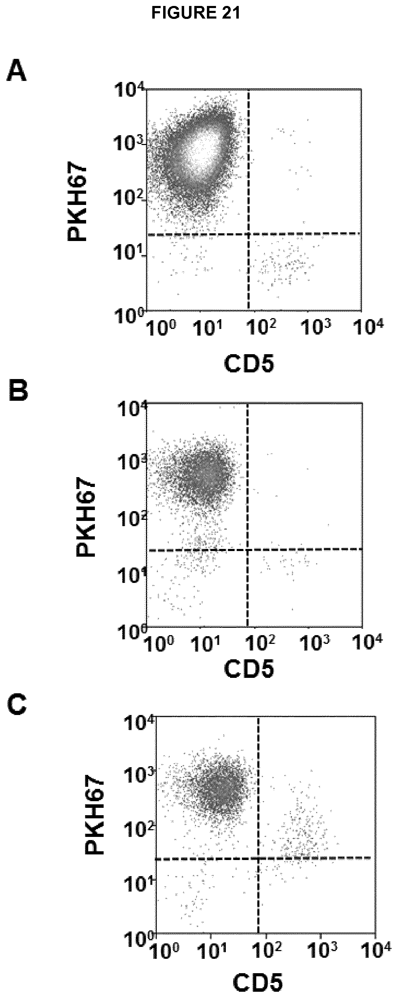

**Figure 21.** Flow cytometric dot plots (CD5 Vs PKH67) illustrating the trogocytotic capacity of T cells. Figure 21A shows the dot plot of the blast cells (PKH67++/CD5-) and T-cells (PKH67-/CD5++) at the basal time before incubation. Figure 21B shows the dot plot of the blast cells (PKH67++/CD5-) and T-cells (PKH67-/CD5++) after incubation and in the absence of the BsMAb. Figure 21C shows the dot plot of the blast cells (PKH67++/CD5-) and T-cells (PKH67-/CD5++) after incubation and in the presence of the CD123/CD3 BsMAb.

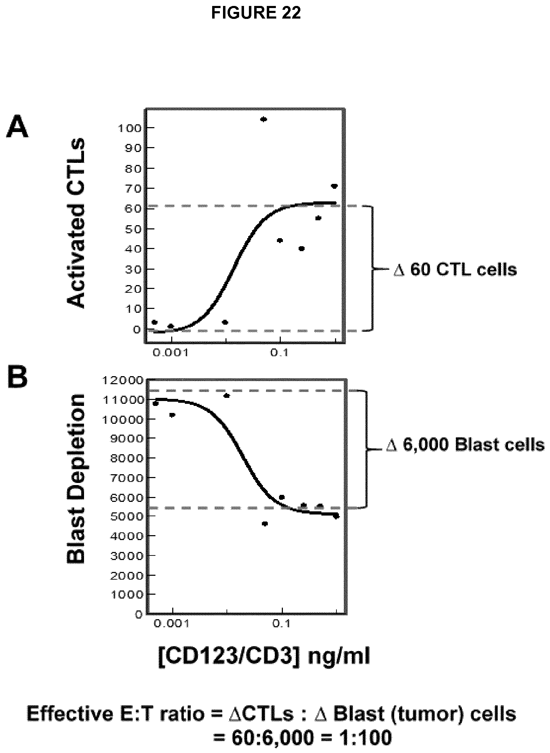

**Figure 22.** The CD123/CD3 BsMAb induce response in a BM sample from an AML patient. Figure 22A shows a dose-response increase in the CTL population, that corresponds with a (Figure 22B) dose-response depletion of blast (tumor) cells. The change ($\Delta$) in absolute counts is used to determine the Effective E:T ratio of 1:100.

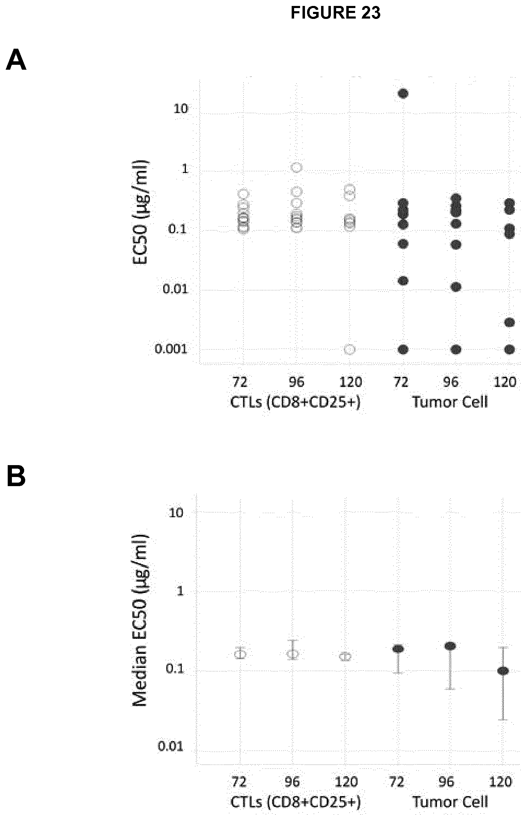

**Figure 23.** Set of graphs showing the EC50 of a CD123/CD3 BsMAb in both CD8+CD25+ CTLs (empty circles) and tumor cells (Filled circles) at different time points (72-96-120 hours) in 13 AML bone marrow samples. Figure 23A shows the distribution of the EC50 values for sample, and Figure 23B shows the median value for each cell population at each time point. The EC50s for both CD8+CD25+ CTLs and tumor cells are relatively similar between the populations and time points.

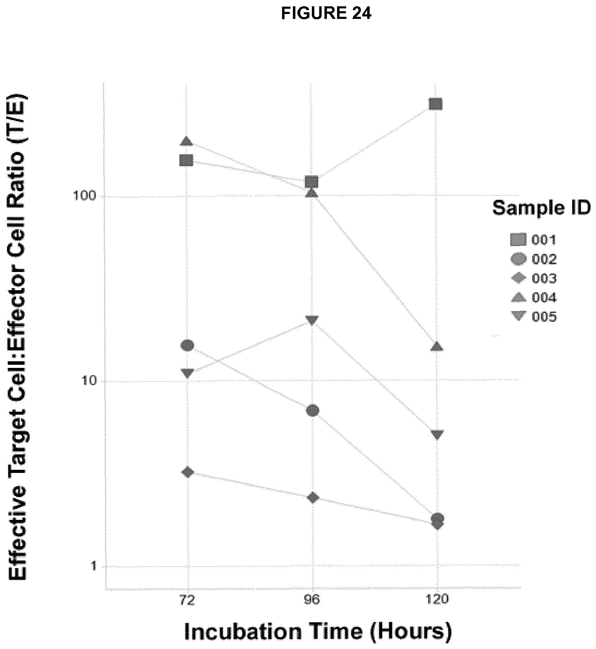

**Figure 24.** Effective T:E ratio of 5 AML samples (selected from those shown in Figure 23, Sample ID 001-005) incubated with a BsMAb CD123/CD3 at different incubation times. The y-axis displays the number of Target cells (tumor cells) for each Effector cell (CTL) at each time point.

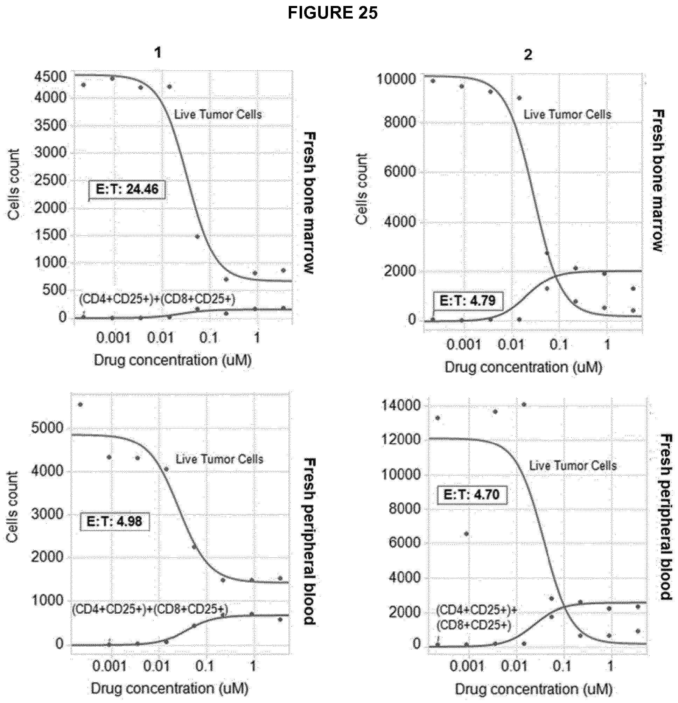

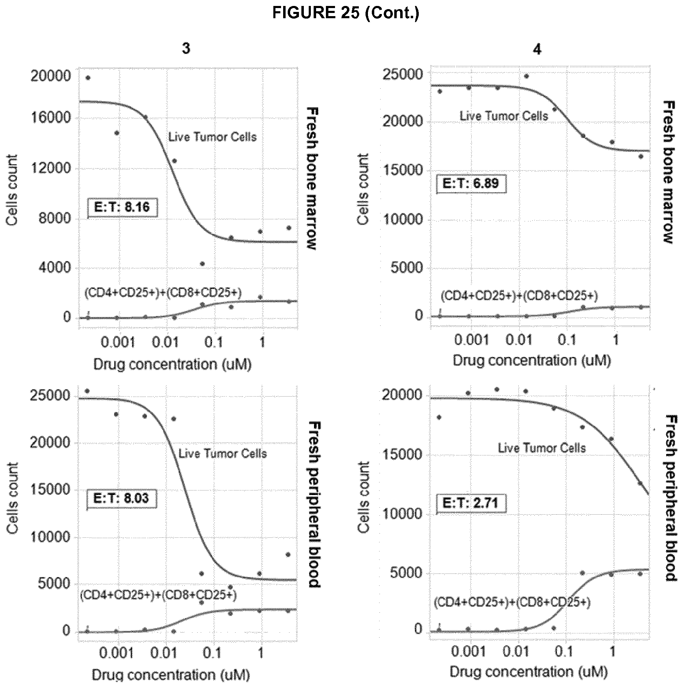

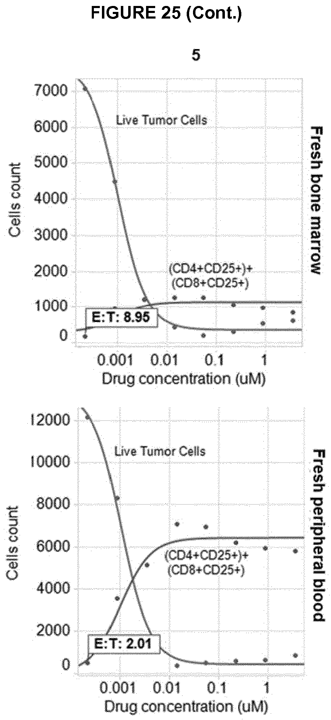

**Figure 25:** Set of graphs showing the effect of a CD123CD3 BsMAb on 5 AML patients (1 - 5) with both paired BM (upper panels) and PB (lower panels) samples. The effective E:T ratio is represented for each sample in the corresponding graph showing higher cytotoxicity in the BM samples.

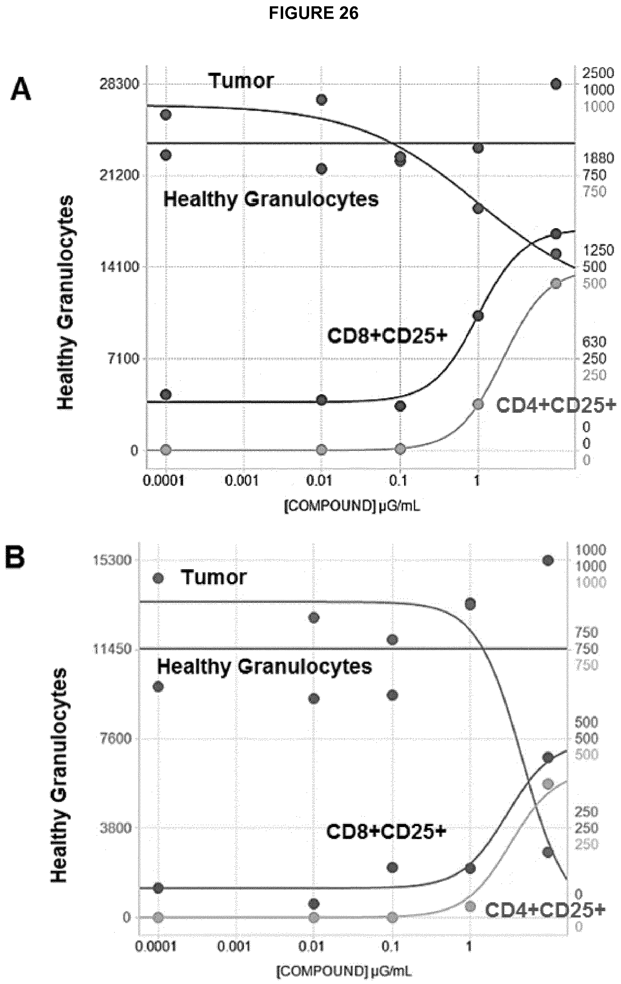

**Figure 26:** Graphs showing the results of a BsMAb on two MM patients (A and B) on different cell populations, including healthy granulocytes. BsMAb show a decrease on tumor cells with an increase of the CTLs (CD8+CD25+) but do not kill healthy granulocytes.

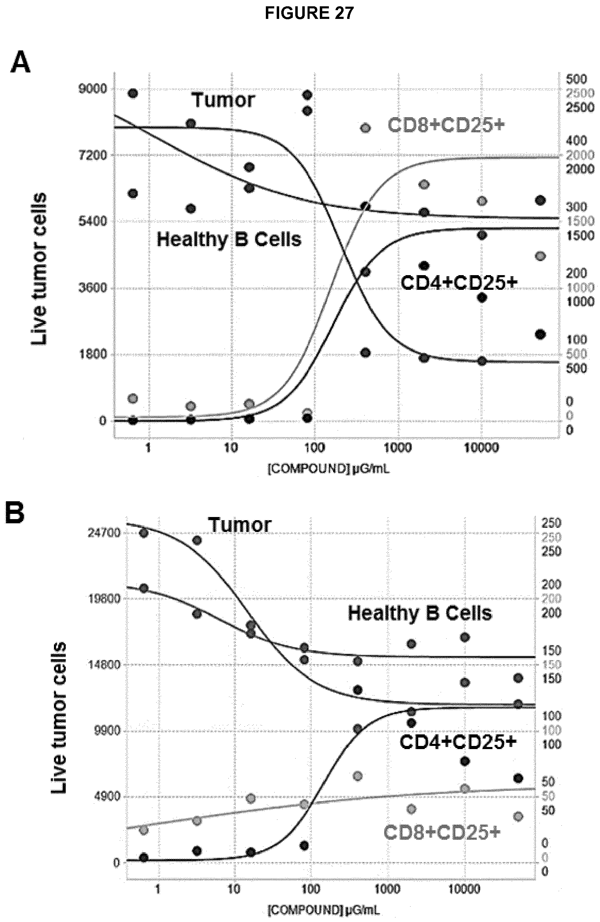

**Figure 27:** Graphs showing the results of a BsMAb on two AML patients (A and B) on different cell populations, including healthy B-cells. BsMAb show a decrease on tumor cells with an increase of the CTLs (CD4+CD25+ and CD8+CD25+) and minimal kill of healthy B-cells.

## Detailed Description of the Invention

The present disclosure relates, at least in part, to a personalized medicine approach to generating and/or selecting immune effector cells that have enhanced cytotoxic activity toward undesired target cells, e.g., cancer cells. Featured herein, for example, is a method for producing immune effector cells (e.g., T cells, e.g., CTLs) that have enhanced cytotoxic activity toward target, e.g., cancer cells. In embodiments, the method comprises bringing immune effector cells (e.g., T cells, e.g., cytotoxic T lymphocytes (CTLs)) in spatial proximity with target cells, e.g., cancer cells. In embodiments, the method comprises using an agent, e.g., a proximity immuno-coaching factor (pICF) to induce the spatial proximity.

Without wishing to be bound by theory, it is believed that the spatial proximity of the immune effector cells with the target cells, e.g., cancer cells, increases the number of immune effector cells that undergo immune cell activation, and some of which undergo a process called trogocytosis, e.g., compared to the number of cells that would undergo trogocytosis in the absence of a pICF. Also, without wishing to be bound by theory, it is believed that T cells (e.g., CTLs) that have undergone trogocytosis (also referred to as trogocytotic T cells) have enhanced cytotoxic activity toward target cells, e.g., cancer cells. Trogocytotic T cells can comprise a number of memory T cells that are poised and highly sensitized to kill the specific target cells (e.g., cancer cells) to which they are exposed during the method described herein.

It is also believed that a percentage of T cells in solid tumors and in hematological malignancies are enriched in tumor infiltrating lymphocytes (TILs), and/or cancer antigen-specific CTLs (i.e., CTLs that recognize antigens specific to cancer cells). It is believed that most if not all of the T cells present inside the mass of a solid tumor are immunosuppressed Tumor-Specific Antigen T cells called TILs (Tumor Infiltrated Lymphocytes). It is also believed that the % of TILs in a solid tumor is an important predictor of clinical response to immunotherapy treatments. These concepts have led to the extended use of the "Basal E:T Ratio", the basal ratio of effector to target cells in a solid tumor, as a key immuno-oncology variable. However, in hematological tissues with cancer cells, from hematological malignancies or hematological tissues of solid tumors, such as blood, bone marrow, spleen, lymph nodes, there are always many T cells present. Thus, the Basal E:T Ratio in these hematological tissues has a very different meaning than in solid tumors. In fact, it is believed that the population of immunosuppressed Tumor-Specific Antigen (TSA) T cells in these hematological tissues with cancer cells is very small. Hence, if the Basal E:T Ratios are calculated following the same approach as in solid tumors, the ratio of total T cells to cancer cells, and the % of TSA T cells is very low, these Basal E:T Ratios may grossly overestimate the number of T cells with the innate capacity to kill effectively cancer cells (TSA) but immunosuppressed. The "Effective E:T Ratio" discovered herein captures this same concept of the ratio of the number of effector T cells with capacity to kill cancer cells effectively, divided by the number of cancer cells; it is an objective measurement in the presence of a pICF of the number of activated, cancer-killing T cells newly generated (the only ones that could kill cancer cells), and the number of cancer cells that have been killed, both relative to control conditions. The overwhelming use of the Basal E:T Ratio as a key variable in publications of bispecific antibodies incubated with samples of hematological malignancies indicates the lack of appreciation of the heterogeneity in T cells of hematological malignancies tissues.

It is believed that a higher percentage of these CTLs exist in microenvironments with a 3-dimensional structure, such as solid tumors, bone marrow, and lymph nodes, while a lower percentage of these CTLs may exist in more fluid environments, such as peripheral blood. The CTLs, e.g., cancer antigen-specific CTLs, may be a preferred starting material for generating CTLs having enhanced cancer killing activity, e.g., by incubating a sample (containing cancer cells and the CTLs) with a pICF. In some examples, the sample can be a microenvironment having a 3-dimensional structure, e.g., solid tumor, bone marrow, or lymph node. In other examples, the sample can be a more fluid microenvironment, such as peripheral blood.

Additionally, without wishing to be bound by theory, it is thought that some of the CTLs, e.g., cancer antigen-specific CTLs, are immunosuppressed by their microenvironment, e.g., the bone marrow or solid tumor. It is also believed that by bringing into direct contact a CTL (e.g., cancer antigen-specific CTLs) with a cancer cell *ex vivo,* a pICF can promote the activation and/or proliferation of the CTL (e.g., cancer antigen-specific CTL). The activation of the CTL (e.g., cancer antigen-specific CTL) may release the CTL from its immunosuppressed state and induce its strong proliferation. This can result in a large number of CTLs in the mixture that specifically recognize a cancer antigen and kill with high efficacy the cancer cells having that specific antigen. In bringing together the cancer cell with the CTL, the pICF may also facilitate the trogocytosis of the CTLs. Thus, it is believed that the trogocytotic T cells in the mixture tend to be those CTLs that have high efficacy of killing specific cancer cells. The population of CTLs having a high efficacy of killing specific cancer cells is also referred to herein as trogocytotic T cells. Advantageously, the use of a pICF *ex vivo* can lead to the generation of such high killing efficacy CTLs even from a sample containing very few cancer antigen-specific CTLs.

Thus, the pICF provides a more efficient method of generating immune effector cells (e.g., T cells) having enhanced target cell killing activity (and method for generating greater numbers of such cells) than previously available techniques, e.g., previously available ACTs. pICFs and trogocytosis and methods to measure their activity and recognize high efficacy cancer-killing T cells are described in greater detail below.

In addition to methods of generating such cells, also provided herein are compositions, e.g., pharmaceutical compositions, comprising immune effector cells (e.g., T cells, e.g., CTLs) that have enhanced cytotoxic activity toward cancer cells (e.g., cancer-killing T cells, e.g., trogocytotic T cells).

Furthermore, without wishing to be bound by theory, it is believed that therapies comprising the immune effector cells (e.g., cancer-killing T cells) described herein are surprisingly effective in killing a variety of cancers, ranging from solid cancers to hematological cancers. This is unlike many previously available ACTs, such as isolated/expanded tumor-infiltrating lymphocytes, which tend to be effective primarily only in highly immunogenic cancers, e.g., melanomas. Thus, particularly surprising is the ability of the immune effector cells (e.g., cancer-killing T cells) described herein to kill and treat cancers in which there typically is a low/minimal immune response against the cancer cells (e.g., unlike melanomas, which are thought to have a higher mutation rate than other cancer types and may thus be more immunogenic).

In embodiments, the immune effector cells, e.g., cancer-killing T cells, e.g., trogocytotic T cells, described herein, the method of producing same, and the methods of use (e.g., as treatment) can have one or more of the following advantages. In some embodiments, the cancer-killing T cells described herein can target (and eliminate/reduce) multiple types of cancer cells. In embodiments, the cancer-killing T cells described herein can be produced without genetic modification of a patient's cells. For example, the cancer-killing T cells described herein can be produced without having to identify specific antigens against which to direct the T cells. In embodiments, cancer-killing T cells described herein can be produced without pre-labeling of cancer cells, e.g., pre-labeling cancer cell membranes with a detectable marker or pre-labeling cancer cells with a specific antigen. In embodiments, the cancer-killing T cells described herein can be produced without pre-activating T cells with an antigen before exposure/incubation with cancer cells. In embodiments, the cancer-killing T cells described herein can be produced by incubating of pICF with a blood sample (e.g., bone marrow, whole blood, or peripheral blood) from a subject without having to separate any cells from the blood sample. For example, the blood sample may contain both the target cells (e.g., cancer cells) and the immune effector cells (e.g., T cells, e.g., CTLs) starting material that is to be targeted to the target cells, such that separate preparations of the cancer cells and the starting immune effector cells are not required.

Another advantage of the methods and compositions herein includes a safety advantage of the activated tumor-antigen specific T cells, *e.g.*, trogocytotic T cells. Without wishing to be bound by theory, it is believed that the activated tumor-antigen specific T cells described herein, e.g., produced using a method described herein, preferentially recognize cancer cells expressing a specific cancer antigen and have reduced reactivity to other cells that do not express the specific cancer antigen, e.g., normal cells. This can confer a safety advantage to these activated tumor-antigen specific T cells, as they would preferentially kill the cancer cells. Without wishing to be bound by theory, it is thought that this specificity is due to the preferred and/or selective activation and proliferation in an *ex vivo* assay using pICFs of activated tumor-antigen specific T cells from CTLs that are already specific for cancer antigen(s).

Also, disclosed herein are methods of selecting the appropriate immunotherapy for a subject, e.g., a patient. For example, disclosed herein are methods of screening for pICFs, e.g., pICFs having optimal activity in generating immune effector cells (e.g., T cells, e.g., CTLs) that have enhanced cytotoxic activity toward cancer cells (e.g., cancer-killing T cells, e.g., trogocytotic T cells). In embodiments, the candidate pICFs are new compounds/molecules not previously described, and these methods are used for drug discovery.

Additionally, disclosed herein are methods of identifying subjects that are more likely to be responsive to treatment using activated tumor-antigen specific T cells, *e.g.*, trogocytotic T cells. Described herein are also methods of selecting populations (e.g., a single clone or multiple clones) of cancer-killing T cells that have the highest cancer-killing activity.

Disclosed herein are also methods of identifying patients that are more likely to respond to cancer therapies, e.g., previously described cancer therapies, e.g., either as monotherapies or in combination with a cancer-killing T cell therapy described herein. The method harnesses the ability to produce highly cytotoxic cancer-killing T cells by using pICFs (as described herein) and applies these cells to an *ex vivo* assay. For example, the method involves generating an *ex vivo* reaction mixture containing activated tumor-antigen specific T cells, e.g., cancer-killing T cells, e.g., trogocytotic T cells (that have enhanced target cell killing activity), where the reaction mixture is generated from components from the patient. The method involve calculating an Effective E:T ratio, in which a high Effective E:T ratio is indicative of potent T cell killing activity and a low Effective E:T ratio is indicative of poor T cell killing activity. The method can involve adding candidate cancer therapies to the reaction mixture to detect a response, e.g., cancer killing activity. Without wishing to be bound by theory, it is believed that the presence of activated tumor-antigen specific T cells, e.g., cancer-killing T cells, e.g., trogocytotic T cells, in the reaction mixture increases the sensitivity of the assay such that cancer therapies that would not have been seen to have cancer-killing activity in previously described *ex vivo* assays would display cancer-killing activity in this more sensitive assay. This personalized medicine assay is also a way to test a large panel of cancer therapies for their potential efficacy in a particular patient to identify targeted therapies and avoid administration of therapies that are less likely to be effective in a particular patient. This approach can potentially identify many more cancer therapies that may be effective in and available to a certain patient than could be identified by previously described *ex vivo* assays.

**Definitions**

As used herein, the articles "a" and "an" refer to one or more than one, *e.g.*, to at least one, of the grammatical object of the article. The use of the words "a" or "an" when used in conjunction with the term "comprising" herein may mean "one," but it is also consistent with the meaning of "one or more," "at least one," and "one or more than one."

As used herein, "about" and "approximately" generally mean an acceptable degree of error for the quantity measured given the nature or precision of the measurements. Exemplary degrees of error are within 20 percent (%), typically, within 10%, and more typically, within 5% of a given range of values.

The term "autologous" refers to any material derived from the same individual to whom it is later to be re-introduced into the individual.

The term "allogeneic" refers to any material derived from a different animal of the same species as the individual to whom the material is introduced.

The term "composition" for the purpose of present specification, the term composition includes "cancer-killing T cells," which term includes activated tumor antigen-specific T cells, including, but not limited to, effector memory T cells, cytotoxic T lymphocytes (CTLs), helper T cells, tumor infiltrating lymphocytes (TILs) and trogocytotic T cells, and pharmaceutical composition thereof.

An "effective amount" of the compound of interest is employed in treatment. The dosage of compounds used in accordance with the invention varies depending on the compound and the condition being treated for example the age, weight, and clinical condition of the recipient patient. Other factors include: the route of administration, the patient, the patient's medical history, the severity of the disease process, and the potency of the particular compound. The dose should be sufficient to ameliorate symptoms or signs of the disease treated without producing unacceptable toxicity to the patient. In general, an effective amount of the compound is that which provides either subjective relief of symptoms or an objectively identifiable improvement as noted by the clinician or other qualified observer.

A "proximity ICF," or "pICF," as used herein, refers to an agent that enhances trogocytosis of an immune effector cell, e.g., a T cell (e.g., CTL), by bringing an immune effector cell, e.g., a T cell, into proximity with a target cell, e.g., a cancer cell. In an embodiment, the proximity ICF binds (e.g., directly binds) to each of the immune effector cell and the target cell. In some embodiments, the proximity ICF is an antibody molecule, e.g., a bispecific antibody molecule that has a first binding specificity for the immune effector cell (e.g., T cell, e.g., CTL) and a second binding specificity for the target cell. Without wishing to be bound by theory, a pICF can aid the sensitization and/or activation of a cytotoxic T cell (CTL), which in turn, is capable of recognizing and/or eliminating a tumor cell. In some embodiments, the pICF increases a population of trogocytotic immune effector cells (e.g., T cells) by at least 0.5%, 1%, 5%, 10%, 25%, 30% or more, e.g., relative to the population of trogocytotic immune effector cells (e.g., T cells, e.g., CTLs) generated from a mixture of immune effector cells and cancer cells in the absence of the pICF.

"Trogocytosis" as used herein refers to a process in which a portion of the cell membrane of a target cell (e.g., antigen presenting cell, e.g., cancer cell) is transferred to an immune effector cell (e.g., T cell, e.g., CTL), thereby forming a "trogocytotic" immune effector cell comprising a portion of the cell membrane from the target cell. In some embodiments, the portion of the cell membrane of the target cell comprises one or more target cell antigens. Thus, trogocytotic immune effector cells can comprise one or more target cell antigens on their cell surface. In other embodiments, the portion of the cell membrane of the target cell compromises membrane fluorescent dyes. Thus, trogocytotic immune effector cells aberrantly express cancer cell surface markers or membrane dyes previously used to stain cancer cells. Without wishing to be bound by theory, it is believed that immune effector cells (e.g., T cells, e.g., CTLs) that have undergone trogocytosis, e.g., captured one or more target cell antigens, are more effective at forming an immune response against (e.g., killing) the target cell, compared to immune effector cell that have not undergone trogocytosis.

"Immune effector cell," as that term is used herein, refers to a cell that is involved in an immune response, e.g., in the promotion of an immune effector response. Examples of immune effector cells include, but are not limited to, T cells, e.g., CD4+ and CD8+ T cells, alpha/beta T cells and gamma/delta T cells, B cells, natural killer (NK) cells, natural killer T (NKT) cells, and mast cells.

"Naïve T cells," as used herein, refer to T cells that comprise antigen-inexperienced cells, e.g., that are precursors of memory cells. In some embodiments, naïve T cells are younger T cells, i.e., that comprise a less differentiated phenotype. In some embodiments, naïve T cells are characterized by expression of CD62L, CD27, CCR7, CD45RA, CD28, and CD127, and the absence of expression of CD57, CD95, CD122, KLRG-1, or CD45RO. In embodiments, naïve T cells are characterized by long telomere length. For example, phenotypic markers associated with naïve T cells are described, e.g., in Maus M (2014), incorporated by reference herein.

"Cytotoxic T lymphocytes" (CTLs) as used herein refer to T cells that have the ability to kill a target cell. In embodiments, CTLs express CD8 on their cell surface. Without wishing to be bound by theory, it is believed that CD8+ T cells become CTLs once they are activated by recognition of an antigen on a target cell. For example, CTL activation occurs when two steps occur: 1) an interaction between an antigen-bound MHC molecule on the target cell and a T cell receptor on the CTL is made; and 2) a costimulatory signal is made by engagement of costimulatory molecules on the T cell and the target cell. CTLs then recognize specific antigens on target cells and induce the destruction of these target cells, e.g., by cell lysis. In embodiments, CTLs target and kill cancer cells and cells that are infected, e.g., with a virus, or that are damaged in other ways. In embodiments, CD4+ T cells can also kill target cells, and thus, "CTL" as used herein can also refer to CD4+ T cells.

"Tumor infiltrating lymphocytes" (TILs) are used herein refer to lymphocytes that have migrated into a tumor. In embodiments, TILs can be cells at different stages of maturation or differentiation, e.g., TILs can include CTLs, Tregs, and/or effector memory T cells, among other types of lymphocytes. In embodiments, the TILs include CTLs that are cancer antigen-specific, i.e., they recognize specific cancer antigens. In embodiments, TILs have tumor killing activity. In embodiments, TILs may include a different composition or different populations of cells compared to lymphocytes isolated from a sample other than a tumor.

"Effector memory T cell" as used herein refers to T cells that respond at a fast timescale to the presence of antigen, e.g., by rapidly producing effector cytokines. For example, upon contact with an antigen, the effector memory T cell secretes a large amount of inflammatory cytokines. In embodiments, an effector memory T cell has the following cell surface phenotype: $CD62L^{low}$, CD44, TCR, CD3, IL-7R (CD127), IL-15R, and $CCR7^{low}$.

"Effective ratio" or "Effective E:T ratio" as used herein refers to the ratio between the cancer-killing T cells and the target cancer cells after exposure to a pICF and/or an immunomodulatory agent. Effective E:T ratio is calculated using the number of cancer-killing T cells (E) and the number of target cancer cells (T) after exposure to a pICF and/or an immunomodulatory agent. In other embodiments, Effective E:T ratio can be calculated for different concentrations of pICF, e.g., at maximum concentration of pICF, at a concentration of pICF that generates a maximum peak in the number of activated or cytotoxic, cancer-killing T cells, or at the EC50 concentration of the respective dose response curves. In embodiments, the Effective E:T ratio can also be expressed as the Effective T:E ratio. As used herein, "Basal E:T ratio" is defined as the ratio between the total number of effector T cells, without specifying their subtype, versus total number of target cells. Thus Basal E:T ratio differs from the "Effective E:T ratio", as Basal E:T ratio refers to the ratio between the total number of T cells and the target cancer cells in the absence of, or before exposure to, a pICF and/or an immunomodulatory agent.

"Regulatory T cells" (Tregs) as used herein refers to T cells generated in the thymus that mediate immunosuppression and tolerogenic responses, e.g., through contact-independent and contact-dependent mechanisms. Some Tregs are inducible Tregs, which are generated from naïve T cells in the periphery. In embodiments, Tregs maintain tolerance to self-antigens and help to reduce autoimmunity. In embodiments, Tregs suppress and/or downregulate proliferation and induction of effector T cells. In embodiments, Tregs express one or more of the following markers on the cell surface: $\alpha\beta$ T cell receptor (TCR), CD3, CD4, CD25, CTLA4, and/or glucocorticoid-induced TNF receptor (GITR). In embodiments, Tregs secrete one or more of the following molecules: IL-10, TGF$\beta$, and/or IL-35.

A "clone" as used herein refers to a population of cells that are derived from the same ancestor cell. In embodiments, the cells within a clone of cells share the same phenotype(s) and genotype(s).

"Antibody molecule" as used herein refers to a protein, e.g., an immunoglobulin chain or fragment thereof, comprising at least one immunoglobulin variable domain sequence. An antibody molecule encompasses antibodies (e.g., full-length antibodies) and antibody fragments. For example, a full-length antibody is an immunoglobulin (Ig) molecule (e.g., an IgG antibody) that is naturally occurring or formed by normal immunoglobulin gene fragment recombinatorial processes). In embodiments, an antibody molecule refers to an immunologically active, antigen-binding portion of an immunoglobulin molecule, such as an antibody fragment. An antibody fragment, e.g., functional fragment, is a portion of an antibody, e.g., Fab, Fab', F(ab')$_2$, F(ab)$_2$, variable fragment (Fv), domain antibody (dAb), or single chain variable fragment (scFv). A functional antibody fragment binds to the same antigen as that recognized by the intact (e.g., full-length) antibody. The terms "antibody fragment" or "functional fragment" also include isolated fragments consisting of the variable regions, such as the "Fv" fragments consisting of the variable regions of the heavy and light chains or recombinant single chain polypeptide molecules in which light and heavy variable regions are connected by a peptide linker ("scFv proteins"). In some embodiments, an antibody fragment does not include portions of antibodies without antigen binding activity, such as Fc fragments or single amino acid residues. Exemplary antibody molecules include full length antibodies and antibody fragments, e.g., dAb (domain antibody), single chain, Fab, Fab', and F(ab')$_2$ fragments, and single chain variable fragments (scFvs).

In embodiments, an antibody molecule is monospecific, e.g., it comprises binding specificity for a single epitope. In some embodiments, an antibody molecule is multispecific, e.g., it comprises a plurality of immunoglobulin variable domain sequences, where a first immunoglobulin variable domain sequence has binding specificity for a first epitope and a second immunoglobulin variable domain sequence has binding specificity for a second epitope. In some embodiments, an antibody molecule is a bispecific antibody molecule.

"Bispecific antibody molecule" as used herein refers to an antibody molecule that has specificity for more than one (e.g., two, three, four, or more) epitope and/or antigen. A bispecific antibody molecule can encompass a variety of formats and is described in greater detail in the *Bispecific antibody molecules* section herein.

"Antigen" (Ag) as used herein refers to a molecule that can provoke an immune response, e.g., involving activation of certain immune cells and/or antibody generation. Any macromolecule, including almost all proteins or peptides, can be an antigen. Antigens can also be derived from genomic recombinant or DNA. For example, any DNA comprising a nucleotide sequence or a partial nucleotide sequence that encodes a protein capable of eliciting an immune response encodes an "antigen". In embodiments, an antigen does not need to be encoded solely by a full-length nucleotide sequence of a gene, nor does an antigen need to be encoded by a gene at all. In embodiments, an antigen can be synthesized or can be derived from a biological sample, e.g., a tissue sample, a tumor sample, a cell, or a fluid with other biological components.

The "antigen-binding site," or "binding portion" of an antibody molecule refers to the part of an antibody molecule, e.g., an immunoglobulin (Ig) molecule, that participates in antigen binding. In embodiments, the antigen binding site is formed by amino acid residues of the variable (V) regions of the heavy (H) and light (L) chains. Three highly divergent stretches within the variable regions of the heavy and light chains, referred to as hypervariable regions, are disposed between more conserved flanking stretches called "framework regions," (FRs). FRs are amino acid sequences that are naturally found between, and adjacent to, hypervariable regions in immunoglobulins. In embodiments, in an antibody molecule, the three hypervariable regions of a light chain and the three hypervariable regions of a heavy chain are disposed relative to each other in three-dimensional space to form an antigen-binding surface, which is complementary to the three-dimensional surface of a bound antigen. The three hypervariable regions of each of the heavy and light chains are referred to as "complementarity-determining regions", or "CDRs". The framework region and CDRs have been defined and described, e.g., in Kabat EA (1991) and Chothia C (1987). Each variable chain (e.g., variable heavy chain and variable light chain) is typically made up of three CDRs and four FRs, arranged from amino-terminus to carboxy-terminus in the amino acid order: FR1, CDR1, FR2, CDR2, FR3, CDR3, and FR4.

"Minimal residual disease (MRD)" as used herein refers to a small population of cells, e.g., diseased cells, e.g., cancerous cells, remaining in a patient during or after treatment, e.g., when the patient is in remission (i.e., with no signs or symptoms of disease). In embodiments, MRD can be a source of cells that causes relapse of the disease, e.g., cancer, in a patient. MRD can be detected using flow cytometry, protein, DNA, or RNA-based assays capable of measuring small numbers of diseased cells in patient samples, e.g., tissue samples.

"Cancer" as used herein can encompass all types of oncogenic processes and/or cancerous growths. In embodiments, cancer includes primary tumors as well as metastatic tissues or malignantly transformed cells, tissues, or organs. In embodiments, cancer encompasses all histopathologies and stages, e.g., stages of invasiveness/severity, of a cancer. In embodiments, cancer includes relapsed and/or resistant cancer. The terms "cancer" and "tumor" can be used interchangeably.

"Sample" or "tissue sample" refers to a biological sample obtained from a tissue or bodily fluid of a subject or patient. The source of the tissue sample can be solid tissue as from a fresh, frozen and/or preserved organ, tissue sample, biopsy, or aspirate; blood or any blood constituents (*e.g.*, serum, plasma); bone marrow or any bone marrow constituents; bodily fluids such as urine, cerebral spinal fluid, whole blood, plasma and serum. The sample can include a non-cellular fraction (*e.g.*, urine, plasma, serum, or other non-cellular body fluid). In other embodiments, the body fluid from which the sample is obtained from an individual comprises blood (*e.g.*, whole blood). In an embodiment, the sample is a whole blood sample, a whole bone marrow sample, a whole peripheral blood sample, or a whole tumor sample obtained from the subject. In embodiments, a "whole" sample, e.g., when referring to a whole blood sample, whole bone marrow sample, or a whole peripheral blood sample, is a sample where substantially no components (e.g., cells) have been removed or isolated from the sample. In one embodiment, the sample, e.g., blood sample, is diluted (e.g., with a physiologically compatible buffer or media) prior to use in the remaining steps of the method. In other embodiments, a "whole" sample, e.g., a whole tissue sample or whole tumor sample, substantially maintains the microenvironment from the tissue of origin, e.g., substantially maintains the structure of the tumor or immune microenvironment. In another embodiment, the sample, e.g., tumor sample, is processed into smaller pieces (e.g., ground, chopped, blended, pulverized, etc.) and diluted (e.g., with a physiologically compatible buffer or media).

"Cell Surface Label" as used herein refers to an agent that interacts, e.g., specifically and/or non-specifically to, a cell surface component, e.g., a cell surface protein, a glycan, a cell membrane. In embodiments, the agent comprises a detectable signal that functions to label the cell surface or the cell itself. In embodiments, the detectable signal is a chemical molecule that emits fluorescence at a known wavelength, e.g., a fluorochrome. In one embodiment, a cell surface label is an antibody that selectively recognizes one or more cell surface targets, wherein the antibody is attached, e.g., chemically attached, to a fluorophore molecule, e.g., also referred to herein as a "fluorescently labeled antibody". In one embodiment, the cell surface label is another macromolecule that can recognize one or more cell surface targets, such as an aptamer. In another embodiment, the cell surface label is a cell tracker dye. In embodiments, a cell tracker dye is a molecule containing a fluorescent molecule, e.g., a fluorochrome, that can distribute or diffuse throughout the cell surface membrane in a non-specific manner. In one embodiment, a cell tracker dye can be amphiphilic, e.g., distributing to the membrane-water interface, lipophilic, or hydrophobic, e.g., covalently attached to lipids that reside in the membrane bilayer.

**Production of Cancer-Killing T Cells**

Provided herein are methods for producing (e.g., making/providing) immune effector cells (e.g., T cells, e.g., CTLs) that have enhanced cancer-killing activity (e.g., cancer-killing T cells).

In embodiments, the method involves providing a T cell and a cancer cell from a subject (e.g., the same subject for both the T cell and the cancer cell or a different subject for the T cell versus the cancer cell).

In embodiments, the T cell and cancer cell are provided in the form of a sample from a subject. The sample can be a blood sample, e.g., a whole blood, peripheral blood, or bone marrow sample. In other embodiments, the sample is from a solid tumor (e.g., sample resected from a primary tumor or a metastasis), a lymph node, or a spleen.

In embodiments, substantially no components (e.g., cells) have been removed or isolated from the sample. For example, the sample, e.g., blood sample, is diluted (e.g., with a physiologically compatible buffer or media) prior to use in the remaining steps of the method. In other examples, the sample, e.g., tumor sample, is processed into smaller pieces (e.g., ground, chopped, blended, pulverized, etc.) and diluted (e.g., with a physiologically compatible buffer or media) prior to use in the remaining steps of the method.

In other embodiments, the T cell and the cancer cell are provided in the different samples from a subject. For example, the T cell is provided in the form of a blood sample, e.g., a whole blood, peripheral blood, or bone marrow sample. In other examples, the T cell is provided in the form of a tumor sample (e.g., sample resected from a primary tumor or a metastasis), e.g., where the T cell comprises a tumor-infiltrating T cell. For example, the cancer cell is provided in the form of a blood sample, e.g., a whole blood, peripheral blood, or bone marrow sample, e.g., where the cancer cell comprises a circulating tumor cell (CTC). In other examples, the cancer cell is provided in the form of a sample from a solid tumor (e.g., sample resected from a primary tumor or a metastasis), a lymph node, or a spleen.

In aspects of the disclosure, the method further involves forming an *ex vivo* reaction mixture with the T cell and the cancer cell, along with a proximity immuno-coaching factor (pICF). Any pICF described herein can be used in the method. pICFs are described in greater detail in the "Proximal immuno-coaching factor (pICF)" section herein. In embodiments, the *ex vivo* reaction mixture is formed under conditions, such as for a period of time, sufficient to allow the T cell to acquire a cell surface marker from the cancer cell (e.g., to allow the T cell to undergo trogocytosis). The method thereby produces a cancer-killing T cell.

Without wishing to be bound by theory, it is believed that in both hematological and solid tumors, there can be different tissues affected with tumor cells in a subject. For example, in solid tumors, metastases can contain tumor cells that have different characteristics, e.g., expression patterns (e.g., different antigen expression patterns), from tumor cells within the primary tumor site. As such, the method can include using a bispecific or multispecific antibody to activate cancer-specific CTLs within each tissue that is affected by cancer cells in a subject's body.

For example, in aspects of the disclosure, the methods described herein include the following steps:

(a) providing a T cell from a subject having a cancer (e.g., a hematological cancer or a solid cancer);
(b) providing a cancer cell, e.g., from the subject;
(c) forming an *ex vivo* reaction mixture comprising the T cell, the cancer cell, and a proximity immuno-coaching factor (pICF), e.g., under conditions (e.g., for a period of time) sufficient to allow the T cell to acquire a cell surface marker from the cancer cell, thereby producing the cancer-killing T cell; and
(d) one, two, three or all of:

i) expanding the cancer-killing T cell from (c);
ii) selecting the cancer-killing T cell from (c);
iii) enriching for the cancer-killing T cell from (c); or
iv) purifying the cancer-killing T cell from (c),

wherein the cancer cell is derived from a primary solid tumor from the subject, is derived from a metastasis from the subject, and/or is a circulating tumor cell (CTC), e.g., from a blood (e.g., whole blood, bone marrow, or peripheral blood) or lymph sample, from the subject.

In another aspect of the disclosure, the method described herein includes the following steps:

(a) providing a sample from a subject having a cancer (e.g., a hematological cancer or a solid cancer), wherein the sample comprises a T cell and a cancer cell;
(b) contacting the sample with a proximity immune-coaching factor (pICF), e.g., under conditions (e.g., for a period of time) sufficient to allow the T cell to acquire a cell surface marker from the cancer cell;
(c) enriching for cancer-killing T cells that have acquired a cell surface marker from the cancer cell,
wherein the sample is derived from a primary solid tumor from the subject, is derived from a metastasis from the subject, and/or is a blood (e.g., whole blood, bone marrow, or peripheral blood) or lymph sample from the subject.

In yet another aspect of the disclosure, the method described herein includes the following steps:

(a) providing a sample from a subject having a cancer (e.g., a hematological cancer or a solid cancer), wherein the sample comprises a cancer cell;
(b) providing a sample, e.g., a blood sample (e.g., peripheral blood sample) from the subject, wherein the sample comprises a T cell;
(c) contacting the sample from step (a) with the sample from step (b) and a proximity immuno-coaching factor (pICF), e.g., under conditions (e.g., for a period of time) sufficient to allow the T cell to acquire a cell surface marker from the cancer cell;
(d) enriching for cancer-killing T cells that have acquired a cell surface marker from the cancer cell,
wherein the sample from step (a) is derived from a primary solid tumor from the subject, is derived from a metastasis from the subject, and/or is a blood (e.g., whole blood, bone marrow, or peripheral blood) or lymph sample from the subject.

In embodiments, a method of producing/generating cancer-killing T cells (e.g., trogocytotic T cells) described herein is repeated using different samples from a given subject, where each repetition includes using a different sample of cancer cell, e.g., primary solid tumor, metastases, blood (e.g., whole blood, bone marrow, or peripheral blood), or lymph.

In embodiments, the method further comprises pooling the trogocytotic T cells generated using each of these different cancer cell samples.

Without wishing to be bound by theory, it is thought that such a method will generate trogocytotic T cells effective against the different kinds of cancer cells that may be present in different tissues of a given subject. Without wishing to be bound by theory, it is believed that such a method can advantageously kill cancer cells throughout a subject's body (e.g., both at a primary tumor and at metastases and perhaps also circulating in the blood) instead of only killing cancer cells at one site within the body.

Additionally, without wishing to be bound by theory, it is believed that CTCs (tumor cells found in peripheral blood of cancer patients, typically solid tumor cancer patients) may be responsible for metastasis and hence are a good target for killing. As such, the methods described herein include incubation of a pICF *ex vivo* with a peripheral blood sample (containing CTCs and T cells), thereby bringing into proximity CTCs with their cognate cancer antigen-specific T cells in order to generate trogocytotic T cells. In other embodiments, e.g., in peripheral blood samples are not sufficiently enriched with cancer antigen-specific T cells, a sample from a 3-dimensional microenvironment (e.g., bone marrow, tumor, metastasis) likely enriched in cancer antigen-specific T cells is used instead of peripheral blood samples. In such cases, for example, a method can include incubating ex *vivo* a pICF and an isolated CTC with a bone marrow, tumor, or metastasis sample (containing cancer antigen-specific T cells) from a subject. This *ex vivo* mixture enables the pICF to bring into spatial proximity the CTCs with the cancer antigen-specific T cells that match those antigens on the CTCs, thereby activating the appropriate T cells to generate trogocytotic T cells. In embodiments, such a method can be repeated using each tissue of the subject affected by cancer, e.g., to maximize the matching of the CTCs with the appropriate cancer antigen-specific T cells.

**Further Processing of cancer-killing T cells**

In accordance with a method described herein, in embodiments, the cancer-killing T cells can be further selected, enriched, purified, and/or expanded.

In embodiments, the cancer-killing T cells described herein, e.g., produced by a method described herein, are selected, e.g., from a reaction mixture. For example, in aspects of the disclosure, the reaction mixture contains a T cell, a cancer cell, and a proximity immuno-coaching factor (pICF). In aspects of the disclosure, the cancer-killing T cells described herein are purified away from the T cell(s) and/or the pICF. In embodiments, the cancer-killing T cells described herein are enriched from the mixture of cells (e.g., cancer cells and/or various types of T cells) in the reaction mixture.

In embodiments, the selection step, purification, and/or the enrichment step comprises using flow cytometry (e.g., fluorescence activated cell sorting (FACS)) or other separation methods such as beads (e.g., magnetic beads). For example, beads can be coated with an antibody or fragment thereof that binds to one or more cancer antigens and/or one or more markers of activated T cells (e.g., CTLs). In this way, cells that bind to such beads would be activated T cells (e.g., CTLs) that express one or more cancer antigens. These cells are likely trogocytotic T cells with enhanced cancer-killing activity. Likewise, in FACS, cells expressing both markers of activated T cells (e.g., CTLs) and markers of cancer cells (e.g., likely trogocytotic T cells) can be separated from other cell types. Negative or positive selection methods can be used for the selection step, purification, and/or the enrichment step.

In embodiments, the cancer-killing T cells described herein, e.g., produced by a method described herein, are expanded, e.g., to generate an amount of cancer-killing T cells for administration into a subject. In embodiments, a preparation of cancer-killing T cells described herein is expanded. In embodiments, the expansion is performed prior to selection, enrichment, or purification of certain T cell populations. In other embodiments, the expansion is performed after selection, enrichment, or purification of certain T cell populations.

Exemplary methods of expanding cells, e.g., cancer-killing T cells, includes those described in US 8,034,334, US 2012/0244133 and Montes M (2005). In embodiments, expansion of the cancer-killing T cells comprises increasing the number of cancer-killing T cells, e.g., in a preparation, e.g., by at least about 2-fold (e.g., at least about 3-, 4-, 5-, 6-, 7-, 8-, 9-, 10-, 15-, 20-, 50-, 100-, 1000-, $10^4$-, $10^5$-, $10^6$-fold, or more). In embodiments, the expansion is performed over the course of at least 2 days, e.g., at least 2, 3, 4, 5, 6, 7, 8, 9, 10, 11, 12, 13, 14, or more days. In some examples, the cells are expanded by culturing the cells in the presence of a cytokine such as IL-2 and/or IL-15, optionally with the addition of an agent that stimulates the T-cell receptor, e.g., an anti-CD3 antibody or fragment thereof.

In embodiments, the selection step, purification step, and/or expansion step comprises the sequential addition of a low, e.g., an insufficient, number of cancer cells. The methods described herein comprising incubating cancer cells, T cells, and a pICF to generate a cytotoxic T cell can generate different clones of cytotoxic T cells. In embodiments, selection of the cytotoxic T cell clones that are the most efficient or most potent at killing cancer cells can be achieved by sequentially adding low, e.g., insufficient, amounts of cancer cells. In an embodiment, a low, or insufficient, number or amount of cancer cells that can be added to a reaction comprising cancer-killing T cells is 50% or less, e.g., 30%, 10%, 1%, 0.1%, or 0.01% or less of the number of activated T cells. In one embodiment, the low, or insufficient, number of cancer cells can be added to the mixture (e.g., comprising cancer cells, T cells, and/or a bispecific or multispecific antibody) at least one or more times (e.g., 2, 3, 4, 5, 6, 7, 8, 9 or 10, times). In one embodiment, the low, or insufficient, number of cancer cells is added every 6-48 hours (e.g., 6 hours, 12 hours, 24 hours, 36 hours, or 48 hours). In an embodiment, the low, or insufficient, number of cancer cells that are added are cancer cells from the patient. In an embodiment, the low, or insufficient, number of cancer cells that are added are not cancer cells from the patient. In an embodiment, the low, or insufficient, number of cancer cells that are added are cancer cells from a cancer cell line.

Without wishing to be bound by theory, it is believed that once the cancer cells in the mixture with the T cells and pICF are eliminated, e.g., by the newly generated cancer-killing T cells, the cancer-killing T cells can become exhausted and decrease in number. However, without wishing to be bound by theory, when a low, e.g., insufficient, number of cancer cells is added to the generated cancer-killing T cells, a subset of the cancer-killing T cells will recognize and kill the newly added cancer cells. Recognition of the cancer cell can occur, for example, through selective recognition and binding of its TCR to the cancer antigen expressed in the cancer cell surface; and the T cell clone with the highest affinity, or fastest $k_{on}$ kinetic constant, to the cancer antigen can bind more strongly and/or faster to the newly added cancer cells, thereby resulting in elimination of the cancer cells and activation of proliferation of the cancer-killing T cell clone. Due to the direct competition between the cancer-killing T cells, the subset of the cancer-killing T cells that are more efficacious will be activated and will proliferate, while the remainder of the cancer-killing T cells that do not recognize the newly added cancer cells will continue the process of exhaustion and self-elimination, thereby leaving on the more efficacious cancer-killing T cells. Thus, without wishing to be bound by theory, the subset of cancer-killing T cells that kills the newly added cancer cells are believed to be the best killers, e.g., the most active or potent cancer-killer T cells. In embodiments, without wishing to be bound by theory, repeating this process of adding a low, e.g., insufficient, number of cancer cells is believed to impose an evolutionary selective pressure towards the most active and more efficacious or potent cancer-killing T cells to preferentially or selectively activate and proliferate. Accordingly, sequential addition of a low, e.g., insufficient, number of cancer cells is useful for the selection and enrichment of the most active and efficacious cancer-killing T cell clones. An exemplary process comprising the sequential addition of low numbers of cancer cells is depicted in **Figure 1.**

In embodiments, the selected, purified, enriched, and/or expanded cells can form a preparation of cancer-killing T cells.

## Proximal Immuno-Coaching Factor (pICF)

pICFs can include, e.g., bispecific antibody molecules, or CDR-grafted scaffolds.

### Bispecific antibody molecules

In embodiments, bispecific antibody molecules can comprise more than one antigen-binding site, where different sites are specific for different antigens. In embodiments, bispecific antibody molecules can bind more than one (e.g., two or more) epitopes on the same antigen. In embodiments, bispecific antibody molecules comprise an antigen-binding site specific for a target cell (e.g., cancer cell) and a different antigen-binding site specific for an immune effector cell (e.g., a T cell, e.g., CTL). Bispecific antibody molecules can be classified into five different structural groups: (i) bispecific immunoglobulin G (BsIgG); (ii) IgG appended with an additional antigen-binding moiety; (iii) bispecific antibody fragments; (iv) bispecific fusion proteins; and (v) bispecific antibody conjugates.

(i) BsIgG is a format that is monovalent for each antigen. Exemplary BsIgG formats include but are not limited to crossMab, DAF (two-in-one), DAF (four-in-one), DutaMab, DT-IgG, knobs-in-holes common LC, knobs-in-holes assembly, charge pair, Fab-arm exchange, SEEDbody, triomab, LUZ-Y, Fcab, κλ-body, orthogonal Fab. See Spiess (2015) at Figure 1. Exemplary BsIgGs include catumaxomab (Fresenius Biotech, Trion Pharma, Neopharm), which contains an anti-CD3 arm and an anti-EpCAM arm; and ertumaxomab (Neovii Biotech, Fresenius Biotech), which targets CD3 and HER2. In some embodiments, BsIgG comprises heavy chains that are engineered for heterodimerization. For example, heavy chains can be engineered for heterodimerization using a "knobs-into-holes" strategy, a SEED platform, a common heavy chain (e.g., in κλ-bodies), and use of heterodimeric Fc regions. See Spiess C (2015). Strategies that have been used to avoid heavy chain pairing of homodimers in BsIgG include knobs-in-holes, duobody, azymetric, charge pair, HA-TF, SEEDbody, and differential protein A affinity. *See Id.* BsIgG can be produced by separate expression of the component antibodies in different host cells and subsequent purification/assembly into a BsIgG. BsIgG can also be produced by expression of the component antibodies in a single host cell. BsIgG can be purified using affinity chromatography, e.g., using protein A and sequential pH elution.

(ii) IgG appended with an additional antigen-binding moiety is another format of bispecific antibody molecules. For example, monospecific IgG can be engineered to have bispecificity by appending an additional antigen-binding unit onto the monospecific IgG, e.g., at the N- or C- terminus of either the heavy or light chain. Exemplary additional antigen-binding units include single domain antibodies (e.g., variable heavy chain or variable light chain), engineered protein scaffolds, and paired antibody variable domains (e.g., single chain variable fragments or variable fragments). See *Id.* Examples of appended IgG formats include dual variable domain IgG (DVD-Ig), IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG,

IgG-2scFv, scFv4-Ig, zybody, and DVI-IgG (four-in-one). See Spiess C (2015), Figure 1. An example of an IgG-scFv is MM-141 (Merrimack Pharmaceuticals), which binds IGF-1R and HER3. Examples of DVD-Ig include ABT-981 (AbbVie), which binds IL-1$\alpha$ and IL-1$\beta$; and ABT-122 (AbbVie), which binds TNF and IL-17A.

(iii) Bispecific antibody fragments (BsAb) are a format of bispecific antibody molecules that lack some or all of the antibody constant domains. For example, some BsAb lack an Fc region. In embodiments, bispecific antibody fragments include heavy and light chain regions that are connected by a peptide linker that permits efficient expression of the BsAb in a single host cell. Exemplary bispecific antibody fragments include but are not limited to nanobody, nanobody-HAS, BiTE, Diabody, DART, TandAb, scDiabody, scDiabody-CH3, Diabody-CH3, triple body, miniantibody, minibody, TriBi minibody, scFv-CH3 KIH, Fab-scFv, scFv-CH-CL-scFv, F(ab')2, F(ab')2-scFv2, scFv-KIH, Fab-scFv-Fc, tetravalent HCAb, scDiabody-Fc, Diabody-Fc, tandem scFv-Fc, and intrabody. *See Id.* For example, the BiTE format comprises tandem scFvs, where the component scFvs bind to CD3 on T cells and a surface antigen on cancer cells. Exemplary BiTEs include blinatumomab (Amgen), which binds CD3 and CD19; solitomab (Amgen), which binds CD3 and EpCAM; MEDI 565 (MedImmune, Amgen), which binds CD3 and CEA; and BAY2010112 (Bayer, Amgen), which binds CD3 and PSMA. Exemplary DARTs include MGD006 (Macrogenics), which binds CD3 and CD123; and MGD007 (Macrogenics), which binds CD3 and gpA33. Exemplary TandAbs include AFM11 (Affimed Therapeutics), which binds CD3 and CD19; and AFM13 (Affimed Therapeutics), which binds CD30 and CD16A. An example of a tandem scFv is rM28 (University Hospital of Tubingen), which binds CD28 and MAPG. Exemplary nanobodies include ozoralizumab (Ablynx), which binds TNF and HSA; ALX-0761 (Merck Serono, Ablynx), which binds IL-17A/F and HSA; ALX-0061 (AbbVie, Ablynx), which binds IL-6R and HSA; ALX-0141 (Ablynx, Eddingpharm), which binds RANKL and HSA. The component fragments of BsAb can be identified/selected using phage display. In some embodiments, the pICF does not comprise blinatumomab.

(iv) Bispecific fusion proteins include antibody fragments linked to other proteins, e.g., to add additional specificity and/or functionality. An example of a bispecific fusion protein is an immTAC, which comprises an anti-CD3 scFv linked to an affinity-matured T-cell receptor that recognizes HLA-presented peptides. In embodiments, the dock-and-lock (DNL) method can be used to generate bispecific antibody molecules with higher valency. Also, fusions to albumin binding proteins or human serum albumin can be extend the serum half-life of antibody fragments. *See Id.*

In embodiments, chemical conjugation, e.g., chemical conjugation of antibodies and/or antibody fragments, can be used to create BsAb molecules. *See Id.* An exemplary bispecific antibody conjugate includes the CovX-body format, in which a low molecular weight drug is conjugated site-specifically to a single reactive lysine in each Fab arm or an antibody or fragment thereof. In embodiments, the conjugation improves the serum half-life of the low molecular weight drug. An exemplary CovX-body is CVX-241 (NCT01004822), which comprises an antibody conjugated to two short peptides inhibiting either VEGF or Ang2. *See Id.*

(v) Bispecific antibody molecules can be produced by recombinant expression, e.g., of at least one or more component, in a host system. Exemplary host systems include eukaryotic cells (e.g., mammalian cells, e.g., CHO cells, or insect cells, e.g., SF9 or S2 cells) and prokaryotic cells (e.g., *E. coli*). Bispecific antibody molecules can be produced by separate expression of the components in different host cells and subsequent purification/assembly. Alternatively, bispecific antibody molecules can be produced by expression of the components in a single host cell. Purification of bispecific antibody molecules can be performed by various methods such as affinity chromatography, e.g., using protein A and sequential pH elution. In other embodiments, affinity tags can be used for purification, e.g., histidine-containing tag, myc tag, or streptavidin tag.

*CDR-grafted scaffolds*

In embodiments, a pICF is a CDR-grafted scaffold domain. In embodiments, the scaffold domain is based on a fibronectin domain, e.g., fibronectin type III domain. The overall fold of the fibronectin type III (Fn3) domain is closely related to that of the smallest functional antibody fragment, the variable domain of the antibody heavy chain. There are three loops at the end of Fn3; the positions of BC, DE and FG loops approximately correspond to those of CDR1, 2 and 3 of the VH domain of an antibody. Fn3 does not have disulfide bonds; and therefore, Fn3 is stable under reducing conditions, unlike antibodies and their fragments (see, e.g., WO 98/56915; WO 01/64942; WO 00/34784). An Fn3 domain can be modified (e.g., using CDRs or hypervariable loops described herein) or varied, e.g., to select domains that bind to an antigen/marker/cell described herein.

In embodiments, a scaffold domain, e.g., a folded domain, is based on an antibody, e.g., a "minibody" scaffold created by deleting three beta strands from a heavy chain variable domain of a monoclonal antibody (see, e.g., Tramontano A (1994), Martin F (1994)). The "minibody" can be used to present two hypervariable loops. In embodiments, the scaffold domain is a V-like domain (see, e.g., WO 99/45110) or a domain derived from tendamistatin, which is a 74 residue, six-strand beta sheet sandwich held together by two disulfide bonds (see, e.g., McConnell SJ (1995)). For example, the loops of tendamistatin can be modified (e.g., using CDRs or hypervariable loops) or varied, e.g., to select domains that bind to a marker/antigen/cell described herein. Another exemplary scaffold domain is a beta-sandwich structure derived from the extracellular domain of CTLA-4 (see, e.g., WO 00/60070).

Other exemplary scaffold domains include but are not limited to T-cell receptors; MHC proteins; extracellular domains (e.g., fibronectin Type III repeats, EGF repeats); protease inhibitors (e.g., Kunitz domains, ecotin, BPTI, and so forth); TPR repeats; trifoil structures; zinc finger domains; DNA-binding proteins; particularly monomeric DNA binding proteins; RNA binding proteins; enzymes, e.g., proteases (particularly inactivated proteases), RNase; chaperones, e.g., thioredoxin, and heat shock proteins; and intracellular signaling domains (such as SH2 and SH3 domains). See, e.g., US 2004/0009530 and US 7,501,121.

In embodiments, a scaffold domain is evaluated and chosen, e.g., by one or more of the following criteria: (1) amino acid sequence, (2) sequences of several homologous domains, (3) 3-dimensional structure, and/or (4) stability data over a range of pH, temperature, salinity, organic solvent, oxidant concentration. In embodiments, the scaffold domain is a small, stable protein domain, e.g., a protein of less than 100 amino acids (e.g., 100, 70, 50, 40 or 30 amino acids). The domain may include one or more disulfide bonds or may chelate a metal, e.g., zinc.

## Other Compositions and Methods of Enhancing T Cell Activity

In accordance with the compositions and methods described herein, in embodiments, other immunomodulatory agents can be used in addition to a pICF to enhance T cell activity, e.g., trogocytosis. These immunomodulatory agents include, but are not limited to, immune checkpoint inhibitors, agonists of T cells, and other immunomodulatory drugs.

In aspects of the disclosure, alternatively, other agents for enhancing T cell activity, e.g., trogocytosis, (e.g., use of immunomodulatory agents, such as immune checkpoint inhibitors, agonists of T cells, and other immunomodulatory drugs) can be used instead of a pICF.

*Immune checkpoint inhibitors*

In embodiments, methods described herein comprise use of an immune checkpoint inhibitor, e.g., in a reaction mixture with a cancer cell and an immune effector cell (e.g., T cell, e.g., CTL), e.g., in addition to a pICF. In embodiments, methods described herein comprise contacting a cancer cell and an immune effector cell (e.g., T cell, e.g., CTL) with an immune checkpoint inhibitor. The methods can also be used in a therapeutic protocol *in vivo.*

In embodiments, an immune checkpoint inhibitor inhibits a checkpoint molecule. Checkpoint molecules can, in some cases, reduce the ability of a CAR-expressing cell to mount an immune effector response. Exemplary checkpoint molecules include but are not limited to CTLA4, PD1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1, and A2aR. See, e.g., Pardoll DM (2012).

In embodiments, the immune checkpoint inhibitor is a PD-1 inhibitor, e.g., an anti-PD-1 antibody such as Nivolumab, Pembrolizumab or Pidilizumab. Nivolumab (also called MDX-1106, MDX-1106-04, ONO-4538, or BMS-936558) is a fully human IgG4 monoclonal antibody that specifically inhibits PD1. See, e.g., US 8,008,449 and WO2006/121168. Pembrolizumab (also called Lambrolizumab, MK-3475, MK03475, SCH-900475 or KEYTRUDA®; Merck) is a humanized IgG4 monoclonal antibody that binds to PD-1. See, e.g., Hamid O (2013), US 8,354,509 and WO2009/114335. Pidilizumab (also called CT-011 or Cure Tech) is a humanized IgG1k monoclonal antibody that binds to PD1. See, e.g., WO2009/101611. In one embodiment, the inhibitor of PD-1 is an antibody molecule having a sequence substantially identical or similar thereto, e.g., a sequence at least 85%, 90%, 95% identical or higher to the sequence of Nivolumab, Pembrolizumab or Pidilizumab. Additional anti-PD1 antibodies, e.g., AMP 514 (Amplimmune), are described, e.g., in US 8,609,089, US 2010/028330, and/or US 2012/0114649.

In some embodiments, the PD-1 inhibitor is an immunoadhesin, e.g., an immunoadhesin comprising an extracellular/PD-1 binding portion of a PD-1 ligand (e.g., PD-L1 or PD-L2) that is fused to a constant region (*e.g.*, an Fc region of an immunoglobulin). In embodiments, the PD-1 inhibitor is AMP-224 (B7-DClg, *e.g.*, described in WO2011/066342 and WO2010/027827), a PD-L2 Fc fusion soluble receptor that blocks the interaction between B7-H1 and PD-1.

In embodiments, the immune checkpoint inhibitor is a PD-L1 inhibitor, e.g., an antibody molecule. In some embodiments, the PD-L1 inhibitor is YW243.55.S70, MPDL3280A, MEDI-4736, MSB-0010718C, or MDX-1105. In some embodiments, the anti-PD-L1 antibody is MSB0010718C (also called A09-246-2; Merck Serono), which is a monoclonal antibody that binds to PD-L1. Exemplary humanized anti-PD-L1 antibodies are described, e.g., in WO2013/079174. In one embodiment, the PD-L1 inhibitor is an anti-PD-L1 antibody, e.g., YW243.55.S70. The YW243.55.S70 antibody is described, e.g., in WO 2010/077634. In one embodiment, the PD-L1 inhibitor is MDX-1105 (also called BMS-936559), which is described, e.g., in WO2007/005874. In one embodiment, the PD-L1 inhibitor is MDPL3280A (Genentech / Roche), which is a human Fc-optimized IgG1 monoclonal antibody against PD-L1. See, e.g., US 7,943,743 and US 2012/0039906. In one embodiment, the inhibitor of PD-L1 is an antibody molecule having a sequence substantially identical or similar thereto, e.g., a sequence at least 85%, 90%, 95% identical or higher to the sequence of YW243.55.S70, MPDL3280A, MEDI-4736, MSB-0010718C, or MDX-1105.

In embodiments, the immune checkpoint inhibitor is a PD-L2 inhibitor, e.g., AMP-224 (which is a PD-L2 Fc fusion soluble receptor that blocks the interaction between PD1 and B7-H1. See, e.g., WO2010/027827 and WO2011/066342.

In one embodiment, the immune checkpoint inhibitor is a LAG-3 inhibitor, e.g., an anti-LAG-3 antibody molecule. In embodiments, the anti-LAG-3 antibody is BMS-986016 (also called BMS986016; Bristol-Myers Squibb). BMS-986016 and other humanized anti-LAG-3 antibodies are described, e.g., in US 2011/0150892, WO2010/019570, and WO2014/008218.

In embodiments, the immune checkpoint inhibitor is a TIM-3 inhibitor, e.g., anti-TIM3 antibody molecule, e.g., described in US 8,552,156, WO 2011/155607, EP2581113 and US 2014/044728.

In embodiments, the immune checkpoint inhibitor is a CTLA-4 inhibitor, e.g., anti-CTLA-4 antibody molecule. Exemplary anti-CTLA4 antibodies include Tremelimumab (IgG2 monoclonal antibody from Pfizer, formerly known as ticilimumab, CP-675,206); and Ipilimumab (also called MDX-010, CAS No. 477202-00-9). Other exemplary anti-CTLA-4 antibodies are described, e.g., in US 5,811,097.

*Agonists of a T cell (e.g., Agonistic antibody)*

In embodiments, compositions and methods described herein comprise use of an agonist of T cells (e.g., agonistic antibody), e.g., in a reaction mixture with a cancer cell and an immune effector cell (e.g., T cell, e.g., CTL), e.g., in addition to a pICF. In embodiments, methods described herein comprise contacting a cancer cell and an immune effector cell (e.g., T cell, e.g., CTL) with an agonist of T cells (e.g., agonistic antibody).

In embodiments, the agonist of T cells is an agonistic antibody or fragment thereof or an activator/agonist of a costimulatory molecule. In embodiments, the agonist of T cells comprises or is a costimulatory molecule. A costimulatory molecule is a cell surface molecule required for an efficient response of a lymphocyte, e.g., T cell, to an antigen. In embodiments, a costimulatory molecule is a molecule other than an antigen receptor or its ligands. Without wishing to be bound by theory, costimulation is believed to enhance expansion, survival, and effector function of T cells (e.g., enhance T cell persistence and/or anti-cancer activity. See, e.g., Song DJ (2012). Exemplary costimulatory molecules include but are not limited to CD28, ICOS (CD278), BTLA, LIGHT, HVEM (LIGHTR), CD160 (BY55), OX40, CD27, CD2, CD7, CD40, CD30, 4-1BB (CD137), ICAM-1, B7-1, a toll-like receptor, LFA-1 (CD11a/CD18), GITR, BAFFR, B7-H3, a signalling lymphocytic activation molecules (SLAM protein), SLAMF7, SLAM (SLAMF1, CD150, IPO-3), SLAMF4 (CD244, 2B4), an integrin, IL2R beta, ITGA4, a MHC class I molecule, a TNF receptor, CD49D, CD49f, LFA-1, CD29, CD18, TNFR2, CD84, RANKL, CD229, CD69, CD100 (SEMA4D), and SLAMF6 (NTB-A, Ly108).

In some embodiments, the agonist of T cells is an agonistic antibody or fragment thereof to CD137, GITR, or CD40.

Exemplary agonistic antibodies are described, e.g., in Scott AM (2012).

*Other immunomodulatory drugs*

In embodiments, compositions and methods described herein comprise an immunomodulatory drug, e.g., lenalidomide, e.g., in a reaction mixture with a cancer cell and an immune effector cell (e.g., T cell, e.g., CTL), e.g., in addition to a pICF. In embodiments, methods described herein comprise contacting a cancer cell and an immune effector cell (e.g., T cell, e.g., CTL) with an agonist of T cells (e.g., agonistic antibody).

In some embodiments, the ICF is an immunomodulatory drug, such as lenalidomide.

In one embodiment, the immunomodulatory agent is an inhibitor of MDSCs and/or Treg cells. Without wishing to be bound by theory, MDSCs and regulatory T (Treg) cells are important components of the immune suppressive tumor microenvironment. Experimental evidence has revealed that MDSCs can modulate the development and induction of Treg cells. For example, MDSCs can suppress T cell effector functions in various ways. Several factors can modulate the expression levels of Arginine, NADPH oxidase and NOS in MDSC subsets, with the final effect on the microenvironment including depletion of l-arginine, release of RNS and ROS (with ONOO-and $H_2O_2$ being the most prevalent molecules, respectively) or unopposed production of high NO levels. Moreover, l-cysteine can be sequestered by MDSCs. All of these molecules influence the intracellular signaling pathways that control T cell proliferation after antigen stimulation. MDSC-mediated immune suppression can also be associated with the expansion of Treg cell populations, inhibition of the T-cell proliferation and promotion of T-cell apoptosis.

In one embodiment, the immunomodulatory agent is lenalidomide.

In other embodiments, the immunomodulatory agent activates an immune response to a tumor specific antigen, e.g., it is a vaccine (e.g., a vaccine against targets such as gp100, MUC1 or MAGEA3.

In other embodiments, the immunomodulatory agent is a cytokine, e.g., a recombinant cytokine chosen from one or more of GM-CSF, IL-7, IL-12, IL-15, IL-18 or IL-21.

In other embodiments, the immunomodulatory agent is an autologous T cell, e.g., a tumor-targeted extracellular and intracellular tumor-specific antigen (e.g., a CAR T cell or a TCR T cell).

In yet other embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Exemplary IDO inhibitors include INCB24360, 1-Methyl tryptophan inhibitor, and NLG919. Exemplary ARG1/ARG2 inhibitors include Compound 9, NCX-4016, and AT38. Exemplary PDE5 inhibitors include Tadalafil. Exemplary agents that modulate tumor extracellular ATP include agonist or antagonist of P2X7, and antagonist of $P2Y_{11}$. Exemplary agents that modulate adenosine signalling include antagonists of $A_{2A}$ receptor (e.g., SCH58261 and SCH420814), and antagonists of $A_{2B}$ receptor (e.g., PSB1115). Modulators of chemokines and chemokine receptors, such as CXCR1, CXCR2, CXCR4, CCR2 and CCR5 include, but are not limited to, CXCR2-specific antibodies, Plerixafor, PF-4136309 and Maraviroc. Modulators of TLRs such as TLR4 (e.g., OM-174, a TLR4 agonist), TLR7 (e.g., Imiquimod, 852A, a TLR7/8 agonist), TLR8 (e.g., VTX-2337, a TLR8 agonist) and TLR9 (e.g., IMO-2055, a TLR9 agonist). Exemplary kinase inhibitors include, but are not limited to, inhibitors of ALK, BRAF, RON, CSF1, Pl3K-delta and PI3K-gamma.

Additional examples of immunomodulatory agents are further described in, *e.g.*, Adams JL (2015) and Serafini P (2008).

## Evaluation of Cancer-Killing T Cells

In accordance with a method described herein, in embodiments, cancer-killing T cells (e.g., preparations of cancer-killing T cells, e.g., selected, purified, enriched, and/or expanded cells) can be characterized or evaluated in a number of ways.

For example, the cancer-killing T cells can be characterized for expression of various cancer cell and/or effector T cell markers, e.g., with panels of antibodies, e.g., monoclonal antibodies. In embodiments, the cells are characterized for expression of markers such as PD-1 and TIM-3, among other immune checkpoint molecules (e.g., immune checkpoint molecules described herein). In embodiments, the presence of expression of PD-1 and/or TIM-3 on the cells can indicate that the cancer-killing T cells are more tumor immunoreactive.

In embodiments, the cancer-killing T cells can be evaluated for their reactivity to cancer cells, e.g., *in vitro* or *ex vivo*. Without wishing to be bound by theory, it is believed that reactivity to cancer cells, e.g., in vitro or *ex vivo,* is a measure of how effective the cancer-killing T cells will be at killing cancer cells *in vivo.* In embodiments, reactivity to cancer cells can be assessed by contacting the cancer-killing T cells with cancer cells, e.g., cancer-derived cell lines or primary cancer samples.

In embodiments, the primary cancer samples include cells isolated from a hematological malignancy in a subject, e.g., isolated from a blood sample (e.g., peripheral blood or bone marrow) of a subject having a hematological malignancy. For example, the cancer-killing T cells and the cancer cells are contacted by co-culturing, e.g., at a predetermined T cell:cancer cell ratio. Exemplary T cell:cancer cell ratios include about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher). In embodiments, the reactivity can be assessed a period of time of 1-36 hours after co-culture (e.g., 1-36 hours, 1-6 hours, 6-12 hours, 12-24 hours, or 24-36 hours). Reactivity can be assessed by quantifying the amount of interferon-gamma released by the cells and/or the percentage of T cells that express 4-1BB. In embodiments, a higher level of interferon-gamma and/or 4-1BB compared to a control level indicates that the cancer-killing T cells are reactive to the cancer cells. Alternatively, markers for specific tumor lineages can be evaluated, including but not limited to, CD107a, granzyme B, perforin, and other specific lineage tumours markers.

In other embodiments, reactivity is assessed by first labeling the cancer cells with a marker (e.g., radioactive marker, e.g., $^{51}Cr$, a fluorescent marker, or other molecule) prior to co-culture with the cancer-killing T cells. After co-culture of labeled cancer cells with cancer-killing T cells, the amount of the marker released into the media (e.g., an indicator of extent of cancer cell lysis) is a measure of the extent of cancer cell death. The amount of radioactive marker, e.g., $^{51}Cr$, can be quantified by using any method to detect and quantify radioactivity. The amount of a fluorescent marker can be quantified using any method to detect and quantify fluorescence. The amount of a marker, e.g., a fluorescent marker or other molecule, can also be quantified using an antibody-based assay (e.g., ELISA). In embodiments, a higher level of the release marker from the cancer cells compared to a control level indicates that the cancer-killing T cells are reactive to the cancer cells.

In embodiments, the control level can be a level of interferon-gamma and/or 4-1BB, and/or marker generated in a similar assay in the absence of a cancer-killing T cell, in the absence of cancer cells, or in the absence of labeled cancer cells.

Also, provided herein are methods for measuring the trogocytosis of the cancer-killing T cells using cell surface labeling. Measuring the trogocytosis of the cancer-killing T cells can be useful in the selection, characterization, and evaluation of the cancer-killing T cells produced by any of the methods described herein. Assays for measuring trogocytosis can also be useful in the screening assays.

In embodiments, trogocytosis is assessed by 1) contacting a cancer cell with a cell surface label, e.g., a fluorescently-labelled antibody or fragment thereof, or a cell tracker dye, thereby labeling the cancer cell; 2) contacting a T cell with the labelled cancer cell; and 3) measuring the trogocytosis by determining the T cells that have incorporated the cell surface label from the cancer cell. In one embodiment, the cell surface label is a fluorescently labeled antibody or fragment thereof that specifically binds to a target antigen, e.g., a cancer cell surface marker. In one embodiment, the cell surface label is a cell tracker dye that non-specifically diffuses throughout and/or distributes within the cell membrane.

The difference between fluorescently labelled antibodies and cell tracker dyes is shown in **Figure 2:** fluorescently-labelled antibodies bind to specific antigens expressed in the cell surface, e.g., cell surface markers, while cell tracker dyes distribute throughout the cell membrane much less selectively, essentially nonspecifically labelling all of the surface membrane of the cell.

In embodiments, when trogocytosis occurs, there is extensive contact of the cell membrane surface between a cancer-killing T cell and a target cancer cell in the immune synapse created between the cells, prior to the T cell inserting its toxic factors into the cancer. **Figure 3** is a picture depicting this process, where the T cell extends its tentacles like an octopus capturing the cancer cells. This contact involves deep overlap of the respective cell membranes involving patches of membrane across both cells. In the trogocytosis, the T cells takes up some of these membrane patches, along with any cell surface labels, e.g., fluorescently-labelled antibodies or cell tracker dyes, present in these membrane patches.

The trogocytotic process is schematically depicted in **Figure 4:** the top panel shows labelling with fluorescently-labelled antibodies that recognize, e.g., bind to, specific cell surface markers on the cancer cells, and the lower panel that shows the non-specific labelling of the cancer cell membrane with cell tracker dye. As the immune synapse occurs (center of the figure), after the cells separates the cytotoxic T cells ends up with some of the patches of the cancer cell surface membrane that formed part of the immune synapse on its own membrane surface.

An advantage to using fluorescently-labelled antibodies includes the identification and tracking of trogocytotic T cells that have incorporated a specific cell surface marker from a cancer cell. However, without wishing to be bound by theory, in some embodiments, use of fluorescently-labelled antibodies may not be able to detect trogocytosis. In embodiments, the number of cancer cell fluorescent antibodies taken up in the T cell can depend on their relative numbers on the membrane patches of the immune synapse. For example, in some embodiments, the density or number of cell surface markers on the target cell is too low for detection of any trogocytosis that may occur, e.g., there is not enough labelled antibody that recognizes a target for a detectable signal or not enough labelled antibody that is incorporated into the T cell after a trogocytotic event to be detected. In another example, in an embodiment, the fluorochromes linked to the antibody do not emit sufficient detectable signal, and thus, cannot be detected in a trogocytotic event where a small fraction of the labelled antibody targets is taken up by the cancer-killing T cell. In another example, in an embodiment, a fluorescently-labelled antibody bound to a cell surface marker may become internalized, which can result in substantially lowering the fluorescence signal to below the detection limit.

Cell tracker dyes include lipophilic or amphiphilic fluorochromes that do not stay in the aqueous medium, but rather distribute throughout the hydrophobic surface membrane of the cells. Thus, in contrast to fluorescently-labelled antibodies, in embodiments where cell tracker dyes are used, any patches of membrane of the cancer cell taken by the T cells will carry the fluorescent molecules and can be detectable. In embodiments where high doses of cell tracker dyes are used, the number of fluorescent molecules that is incorporated into the cell membrane of the T cells by trogocytosis can be higher, e.g., substantially higher, than the number of fluorescently labelled antibodies to specific cancer cell targets.

Due to the non-specific nature of the cell tracker dye in labeling cell membranes, use of cell tracker dyes in samples that contain both cancer cells and T cells (e.g., in whole samples as used herein) can cause the labelling of both the cancer cells and the T cells, and therefore, can prevent accurate measurement of trogocytotic events. Thus, in such embodiments, the cell tracker dye is added selectively only to the cancer cells, e.g., to the cancer cells in the absence of T cells or cancer-killing T cells. In embodiments where cell tracker dyes are used and where the samples contain both cancer cells and T cells, the cell tracker dye is not added to the sample directly. In aspects of the disclosure, the cancer cells, T cells, and a pICF are provided under the conditions described herein to generate cancer-killing T cells. In an aspect of the disclosure, the pICF can be washed away from the cancer cells and T cells, e.g., cancer-killing T cells. In an embodiment, after the generated cancer-killing T cells kill all or almost all of the cancer cells, a labelled cancer cell, or a population of labelled cancer cells, can be added to the newly generated cancer-killing T cells. In embodiments, the labelled cancer cell or a population of labelled cancer cells can be cancer cells from the patient (e.g., directly from the patient or from a cryopreserved and thawed sample) or from a cancer cell line that has been labelled with cell tracker dye. In embodiments, without wishing to be bound by theory, addition of the labelled cancer cells with the generated cancer-killing T cells can reactivate the cancer-killing T cells and can induce proliferation, and accordingly, trogocytosis can be measured by detecting the signal emitted from the cell tracker dye. This exemplary process is shown in **Figure 5.**

Also, disclosed herein are methods of selecting the most effective cancer-killing T cells, e.g., trogocytotic T cells, e.g., for a specific patient.

In embodiments, the methods described herein comprise evaluating the cancer-killing T cell or preparation thereof for its likelihood to be efficacious *in vivo,* e.g., as an adoptive cell therapy. In embodiments, the methods comprise determining one or more of the following parameters: increased cancer cell killing activity, reduced toxicity, reduced off-target effect, increased viability, or increased proliferation. Without wishing to be bound by theory, it is believed that cancer-killing T cells (or preparations thereof) that have increased cancer cell killing activity, reduced toxicity, reduced off-target effect, increased viability, and/or increased proliferation are more likely to be efficacious *in vivo,* e.g., as an adoptive cell therapy.

The reduced toxicity of the cancer-killing T cell or preparation thereof are cells which kill significantly less non-pathological cells, i.e. they kill more selectively. This can be measured by labeling non-pathological cells and showing more selective cancer cell killing when compared to a reference, wherein said reference can be either different patient samples for the same cancer type, or different cell subsets (e.g. clones) within the same patient sample (e.g. trogocytotic).

The most common toxicity observed in cellular therapies is called Cytokine Storm, also known as cytokine cascade and hypercytokinemia. It is a potentially fatal immune reaction that arises when the cytokines released by cancer-killing T cells in the process of killing by cell lysis cancer cells are released outside the cells, resulting in highly elevated levels of various cytokines. In embodiments, the cancer-killing T cell or preparation thereof comprises cells having reduced toxicity because they generate less cytokines in the supernatant and/or intracellularly. In aspects of the disclosure, the cancer-killing T cell or preparation thereof comprises cells having both and simultaneously higher cancer-killing activity and reduced toxicity, because they generate less cytokines in the supernatant and/or intracellularly per unit of cancer cell killing, that is once the types and/or levels of cytokines released is normalized by the quantitative estimation of cancer cell killing activity such as Effective E:T Ratios, EC50, Emax, kinetics, or a combination of these factors.

In embodiments, methods described herein further comprise determining the cancer-killing activity of a cancer-killing T cell (e.g., selected, enriched, purified, and/or expanded) or preparation thereof. Cancer-killing activity can be determined by methods such as those comprising the following:

(a) contacting the cancer-killing T cells (or preparation thereof) with target cells that are derived from a cancer under conditions (e.g., for a period of time) sufficient to allow the cancer-killing T cells to kill the target cells; and
(b) determining the number of target cells after step (a).

In embodiments, a decrease in the number of target cells after the contacting step compared to the number of target cells before the contacting step indicates that the cancer-killing T cells are effective in killing cancer cells.

In embodiments, the activity of the cancer-killing T cells is tested against cancer cells from the same patient as those from which the T cells were isolated. In a further embodiment, the activity of the cancer-killing T cells is tested against cancer cells from a different patient (i.e., patient other than the one from which the T cells were isolated), e.g., that has the same type of cancer as the patient from which the T cells were isolated. In embodiments, the activity of the cancer-killing T cells is tested against cells lines derived from the same type of cancer as that in the patient from which the T cells were isolated.

In embodiments, the method can further comprise determining the number of cancer-killing T cells after step (a). In embodiments, an increase in the number of cancer-killing T cells compared to the number of cancer-killing T cells before the contacting step indicates that the cancer-killing T cells have increased viability and/or proliferation and may be more effective in killing cancer cells.

In embodiments, the evaluation and/or determination steps can be performed before and/or after a selection, enrichment, purification, or expansion step described herein. In embodiments, the cancer-killing T cells (or preparations thereof) that are determined to be more likely to be efficacious *in vivo* are expanded. In embodiments, additional expansion of the cancer-killing T cells can be achieved by contacting the cancer-killing T cells with cancer cells, e.g. cancer-derived cell lines or primary cancer samples. In one embodiment, a low, or insufficient, number of cancer cells are added to the cancer-killing T cells as described herein. In one embodiment, the cancer cells are added to the cancer-killing T cells one or more times, e.g., several times, e.g., sequentially. In one embodiment, each addition of the cancer cells, e.g., a low number of cancer cells, is performed when all or some portion of the cancer cells used in that contacting step are eliminated, e.g., killed. In embodiments, without wishing to be bound by theory, each addition of the cancer cells induces further expansion of the cancer-killing T cells and/or selective expansion of a particular cancer-killing T cell clone.

In some embodiments, cancer-killing T cells described herein (or preparations thereof), e.g., produced by methods described herein, contain more than one clone of a T cell. In embodiments, some clones may have higher cancer-killing activity than others.

In embodiments, clones of cancer-killing T cells containing the greatest activity (e.g., in killing cancer cells) are selected. In embodiments, clones can be separated by using limiting dilution or flow cytometry methods, e.g., to separate single cells from each other, e.g., plated into separate wells. In embodiments, the single cells are expanded to produce populations of each clone. Each clone can be evaluated for its likelihood to be efficacious *in vivo,* e.g., by determining cancer-killing activity and optionally, other parameters described herein. In embodiments, those clones exhibiting highest cancer-killing activity are further expanded and/or stored. In embodiments, the most active cancer-killing T cell clones are selected by adding a low, e.g., insufficient, number of cancer cells, e.g., from the same patient or a cancer cell line, sequentially, e.g., each time the existing cancer cells are eliminated. In embodiments, the most active T cell clone is among the first T cells that recognize, e.g., bind, and eliminate the few cancer cells, there by preferentially inducing the proliferation of the most active T cell clone. Accordingly, without wishing to be bound by theory, the aforementioned method is believed to enrich for the most active cancer-killing T cells of the T cell pool over time. Optionally, multiple clones exhibiting high cancer-killing activity are pooled together and, e.g., further expanded and/or stored.

In embodiments, cancer-killing T cells described herein (or preparations thereof) contain a single clone of a T cell.

In embodiments, cancer-killing T cells described herein are prepared according to Good Manufacturing Practice (GMP). For example, the *ex-vivo* reaction mixture described herein, e.g., used in the production of cancer-killing T cells, is prepared according to Good Manufacturing Practice (GMP). In embodiments, the cancer-killing T cells are prepared using an automated flow cytometry platform embedded in a GMP system or facility. In embodiments, the T cell, the cancer cell, or both, used in the ex-vivo reaction mixture, are obtained from a hospital or a health care provider. In embodiments, the expansion, selection, purification, and/or enrichment of the cancer-killing T cells is performed according to Good Manufacturing Practice (GMP). In embodiments, methods described herein further comprise sending the cancer-killing T cell (e.g., produced by GMP) to a hospital or a health care provider.

## Evaluation of Cancer-Killing T cell and Subjects' Responsiveness

Provided herein are methods of evaluating the potential of a subject to produce cancer-killing T cells, e.g., trogocytotic T cells. Also, provided herein are methods of evaluating a subject's responsiveness to cancer-killing T cells.

In aspects of the disclosure, the methods comprise (a) providing a T cell from a subject having a cancer; (b) providing a cancer cell from the subject; and (c) forming an *ex vivo* reaction mixture comprising the T cell, the cancer cell, and a pICF, e.g., where the *ex vivo* reaction mixture is formed under conditions, e.g., for a period of time, sufficient to allow the T cell to acquire a cell surface marker from the cancer cell, e.g., sufficient to permit production of trogocytotic T cells that have cytotoxic activity toward the cancer cell.

In aspects of the disclosure, the activity of a candidate pICF and/or immunomodulator, and the T cells generated, is determined using an *ex vivo/in vitro* assay to measure dose response curves, whose mathematical fitting enable quantitative parameters to estimate the activity, selected from at least one from EC50, Effective E:T ratio, Emax or kinetics. These parameters can be used to evaluate the Cancer-killing T cells and the responsiveness of individual subjects.

- EC50 of the T cell proliferation determines the concentration of pICF which generates 50% of the activated cancer-killing T cells in a sample. The EC50 of T cell activation is similar to the EC50 of cancer cell depletion.
- The Effective E:T ratio represents the activity of the cancer-killing T cells generated (effective T cells) on the cancer cells (target cells). High Effective E:T Ratios predict sensitive patients to the cancer-killing T cells as autologous cell therapy, and low Effective E:T Ratios predict resistant patients to these cancer-killing T cells as autologous cell therapy.
- Emax of the dose response curves of the cancer cells determines the percentage (%) of cancer cells alive at high doses of the pICF at a given incubation time. Longer incubations allow cancer-killing T cells to kill more cancer cells. The activated cancer-killing T cells need to kill 100% of cancer cells for these T cells to be a suitable monotherapy treatment for a patient. When killing cancer cells is significantly lower than 100%, and this does not improve at longer incubation times, those cancer cells alive are resistant and can be clinically informative to determine a treatment. In order to revert the resistant phenotype, addition of additional immunomodulatory agents, such as immune check point inhibitors, may overcome this immunosuppression and thus overcome this resistance.

The time-dependent kinetics of the cancer-killing T cell activity. The efficiency of the T cell activation and cancer cell depletion is different in each subject for the same pICF. Cancer-killing T cells which kill cancer cells faster are likely to be more efficacious in cellular therapies. Faster activity is correlated with faster effects against the cancer cells in a patient, a positive outcome that reflects higher sensitivity. Faster activity means also higher affinity towards cancer cells when the cancer-killing T cells are CD8+ Tumor-Specific Antigen T cells that recognize cancer cells through a MHC-I mechanism.

In embodiments, the method further comprises identifying the trogocytotic T cells in the reaction mixture. In embodiments, the method further comprises quantifying the trogocytotic T cells in the reaction mixture. The identification and/or quantification can involve the identification of cells expressing one or more markers from the cancer cell. Additionally, or alternatively, the identification and/or quantification involves the identification of cells expressing one or more markers of a CTL, e.g., effector memory CTL. The identification and/or quantification of CTL markers can be performed using labeled antibody molecules (e.g., fluorescently labeled or radioactively labeled antibody molecules). In embodiments, the identification and/or quantification of trogocytotic markers can also be performed using a cell surface label, e.g., a fluorescent cell tracker dye that distributes in or throughout the target cell membrane. In such embodiments, the cell surface label, e.g., cell tracker dye, is added to the cancer cell but not to the cancer-killing T cells in reactions for generating trogocytotic T cells, e.g., reactions comprising cancer cells, T cells, and pICF. In some examples, flow cytometry can be used to identify (and optionally quantify) the expression of a marker on the cells in the reaction mixture. In embodiments, flow cytometry can be used to quantify the number of cells having particular expression patterns, e.g., expression pattern indicative of cancer-killing T cells (e.g., trogocytotic T cells). In embodiments, a higher number of trogocytotic T cells generated using components (e.g., T cells and cancer cells) (e.g., compared to a reference level) from a subject indicates that the subject is capable of producing cancer-killing T cells, e.g., trogocytotic T cells. In embodiments, a higher number of trogocytotic T cells generated using components (e.g., T cells and cancer cells) from a subject (e.g., compared to a reference level) indicates that the subject is responsive to therapy comprising a cancer-killing T cell, e.g., as an adoptive cell therapy. In embodiments, a reference level can be the number of cancer-killing T cells (e.g., trogocytotic T cells) generated in a similar reaction mixture in the absence of one or more of a T cell, a cancer cell, or a pICF. For example, a reference level can be the number of cancer-killing T cells (e.g., trogocytotic T cells) generated in a similar reaction mixture in the absence of a pICF. In other embodiments, a reference level is the number of cancer-killing T cells (e.g., trogocytotic T cells) generated in the *ex vivo* reaction mixture after a period of time that is insufficient to allow the T cell to acquire a cell surface marker from the cancer cell.

In embodiments, the ratio of cancer-killing T cells to target cells (e.g., cancer cells) that lead to cancer cell death can be an indicator of a subject's responsiveness to the cancer-killing T cells. Without wishing to be bound by theory, it is believed that a higher target cell:cancer-killing T cell ratio can indicate more activated (e.g. trogocytic) T cells produced (e.g., using a method described herein), and can in turn indicate that the subject is a better responder to the cancer-killing T cells (and possibly a better responder to the pICF used to produce the cancer-killing T cells).

## Pharmaceutical Compositions and Methods of Treatment

Pharmaceutical compositions disclosed herein can comprise a cancer-killing T cell, includes activated tumor antigen-specific T cells, including, but not limited to, effector memory T cells, cytotoxic T lymphocytes (CTLs), helper T cells, tumor infiltrating lymphocytes (TILs) and trogocytotic T cells or preparation thereof, as described herein, in combination with one or more physiologically or pharmaceutically acceptable carriers, diluents, or excipients. For example, the pharmaceutical composition can comprise buffers (e.g., neutral buffered saline, phosphate buffered saline; polypeptides/amino acids (e.g., glycine); anticoagulants (e.g. Heparin); proteins; antioxidants; carbohydrates (e.g., glucose, mannose, sucrose or dextran, mannitol); adjuvants (e.g., aluminium hydroxide); chelating agents (e.g., EDTA or glutathione); and/or preservatives. In embodiments, the pharmaceutical composition is substantially free of a contaminant, such as mycoplasma, endotoxin, lentivirus or components thereof, magnetic beads, bacteria, fungi, bovine serum albumin, bovine serum, and/or plasmid or vector components. In embodiments, the pharmaceutical composition comprises cancer-killing T cells that are prepared according to Good Manufacturing Practice (GMP).

In embodiments, the pharmaceutical composition is a purified preparation. For example, the pharmaceutical composition is substantially free of, e.g., there are no detectable levels of a contaminant. In embodiments, the contaminant comprises endotoxin, mycoplasma, p24, VSV-G nucleic acid, HIV gag, replication competent lentivirus (RCL), residual pICF, antibodies, bovine serum albumin, bovine serum, pooled human serum, culture media components, vector packaging cell or plasmid components, a bacterium or fungus. In one embodiment, the bacterium is at least one selected from the group consisting of *Alcaligenes faecalis, Candida albicans, Escherichia coli, Haemophilus influenza, Neisseria meningitides, Pseudomonas aeruginosa, Staphylococcus aureus, Streptococcus pneumonia,* and *Streptococcus pyogenes* group A.

In certain embodiments, the pharmaceutical composition comprises a detectable (e.g., trace) amount of a pICF,e.g., a pICF described herein. In embodiments, the pICF is present at a concentration of less than 10% by weight, e.g., less than 10%, 9%, 8%, 7%, 6%, 5%, 4%, 3%, 2%, 1%, 0.5%, 0.1%, 0.05%, 0.01%, or less by weight (e.g., but no less than 0.0001% by weight).

In certain embodiments, the pharmaceutical composition comprises a detectable (e.g., trace) amount of a cell surface label, e.g., a fluorescent cell surface label, such as an antibody cell surface label or a cell tracker dye as described herein. In embodiments, the cell surface label, e.g., fluorescent cell surface label, is present at a concentration of less than 10% by weight, e.g., less than 10%, 9%, 8%, 7%, 6%, 5%, 4%, 3%, 2%, 1%, 0.5%, 0.1%, 0.05%, 0.01%, or less by weight (e.g., but no less than 0.0001% by weight).

In embodiments, the pharmaceutical composition comprises a cancer-killing T cell (or preparation thereof) prepared using a method described herein.

Methods described herein include treating a cancer in a subject by using a cancer-killing T cell (or preparation thereof) described herein, e.g., using a pharmaceutical composition described herein. Also disclosed are methods for reducing or ameliorating a symptom of a cancer in a subject as well as methods for inhibiting the growth of a cancer and/or killing one or more cancer cells. In aspects of the disclosure, the methods described herein decrease the size of a tumor and/or decrease the number of cancer cells in a subject administered with a cancer-killing T cell described herein or a pharmaceutical composition described herein.

In embodiments, the cancer is a hematological cancer. In embodiments, the hematological cancer is a leukemia or a lymphoma. Exemplary hematological cancers include but are not limited to a Hodgkin's lymphoma, Non-Hodgkin's lymphoma (e.g., B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia. In embodiments, the cancer is other than acute myeloid leukemia (AML).

In embodiments, the cancer is a solid cancer. Exemplary solid cancers include but are not limited to ovarian cancer, rectal cancer, stomach cancer, testicular cancer, cancer of the anal region, uterine cancer, colon cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not a melanoma.

In embodiments, the subject to be treated by cancer-killing T cells is the same as the subject from which T cells and/or cancer cells were isolated for the production of the cancer-killing T cells. In embodiments, the subject to be treated by cancer-killing T cells is different from the subject from which T cells and/or cancer cells were isolated for the production of the cancer-killing T cells. In embodiments, both subjects have or have had the same type of cancer.

In embodiments, the cancer-killing T cells (or pharmaceutical composition) are administered in a manner appropriate to the disease to be treated or prevented. The quantity and frequency of administration will be determined by such factors as the condition of the patient, and the type and severity of the patient's disease. Appropriate dosages may be determined by clinical trials. For example, when "an effective amount" or "a therapeutic amount" is indicated, the precise amount of the pharmaceutical composition (or cancer-killing T cells) to be administered can be determined by a physician with consideration of individual differences in tumor size, extent of infection or metastasis, age, weight, and condition of the subject. In embodiments, the pharmaceutical composition described herein can be administered at a dosage of $10^4$ to $10^9$ cells/kg body weight, e.g., $10^5$ to $10^6$ cells/kg body weight, including all integer values within those ranges. In embodiments, the pharmaceutical composition described herein can be administered multiple times at these dosages. In embodiments, the pharmaceutical composition described herein can be administered using infusion techniques described in immunotherapy (see, *e.g.*, Rosenberg SA (1988)).

In embodiments, the cancer-killing T cells (or preparations thereof) or pharmaceutical composition is administered to the subject parenterally. In embodiments, the cells are administered to the subject intravenously, subcutaneously, intratumorally, intranodally, intramuscularly, intradermally, or intraperitoneally. In embodiments, the cells are administered, e.g., injected, directly into a tumor or lymph node. In embodiments, the cells are administered as an infusion (e.g., as described in Rosenberg SA (1988)) or an intravenous push. In embodiments, the cells are administered as an injectable depot formulation.

In embodiments, a single dose of cancer-killing T cells (or pharmaceutical composition) is administered to a subject. In embodiments, multiple doses of cancer-killing T cells are administered to a subject. In embodiments, the time period between each dose is at least 12 hours, e.g., at least 12, 24, 36, 48, 72, 96 h or more, or at least 1, 2, 3, 4, 5, 6, 7 days or more, or at least 1, 2, 3, 4 weeks or more.

In embodiments, a single dose comprises $10^3$ to $10^{11}$ cancer-killing T cells (e.g., $10^3$ to $10^4$, $10^4$ to $10^5$, $10^5$ to $10^6$, $10^6$ to $10^7$, $10^7$ to $10^8$, $10^8$ to $10^9$, $10^9$ to $10^{10}$, or $10^{10}$ to $10^{11}$ cancer-killing T cells).

In embodiments, each dose of a multiple dose regimen comprises $10^3$ to $10^{11}$ cancer-killing T cells (e.g., $10^3$ to $10^4$, $10^4$ to $10^5$, $10^5$ to $10^6$, $10^6$ to $10^7$, $10^7$ to $10^8$, $10^8$ to $10^9$, $10^9$ to $10^{10}$, or $10^{10}$ to $10^{11}$ cancer-killing T cells).

In embodiments, the cancer-killing T cells or preparations thereof (or pharmaceutical composition) decrease the number of or percentage of cancer cells in a subject, e.g., by at least 25%, at least 30%, at least 40%, at least 50%, at least 65%, at least 75%, at least 85%, at least 95%, or at least 99% relative to a negative control.

In embodiments, the subject is a mammal. In embodiments, the subject is a human, monkey, pig, dog, cat, cow, sheep, goat, rabbit, rat, or mouse. In embodiments, the subject is a human. In embodiments, the subject is a pediatric subject, e.g., less than 18 years of age, e.g., less than 17, 16, 15, 14, 13, 12, 11, 10, 9, 8, 7, 6, 5, 4, 3, 2, 1 or less years of age. In embodiments, the subject is an adult, e.g., at least 18 years of age, e.g., at least 19, 20, 21, 22, 23, 24, 25, 25-30, 30-35, 35-40, 40-50, 50-60, 60-70, 70-80, or 80-90 years of age.

**Combination Therapies**

In accordance with a method described herein, in some embodiments, an effector T cell described herein, e.g.,

effector T cell population described herein (e.g., trogocytotic T cell) can be used in combination with a second therapeutic agent or procedure.

In embodiments, the effector T cell and the second therapeutic agent or procedure are administered/performed after a subject has been diagnosed with a cancer, e.g., before the cancer has been eliminated from the subject. In embodiments, the effector T cell and the second therapeutic agent or procedure are administered/performed simultaneously or concurrently. For example, the delivery of one treatment is still occurring when the delivery of the second commences, e.g., there is an overlap in administration of the treatments. In other embodiments, the effector T cell and the second therapeutic agent or procedure are administered/performed sequentially. For example, the delivery of one treatment ceases before the delivery of the other treatment begins.

In embodiments, combination therapy leads to more effective treatment, e.g., more effective killing of cancer cells. In embodiments, the combination of the first and second treatment is more effective (e.g., leads to a greater reduction in symptoms and/or cancer cells) than the first or second treatment alone. In embodiments, the combination therapy permits use of a lower dose of the first or the second treatment compared to the dose of the first or second treatment normally required to achieve similar effects when administered as a monotherapy. In embodiments, the combination therapy has a partially additive effect, wholly additive effect, or greater than additive effect.

In embodiments, the second therapeutic agent or procedure includes a therapy described in the "Other methods of enhancing T cell activity" section herein. In embodiments, the second therapeutic agent includes an immune checkpoint inhibitor (e.g., an immune checkpoint inhibitor described herein), an agonist of a T cell (e.g., an agonist of a T cell described herein), and/or other immunomodulatory drug (e.g., lenalidomide) as described herein.

### Screening Assays for New pICFs and Immunomodulators

Disclosed herein are methods of screening for candidate pICFs and/or candidate immunomodulators. For example, the methods involve evaluating the efficacy, e.g., *ex vivo* efficacy, of pICFs. Methods herein include screening of multiple pICF and/or immunomodulator candidates and/or their combinations in order to identify the most effective set of pICFs and/or immunomodulators for a specific tumor/cancer type of a specific patient.

In aspects of the disclosure, the methods comprise a cell based assay and can involve an automated sample preparation and automated evaluation, e.g., by flow cytometry, e.g., using the ExviTech platform. See, e.g., US 8,703,491, US 2013/0109101A1, US 2010/0298255A1, US 8,313,948 and Bennett TA (2014). For example, use of an automated platform, e.g., automated flow cytometry platform, can enable the evaluation of hundreds or thousands of different pICFs and/or immunomodulators, and this evaluation can be made *ex vivo.* The use of flow cytometry methods permits the evaluation of individual cells and also the sorting of specific cell populations. Immune cells can be stained with antibodies that bind to cell type specific cell surface markers. Target cancer cells can be stained with cell surface labels, e.g., antibodies that bind to cell type-specific cell surface markers or cell tracker dyes that distribute in the target cell membrane. Cells can also be stained for molecules present in the interior of a cell, allowing for the characterization of cells by their production of proteins, e.g., interleukins or interferons.

In aspects of the disclosure, candidate pICFs and/or immunomodulators can be screened using an automated flow cytometry platform, such as the ExviTech platform. *See Id.* This platform permits the determination of the cancer-killing (e.g. trogocytotic) potential of hundreds or thousands of pICFs and/or immunomodulators. The cancer-killing potential of pICF and/or immunomodulators can also be measured by ratios of target cancer cells to cancer-killing T cells as described herein. The platform also allows for the screening of many combinations of the pICFs and/or immunomodulators.

In aspects of the disclosure, the screening method comprises incubating one or more candidate pICFs and/or immunomodulators with cancer cells and T cells. In aspects of the disclosure, the method comprises incubating one or more candidate pICFs and/or immunomodulators with a sample, e.g., blood sample, where the blood sample contains both cancer cells and T cells. In aspects of the disclosure, the method comprises incubating one or more candidate pICFs and/or immunomodulators with a tumor sample, where the tumor sample contains both cancer cells and T cells. In other aspects of the disclosure, the cancer cells and T cells are from different samples, e.g., the cancer cells are from a tumor sample and the T cells are from a blood sample.

In aspects of the disclosure, the sample comprises a blood sample, e.g., whole blood sample, peripheral blood, or bone marrow. In another aspect of the disclosure, the sample is obtained from a lymph node or a spleen. In aspects of the disclosure, the sample is obtained from any other tissue that is involved in a malignancy, e.g., hematological malignancy or solid cancer. In aspects of the disclosure, samples are used in the method described herein soon after they are obtained. Alternatively, samples may be treated with a chemical to avoid coagulation and analyzed at a later time point. In one aspect of the disclosure, a blood sample is treated with heparin to avoid coagulation. In another aspect of the disclosure, a blood sample is treated with EDTA to avoid coagulation. In another aspect of the disclosure, a blood sample is treated with an anticoagulant, including but not limited to a thrombin inhibitor, to avoid coagulation. In aspects of the disclosure, the sample is used without purification or separation steps, e.g., so that the cellular environment is more similar to the *in vivo* environment.

In aspects of the disclosure, the incubation time is sufficient for the T cell to acquire a cell surface marker from the cancer cell, e.g., to undergo trogocytosis to form a trogocytotic T cell, e.g., that kills the cancer cell. In aspects of the disclosure, the incubation time is sufficient for the pICF and/or immunomodulators to induce a significantly higher Effective E:T ratio between eliminated cancer cells and CD8 and/or CD4 activated T cells. In aspects of the disclosure, the incubation time is at least 12 hours (e.g., at least 12, 24, 36, 48, 72, 96, 120 h, or more). In aspects of the disclosure, a second or subsequent sets of cancer cells are added after the first reaction mixture of cancer cells, T cells and a pICF generates cancer-killing T cells *ex vivo,* and in these cases the incubation time is shorter, e.g., at least 1 hour (e.g. at least 2, 3, 4, 5, 6, 7, 8, 9, 10, 11, 12, 24 h, or more).

Hundreds or thousands of candidate pICFs and/or immunomodulators can be evaluated or screened. The methods described herein are capable of analyzing large numbers of candidate pICFs and/or immunomodulators (e.g., combinations of candidate pICFs and/or immunomodulators) at various concentrations in the form of aliquots to assess a large number of variables for a personalized medicine regimen (e.g., for personalized production of and use of cancer-killing T cells). In one aspect of the disclosure, the method analyzes about 5-500 aliquots (e.g., 5, 10, 15, 20, 25, 30, 35, 40, 45, 50, 100, 200, 500, or more) (optionally per candidate pICF and/or immunomodulator), or a range defined by any two of the preceding values. In another aspect of the disclosure, the method analyzes about 96 or more aliquots. Additionally, the number of candidate pICFs and/or immunomodulators can vary along with the number of aliquots. In one aspect of the disclosure, both the number of aliquots and the number of different candidate pICFs and/or immunomodulators are each greater than about 5 (e.g., 5, 10, 15, 20, 25, 30, 35, or 40), or a range defined by any two of the preceding values. In another aspect of the disclosure, both the number of aliquots and the number of different candidate pICFs and/or immunomodulators are each greater than about 50. In another aspect of the disclosure, both the number of aliquots and the number of different candidate pICFs and/or immunomodulators are each greater than about 96. In another aspect of the disclosure, the active ingredients of approved drugs known in the art can be screened in the assays described herein to identify potential pICFs and/or immunomodulators. Such approved drugs are safe in humans and can be used to generate the cancer-killing T cells for administration to a patient.

In aspects of the disclosure, the screening method comprises identifying (e.g., and quantifying) the target cell (e.g., cancer cell) population. Alternatively, or in addition, the screening method comprises identifying the effector cell population (e.g., trogocytotic T cell population). Cell populations can be identified by using antibodies, e.g., monoclonal antibodies, directed toward specific cell surface or intracellular markers, e.g., that are conjugate to detection labels, such as fluorescent tags. In aspects of the disclosure, cell surface markers include cluster of differentiation (CD) markers, which are used for the identification of hematological malignancies (e.g., leukemia, multiple myeloma, lymphoma) and of leukocytes. CD markers are also used to identify and diagnose solid tumors. Flow cytometry can be used for detection and quantification of cell populations. Cell surface labels, e.g., cell tracker dyes, can also be used to label the surface membrane of cancer cells to measure trogocytosis by cancer-killing T cells. Immunohistochemistry can also be used to detect certain cell markers, e.g., to identify cell populations.

In aspects of the disclosure, the effect of a candidate pICF and/or immunomodulator on the population(s) and subpopulation(s) of effector cells (e.g., T cells) is determined. For example, the sample may contain various types of T cells before incubation with the pICF and/or the immunomodulator and may contain different combinations of or different levels of various T cell types after incubation with the pICF and/or immunomodulator. In aspects of the disclosure, incubation with the pICF and/or immunomodulator can lead to formation of and/or increase in the numbers of trogocytotic T cells. In aspects of the disclosure, the percentage of T cells that become trogocytotic (and express markers from both effector T cells and cancer cells) after incubation with the pICF and/or immunomodulator is measured.

In aspects of the disclosure, the effect of a candidate pICF and/or immunomodulator on the target cell (e.g., cancer cell) population is measured. The measurement can involve measuring cell depletion, e.g., quantifying the cell counts in the well(s) containing pICF or immunomodulator to the well(s) containing a negative control.

In aspects of the disclosure, the effect of a candidate pICF and/or immunomodulator on the effector cell (e.g., trogocytotic T cell) population is measured. The measurement can involve cell proliferation analysis, e.g., comparing the cell counts in the well(s) containing pICF and/or immunomodulator to the well(s) containing a negative control.

In aspects of the disclosure, candidate pICFs and/or immunomodulators that lead to (i) depletion of target (e.g., cancer) cells; (ii) formation of or increase in levels of trogocytotic T cells (e.g., that contain markers from cancer cells and markers from effector T cells, e.g., CTLs), and/or (iii) proliferation of effector T cells (e.g., CTLs) are identified as effective pICFs and/or immunomodulators, e.g., effective in generating T cells with enhanced cancer-killing activity.

In aspects of the disclosure, candidate pICFs and/or immunomodulators are evaluated in comparison with a reference, e.g., a pICF and/or immunomodulator described herein. In aspects of the disclosure, a candidate pICF that leads to a similar or greater depletion of target (e.g., cancer) cells compared to the reference is identified as an effective pICF and/or immunomodulator. In aspects of the disclosure, a candidate pICF that leads to a similar or greater formation of or increase in levels of trogocytotic T cells (e.g., that contain markers from cancer cells and markers from effector T cells, e.g., CTLs) is identified as an effective pICF and/or immunomodulator. In aspects of the disclosure, a candidate pICF and/or immunomodulator that leads to a similar or greater extent of proliferation of the cancer-killing T cells is identified as effective pICF and/or immunomodulator.

In aspects of the disclosure, the activity of a candidate pICF and/or immunomodulator, and the T cells generated, is determined using an *ex vivo/in vitro* assay to measure dose response curves, whose mathematical fitting enable quantitative parameters to estimate the activity, selected from at least one from EC50, Effective E:T ratio, Emax or kinetics.

- EC50 of the T cell proliferation determines the concentration of pICF which generates 50% of the activated cancer-killing T cells in a sample. The EC50 of T cell activation is similar to the EC50 of cancer cell depletion.
- The Effective E:T ratio represents the activity of the cancer-killing T cells generated (Effective T cells) on the cancer cells (target cells). High Effective E:T Ratios predict sensitive patients to the cancer-killing T cells as autologous cell therapy, and low Effective E:T Ratios predict resistant patients to these cancer-killing T cells as autologous cell therapy.
- Emax of the dose response curves of the cancer cells determines the percentage (%) of cancer cells alive at high doses of the pICF at a given incubation time. Longer incubations allow cancer-killing T cells to kill more cancer cells. The activated cancer-killing T cells need to kill 100% of cancer cells for these T cells to be a suitable monotherapy treatment for a patient. When killing cancer cells is significantly lower than 100%, and this does not improve at longer incubation times, those cancer cells alive are resistant and can be clinically informative to determine a treatment. In order to revert the resistant phenotype, addition of additional immunomodulatory agents, such as immune check point inhibitors, may overcome this immunosuppression and thus overcome this resistance.

1. Adding *ex vivo* additional immunomodulatory agents to relieve immunosuppression is especially meaningful for cancer-killing T cells generated for subsequent cellular therapy. The reason is that this enables the combination of multiple immunotherapy agents, immunomodulatory agents, including multiple mechanism of action, up to 5, 10, or 20 agents, contrary to the combined treatments described in the prior art, which only allow the combination of 2-3 immunotherapy drugs administered simultaneously to the subject *in vivo* due to the toxicity restrictions which limit poly-immunotherapy. Screening assays as presented herein that measure *ex vivo/in vitro* toxicity of cancer-killing T cells, alone or normalized by their cancer cell killing activity, may be suitable to identify the optimal combination of immunotherapies incubated *ex vivo/in vitro* that limit their combined toxicity while enhancing their combined activity. Hence, cancer-killing T cells generated ex vivo for cellular therapy can exploit the advantages of poly-immunotherapy.
2. Adding immunomodulatory agents to the cancer-killing T cells *in vivo,* as additional immuno-therapeutic treatment, resulting in a combination treatment. Screening assays as presented herein that measure *ex vivo/in vitro* toxicity of cancer-killing T cells, alone or normalized by their cancer cell killing activity, may be suitable to predict the optimal combination of immunotherapies to treat a patient by limiting their combined toxicity while enhancing their combined activity.

- The time-dependent kinetics of the cancer-killing T cell activity. The efficiency of the T cell activation and cancer cell depletion is different in each subject for the same pICF. Cancer-killing T cells which kill cancer cells faster are likely to be more efficacious in cellular therapies. Faster activity is correlated with faster effects against the cancer cells in a patient, a positive outcome that reflects higher sensitivity. Faster activity means also higher affinity towards cancer cells when the cancer-killing T cells are CD8+ Tumor-Specific Antigen T cells that recognize cancer cells through a MHC-I mechanism.

In aspects of the disclosure, the cancer-killing T cell preparation comprises cells having less toxicity *ex vivo/in vitro* because they kill significantly less non-pathological cells, i.e. they kill more selectively. This can be measured by labeling non-pathological cells and showing more selective cancer cell killing when compared to a reference, wherein said reference can be either different patient samples for the same cancer type, or different cell subsets (e.g. clones) within the same patient sample (e.g. trogocytotic).

The most common toxicity observed in cellular therapies is called Cytokine Storm, also known as cytokine cascade and hypercytokinemia. It is a potentially fatal immune reaction that arises when the cytokines released by cancer-killing T cells in the process of killing by cell lysis cancer cells are released outside the cells, resulting in highly elevated levels of various cytokines. In aspects of the disclosure, the cancer-killing T cell preparation comprises cells having less toxicity *ex vivo/in vitro* because they generate less cytokines in the supernatant and/or intracellularly. In aspects of the disclosure, the cancer-killing T cell preparation comprises cells having both and simultaneously higher cancer-killing activity and less toxicity *ex vivo/in vitro,* because they generate less cytokines in the supernatant and/or intracellularly per unit of cancer cell killing, that is once the types and/or levels of cytokines released is normalized by the quantitative estimation of cancer cell killing activity such as Effective E:T Ratios, EC50, Emax, kinetics, or a combination of these factors.

In aspects of the disclosure, the efficacy, e.g., potency, activity, of a candidate pICF and/or immunomodulator, is determined using an *ex vivo/in vitro* assay using different ratios of cancer-killing T cell:target cell, e.g., where a target cell can be a cancer cell. In some aspects of the disclosure, the assay involves providing a cancer-killing T cell or a preparation thereof, e.g., produced according to a method described herein. In aspects of the disclosure, the assay further involves a step (a) forming a plurality of ex vivo reaction mixtures comprising a candidate pICF(s) and/or immunomodulator(s), a target cell (e.g., cancer cell), and the cancer-killing T cell or preparation thereof under conditions (e.g., for a period of time and for certain concentrations of the candidate pICF and/or immunomodulatory agent) sufficient to allow the cancer-killing T cells to kill the target cells. In aspects of the disclosure, the *ex vivo* reaction mixtures comprise a plurality of target cell to T cell ratios. The assay can also involve a step (b) for each target cell to T cell ratio, determining the number of target cells after step (a), and optionally determining the number of cancer-killing T cells after step (a). In aspects of the disclosure, the assay further comprises a step (c) correlating the target cell to T cell ratio from step (a) with the number of target cells in step (b). In aspects of the disclosure, a high target cell to T cell ratio from step (a) (e.g., higher ratio than a reference ratio) that results in fewer target cells after step (a) indicates that the candidate pICF and/or immunomodulator is an effective pICF and/or immunomodulator (e.g., a potent pICF and/or immunomodulator) for use in producing a cancer-killing T cell from the subject.

In aspects of the disclosure, the reference ratio is a predetermined ratio, e.g., about 1:3 to 1:10, e.g., about 1:3, 1:4, 1:5, 1:6, 1:7, 1:8, 1:9, or 1:10. In aspects of the disclosure, the high target cell to T cell ratio from step (b) is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In aspects of the disclosure, fewer target cells after step (a) is indicated by a lower number of target cells in step (b) compared to a control value (e.g., lower by at least 1.5-fold, e.g., at least 2-, 3-, 4-, 6-, 8-, 10-, 25-, 50-, 100-, 150-, 200-, 500-, 1000-, or more). In aspects of the disclosure, the control value is the number of target cells before the formation of the *ex vivo* mixtures, or the number of target cells in the *ex vivo* mixtures after a period of time insufficient to allow the cancer-killing T cells to kill the target cells. In aspects of the disclosure, the control value is the number of target cells in the *ex vivo* mixtures incubated for the same period of time without a pICF, or with a null control of the pICF that contains only the T cell interacting arm (e.g. CD3).

In aspects of the disclosure, the cancer-killing T cell or preparation thereof comprises a T cell, e.g., CTL, that is CD8+ and CD25+ and/or a T cell that is CD4+ and CD25+.

In aspects of the disclosure, the method (e.g., step (b) of the method is performed in an automated platform, e.g., an automated flow cytometry platform described herein, e.g., the ExviTech platform described herein.

In aspects of the disclosure, effective candidate pICFs and/or immunomodulators are used in a method described herein, e.g., method of producing cancer-killing T cells described herein, method of treatment described herein, method of evaluating cancer treatments described herein, and/or method of identifying patients responsive to cancer-killing T cells described herein.

## Evaluation Assays for Cancer Treatments

Provided herein are methods of evaluating whether or not a patient will respond to a certain cancer treatment. In embodiments, the methods of evaluating include methods of screening for cancer treatments that would likely be effective in a particular patient.

In embodiments, a number of types of cancer treatments (e.g. a chemotherapy, a targeted anti-cancer therapy, an oncolytic drug, a cytotoxic agent, an immune-based therapy, a cytokine, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy) can be evaluated. In embodiments, a cancer treatment to be evaluated includes but is not limited to an immune checkpoint inhibitor, e.g., an inhibitor of one or more of: CTLA4, PD1, PDL1, PDL2, B7-H3, B7-H4, TIM3, LAG3, BTLA, CD80, CD86, or HVEM. Exemplary immune checkpoint inhibitors include ipilimumab, tremelimumab, MDX-1106, MK3475, CT-011, AMP-224, MDX-1105, IMP321, or MGA271. In embodiments, a cancer treatment to be evaluated includes an agonist of T cells, e.g., an antibody or fragment thereof to CD137, CD40, and/or glucocorticoid-induced TNF receptor (GITR). In embodiments, a cancer treatment to be evaluated includes an immunomodulatory agent such as lenolidomide. Any of the immunomodulatory agents described herein can be evaluated.

In aspects of the disclosure, the method of evaluating comprises: (a) providing a T cell from a subject having a cancer (e.g., a hematological cancer or a solid cancer); (b) providing a cancer cell, e.g., from the subject; (c) forming an *ex vivo* reaction mixture comprising the T cell, the cancer cell, and a proximity immuno-coaching factor (pICF), e.g., under conditions (e.g., for a period of time) sufficient to allow the T cell to acquire a cell surface marker from the cancer cell; and (d) contacting the *ex vivo* reaction mixture with a candidate cancer treatment or combination of cancer treatments. The method further comprises determining one or more parameters indicating effectiveness of the candidate cancer treatment(s) in killing cancer cells in the particular patient.

Exemplary pICFs are described in detail in the "proximal Immuno-Coaching Factor (pICF)" section herein.

In embodiments, the T cell and the cancer cell are from the same sample, e.g., from the patient to be evaluated (for responsiveness to cancer treatment). For example, a blood sample (e.g., comprising both the cancer cell and the T cell) from the patient to be evaluated is provided as the sample. Alternatively, a tumor sample (e.g., comprising both the cancer cell and the T cell, e.g., tumor infiltrating T cell) from the patient to be evaluated is provided. In embodiments, the method does not comprise removing any components (e.g., cell components) from the sample, e.g., blood sample or the tumor sample, before forming the ex *vivo* reaction mixture. In embodiments, the blood sample or tumor sample can be a freshly isolated sample or a frozen and thawed sample.

In embodiments, the sample comprises a blood sample, e.g., whole blood sample, peripheral blood, or bone marrow. In another embodiment, the sample is obtained from a lymph node or a spleen. In embodiments, the sample is obtained from any other tissue that is involved in a malignancy, e.g., hematological malignancy or solid cancer. In embodiments, samples are used in the method described herein soon after they are obtained. Alternatively, samples may be treated with a chemical to avoid coagulation and analyzed at a later time point. In one embodiment, a blood sample is treated with heparin to avoid coagulation. In another embodiment, a blood sample is treated with EDTA to avoid coagulation. In another embodiment, a blood sample is treated with an anticoagulant, including but not limited to a thrombin inhibitor, to avoid coagulation. In embodiments, the sample is used without purification or separation steps, e.g., so that the cellular environment is more similar to *the in vivo* environment.

In embodiments, the reaction mixture is carried out in a container, e.g., a well of a multi-well dish or plate (e.g., a microplate, e.g., comprising 6, 12, 24, 48, or 96 wells), or an assay tube.

In embodiments, the method (e.g., by including the pICF) generates a population of trogocytotic T cells that have enhanced cancer-killing activity. Without wishing to be bound by theory, it is believed that by generating this population of enhanced cancer-killing T cells, the assay is more sensitive in detecting effects of cancer treatments than in other types of assays not containing such cancer-killing T cells.

In embodiments, the method of evaluating can be performed in a high throughput matter, e.g., can involve screening for cancer treatments that would likely be effective in a particular patient. In embodiments, screening methods comprise a cell based assay and can involve an automated sample preparation and automated evaluation, e.g., by flow cytometry, e.g., using the ExviTech platform. For example, use of an automated platform, e.g., automated flow cytometry platform, can enable the evaluation of hundreds or thousands of different cancer treatments, and this evaluation can be made *ex vivo.* In embodiments, candidate cancer treatments can be screened using an automated flow cytometry platform, such as the ExviTech platform. The platform also allows for the screening of many combinations of the cancer treatments.

Hundreds or thousands of candidate cancer treatments can be sampled. The methods described herein are capable of analyzing large numbers of candidate cancer treatments (e.g., combinations of candidate cancer treatments) at various concentrations in the form of aliquots to assess a large number of variables. In one embodiment, the method analyzes about 5 - 500 aliquots (e.g., 5, 10, 15, 20, 25, 30, 35, 40, 45, 50, 100, 200, 500, or more) (optionally per cancer treatment), or a range defined by any two of the preceding values. In another embodiment, the method analyzes about 96 or more aliquots. Additionally, the number of cancer treatments can vary along with the number of aliquots. In one embodiment, both the number of aliquots and the number of different candidate cancer treatments are each greater than about 5 - 40 (e.g., 5, 10, 15, 20, 25, 30, 35, or 40), or a range defined by any two of the preceding values. In another embodiment, both the number of aliquots and the number of different candidate cancer treatments are each greater than about 50. In another embodiment, both the number of aliquots and the number of different candidate cancer treatments are each greater than about 96.

The effects of candidate cancer treatments on cancer cells from the patient to be evaluated can be determined by the extent of cancer-killing that occurs in the presence of the candidate treatment compared to in the absence of the candidate treatment. The extent of cancer-killing can be determined using methods described herein. In embodiments, cancer-killing activity is determined by measuring the Effective E:T ratio between target cancer cells eliminated and activated T cells (cancer-killing T cells), as described herein and referred to as Effective E:T ratio. For example, cancer cells can be identified by detection of cancer-specific cell markers, e.g., by using flow cytometry, and then quantified.

In embodiments, a candidate cancer treatment that leads to a greater extent of cancer-killing (e.g., lower numbers of cancer cells after treatment than before, or lower numbers of cancer cells compared to samples containing a negative control treatment) (e.g., lower numbers of cancer cells by at least 10% (e.g., 10%, 20%, 30%, 40%, 50%, 60%, 70%, 80%, 90%, 1.5-fold, 2-fold, 5-fold, 10-fold, 20-fold, 50-fold, 100-fold, or more) in the assay indicates that the patient is likely to be sensitive to or responsive to the cancer treatment, i.e., the cancer treatment is likely to effectively kill cancer cells and/or reduce tumor burden in the patient.

In embodiments, a candidate cancer treatment that leads to a greater extent of cancer-killing (e.g., lower numbers of cancer cells after treatment than before, or lower numbers of cancer cells compared to samples containing a negative control treatment) (e.g., lower numbers of cancer cells by at least 10% (e.g., 10%, 20%, 30%, 40%, 50%, 60%, 70%, 80%, 90%, 1.5-fold, 2-fold, 5-fold, 10-fold, 20-fold, 50-fold, 100-fold, or more) in the assay indicates that the cancer treatment(s) are likely to be effective in treating the cancer in the patient.

In embodiments, the method further comprises preparing and/or providing a report of the responsiveness of the patient to various candidate cancer treatments. In embodiments, the report is provided to a patient or to another person or entity, e.g., a caregiver, *e.g.*, a physician, *e.g.*, an oncologist, a hospital, clinic, third-party payor, insurance company or government office. In another embodiment, the report is provided to a party responsible for interpreting or determining the effect of the candidate cancer treatment on cancer cells (e.g., extent of cancer-killing).

In embodiments, the report can be in an electronic, web-based, or paper form. The report can include an output from the method, *e.g.*, the identification of cancer cells, the quantification of cancer cells, and the extent of cancer cell killing corresponding to each cancer treatment or combination of cancer treatments.

In one embodiment, a report is generated, such as in paper or electronic form, which identifies the extent of cancer cell death and the associated cancer treatment that led to the effect.

Such information can include information on potential or suggested cancer treatments. The report can include information on the likely effectiveness of a cancer treatment, the acceptability of a cancer treatment, or the advisability of applying the cancer treatment to the patient. For example, the report can include information, or a recommendation on, the administration of a cancer treatment, *e.g.*, the administration at a preselected dosage or in a preselected treatment regimen, *e.g.*, in combination with other drugs, to the patient. In an embodiment, not all candidate cancer treatments tested in the method are identified in the report. For example, the report can be limited to cancer treatments likely to be effective in the patient. In other examples, the report can omit cancer treatment unlikely to be effective in the patient. The report can be delivered, *e.g.*, to an entity described herein, within 3 - 21 days (e.g., 3, 4, 5, 6, 7, 14, or 21 days) from receipt of the sample by the entity practicing the method.

## **EXAMPLES**

### Example 1. Effect of bispecific antibodies in multiple myeloma (MM)

In embodiments, samples obtained from patients with hematological malignancies are useful for testing the effect of a BsMAb to a specific tumor target on malignant cells and a T cell target (CD3) on CTLs, as such samples contain both the target (malignant) cells and the effector (CTLs) cells. Bone marrow (BM) samples were obtained from patients who had been diagnosed with multiple myeloma. All samples were from adult patients, over 18 years of age, who gave informed consent for study participation.

Bispecific monoclonal antibodies (BsMAb) and control antibodies were added to 96-well plates. Each BM sample was diluted with culture media, in its entirety (i.e., without first separating cells or other components from the BM), and plated into the 96-well plates comprised of wells containing either the BsMAb, control antibodies, or neither, with all conditions tested in duplicate. Samples were incubated for 96 hours to promote the proliferation of the CTLs in the sample. Just prior to analysis, the red cells were lysed, and antibodies that define the malignant cell population and the CTL population were added to the plates: CD138-PE (Cytognos, Ref: CYT-138PE4, clone B-A38) / CD38PerCP (Immunostep, Ref: 38PP-100T, clone GR7A4) / CD45-PB (BD-Pharmingen, Ref: 560367, clone 30-F11) / Annexin V-FITC (Immunostep, Ref: ANXVF) / CD56-APC (BD-Pharmingen, Ref:341027, clone NCAM16,2) / CD3PE (Cytognos, Ref: CYT-3PE5, clone 33-2A3) / CD4PB (IMMUNOSTEP, Ref: 4CFB-100T, clone HP2/6) / CD8 FITC(Cytognos, Ref: CYT-8F8, clone UCHT-4) / CD25 APC (Biolegend, Ref: 302610, clone BC96) / CD69 PerCP (Biolegend, Ref: 310928, clone FN50). Annexin V-FITC was added to monitor the level of apoptosis. Plates were analyzed using the ExviTech flow cytometry based platform. Cells were identified using a gating strategy based on forward scatter (FSC), side scatter (SSC) and the expression of various surface markers including CD38, CD138 and CD56. The percentage of live cells was determined by comparing the number of live cells in wells with the BsMAb to the number of live cells in control-well without the BsMAb.

**Figure 6** displays a graph of a representative sample showing both the count of the malignant cells and CTLs after the 96-hour incubation with various concentrations of the BsMAb (drug) displayed on the x-axis. There was a BsMAb dose-response effect in the increase in proliferation of the CTLs (continuous line, triangles) that corresponded to a decrease of the malignant cell population (dotted line, squares). The decrease in the malignant cell population occurred through a mechanism separate from apoptosis, suggesting a non-apoptotic cell death consistent with T cell lysis. Control mAbs that target only the malignant cells or the T cell population have no effect. For this sample the basal (without BsMAb) E:T ratio is 1:2.7, different than the Effective (with BsMAb) E:T ratio that is 1:6.1. This demonstrates that the functionality of BsMAbs can be assessed *ex vivo* and that the proliferation of CTLs was promoted in whole bone marrow samples in the presence of the BsMAb (with one arm being anti CD3), without having to previously isolate the lymphocytes from the whole bone marrow sample.

### Example 2. Effect of BsMAbs on Target and Effector Cells in chronic lymphoid leukemia (CLL)

In this example, the effect of BsMAbs on target and effector cells was tested in a different indication and a different sample type than those tested in Example 1. Peripheral blood (PB) samples from patients who have been diagnosed with CLL were used. All samples were from adult patients, over 18 years of age, who gave informed consent for study participation.

A BsMAb that targets CD19 on malignant cells and CD3 on CTLs as well as control antibodies were added to 96-well plates. Each PB sample was diluted with culture media, in its entirety (i.e., without first separating cells or other components from the PB), and plated into the 96-well plates comprised of wells containing either the BsMAb, control antibodies, or neither, with all conditions tested in duplicate. Samples were incubated for 144 hours to promote the proliferation of the CTLs in the sample. Just prior to analysis, the red cells were lysed, and antibodies that define the malignant cell population and the CTL population were added to the plates. An 8 color-panel was used to determine the effect of the CD3/CD19 BsMAb on each live population: CD3PB (BD-Pharmingen, Ref:558117, clone UCHT1) / CD45-PO (Invitrogen, Ref: MHCD4530, clone HI30) / Annexin V-FITC (Immunostep, Ref: ANXVF) / CD19-PE (Ebioscience, Ref: 12-0199-42, clone HIB19) / CD4-PECY5 (Biolegend, Ref: 317412, clone OKT4) / CD5-PECY7 (Biolegend, Ref: 300622, clone UCHT2) / CD8 APC (BD, Ref: 555369, clone RPA-T8) /CD25 APCH7 (BD-Pharmingen, Ref: 560225, clone MA251). Annexin V-FITC was added to monitor the level of apoptosis and fully quantitate the number of live cells. The number of live leukemic B cells (CD45+CD19+, squares) and the number of live CTLs (CD8+CD25+, triangles) are displayed on Y-axis. The X-axis represents 8 concentrations of the CD3CD19 BsMAb (ng/ml). Plates were analyzed using the ExviTech flow cytometry based platform. Cells were identified using a gating strategy based on forward scatter (FSC), side scatter (SSC) and the expression or lack of expression of the various surface markers. The percentage of live cells was determined by comparing the number of live cells in wells with the BsMAb to the number of live cells in control well without the BsMAb.

**Figure 7** displays a graph of a representative sample showing both the count of the malignant B-cells and CTLs after the 144-hour incubation with various concentrations of the BsMAb (drug). There was a dose-response effect in the increase in proliferation of the CTLs (triangles) that correspond to a decrease of the malignant cell population (squares). The decrease in the malignant cell population occurred through a mechanism separate from apoptosis, suggesting a non-apoptotic cell death consistent with T cell lysis. Control mAbs that target only the malignant cells or the T cell population had no effect. For this sample the basal (without BsMAb) E:T ratio is 1:30.1, different than the Effective (with BsMAb) E:T ratio that was 1:5.7. These results demonstrate that the functionality of BsMAbs can be assessed *ex vivo* using whole peripheral blood as well as whole bone marrow. The proliferation of CTLs was promoted in whole PB samples in the presence of the CD19/CD3 BsMAb without having to previously isolate the lymphocytes from the PB sample.

Example 3. Effect of BsMAbs on Target and Effector Cells in acute myeloid leukemia (AML)

In this example, the effect of BsMAbs on target and effector cells was tested in a different indication than those of Examples 1 and 2. Bone marrow (BM) samples were obtained from patients who had been diagnosed with AML. All samples were from adult patients, over 18 years of age, who gave informed consent for study participation.

The BsMAb that targets CD123 on malignant cell and CD3 on the T cell population, as well as control antibodies, were added to 96-well plates. Each BM sample was diluted with culture media, in its entirety (i.e., without first separating cells or other components from the BM), and plated into the 96-well plates comprised of wells containing either the BsMAb, control antibodies, or neither, with all conditions tested in duplicate. Samples were incubated for 120 hours to promote the proliferation of the CTLs in the sample. Just prior to analysis, the red cells were lysed, and antibodies that define the malignant cell population and the CTL population were added to the plates: CD3PB (BD-Pharmingen, Ref:558117, clone UCHT1) / CD45-PO (Invitrogen, Ref: MHCD4530, clone HI30) / Annexin V-FITC (Immunostep, Ref: ANXVF) / CD117-PE (Biolegend, Ref: 313204, clone 104D2) or CD33PE (BD-Pharmingen, Ref: 555450, clone WM53 / CD4-PECY5 (Biolegend, Ref: 317412, clone OKT4) / CD5-PECY7 (Biolegend, Ref: 300622, clone UCHT2) / CD8 APC (BD, Ref: 555369, clone RPA-T8) /CD25 APCH7 (BD-Pharmingen, Ref: 560225, clone M-A251). Annexin V-FITC was to monitor the level of apoptosis.

Plates were analyzed using the ExviTech flow cytometry based platform. Cells were identified using a gating strategy based on forward scatter (FSC), side scatter (SSC) and the expression or lack of expression of the various surface markers. The percentage of live cells was determined by comparing the number of live cells in wells with the BsMAb to the number of live cells in control well without the BsMAb.

**Figure 8** displays graphs of three representative samples showing both the count of the malignant cells (Live blast cells) and CTLs (CD8+CD25+) after the 120-hour incubation with various concentrations of the BsMAb (drug) as displayed on the x-axis. There was a dose-response effect in the increase in proliferation of the CTLs (grey line, triangles) that corresponded to a decrease of the malignant cell population (black line, squares) for each sample. The decrease in the malignant cell population occurred through a mechanism separate from apoptosis, suggesting a non-apoptotic cell death consistent with T cell lysis. Control mAbs that target only the malignant cells or the T cell population have no effect. In this example, the level of decrease in target cells (AML blast cells) varied from sample to sample, as did the increase in the effector cell (malignant blast cell) population. The Effective E:T ratio also varied between the different patient samples, from 1:0.8 to 1:7.1, which means that for one sample, less than 1 cancer is killed by each activated T cell (1:0.8), while for another sample, more than 7 cancer cells are killed by each activated T cell (1:7.1). This demonstrates a way to identify the patients that have a favorable Effective E:T ratio, the ratio after incubation with the BsMAb. In embodiments, a favorable Effective E:T ratio can correspond to a patient for which Adoptive Cell Therapy may be particularly effective. This further demonstrates that the functionality of BsMAbs could be assessed *ex vivo* and that the proliferation of CTLs was promoted in whole bone marrow samples in the presence of the BsMAb (with one arm being anti CD3), without having to previously isolate the lymphocytes from the whole bone marrow sample.

Example 4. Flow Cytometric Identification of a Trogocytotic Effector Cell Population Ideal for ACT

In some embodiments, a population of T cells that displayed BsMAb dose-dependent proliferation could potentially have markers for both CTLs and the malignant plasma cell population. In such embodiments, this population of CTLs may have actively engaged malignant cells, and when lysing the malignant cells, some of the cell surface markers of the malignant cells can become embedded in the membrane of the CTL in the trogocytosis process. This population is referred to herein as "trogocytotic CTLs," and they are a subset of the activated T cells that interact with, and most likely eliminate, the malignant cells. As the CTLs experience close contact with the malignant cells, in some embodiments, they are exposed to specific antigens on the malignant cells and thus would be primed to generate receptors specific to these antigens. In other embodiments, these CTLs recognize a plurality of antigens in the malignant cells. Without wishing to be bound by theory, it is believed that this population of trogocytotic CTLs could be expanded and used in the recognition (and killing) of malignant cells in a host.

To demonstrate this effect, a BM sample from an AML patient was analyzed with a full array of antibodies against CD markers to delineate the malignant cells and markers to identify subsets of the lymphocytic cell populations. Wells were evaluated for the presence of a trogocytotic population defined as T-cells (CD3+) expressing aberrant blast markers (CD117+) in the membrane surface after a 120 hours CD123/CD3 BsMAb exposure in the AML sample.

**Figure 9A** shows a gate (square) that defines the T-cells population (grey color) using side scatter (SSC) and CD3 expression. **Figure 9B** shows a dot plot after 120-hour incubation in the absence of the BsMAb of the T-cell (CD3+/CD117-) population (square gate, grey color) and AML blast cell (square gate, black color) population (CD117+/CD3-). In the absence of the BsMAb there is no apparent trogocytotic cells as would be seen in the circular gate that depicts a CD117+/CD3+ population. **Figure 9C** shows the same gates as Figure 9B but after a 120-hours incubation in the presence of the CD123/CD3 BsMAb. At this time point and in wells containing a concentration of the BsMAb > 0.05 $\mu$g/ml, there was a clear elimination of the malignant blast cells (CD117+/CD3-), and a clear increase in the numbers of T-cells (CD3+/CD117-). Additionally, the appearance of a new T-cell population (CD117+/CD3+, circle), expressing the AML blast cell marker CD117 was observed. This population is defined as the trogocytotic CTL population.

The effectiveness of various pICFs, e.g., various BsMAbs, can be tested *ex vivo,* e.g., in an assay such as that described in this example. In embodiments, the existence of a CTL population that may be superior in targeting malignant cells can be detected. In embodiments, this population can be expanded, and the CTL cell preparation can be used as a medicament, particularly in the ACT treatment of malignancies, e.g., hematological malignancies.

Example 5. Evaluation of the killing capacity of the CTL population generated through the use of a BsMAb antibody

In some embodiments, trogocytotic CTLs generated through the use of a BsMAb can have a greater capacity to eliminate a malignant cell population from the same host. In embodiments, the trogocytotic population would be isolated from the rest of the sample, for example by using Fluorescence Activated Cell Sorting (FACS). To examine this, cryopreserved samples containing the leukocytes of a patient diagnosed with AML were tested.

Figures 10A, 10B, and 10C show the flow cytometric dot plots (CD117vsCD3) of an experiment demonstrating the killing capacity of a newly generated CTL population after exposure to a CD123/CD3 BsMAb for 120 hours. First, one tube of cryopreserved cells from an AML patient was exposed to a concentration of 0.875 $\mu$g/ml of the CD123CD3 BsMAb for 120 hours. The resultant cells were washed to remove any excess BsMAb, and the cells were incubated overnight at 37°C with 5% $CO_2$ with humidity. A second tube of cryopreserved cells from the same patient was thawed and this sample was mixed with the sample that was previously generated by exposure to the BsMAb.

**Figure 10A** shows the dot plot (CD117vsCD3) of the results from the first AML sample after a 120 hour BsMAb exposure, where the AML blast cells (CD117+/CD3-) have been eliminated leaving only the newly generated T-cells (CD3+/CD117).

**Figure 10B** shows the dot plot (CD117/CD3) of the new cryopreserved vial from the same patient with the presence of blast cells (CD117+/CD3-) and a low level of residual T-cells (CD117-/CD3+). The sample of cells shown in Figure 10A looked similar to Figure 10B prior to exposure to the BsMAb. Once the samples were mixed, they were incubated for 2 hours, 4 hours and 24 hours, in the presence and absence of 0.875 $\mu$g/ml of CD123/CD3 BsMAb. The newly thawed cells were also tested alone, without the addition of the pretreated (Figure 5A) cells.

**Figure 10C** shows the dot plot (CD117/CD3) of the mixture of the Figure 10A and Figure 10B cell population at the 0-time point, where the presence of both T cells (CD3+/CD117-) and new AML blast cells (CD117+/CD3-) can be seen.

**Figure 10D** is the dot plot (CD117/CD3) after a 24-hour incubation, where clearly, the AML blast cells (CD117+/CD3-) were depleted in the presence of the previously generated T-cell population. This was done in the absence of the CD123/CD3 BsMAb. The previously generated T cell population was able to kill the AML blast population without assistance from the BsMAb. This demonstrates that the CTLs generated by an initial exposure to both the BsMAb and malignant cells retained the ability to kill the malignant population in the absence of the BsMAb.

The graph in **Figure 11** shows the kinetic effects of the previously generated CTL to deplete the AML blast cells from the new vial over time. The medium gray dashed line represents the control population of the AML blasts (from Figure 10B) without the presence of the T cells from Figure 10A. The results show a slight spontaneous depletion of the blast population over time. The black circles represent the AML blast cells that were mixed with the T cells from Figure 10A. There was a very rapid depletion of the AML blast cells by the previously generated T cell population, with almost 50% of the blast gone in the first 2 hours. By the 24-hour point, almost 100% of the Blast population was depleted. Addition of the CD123/CD3 BsMAb (light grey squares) had no effect. These results happened over a time period that is too short for it to be the result of the T cell population present in the new vial (the cells in Figure 10B).

Example 6. Measurement of activity of activated T cells induced by pICF by dose responses of different E:T ratios

A method to measure the activity of a drug is to generate dose response curves evaluating its activity at different concentrations, fitting the data to operational pharmacodynamic mathematical models that describe the activity and enables its quantitation by standard parameters.

Incubating T cells and tumor cells with a pICF induces the activation and proliferation of cancer-killing T cells, which are the medical agent that kills tumor cells.

In a first step, the activated cancer-killing T cells are generated by incubating (e.g. for 120 hours) a patient sample (e.g. blood, bone marrow) with the pICF, resulting in a lower number of tumor cells because the cancer-killing T cells eliminate a number of tumor cells. In a subsequent step, in some cases, the pICF can be optionally washed away, and the cancer-killing T cells groups, CD4+CD25+ and CD8+CD25+, can be optionally sorted by FACS (Fluorescence Activated Cell Sorter) as pure populations. Alternatively, the same scheme can be used without washing away the pICF and/or without sorting by FACS.

A second patient sample, e.g., aliquot of the same patient sample, can then be added to the groups of cancer-killing T cells and isolated, or added to the entire batch of leftover cells. Because the cancer-killing T cells represent the medicinal agent, adding it in different amounts, in particular in different E:T ratios, to the second aliquot (the patient sample), enables measuring dose response curves and use of standard pharmacodynamics modeling approaches. In some examples, the number of tumor cells is fixed and the number of cancer-killing T cells added is varied to generate a dose response curve at different E:T ratios. This is a pharmacodynamics approach to measure drug activity.

This example also describes the use of a bispecific antibody, CD3xCD123, as pICF, on blast cells from an AML sample. T-Lymphocytes were generated from a frozen AML sample after a 120-hour incubation at 37°C in humidified air containing 5% $CO_2$ and the presence of the CD123xCD3 BITE (Creative Biolabs). After this period, activated (CD25+) T-cells (CD8+ and CD4+) were sorted by fluorescence activated cell sorting (FACS sorting) using a FACS Aria III flow cytometer (BD). The purity of the sorted cell populations was higher than 99%. Once the effector T-cells were purified, a new vial from the same patient was thawed to evaluate the cytotoxicity of the sorted populations.

The effector T-cells (CD8+CD25+ or CD4+CD25+ or both simultaneously in the same 50/50 proportion) were mixed with a constant number of blast cells at different effector:target (E:T) ratios starting at a ratio close to 1:1. Both cell types (effectors and targets) were seeded for another 24h incubation at 37°C in humidified air containing 5% $CO_2$ without the presence of the CD123xCD3 BITE.

**Figure 12** demonstrates the killing capacity of the sorted CD8+CD25+ or CD4+CD25+ cells after CD123xCD3 BITE exposure in a cell dose dependent manner, reaching the maximum killing capacity in a ratio close to 1:1. The x-axis represents the E:T ratio (**Figure 12A**) or the number of activated T cells (Figure 12B). The graphs are identical in both representations, given that the number of blasts is fixed, and the y-axis represents the percentage of live blast cells after a 24-hour incubation at the different ratios or T cell counts.

Different pharmacodynamic modelling and simulations strategies are used to characterize the immunomodulation and tumor cell depletion activities. These models permit the estimation of typical population parameters as well as an accurate evaluation of activities for each single individual. Simplest models evaluate the relationship between the activation of T cells and the depletion of tumor cells, or the direct relationship of any of these variables with the drug concentration. More complex situations can be modelled, such as the relationship of the final response with the basal E:T ratio (NK), or the ratio of the maximum number of activated T cells (CD8+CD25+ or CD4+CD25+) and basal blasts, or the ratio of activated T cells and depleted blasts at a particular reference drug concentration point (including the Effective E:T ratio described herein). The relationship accounts for the effect at different concentrations of the drugs and different incubation times. If measurements are limited to one per sample, such as only the original E:T ratio as it arrives, or the maximum number of activated T cells, different modelling approaches may be used. For example, a population modelling approach can be used, where the measurements from each sample contributes to build up a continuous model function valid for all samples. Nested models can also be applied where the dependent variable for one model (e.g., activation of T cells versus drug concentration and time) is used as an input for a second model where a second variable is analyzed (e.g., tumor cell depletion versus activated T cells).

Standard pharmacodynamics models can be applied to fit the data sets described herein, e.g., in this example, to operational models to quantify their respective activities. Because cancer-cell killing results from binding of T cell receptors in activated T cells to tumor target cells, simple models of ligand-receptor binding are applied. Equilibrium models are used to fit binding data (y) as a function of the ligand concentration (x, T cell counts). A maximum binding capacity ($BL_{max}$) and the affinity for binding of the receptor ($K_d$) are used. This model follows the hyperbolic equation:

$$y = C + BL\max\left(1 - \left(\frac{x}{Kd + x}\right)\right)$$

The data points for each of the 3 subsets of cancer-killing T cells (CD8+CD25+, CD4+CD25+ and CD8+CD25+/CD8+CD25+) fitted with this model are shown in **Figure 13 (A-C).** The fitted graphs shown in **Figure 13** show a significant difference in the activity between these 3 subsets. This difference can be appreciated after quantifying the activity, enabled by the fitting effort. **Table 1** below shows the standard activity values of each T cell subset; potency or IC50, the concentration (number of activated T cells) inducing 50% tumor cell killing. $E_0$ or % survival at the point the fitted curve crosses zero on the X-axis, i.e. in the absence of T cells, similar but not identical to the control wells without activated T cells added. Emax or % survival at maximum dose of activated T cells. AUC (Area Under the Curve) integrates the area under the fitted curve (limited from 0 to 1000 T cells to avoid artifacts at very large and unrealistic T cell numbers or ratios). The AUC captures the overall accumulated activity of all activated T cells. There was significantly higher potency of CD8+CD25+ versus CD4+CD8+: 67 versus 336, or 5-fold. This means that to kill the same number of tumor cells, it takes 5 times less T cells for CD8 vs CD4 activated T cells. However, the CD8 subset leaves 13.7% of the tumor cells alive while the CD4 subset kills all of them. The AUC from CD8 is substantially less than the AUC from CD4. The mixture of CD4/CD8 at equal proportions has intermediate values. Thus, CD8+CD25+ appears better at killing cancer cells, although the CD4+CD25+ subset adds additional activity in terms of killing all cancer cells, including some (13.7%) that CD8+CD25+ cells could not kill in this 24 h timeframe.

**Table 1**

|  | IC50 (T-Cells#) | E0 (Survival %) | Emax (% Survival) | AUC (0-1000) |
|---|---|---|---|---|
| CD8+CD25+ | 67 | 84.8 | 13.7 | 29392.1 |
| CD4+CD25+ | 336 | 96.5 | 0.0 | 44769.0 |
| (CD4+CD25+)+(CD8+CD25+) [1:1] | 164 | 138.4 | 0.0 | 44499.9 |

This example shows the power of modelling approaches, which are rarely used in immuno-oncology *ex vivo* assays. Quantification of the cancer-killing activity by these models empower the *ex vivo* assays to be more precise, making them better assays to screen new and combinations of ICFs and/or immunomodulators, better assays to personalize immune-oncological drug treatments, alone or in combinations, and better assays to identify the appropriate cancer-killing T cells for Autologous Cell Therapy, alone and in combinations. Pharmacodynamic modelling and simulations have been widely applied to different phases of drug development. See, e.g., Upton RN (2014) and Mould DR (2013).

Multiple applications have been described as well in oncology. See, e.g., Buil-Bruna N (2016). Described herein are pharmacodynamic modelling and simulations strategies to characterize immunomodulation and tumor cell depletion activities. Structural models can cover standard response exposure relationships through lineal, sigmoid or log-lineal models and including covariates studies. See, e.g., Upton RN (2014), Mould DR (2013) and The Guidance for Industry Population Pharmacokinetics (1999).

For example, models described above evaluate the relationship between the activation of T cells and the depletion of tumor cells, as well as the direct relationship of any of these variables with the drug concentration. Additional models study the relationship of the final response with the relative E:T ratios using activated cells as used above, or the ratio of the maximum number of activated T cells (CD8+CD25+ or CD4+CD25+) and basal blasts, or the ratio of activated T cells and depleted blasts at a particular reference drug concentration point (including the Effective E:T ratio proposed herein). Models will be developed considering different covariates as the effect at different drug exposure levels or different incubation times. These models allow for estimation of typical population parameters as well as accurate estimation of activities for each single individual.

There are modelling approaches also without measuring multiple E:T ratios as in the example shown above. For instance, when only using the data that results from the initial incubation with the pICF without adding a second part of sample varying conditions. In these cases, only one value of several parameters such as the Effective E:T Ratio is available. Yet this data has less manipulation than mixing different E:T ratios on a second part of the sample and is thus also considered. The possible limitation that only one ratio is available per sample can be overcome through a single-through sample design where the single data point from each sample contributes to build up a continuous model function valid for all samples.

Example 7. Measurement of activity of ICF and/or Immunomodulators by measuring their effect in dose response curves on activated T cells induced by pICF

Exemplary immunomodulators are described herein, e.g., and include immune checkpoint inhibitors (PD1 or CTL4), Treg inhibitors, and MDSC inhibitors.

A similar scheme as described above, adding a second patient sample, e.g., second aliquot of the same patient sample, after incubation with the pICF has generated activated cancer-killing T cells, is used to evaluate other components of the immune-oncology response. E:T ratios described herein, can be added and can also include a potential ICF or immunomodulator.

This scheme is applicable whether or not the molecule has activity in enhancing the cancer-killing activity by itself. Such activity can be better observed once the activated T cells have been generated by the pICF. For example, immune check point molecules such as PD1 or CTL4 become expressed on the cell surface of T cells only after several days of incubation with a pICF. Thus, a suitable assay is to add immune check point inhibitors such as anti-PD1 antibodies after their targets have been expressed on the T cell surface. For example, the dose response curves can be measured with or without a dose of an immune check point inhibitor, such as anti-PD1 antibodies. If adding this anti-PD1 antibody enhances the immune response towards the tumor cells, by increasing the number of CD4 and/or CD8 activated cells, and/or by improving the E:T ratios, and/or by enhancing the killing activity of these T cells, the immune checkpoint inhibitor, e.g., anti-PD1 antibody is considered an ICF. Enhancement of activity can be measured by specific parameters in a pharmacodynamics models as shown in Example 6, such as EC50, Emax, and AUC. If a molecule such as an anti-PD1 antibody enhances the activity of cancer killing T cells, it is considered an ICF.

Different ICFs or immunomodulators act on cell types other than the activated T cells or tumor cells, as described above. Examples are immunosuppressive regulatory T cells called Tregs, and myeloid derived suppressor cells (MDSCs). To measure the activity of these compounds as ICFs or immunomodulators, this same assay can be used, i.e., adding a fixed dose of these compounds to the dose response of activated T cells. Sometimes, the number of Tregs and MDSCs in these *ex vivo* samples might be low or insufficient for such measurements. In these cases, the scheme can be further extended by adding to the second part of the sample a fixed amount of Tregs or MDSCs, or both, that is sufficient to measure the effect of these ICF and immunomodulator compounds properly.

In some examples, sorted and/or purified Tregs and/or MDSCs (which negative regulate the cytotoxic effect or cell expansion of T cells) can be added to the co-culture. Once the different populations (activated T cells after BITE exposure, Tregs or MDSC) are FACS-sorted, the sorted cells can be mixed in a co-culture experiment, varying the ratio from each one (T cells:Tregs:Blasts; or T cells:MDSC:Blasts) and evaluating the lysis capacity of the T cells, e.g., in frozen vials in the presence of these suppressive cells.

In addition, immune checkpoint inhibitors (e.g., as described herein, e.g., inhibitors of PD-1 and/or CTL4) can be added to the different co-cultures models to evaluate their effects, e.g., evaluated their ability to enhance blast lysis or T-cell expansion.

The methods described herein are efficacious assays to screen new ICFs or immunomodulators, compare them directly against each other or in various combinations (e.g., among themselves and/or with other type of molecules such as standard of care).

Example 8. Selecting and/or enriching activated CTLs

In this example, the ability to select and enrich a population of activated CTLs is demonstrated. Two BM samples were obtained from patients diagnosed with AML. Samples were from adult patients, over 18 years of age, who gave informed consent for study participation.

A part of each sample was prepared and tested to determine the Basal number of activated CTLs present. Prior to analysis, RBCs were lysed and antibodies that define the active CTL population were added to the plates (CD138-PE (Cytognos, Ref: CYT-138PE4, clone B-A38) / CD38PerCP (Immunostep, Ref: 38PP-100T, clone GR7A4) / CD45-PB (BD-Pharmingen, Ref: 560367, clone 30-F11) / CD56-APC (BD-Pharmingen, Ref:341027, clone NCAM16,2) / CD3PE (Cytognos, Ref: CYT-3PE5, clone 33-2A3) / CD4PB (IMMUNOSTEP, Ref: 4CFB-100T, clone HP2/6) / CD8 FITC(Cytognos, Ref: CYT-8F8, clone UCHT-4) / CD25 APC (Biolegend, Ref: 302610, clone BC96) / CD69 PerCP (Biolegend, Ref: 310928, clone FN50)), to monitor the level of apoptosis, Annexin V-FITC (Immunostep, Ref: ANXVF) was also added.

Plates were analyzed using the ExviTech flow cytometry based platform. See, e.g., US 8,703,491, US 8,313,948 and Bennett TA (2014).

Bone marrow samples were diluted with culture media, in their entirety (i.e., without first separating cells or other components from the BM), and plated into 96-well plates comprised of wells containing either CD123/CD3 BsMAb (Creative Biolabs, Shirley, NY), control antibodies, or neither, with all conditions tested in duplicate. Samples were incubated for 120 hours to promote the proliferation of the CTLs in the sample. At the end of the incubation period the plates were processed as outlined above and analyzed using the ExviTech. Additionally, a portion of each sample was sorted by fluorescence activated cell sorting (FACS sorting) using a FACS Aria III flow cytometer (BD, San Jose, CA) to enrich the activated CTL population. Gates were set to sort cells that were both CD5 and CD25 positive.

The results are shown in Figure 14, where the dot plots from sample 1 display CD5 intensity on the X-axis and CD25 intensity on the Y-axis. **Figure 14A** is the Base point, showing a very low level of CD5+CD25+ cells (upper right quadrant). **Figure 14B** shows the sample after it has been incubated with the CD123/CD3 BsMAb, where a large percentage of the cells are activated CTLs, but other cell subtypes are still present. After this population was sorted (**Figure 14C**), over 99% of the cells are activated CTLs. The percentage of CD5+ and CD25+ cells for each sample and each condition is shown in **Figure 14DJ**. This demonstrates how activated CTL can be generated and enriched by selective sorting.

Example 9. Effect of trogocytic cancer-killing T cells

This example describes the use of a BsMAb as an pICF, on blast cells from an AML sample. Activated T-Lymphocytes were generated from an AML sample in which the leukocyte population, containing both malignant and normal cells, were previously isolated and cryopreserved. The sample was obtained from an adult patient, over 18 years of age, who gave informed consent for study participation.

The sample was thawed and mixed with culture media containing a CD123/CD3 BsMAb (Creative Biolabs, Shirley, NY). The sample was incubated for 120 hours at 37°C in humidified air containing 5% $CO_2$. The CD3 arm binds and help to activate a T cell, while the CD123 arm binds a malignant AML cell, bringing it into proximity with the now activated CTL.

After incubation, the sample was washed, then exposed to a panel of antibodies to identify activated CTLs that had undergone trogocytosis verses CTLs that had not. The antibody cocktail used was CD3PB (BD-Pharmingen, Ref:558117, clone UCHT1), CD25 APC (Biolegend, Ref: 302610, clone BC96), and CD123 (ref) along with Annexin V-FITC (Immunostep, Ref: ANXVF). CD3 and CD25 would identify the CTLs and CD123 is a marker on malignant AML cells that if present on a CTL would indicate a trogocytic CTL. The sample was sorted by fluorescence activated cell sorting (FACS sorting), using a FACS Aria III flow cytometer (BD), into two populations: CD3+CD25+CD123- (**Figure 15A**) and trogocytic CD3+CD25+CD123+ (**Figure 15B**). The purity of the sorted cell populations was higher than 99%. Once the effector T cells were purified, a new cryopreserved vial from the same patient was thawed to evaluate the cytotoxicity of the sorted populations.

The same number of effector T cells from both populations (CD123+ and CD123-) were mixed with cells from the newly thawed sample that contains malignant blast cells. Both cell types (effectors and targets) were seeded for another 24h incubation at 37°C in humidified air containing 5% $CO_2$ without the presence of the CD123/CD3 BsMAb. As can be seen in the **Figure 15C,** the trogocytic CTLs (CD123+) population killed about 10% more malignant blast cells than the CD123- population of cells (61% vs 72% survival). As even this simple preparation conferred some advantages optimization of the different parameters of this experiment may show more distinct advantages.

Example 10. Inter-patient variability in BsMAb induced generation of CTLs and AML blast depletion

This example evaluates the *ex vivo* effect of a CD123/CD3 BsMAb (Creative Biolabs, Shirley, NY) on 17 BM samples from AML patients. All samples were from adult patients, over 18 years of age, who gave informed consent for study participation.

Samples were received within 24-48 hours after collection. BM samples were diluted with culture media, in their entirety (i.e., without first separating cells or other components from the BM), and plated into 96-well plates comprised of wells containing either CD123/CD3 BsMAb, control antibodies, or neither, with all conditions tested in duplicate. Samples were incubated for either 72, 96 or 120 hours. Prior to analysis, RBCs were lysed and antibodies that define the active CTL population (CD3PB (BD-Pharmingen, Ref:558117, clone UCHT1) / CD4-PECY5 (Biolegend, Ref: 317412, clone OKT4) / CD5-PECY7 (Biolegend, Ref: 300622, clone UCHT2) CD8 APC (BD, Ref: 555369, clone RPA-T8) /CD25 APCH7 (BD-Pharmingen, Ref: 560225, clone M-A251) / CD45-PO (Invitrogen, Ref: MHCD4530, clone HI30)) and the AML blast population (CD33PE (BD-Pharmingen, Ref: 555450, clone WM53 or CD117-PE (Biolegend, Ref: 313204, clone 104D2)) were added to the plates, to monitor the level of apoptosis, Annexin V-FITC (Immunostep, Ref: ANXVF) was also added. Plates were analyzed using the ExviTech flow cytometry based platform. See, e.g., US 8,703,491 B2, US 8,313,948 B2, and Bennett et al. Clin. Lymphoma, Myeloma, and Leukemia. 14.4(2014):305-18.

The CD123/CD3 BsMAb binds CD123 on myeloid cells, including malignant AML blast cells, and recognizes CD3 on T cells. Binding of CD3 and proximity of the AML blasts activates CTLs resulting in lysis of the AML blasts. As the BM samples from the AML patients contain both AML blast cells and T cells, the effect of the BsMAb can be measured on both populations simultaneously in each sample. The CD123/CD3 BsMAb demonstrated AML blast killing in a time- and dose-dependent manner (**Figure 16A**). Similarly, there is also a time- and dose-dependent T-cell activation and proliferation of CTLs (**Figure 16B**). Each line in the graphs represent an individual patient. From these results, it can be seen that there is a very high level of interpatient variability in both the kinetics and efficiency of the blast depletion (A) and the proliferation of the CLT population (B). This type of kinetic analysis, where pharmacological data can be determined from dose-response data (e.g. EC50 and Emax) over several time periods, may be useful as another quantitative assessment of the effectiveness of a BsMAb or other ICFs. Further work needs to be done to see if there is a correlation between these results and the clinical response of the patients, however, this type of data may someday be used to determine if a specific BsMAb may be fit for a specific patient.

Example 11. Measurement of BsMAb activity in Minimal Residual Disease (MRD) sample

A bone marrow (BM) sample was obtained from an AML patient with MRD. Sample was from an adult patient, over 18 years of age, who gave informed consent for study participation.

The sample was tested upon receipt and the AML blast population was found to be less than 5% of monocytic population. The BM sample was diluted with culture media, in its entirety (i.e., without first separating cells or other components from the BM), and plated into 96-well plates comprised of wells containing either CD123/CD3 BsMAb, control antibodies, or neither. As the quantity of the sample was small, only a single dose of CD123/CD3 BsMAb was tested, 3 $\mu$g/ml, at 3 time points, 72, 96 and 120 hours. All conditions were tested in triplicate.

Just prior to analysis, at each time point, the red cells were lysed, and antibodies that define the malignant cell population and the CTL population were added to the plates: CD3PB (BD-Pharmingen, Ref:558117, clone UCHT1) / CD45-PO (Invitrogen, Ref: MHCD4530, clone HI30) / Annexin V-FITC (Immunostep, Ref: ANXVF) / CD117-PE (Biolegend, Ref: 313204, clone 104D2) or CD33PE (BD-Pharmingen, Ref: 555450, clone WM53 / CD4-PECY5 (Biolegend, Ref: 317412, clone OKT4) / CD5-PECY7 (Biolegend, Ref: 300622, clone UCHT2) / CD8 APC (BD, Ref: 555369, clone RPA-T8) /CD25 APCH7 (BD-Pharmingen, Ref: 560225, clone M-A251). Annexin V-FITC was to monitor the level of apoptosis.

Plates were analyzed using the ExviTech flow cytometry based platform. See, e.g., See, e.g., US 8,703,491, US 8,313,948, and Bennett TA (2014). Cells were identified using a gating strategy based on forward scatter (FSC), side scatter (SSC) and the expression or lack of expression of the various surface markers.

Figure 17 represents in Y-axis the absolute cell count of both the AML blast cells (**Figure 17A**) or activated CTL cells (CD25+, **Figure 17B**) over time (X-axis). The dashed line represents the control sample without BsMAb and the solid line represent the CD123/CD3 BsMAb data. As can be seen in Figure 17, even when the percentage of the tumor cells is minimally expressed in the whole sample, activated CTLs are still generated in a time dependent manner (Figure 17B). Additionally, the few blast cells present are also depleted in a time-dependent manner (Figure 17A).

Example 12. Characterization of Human T Lymphocyte populations before and after exposure to a BsMAb

This example evaluates the phenotype of human T Lymphocytes after a BsMAb exposure. Bone marrow (BM) samples were extracted from 4 patients diagnosed with AML. Samples were from adult patients, over 18 years of age, who gave informed consent for study participation.

Samples were diluted in culture medium (Roswell Park Memorial Institute (RPMI) 1640 (Sigma, St Louis, MO) media supplemented with 20% (vol/vol) FBS (Thermo Scientific, Waltham, MA), 2% Hepes, 1% antibiotic (Zell Shield, Labclinics, Barcelona, Spain) and 1% L-Glutamine 200mM (Lonzo, Hopkinton, MA)), the CD123/CD3 BsMAb (Creative Biolabs) was added at a concentration of 0.21875$\mu$g/ml. Samples were incubated for 120 hours to promote the proliferation of the CTLs in the sample.

In order to characterize the T-cell phenotype, the following labeling antibodies were used: CD4 (Immunostep, Salamanca, Spain), CD27 (BD Pharmingen), CD8 (Cytognos, Salamanca, Spain), CD62L (BD Pharmingen,), CD45RA (QuantoBio, Beijing, China), CD5

(Biolegend, San Diego, CA), CD25 (Biolegend, San Diego, CA), CCR7 (Biolegend, San Diego, CA), CD28 (BD Pharmingen) and CD57 (BD Biosciences). **Figure 18** represents a cancer-killing T cell preparation before and after the BsMAb exposure. This figure is one of the samples tested, and is representative of all samples, as there were only slight variations between them. Flow cytometric dot plots are shown, each representing the intensity of different CD markers on the axis. At the basal time, before addition of the BsMAb (Figures 18 A-D), we observed very few CD25+ cells (**Figure 18A**). CD25 is an activation marker thus this low expression is expected. **Figure 18B** shows the distribution of the different T cell population phenotypes in the samples as follows: 46% naive T-cells (Naive, CD45RA$^+$/CCR7$^+$/CD27$^+$), 43% central memory T-cells (CM, CD45RA$^-$/CCR7$^+$/CD27$^+$), 9% effector memory T-cells (EM, CD45RA$^-$/CCR7$^-$/CD27$^{+/-}$), and 2% terminal effector memory T-cells (E, CD45RA$^+$/CCR7$^-$/CD27$^-$), as also described elsewhere (Larbi A 2014). **Figures 18C** and **18D** show expression of markers CCR7, CD62L, CD28 and CD57, that also correspond to with what is expected in non-activated T cell populations. Expression of these same markers after a 120-hour incubation with a CD123/CD3 BsMAb is shown in Figures 18 E-H. As seen in **Figure 18E,** 97% of cells now express the activation marker CD25. There has also been a major shift in the distribution of the various subpopulations (**Figure 18F**), with 90% of cells now falling in the central memory T-cell population (CM, CD45RA$^-$/CCR7$^+$/CD27$^+$). This distribution was similar for all samples, after incubation the CM population was equal to or greater than 90%. **Figure 18G** shows that CCR7 expression remains high and that CD62L expression remains mixed, but is starting to drop. In **Figure 18H,** CD28 is slightly increased and CD57 expression is greatly increased, which also shows the shift to an activated memory cell population.

Example 13. Measure of BsMAb activity using a physiological compatible media (use of autologous plasma)

To evaluate the impact of a physiologically more relevant media on the pICF (BsMAb) activity, one bone marrow BM sample from an AML subject was tested in duplicate with the following conditions:

 i) Media containing Roswell Park Memorial Institute (RPMI) 1640 (Sigma, St Louis, MO), supplemented with 20% (vol/vol) FBS (Thermo Scientific, Waltham, MA), 2% Hepes, 1% antibiotic (Zell Shield, Labclinics, Barcelona, Spain) and 1% L-Glutamine 200mM (Lonzo, Hopkinton, MA).
 ii) Media containing RPMI 1640, supplemented with 20% (vol/vol) autologous serum obtained after centrifugation, 2% Hepes, 1% antibiotic and 1% L-Glutamine 200mM.

BM Sample was obtained from an adult patient suffering from AML over 18 years of age, who gave informed consent for study participation. For both conditions, eight concentrations of CD123/CD3 BsMAb (Creative Biolabs) were incubated for 72 or 120 hours at 37°C in humidified air containing 5% $CO_2$.

Following the incubation, a multicolor flow panel with Annexin-V PB (Immunostep, Salamanca, Spain), CD45PO (Invitrogen, Carlsbad, CA), CD8-FITC (Cytognos, Salamanca, Spain), CD117-PE (Becton Dickinson, San Jose, CA), CD4PerCP (Biolegend, San Diego, CA), CD5-PE-Cya7 (Biolegend, San Diego, CA) and CD25-APC (Biolegend, San Diego, CA) was performed to evaluate simultaneously blast depletion and T cell activation.

As shown in Figure 19, in normal media (grey line) or media supplemented with 20% autologous plasma (black line), blast depletion is similar for both assay conditions at 72h or 120h (Figure 19A and 19B respectively). However, a higher T-cell activation (CTL) and/or expansion is observed at 72h for normal media (Figure 19C), but at 120h both conditions show a similar result (Figure 19D).

Table 2 shows a quantitative approach which reveals a lower Effective E:T ratio with the normal (1:8) vs autologous plasma media (1:21) at the 72h time point. Even though the number of CTLs generated with the autologous plasma was lower, they were as effective at killing the same number of tumor cells as the greater number of CTLs generated with the normal media. This suggests that while something in the plasma reduced the number of CTLs generated, it also produced CTLs that were more effective.

However, the longer incubation reduces this difference in effective killing capacity. Possibly, the autologous plasma CTL population expansion started more slowly, but then had time to catch up. This illustrates that the use of a more physiological similar media generates CTLs that more effectively eliminate tumor cells, and may give more valuable information regarding how a patient would respond to a treatment.

**Table 2**

| Media | Time | # Blast | #T-Cells CD25+ | ΔLeukemic | Effective E:T |
|---|---|---|---|---|---|
| **CTROL** | 72h | 11162 | 4 | | |
| **Normal** | 72h | 4191 | 845 | 6971 | 1:8 |
| **Autologous Plasma** | 72h | 4367 | 529 | 6795 | 1:21 |
| **CTROL** | 120h | 7052 | 27 | | |
| **Normal** | 120h | 1233 | 16270 | 5819 | 0.35 |
| **Autologous Plasma** | 120h | 902 | 13749 | 6150 | 0.44 |

Example 14. Measurement of BsMAb activity in a Solid Tumor sample

This example evaluates the *ex vivo* effect of a EpCAM/CD3 BsMAb (G&P Biosciences, Santa Clara, CA) on a melanoma sample acquired from a Tumor BioBank (Molecular Response, San Diego, CA) with the simultaneous presence of cancer cells and autologous T-cells.

Sample was thawed and plated into 96-well plates comprised of wells containing either 3 different concentrations of EpCAM/CD3 BsMAb (3, 2, 1 $\mu$g/ml) and control antibodies. Sample was incubated with culture medium (Roswell Park Memorial Institute (RPMI) 1640 (Sigma, St Louis, MO) media supplemented with 20% (vol/vol) FBS (Thermo Scientific, Waltham, MA), 2% Hepes, 1% antibiotic (Zell Shield, Labclinics, Barcelona, Spain) and 1% L-Glutamine 200mM (Lonzo, Hopkinton, MA). After 144h of incubation, all cells were harvested with 0.05% trypsin/EDTA and spun down by centrifugation. Prior to analysis, a multicolor flow cytometry panel was added to define both tumor and the T-cell population: Annexin V (Immunostep), CD45 PO (Invitrogen), CD8 (Cytognos), Tag72 (Bionova), CD4 (Biolegend), CD5 (Biolegend), EpCAM (BD, Biosciences) and CD25 (Biolegend).

As shown in the Figure 20, in the presence of EpCAM/CD3 (grey histograms) but not in the control wells tumor cells were killed in a dose-dependent manner (**Figure 20A**). In addition, this increase in tumor cell depletion Is associated with an increase of the CD25 activation marker on the T-cells (**Figure 20B**).

Example 15. Effect of trogocytotic cancer-killing T cells in an AML sample

This example demonstrates that activated CTLs that have undergone trogocytosis have a high killing capacity.

A cryopreserved AML sample was stained with Annexin V (Immunostep), CD45 (Invitrogen), CD33 (BD, Biosciences) and CD5 (Biolegend). Live Blast cells (Annexin-/CD45+/CD33++) and Live T-cell (Annexin-/CD45++/CD5+) populations were sorted by fluorescence activated cell sorting (FACS sorting) using a FACS Aria III flow cytometer (BD, San Jose, CA) with a cell purity> 99%. Sample was obtained from an adult patient diagnosed with AML, over 18 years of age, who gave informed consent for study participation.

Blast cells were stained with the cell surface dye PKH67 (Sigma Aldrich, St. Louis, MO) and incubated for 144h with the autologous T cells at different Effector: Target ratios in triplicate, in the presence or absence of the CD123-CD3 BsMAb. PKH67 is a cell membrane labelling dye. For this particular assay, only the blast cells were stained with the dye but not the T-cells. After the BsMAb exposure, it was expected that T-cells would acquire this marker from the blasts during cell to cell interaction induced by the BsMAb, which would show trogocytosis had occurred.

Cells were incubated with culture medium (Roswell Park Memorial Institute (RPMI) 1640 (Sigma, St Louis, MO) supplemented with 20% (vol/vol) FBS (Thermo Scientific, Waltham, MA), 2% Hepes, 1% antibiotic (Zell Shield, Labclinics, Barcelona, Spain) and 1% L-Glutamine 200mM (Lonzo, Hopkinton, MA). Prior to analysis, a multicolor flow cytometry panel was added to define both tumor and the T-cell population: Annexin V (Immunostep), CD45 (Invitrogen), CD8 (Cytognos), CD33 (BD, Biosciences), CD4 (Biolegend), CD5 (Biolegend), and CD25 (Biolegend).

**Figures 21A, 21B** and **21C** show the flow cytometric dot plots (CD5/PKH67) of the experiment illustrating the trogocytotic capacity of the T-cells. Live Blast cells and T-cells are simultaneously represented. Figure 21A shows the dot plot of the blast cells (PKH67++/CD5-, upper left quadrant) and a few T-cells (PKH67-/CD5++, lower right quadrant) at the basal time before incubation. At this time point, no double positive T-cells (PKH67++/CD5++) were observed. Figure 21B shows the dot plot of the blast cells (PKH67++/CD5-) and T-cells (PKH67-/CD5++) after incubation and in the absence of the BsMAb. At this time point and the absence of the BsMAb, no double positive T-cells (PKH67++/CD5++, upper right quadrant) were observed. Figure 21C shows the dot plot of the blast cells (PKH67++/CD5-) and T-cells (CD5++) after incubation and in the presence of the BsMAb. As can be seen, most of the activated T-cells (CTLs) appear in the upper right quadrant, showing that they have picked up tracker dye in their interaction with the tumor cells. The trogocytic CTLs generated in this sample. in the presence of the BsMAb is 78% of the CD5++ population compared to only 9% of the control.

A second cryopreserved tube from the same AML patient was used to quantify the depletion of blast cells and T cell activation in a dose-response manner. The sample was diluted with the same culture medium used above, and added to plates containing 8 different concentrations of a CD123/CD3 BsMAb (0.5, 1, 10, 50, 100, 250, 500, and 1000 ng/ml). Plates were incubated for 144 hours. Prior to analysis the same multicolor flow cytometry panel of markers used above was added to the plates to identify both tumor cells and the CTL population. Figure 22A shows how the CD123/CD3 BsMAb induced an increase in the CTL population in a dose-response manner, that corresponds with a dose-response depletion of blast (tumor) cells. The Effective E:T ratio for this sample is 60:6,000, or 1:100, demonstrating that the CTLs that were generated are very efficient at killing the tumor cells. The Effective E:T ratio may prove to be an additional tool used to determine the robustness of a BsMAb or other ICF activity specifically for each patient.

Example 16. *Ex vivo* measurement of the pharmacological activity of cancer-killing T cells

There are four parameters to measure quantitatively the activity of cancer-killing T cells generated by pICFs in patient samples and determine the pharmacological activity of cancer-killing T cells to be used in cellular therapy.

The EC50 of T cell proliferation correspond to the concentration of pICF which generates 50% of the activated, cancer-killing T cells. Figure 16B shows superposed dose response curves of activated T cells from several independent subjects as a function of the dose of pICF BsMAb CD3xCD123. Note that the EC50 of T cell activation is similar to the EC50 of depletion of cancer cells (Figure 16 A) because they represent the same process: how the pICF induces activated T cells turn into cancer-killing cells that depletes cancer cells. This equivalency between the EC50 of T cell activation and cancer cell depletion can be observed also in Figures 6, 7, and 8 of the present specification.

This EC50 of T Cell activation is very similar across multiple samples of patients diagnosed with the same disease (Figure 16A and Figure 16B). Therefore, this parameter is informative of patient sensitivity only for the few cases where the EC50 is significantly different from the majority of the samples. If the EC50 is significantly lower, it should be interpreted that for said specific patient T cells will be activated at a lower dose of pICF. On the contrary, if the EC50 value is significantly higher, the interpretation is that for said specific patient, T cells will be activated at a higher dose of pICF, and thus higher doses of the pICF should be used during the incubation to generate cancer-killing T cells.

The Effective E:T Ratio represents the activity of the cancer-killing T cell generated by the pICF. Thus, it is a key parameter to decide whether to treat patients with the activated T cells obtained by the process disclosed herein. This parameter can be validated by measuring dose responses adding precise numbers of activated cancer-killing T cells generated after incubation with the pICF, previously isolated by FACS sorting as shown in Figures 12 and 13.

High Effective E:T Ratios predict sensitive patients to these cancer-killing T cells as autologous cell therapy, and low Effective E:T Ratios predict resistant patients to these cancer-killing T cells as autologous cell therapy.

Emax or Efficacy measures the % cancer cells left alive at high doses of pICF. This parameter depends on the incubation time, and for clinical decisions regarding T cell treatment a long incubation time is necessary, at which the activated T cells kill all the cancer cells for the majority of the samples; at this incubation time the small subset of samples that show a resistant subset of cancer cells is clinically informative. The activated cancer-killing T cells must kill 100% of cancer cells for these cancer-killing T cells in order to be a suitable monotherapy treatment for a patient. On the contrary, if activated T cells cannot kill all cancer cells (Figure 16A), and there is a subset of resistant cancer cells, cancer-killing T cells as monotherapy would be predicted to leave these cancer cells alive, and therefore it will not be considered an appropriate treatment. It is possible the combination of additional drugs or other ICFs in a treatment which will revert the resistant phenotype of cancer cells.

When such a subset of cancer cells is identified as resistant to cancer-killing T cells, these resistant cancer cells are immunosuppressed, because they are resistant to activated autologous T cell therapy. Therefore, the addition of additional immunomodulatory agents such as immune check point inhibitors, can overcome the immunosuppression and consequently overcome the cancer cells resistance to the treatment. These additional immunomodulatory agents can be added in two different ways:

a) Adding *ex vivo* to relieve immunosuppression and enable the generation of fully cytotoxic cancer-killing T cells. This is especially meaningful for cancer-killing T cells generated for subsequent cellular therapy. They can be added to the same wells incubating with a pICF, or in a second step after the incubation with the pICF. Adding immunomodulatory agents *ex vivo,* enables the combination of multiple agents, including agents with multiple mechanisms of action, this is up to 5, 10, or 20 agents. These numbers are much higher than the usual 2-3 immunotherapy drugs that can be administered simultaneously to a clinical patient *in vivo,* where toxicity restrictions limit poly-immunotherapy. Hence, *ex vivo* generation of cancer-killing T cells for cellular therapy can exploit the poly-immunotherapy better than direct patient clinical drug treatment.

b) Can be administered to a patient in combination with the cellular therapy, so that they relieve the immunosuppression in the patient's body. This is especially meaningful for targets on cancer cells that render these cancer cells resistant to the cancer-killing T cells to be administered as a cellular therapy to the patient.

Any of the 3 parameters mentioned above to measure the activity of cancer-killing T cells may change as a function of incubation time and therefore the kinetics of the reactions may also provide meaningful information. A time-dependent parameter reflects the kinetics of these processes. Some examples of meaningful interpretations are described below.

Figure 16 show that there is a very high level of interpatient variability in the kinetics and efficiency of the T cell activation and cancer cell depletion across AML samples for the same BsAbCD3xCD123.

Cancer-killing T cells that kill cancer cells faster are likely to be more efficacious cellular therapies. Faster activity may mean faster effects against the cancer in the patient, a positive outcome that reflects higher sensitivity. Faster activity may also mean higher affinity towards the cancer cells if the cancer-killing T cells are CD8+ Tumor-Specific Antigen T cells that recognize cancer cells through a MHC-I mechanism. Figure 11 shows the kinetics of cancer cell killing of cancer-killing T cells from a given sample.

When T cells become activated by the pICF at earlier time points, it may need some dividing cycles to maximize its killing efficacy. On the other hand, after cancer-killing T cells kill cancer cells, and there are no more cancer cells left, these T cells become exhausted, decreasing sharply in numbers, activation state, and killing capacity. Sometimes there is a subset of cancer resistant cells that cannot be killed by increasing the incubation time, yet the cancer-killing T cells do recognize them and induces their proliferation, thus increasing numbers of cancer killing T cells without changing the number of cancer cells thus changing the Effective E:T Ratio at these longer incubation times. Therefore, to measure the activity of cancer-killing T cells it is important to focus on the time after activation, when proliferation is associated with cancer cell killing and before they reach exhaustion or a subset of T cell resistant cancer cells is found.

To demonstrate these concepts, 13 AML bone marrow samples were obtained from adult patients, over 18 years of age, who gave informed consent for study participation. Samples were incubated with culture media for either 72, 96 or 120 hours and added to plates containing 8 different concentrations of a CD123/CD3 BsMAb. Note that not all the patients were analyzed at all the times. Following the incubation, a multicolor flow panel with Annexin-V (Immunostep, Salamanca, Spain), CD45 (Invitrogen, Carlsbad, CA), CD8 (Cytognos, Salamanca, Spain), CD117 (Becton Dickinson, San Jose, CA), CD64 (Biolegend, San Diego, CA), CD34 (Becton Dickinson, San Jose, CA), CD33 (Becton Dickinson, San Jose, CA), HLA-DR (Immunostep, Salamanca, Spain) CD4PerCP (Biolegend, San Diego, CA), CD5-PE-Cya7 (Biolegend, San Diego, CA) and CD25-APC (Biolegend, San Diego, CA) was performed to evaluate simultaneously blast depletion and T cell activation. Appropriate blast markers were used for each particular sample.

Table 3 shows the statistical parameters of EC50, Emax (absolute cell counts at the highest concentration of MsMAb), and E0 (absolute cell count in the absence of BsMAb) for both CD8+ CTLs and tumor cells. As can be seen in Table 3, and also in Figure 23, the EC50s of both the CTL and Tumor cell populations are similar. **Figure 23A** shows the EC50 for both the CTL and Tumor cell populations for each sample at each time point, which displays some consistency, but variations can be seen at all time points. Individual variations are to be expected, however **Figure 23B** shows that the median EC50 values are very similar for each population, as well as for each time point.

The Effective E:T ratio is determined by calculating the change in the absolute count of Effector cell and the change in the absolute number of Target cells. The Emax and E0 values are used to quantify this. Here the Effector cells are the CTL population and the Targets are the tumor cells. The Effective E:T ratio represents the change in values induced by an ICF (e.g. a BsMAb).

**Figure 24** shows a version of the T:E ratio for 5 representative AML samples from above. The y-axis displays the number of Target cells present per each Effector Cell (T/E) for each time point (72, 96 or 120 hours). As the CD8+ CTLs increase over time, generally there is a decrease of the Effective T:E ratio over longer incubation times.

This example demonstrates the relevance of quantitatively measuring the activity of the cancer-killing T cells in a cell culture model where tumor depletion and T-cell expansion can occur. The analysis of multiple key parameters including EC50, Emax, and the Effective E:T ratio in a kinetic manner gives valuable information for both tumor and CTL populations. The data generated provides a robust tool to quantify the activity and effectiveness of ICFs.

**Table 3**

| | Population | Time (h.) | N | Median | Avg | Min | Max | StdDev |
|---|---|---|---|---|---|---|---|---|
| EC50 (µGr/mL) | Live T-Lymph Cells CD8+CD25+ | 72 | 13 | 0.16 | 0.19 | 0.11 | 0.43 | 0.09 |
| | | 96 | 11 | 0.17 | 0.29 | 0.11 | 1.19 | 0.32 |
| | | 120 | 9 | 0.15 | 0.2 | 0 | 0.51 | 0.15 |
| | Live tumor cells | 72 | 11 | 0.19 | 2.05 | 0 | 21.08 | 6.31 |
| | | 96 | 9 | 0.21 | 0.16 | 0 | 0.36 | 0.12 |
| | | 120 | 6 | 0.1 | 0.12 | 0 | 0.3 | 0.12 |
| Emax Cell # | Live T-Lymph Cells CD8+CD25+ | 72 | 13 | 114 | 438.62 | 40 | 1970 | 637.55 |
| | | 96 | 11 | 263 | 600.55 | 71 | 2267 | 725.49 |
| | | 120 | 9 | 829 | 1254.67 | 53 | 2857 | 1056.41 |
| | Live tumor cells | 72 | 11 | 1362.4 | 2567.24 | 0 | 11859.22 | 3749.35 |
| | | 96 | 9 | 1630.63 | 3034.86 | 20.09 | 12892.9 | 4324.83 |
| | | 120 | 6 | 1445.47 | 3414.7 | 0 | 10844.15 | 4349.72 |
| E0 Cell # | Live T-Lymph Cells CD8+CD25+ | 72 | 13 | 0 | 10.62 | 0 | 100 | 27.55 |
| | | 96 | 11 | 0 | 12.45 | 0 | 100 | 30.86 |
| | | 120 | 9 | 0 | 3.67 | 0 | 28 | 9.27 |
| | Live tumor cells | 72 | 11 | 12118.5 | 11110.04 | 218.7 | 25749.3 | 7565.65 |
| | | 96 | 9 | 11271.6 | 11149.57 | 628.2 | 22341.6 | 8020.15 |
| | | 120 | 6 | 10475.2 | 12626.18 | 1476.3 | 30064.5 | 10024 |

Example 17. *Ex vivo* activity of cancer-killing T cells in bone marrow vs peripheral blood samples

This example evaluates the cytotoxic capacity of the CTLs in 10 paired BM and PB AML samples (5 paired BM and PB). Samples were obtained from adult patients, over 18 years of age, who gave informed consent for study participation. Samples were received within 24-48 hours after collection. BM samples were diluted with culture media, in their entirety (i.e., without first separating cells or other components from the BM), and plated into 96-well plates comprised of wells containing 8 concentrations of the BsMAb and control antibodies for 72 hours.

Following the incubation, a multicolor flow panel with Annexin-V (Immunostep), CD45 (Invitrogen), CD8 (Cytognos), CD117 (Becton Dickinson), CD64 (Biolegend), CD34 (Becton Dickinson), CD33 (Becton Dickinson), HLA-DR (Immunostep), CD4 (Biolegend), CD5 (Biolegend), CD25 (Biolegend), CD138 (Cytognos), CD38 (Immunostep,) CD45 (BD-Pharmingen), CD56 (BD-Pharmingen), CD19 (Biolegend) and CD3 (Cytognos) was performed to evaluate simultaneously tumor depletion and T-cell activation. Appropriate tumor markers were used for each particular sample.

The inventors have hypothesized that pICF can activate immunosuppressed Tumor-Specific Antigen (TSA) T Cells, and that these activated T cells have higher cancer cell killing than other normal T cells also activated by the pICF. Additionally, they have also described the immunosuppressive microenvironment present in tissues with a 3-dimensional structure such as bone marrow, as opposed to peripheral blood. Then, if this hypothesis is correct the activity of cancer-killing T cells in bone marrow samples should be higher than those in peripheral blood samples of the same cancer patient.

Paired samples from bone marrow and peripheral blood (of the same patient) from 5 AML patients were incubated with a CD3xCD123. **Figure 25** shows the dose-response curves for T cell activation and cancer cell depletion in both paired samples (S1 - S5, upper vs lower panels) for each patient. A similar pattern is observed for each blood and bone marrow sample for the same patient, in terms of EC50s and Emax. Different samples do show a different behavior, especially in terms of their Emax.

The activity of cancer-killing T cells is calculated by Effective E:T Ratios included in each graph off Figure 25 and **Table 4** below. Bone marrow samples have higher Effective E:T Ratios than their correspondent blood counterparts in samples 1, 4, and 5. Bone marrow samples for samples 2 and 3 have the same Effective E:T Ratios in bone marrow and blood. Thus, the expected results are confirmed in that bone marrow cancer-killing T cells have either higher or similar cancer killing activity than their blood counterparts, not less. This pattern may be a reflection of the presence of immunosuppressed TSA T Cells that can be activated by the pICF, suggesting a higher presence of these T cells in bone marrow samples with higher Effective E:T Ratios in bone marrow vs blood paired samples. This may resemble the measurement of %TILs in solid tumor samples as a known clinical predictor of immuno-therapy response.

**Table 4**

|  | Sample 1 | Sample 2 | Sample 3 | Sample 4 | Sample 5 |
|---|---|---|---|---|---|
| **Bone Marrow** | 25 | 5 | 8 | 7 | 9 |
| **Peripheral Blood** | 5 | 5 | 8 | 3 | 2 |
| **Difference** | 5-fold | Same | same | 2.3-fold | 4.5-fold |

Example 18. *Ex vivo* selective killing of cancer-killing T cells

This example evaluates the selective capacity of the CTLs on residual normal cells from two MM and two AML patients. Samples were obtained from adult patients, over 18 years of age, who gave informed consent for study participation. Samples were received within 24-48 hours after collection. BM samples were diluted with culture media, in their entirety (i.e., without first separating cells or other components from the BM), and plated into 96-well plates comprised of wells containing 8 concentrations of the BsMAb and control antibodies for 72 hours.

Following the incubation, a multicolor flow panel with Annexin-V (Immunostep), CD45 (Invitrogen), CD8 (Cytognos), CD117 (Becton Dickinson), CD64 (Biolegend), CD34 (Becton Dickinson), CD33 (Becton Dickinson), HLA-DR (Immunostep) CD4 (Biolegend), CD5 (Biolegend), CD25 (Biolegend), CD138 (Cytognos), CD38 (Immunostep,) CD45 (BD-Pharmingen), CD56 (BD-Pharmingen), CD19 (Biolegend) and CD3 (Cytognos) was performed to evaluate simultaneously tumor depletion, T-cell activation and residual normal cells. This latter population corresponds to B-cells (CD19+) in the AML patients and granulocytes (CD45+/SSChigh/FSChigh) for the MM patients. Appropriate tumor markers were used for each particular sample.

The inventors have hypothesized that the pICF can activate immunosuppressed Tumor-Specific Antigen (TSA) T Cells in bone marrow samples of hematological malignancies, contrary to the common understanding that bispecific antibodies activate T cells by bringing them in proximity to the cancer cells in an antigen-independent mechanism of action. If the activated T cells are TSA T Cells, they should recognize and kill selectively cancer cells. On the contrary, if activated T cells kill in an unselective antigen-independent manner, they should kill also non-pathological cells. This can be evaluated in the incubation experiments with pICF bispecific antibodies for MM (Figure 26) and AML (Figure 27) analyzing their corresponding non-pathological cells at the same time as the cancer cells.

**Figure 26** shows two MM bone marrow samples (A and B) in which the pICF induces T cell activation (CD8+CD25+) and cancer cell killing (decrease of tumor cells). When the population of granulocytes is added to these graphs, considered the most numerous non-pathological cells, their numbers stay quite similar after incubation with the pICF without significant killing, while the cancer cells are killed. The large number of granulocytes in each well of these samples, approximately 22.000 and 11.500 respectively, indicates that this result is statistically very significant. These results show the cancer-killing T cells kill cancer cells with selectivity.

**Figure 27** shows two AML bone marrow samples (A and B) in which the pICF induces T cell activation and cancer cell killing. When a population of B cells is added to these graphs, considered the most numerous non-pathological cells, their numbers do not decrease substantially after incubation with the pICF, while the cancer cells are killed. The low numbers of B cells, approximately 450 and 200 respectively, are less statistically significant than the granulocytes in the MM samples, yet consistent with the MM results. These results show the cancer-killing T cells kill cancer cells with selectivity.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims are introduced into another claim. For example, any claim that is dependent on another claim can be modified to include one or more limitations found in any other claim that is dependent on the same base claim. Where elements are presented as lists, *e.g.*, in Markush group format, each subgroup of the elements is also disclosed, and any element(s) can be removed from the group. It should be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

**BIBLIOGRAPHY**

**Non-patent literature**

- Adams JL et al. (2015). Big opportunities for small molecules in immuno-oncology. Nat Rev Drug Discov 14(9):603-22.
- Bennett TA et al. (2014). Pharmacological profiles of acute myeloid leukemia treatments in patient samples by automated flow cytometry: a bridge to individualized medicine. Clin. Lymphoma Myeloma Leuk. 14(4):305-18.
- Buil-Bruna N et al. (2016). Bringing Model-Based Prediction to Oncology Clinical Practice: A Review of Pharmacometrics Principles and Applications. The Oncologist 21(2):220-32.
- Chothia C et al. (1987). Canonical structures for the hypervariable regions of immunoglobulins. J Mol Biol 196(4):901-17.
- Hamid O et al. (2013). Safety and tumor responses with lambrolizumab (anti-PD-1) in melanoma. N Engl J Med 369(2):134-44.
- Kabat EA et al. (1991). Sequences of Proteins of Immunological Interest. US Department of Health and Human Services Fifth Edition. NIH Publication No. 91-3242.
- Larbi A and Fulop T (2014). From "truly naïve" to "exhausted senescent" T cells: when markers predict functionality. Cytometry Part A 85(1): 25-35.
- Martin F et al. (1994). The affinity-selection of a minibody polypeptide inhibitor of human interleukin-6. EMBO J 13(22):5303-9.
- Maus MV et al. (2014). Adoptive immunotherapy for cancer or viruses. Annu Rev Immunol 32:189-225.
- McConnell SJ et al. (1995). Tendamistat as a scaffold for conformationally constrained peptide libraries. J Mol. Biol 250(4):460-70.
- Montes M et al. (2005). Optimum in vitro expansion of human antigen-specific CD8 T cells for adoptive transfer therapy. Clin Exp Immunol 142(2):292-302.
- Mould DR et al. (2013). Basic Concepts in Population Modeling, Simulation, and Model-Based Drug Development - Part 2: Introduction to Pharmacokinetics Modeling Methods. CPT Pharmacometrics Syst Pharmacol 2(4):e38.
- Pardoll DM. (2012). The blockade of immune checkpoints in cancer immunotherapy. Nat Rev Cancer 12(4):252-64.
- Rosenberg SA et al. (1988). Use of tumor-infiltrating lymphocytes and interleukin-2 in the immunotherapy of patients with metastatic melanoma. A preliminary report. N Eng J Med 19(25):1676-80.
- Scott AM et al. (2012). Monoclonal antibodies in cancer therapy. Cancer Immun 12:14.
- Serafini P et al. (2008). Myeloid-derived suppressor cells promote cross-tolerance in B-cell lymphoma by expanding regulatory T cells. Cancer Res 68(13):5439-49.
- Song DG et al. (2012). CD27 costimulation augments the survival and antitumor activity of redirected human T cells in vivo. Blood 119(3):696-706.
- Spiess C et al. (2015). Alternative molecular formats and therapeutic applications for bispecific antibodies. Mol Immunol 67:95-106.
- Tramontano A et al. (1994). The making of the minibody: an engineered beta-protein for the display of conformationally constrained peptides. J Mol. Recognit 7(1):9-24.
- Upton RN et al. (2014). Basic Concepts in Population Modeling, Simulation, and Model-Based Drug Development: Part 3-Introduction to Pharmacodynamic Modeling Methods. CPT Pharmacometrics Syst Pharmacol 3(1): e88.
- Guidance for Industry Population Pharmacokinetics (1999). US Department of Health and Human Services Food and Drug Administration; Center for Drug Evaluation and Research (CDER) and Center for Biologics Evaluation and Research (CBER).
- http://www.fda.gov/downloads/drugs/guidancecomplianceregulatoryinformation/guida nces/ucm072137.pdf).

**Claims**

1. An *in vitro* method of producing an activated T cell or an activated T cell preparation, comprising:

   (a) providing a sample comprising at least one T cell from a subject having a cancer;
   (b) providing a sample comprising at least one cancer cell from a subject, wherein the type of cancer of the cancer cell is the same as the type of cancer the subject providing the sample of step (a) has;

(c) forming an *ex vivo* reaction mixture comprising the at least one T cell, the at least one cancer cell, and a bispecific or multispecific antibody having a first element providing affinity for the T cell and a second element having affinity for the cancer cell and incubating the mixture under conditions and for a period of time sufficient to activate the T cell, thereby producing at least one activated T cell; and

(d) selecting the activated T cell from (c), wherein the selection of the activated T cell is based on its increased cancer cell killing activity determined by measuring an Effective E:T ratio, wherein said Effective E:T ratio is a ratio between effector cells (E) to target cells (T), effector cells (E) being defined as activated T cells and target cells (T) being defined as cancer cells killed by said activated T cells.

2. The method of claim 1, wherein in step (c) the *ex vivo* reaction mixture is incubated under conditions and for a period of time sufficient to allow the at least one T cell to acquire a cell surface marker from the at least one cancer cell.

3. The method of claim 1 or 2, further comprising one, two or all of the following in vitro steps:

(i) expanding the activated T cells from (d);
(ii) enriching for the activated T cells from (d); or
(iii) purifying the activated T cells from (d).

4. The method of any of claims 1-3, wherein the bispecific or multispecific antibody is selected from: a bispecific immunoglobulin (BsIgG), an immunoglobulin operatively linked to additional antigen-binding molecule, a bispecific antibody (BsAb) fragment comprising at least two scFv fragments operative linked by a linker selected from a dual-affinity retargeting (DART) antibody fragment or a Bispecific T cell Engager (BiTE), a bispecific fusion protein, or a BsAb conjugate.

5. The method of any of claims 1-4, wherein the bispecific or multispecific antibody is selected from the list consisting of BsMAb CD123/CD3, BsMAb CD19/CD3 and EpCAM/CD3.

6. The method of any of claims 3-5, wherein the selecting step (d) and/or enriching step (ii) comprises using a fluorescently labeled compound that binds to (i) at least one cancer antigen, or diffuses into the cancer cell membrane or (ii) at least one marker of activated T cells, or both (i) and (ii); or using a bead coated with an antibody or fragment thereof that binds to (i) at least one cancer antigen or (ii) at least one marker of activated T cells, or both (i) and (ii).

7. The method of any of claims 1-6, wherein the activated T cell or preparation comprises at least one CD8+ T cell and/or at least one CD25+ T cell, and/or at least one CD8+/CD25+ T cell and/or at least one CD4+/CD25+ T cell, and or at least one cytotoxic T lymphocyte (CTL) or at least one tumor infiltrating lymphocyte (TIL) and/or at least one trogocytotic T cell.

8. The method of any of claims 1-7, further comprising separating individual clones from the activated T cell preparation, wherein the separating step comprises clonal expansion of single cells by:

(i) separating the preparation of activated T cells into single cells and
(ii) expanding the single cells to generate at least one preparation of activated T cells.

9. The method of any of claims 1-8, wherein step (a) and step (b) comprise providing one sample comprising both the cancer cell and the T cell.

10. The method of any of claims 1-9, wherein the sample (a) is derived from a tissue with a microenvironment, wherein substantially no components have been removed or isolated from the sample, selected from: whole blood, peripheral blood, bone marrow, lymph node, a biopsy of a primary tumor, or a biopsy of a metastasis or spleen.

11. The method of any of claims 1-10, wherein the cancer of sample (b) is a hematological cancer selected from: Hodgkin's lymphoma, Non-Hodgkin's lymphoma (B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.

12. The method of any of claims 1-11, wherein the cancer is a solid cancer selected from: ovarian cancer, rectal cancer, stomach cancer, testicular cancer, cancer of the anal region, uterine cancer, colon cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

13. The method of any of claims 1-12, wherein the subject providing sample (a) and/or sample (b):

(i) has not received a prior treatment for the cancer;
(ii) has received at least one previous treatment for the cancer; or
(iii) has minimal residual disease (MRD).

14. The method of any of claims 1-13, further comprising evaluating the cancer-killing activity of the activated T cell or activated T cell preparation.

15. The method of claim 14, wherein evaluating comprises:

(a) providing an activated T cell or a preparation thereof obtainable according to the method of claims 1-14;
(b) providing a cancer cell, wherein the cancer cell is from the same subject;
(c) contacting the activated T cell or the preparation thereof with the cancer cells for a period of time sufficient to allow the activated T cell to kill the cancer cells;
(d) determining the level of cancer cells after step (c), and optionally determining the level of activated T cells after step (c); and optionally,
(e) determining the ratio of either cancer cell to T cell, or T cell to cancer cell, from step (d).

16. The method of claim 15, wherein a decrease in the level or amount of cancer cells, relative to a reference level, is indicative of increased cancer cell killing, or wherein a reduced change or no substantial change in the level or amount of cancer cells relative to a reference level, is indicative of decreased cancer cell killing.

17. The method of claim 15 or 16, wherein an Effective E:T ratio of 1:10 or lower indicates that the activated T cell or preparation thereof is an effective killer of cancer cells, and wherein a ratio of 1:1, 1:3, or 1:5 is indicative of a poor T cell killing activity.

18. The method of any of claims 1-17, wherein Effective E:T ratio is calculated from values derived from mathematical fitting of the experimental values of a dose response curve of multiple doses of the bispecific or multispecific antibody.

19. The method of any of claims 1-18, wherein in step (c) immunomodulatory agents are added to the mixture, wherein said immunomodulatory agents are selected from the group consisting of immune checkpoint inhibitors, agonists of T cells and other immunomodulatory drugs.

20. The method of claim 19, wherein the immune checkpoint inhibitors inhibit immune checkpoints selected from the group consisting of CTLA4, PD-1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1 and A2aR.

21. A composition comprising activated T cells obtainable by the method of any of claims 1-20, wherein said activated T cells have an Effective E:T ratio of 1:10 or lower and wherein said Effective E:T ratio is a ratio between effector cells (E) to target cells (T), effector cells (E) being defined as the number of activated T cells (E) activated by a bispecific or multispecific antibody and target cells (T) being defined as the number of cancer cells killed by said activated T cells.

22. A pharmaceutical composition comprising the composition of activated T cells of claim 21 and a pharmaceutically acceptable carrier.

23. The composition of activated T cells of claim 21, or the pharmaceutical composition of claim 22, wherein said composition or pharmaceutical composition comprises trogocytotic activated T cells.

24. The composition of activated T cells of claim 21 or 23, or the pharmaceutical composition of claim 22 or 23, wherein the activated T cells comprise at least 100 copies of the cancer cell surface marker and also comprise a detectable amount of bispecific or multispecific antibody.

25. The composition of activated T cells of any of claims 21, 23 or 24, or the pharmaceutical composition of any of claims 22-24, wherein the activated T cells are cytotoxic T lymphocytes or helper T cells selected from CD8+ T cells or CD4+ T cells.

26. The composition of activated T cells of any of claims 23-25, or the pharmaceutical composition of any of claims 23-25, comprising trogocytotic T cells at a concentration of at least 30% of the total number of cells in the composition or preparation.

27. The composition of activated T cells of any of claims 21, 23-26, or the pharmaceutical composition of any of claims 22-26, comprising immunomodulatory agents selected from the group consisting of immune checkpoint inhibitors, agonists of T cells and other immunomodulatory drugs.

28. The composition of activated T cells of claim 27 or the pharmaceutical composition of claim 27, wherein immune checkpoint inhibitors inhibit immune checkpoints selected from the group consisting of CTLA4, PD-1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1 and A2aR.

29. Pharmaceutical composition according to claim 22 for use in Adoptive Cancer Therapy for treating a subject, wherein the subject is the same subject as that of step (a) and/or wherein the subject is the same subject as that of step (b) and/or wherein the subject is different from the subject as that as step (a) or (b).

30. Pharmaceutical composition for use according to claim 29 in Adoptive Cancer Therapy for treating a subject suffering a hematological cancer selected from chronic lymphocytic leukemia, acute lymphocytic leukemia, Non-Hodgkin's Lymphoma, acute myeloid leukemia, myelodysplastic syndrome or multiple myeloma.

31. An activated T cell obtainable by the method of any of claims 1-20 or the composition of activated T cells of any of claims 21, 23-28 or the pharmaceutical composition of any of claims 22-28 for use in a method for treating a subject having cancer, wherein said method comprises administering an effective amount of said activated T cell, the composition of activated T cells or the pharmaceutical composition, to the subject, wherein the activated T cell obtainable by the method of any of claims 1-20 has an Effective E:T ratio of 1:10 or lower and wherein said Effective E:T ratio is a ratio between effector cells (E) to target cells (T), effector cells (E) being defined as the number of activated T cells (E) activated by a bispecific or multispecific antibody and target cells (T) being defined as the number of cancer cells killed by said activated T cells.

32. The activated T cell, the composition of activated T cells or the pharmaceutical composition, for use in a method of treating a subject having cancer, according to claim 31, wherein said method comprises:

(a) providing a sample from the subject, wherein the sample comprises a T cell and a cancer cell;
(b) forming an *ex vivo* reaction mixture comprising the sample of step (a) and a bispecific or multispecific antibody and incubating the mixture under conditions and for a period of time sufficient to activate the T cell, thereby producing at least one activated T cell;
(c) selecting the activated T cell from (c), wherein the selection of the activated T cell is based on increased cancer cell killing activity determined by measuring an Effective E:T ratio; and
(d) administering an effective amount of the activated T cells to the subject.

33. The activated T cell, the composition of activated T cells or the pharmaceutical composition for use in a method of treating a subject having cancer, according to claim 32, wherein in step (c) of said method, the *ex vivo* reaction mixture is incubated under conditions and for a period of time sufficient to allow the at least one T cell to acquire a cell surface marker from the at least one cancer cell.

34. The activated T cell, the composition of activated T cells or the pharmaceutical composition for use in a method of treating a subject having cancer, according to claim 32 or 33, wherein said method further comprises one, two or all of the following in vitro steps:

> (i) expanding the activated T cells from (d);
> (ii) enriching for the activated T cell from (d); or
> (iii) purifying the activated T cell from (d).

35. The activated T cell, the composition of activated T cells or the pharmaceutical composition for use in a method of treating a subject having cancer, according to any of claims 32-34, wherein said method further comprises administering a second therapeutic agent or procedure.

36. The activated T cell, the composition of activated T cells or the pharmaceutical composition for use in a method of treating a subject having cancer, according to claim 35, wherein in said method the second therapeutic agent or procedure is chosen from at least one of: chemotherapy, a targeted anti-cancer therapy, an oncolytic drug, a cytotoxic agent, an immune-based therapy, a cytokine, a surgical procedure, a radiation procedure, an agonist of T cells, an immune checkpoint inhibitor, an immunomodulatory agent or a combination of multiple immunomodulatory agents, a vaccine, or a cellular immunotherapy.

37. The activated T cell, the composition of activated T cells or the pharmaceutical composition for use, according to claim 36, wherein said immunomodulatory agents are immune checkpoint inhibitors.

38. The activated T cell, the composition of activated T cells or the pharmaceutical composition for use, according to claim 37, wherein the immune checkpoint inhibitors inhibit immune checkpoints selected from the group consisting of CTLA4, PD-1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1 and A2aR.

39. *An in vitro* method of evaluating the responsiveness of a subject to a therapy comprising:

> (a) providing a sample comprising a T cell from a subject having a cancer;
> (b) providing a sample comprising a cancer cell, from the subject;
> (c) forming an *ex vivo* reaction mixture comprising the T cell, the cancer cell, a bispecific or multispecific antibody having a first element providing affinity for the T cell and a second element having affinity for the cancer cell, and the therapy, and incubating the mixture under conditions and for a period of time sufficient to activate the T cell, thereby producing at least one activated T cell;
> (d) selecting the activated T cell from (c), wherein the selection of the activated T cell is based on increased cancer cell killing activity determined by measuring an Effective E:T ratio, wherein said Effective E:T ratio is a ratio between effector cells (E) to target cells (T), effector cells (E) being defined as activated T cells and target cells (T) being defined as cancer cells killed by said activated T cells, and wherein an increase in cell killing activity of the reaction mixture in the presence of the therapy relative to a reference value in absence of the therapy is indicative of a higher responsiveness to the therapy.

40. The method of claim 39, wherein in step (c) the *ex vivo* reaction mixture is incubated under conditions and for a period of time sufficient to allow the at least one T cell to acquire a cell surface marker from the at least one cancer cell.

41. The method of claim 39 or 40, further comprising one, two or all of the following in vitro steps:

> (i) expanding the activated T cells from (d);
> (ii) enriching for the activated T cell from (d); or
> (iii) purifying the activated T cell from (d).

42. The method of any of claims 39-41, wherein step (d) additionally comprises determining the level of cancer cells after step (c), and optionally determining the level of activated T cells after step (c).

43. The method of any of claims 39-42, wherein the bispecific or multispecific antibody is selected from: a bispecific immunoglobulin (BsIgG), an immunoglobulin operatively linked to additional antigen-binding molecule, a bispecific antibody (BsAb) fragment, a bispecific fusion protein, or a BsAb conjugate.

44. The method of any of claims 39-43, wherein the therapy is an immunomodulatory agent or a combination of multiple immunomodulatory agents selected from: an immune checkpoint inhibitor, or an agonist of T cells, or an inhibitor of MDSCs and/or Treg cells, or lenalidomide, or a vaccine, or a cytokine, or an autologous T cell, or a modulator of a component associated with amino acid catabolism, signalling of tumor-derived extracellular ATP, adenosine signalling, adenosine production, chemokine and chemokine receptor, recognition of foreign organisms, or kinase signalling activity, or an inhibitor of IDO, COX2, ARG1, ArG2, iNOS, or phosphodiesterase; or a TLR agonist, or a chemokine antagonist.

45. The method of claim 44, wherein said immunomodulatory agents are immune checkpoint inhibitors.

46. The method of claim 45, wherein the immune checkpoint inhibitors inhibit immune checkpoints selected from the group consisting of CTLA4, PD-1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1 and A2aR.

47. An *in vitro* method of evaluating a subject's ability to generate activated T cells, comprising:

   (a) providing a sample comprising at least one T cell from a subject having a cancer;
   (b) providing a sample comprising at least one cancer cell from the subject;
   (c) forming an *ex vivo* reaction mixture comprising the at least one T cell, the at least one cancer cell, and a bispecific or multispecific antibody having a first element providing affinity for the T cell and a second element having affinity for the cancer cell, and incubating the mixture under conditions and for a period of time sufficient to activate the T cell, thereby producing at least one activated T cell;
   (d) selecting the activated T cell from (c), wherein the selection of the activated T cell is based on increased cancer cell killing activity determined by measuring an Effective E:T ratio, wherein said Effective E:T ratio is a ratio between effector cells (E) to target cells (T), effector cells (E) being defined as activated T cells (E) and target cells (T) being defined as cancer cells (T) killed by said activated T cells; and
   (e) quantifying the level of the activated T cells with a high Effective E:T ratio in the *ex vivo* reaction mixture, wherein an elevated level of activated T cells with a high Effective E:T ratio in the *ex vivo* reaction mixture compared to a reference level of activated T cells, generated in an *ex vivo* reaction mixture in the absence of the T cell, the cancer cell or the bispecific or multispecific antibody, indicates that the subject is capable of generating activated T cells with a high Effective E:T ratio and is identified as being likely to respond to the method of claims 31-38, and a low level of activated T cells with a high Effective E:T ratio in the *ex vivo* reaction mixture indicates that the subject is less capable of generating activated T cells and said subject is identified as being less likely to respond to the method of claims 31-38.

48. The method of claim 47, wherein in step (c) the *ex vivo* reaction mixture is incubated under conditions and for a period of time sufficient to allow the at least one T cell to acquire a cell surface marker from the at least one cancer cell.

49. The method of claim 47 or 48, further comprising one, two or all of the following in vitro steps:

   (i) expanding the activated T cells from (d);
   (ii) enriching for the activated T cell from (d); or
   (iii) purifying the activated T cell from (d).

50. The method of any of claims 47-49, wherein the cancer cell is a cell selected from a hematological cancer, a solid cancer or a metastatic cancer, or a circulating tumor cell or a combination thereof.

51. The method of any of claims 47-50, wherein the T cell is a cell selected from: a peripheral blood sample, a bone marrow sample, a lymph node sample, a tumor sample comprising a CTL and/or a TIL, or a combination thereof.

52. The method of claim 51, wherein the T cell expresses CD8 and/or CD25 and/or the T cell expresses CD4 and/or CD25.

53. The method of any of claims 47-52, wherein the presence of a tumor antigen on the activated T cell is detected.

54. The method of any of claims 47-53, wherein an elevated level of activated T cells in the *ex vivo* reaction mixture indicates that the subject is capable of generating activated T cells.

55. The method of any of claims 47-54, wherein the activated T cells comprises trogocytotic activated T cells.

**EP 3 445 391 B1**

56. The method of any of claims 47-55, wherein an elevated level of cancer cell killing in the presence of the *ex vivo* reaction mixture is indicative of the activity of the activated T cells in the reaction mixture.

57. An *in vitro* method of identifying the effectiveness of an activated T cell or preparation thereof, likely to be effective in killing a cancer cell *in vivo,* comprising:

(a) providing a population of activated T cells using the method of any of claims 1-20; and
(b) further determining at least one parameter selected from the group consisting of: cytotoxic activity of the activated T cells toward the cancer cell, viability of the activated T cells, proliferation of the activated T cells, reduced toxicity, reduced off-target effect, expression of cell surface markers on the activated T cells, level of trogocytotic cells, and pharmacodynamic parameters obtained by fitting dose response curves of activated T cells and cancer cells selected from the group consisting of: EC50, Emax, Basal E:T Ratios, and kinetic parameters.

58. An *in vitro* method of evaluating the potency of an activated T cell or preparation thereof, comprising:

(a) providing an activated T cell or a preparation thereof using the method of any of claims 1-20;
(b) providing cancer cells, wherein the cancer cells are from the subject;
(c) contacting the activated T cell or the preparation thereof with the cancer cells under conditions and for a period of time sufficient to allow the activated T cell to kill the cancer cells; and
(d) determining the level of cancer cells after step (c), and optionally determining the level of activated T cells after step (c);
wherein a decrease in the level or amount of cancer cells is indicative of increased cancer cell killing, and wherein no substantial change in the level or amount of cancer cells is indicative of decreased cancer cell killing.

59. The method of claim 58, wherein the contacting step further comprises the addition of a bispecific or multispecific antibody at increasing dosages to generate a dose response curve.

60. The method of claim 58 or 59, wherein an Effective E:T ratio of 1:10 or lower is indicative of potent cancer cell killing activity.

61. The method of any of claims 58-60, wherein if the T cells have potent cancer cell killing activity, the subject is identified as being a strong responder to the bispecific or multispecific antibody.


**Patentansprüche**

1. Ein *in vitro* Verfahren zur Herstellung einer aktivierten T-Zelle oder einer aktivierten T-Zell-Zubereitung, umfassend:

(a) Bereitstellen einer Probe, die mindestens eine T-Zelle von einem Subjekt, das Krebs hat, umfasst;
(b) Bereitstellen einer Probe, die mindestens eine Krebszelle eines Subjektes umfasst, wobei die Art des Krebses der Krebszelle die gleiche ist wie die Art des Krebses, die das Subjekt hat, das die Probe von Schritt (a) bereitstellt;
(c) Bilden eines *ex vivo* Reaktionsgemischs, das die mindestens eine T-Zelle, die mindestens eine Krebszelle und einen bispezifischen oder multispezifischen Antikörper mit einem ersten Element, das Affinität für die T-Zelle bereitstellt, und einem zweiten Element, das Affinität für die Krebszelle aufweist, umfasst, und Inkubieren des Gemischs unter Bedingungen und für einen Zeitraum, die ausreichen, um die T-Zelle zu aktivieren, wodurch mindestens eine aktivierte T-Zelle erzeugt wird; und
(d) Auswählen der aktivierten T-Zelle aus (c), wobei die Auswahl der aktivierten T-Zelle auf ihrer erhöhten Aktivität zur Abtötung von Krebszellen basiert, die durch Messen eines effektiven E:T-Verhältnisses bestimmt wird, wobei das effektive E:T-Verhältnis ein Verhältnis zwischen Effektorzellen (E) zu Zielzellen (T) ist, wobei Effektorzellen (E) als aktivierte T-Zellen definiert sind und Zielzellen (T) als von den aktivierten T-Zellen abgetötete Krebszellen definiert sind.

2. Verfahren nach Anspruch 1, wobei in Schritt (c) das *ex vivo* Reaktionsgemisch unter Bedingungen und für einen Zeitraum inkubiert wird, die ausreichen, um es der mindestens einen T-Zelle zu ermöglichen, einen Zelloberflächenmarker von der mindestens einen Krebszelle zu erhalten.

3. Verfahren nach Anspruch 1 oder 2, ferner umfassend einen, zwei oder alle der folgenden *in vitro* Schritte:

(i) Expandieren der aktivierten T-Zellen aus (d);
(ii) Anreichern der aktivierten T-Zellen aus (d); oder
(iii) Reinigen der aktivierten T-Zellen aus (d).

4. Verfahren nach einem der Ansprüche 1-3, wobei der bispezifische oder multispezifische Antikörper ausgewählt ist aus: Einem bispezifischen Immunglobulin (BsIgG), einem Immunglobulin, das operativ mit einem zusätzlichen Antigen-bindenden Molekül verknüpft ist, einem bispezifischen Antikörper (BsAb)-Fragment, das mindestens zwei scFv-Fragmente umfasst, die durch einen Linker operativ verknüpft sind, der ausgewählt ist aus einem Dual-Affinitäts-Retargeting (DART)-Antikörperfragment oder einem bispezifischen T-Zell-Engager (BiTE), einem bispezifischen Fusionsprotein oder einem BsAk-Konjugat.

5. Verfahren nach einem der Ansprüche 1-4, wobei der bispezifische oder multispezifische Antikörper aus der Liste ausgewählt ist, die aus BsMAb CD123/CD3, BsMAb CD19/CD3 und EpCAM/CD3 besteht.

6. Verfahren nach einem der Ansprüche 3-5, wobei der Auswahlschritt (d) und/oder Anreicherungsschritt (ii) das Verwenden einer fluoreszenzmarkierten Verbindung umfasst, die an (i) mindestens ein Krebsantigen bindet oder in die Krebszellmembran diffundiert oder (ii) mindestens einen Marker einer aktivierter T-Zellen oder sowohl (i) als auch (ii) bindet; oder das Verwenden eines Kügelchens, das mit einem Antikörper oder einem Fragment davon beschichtet ist, der an (i) mindestens ein Krebsantigen oder (ii) mindestens einen Marker aktivierter T-Zellen oder sowohl (i) als auch (ii) bindet.

7. Verfahren nach einem der Ansprüche 1- 6, wobei die aktivierte T-Zelle oder die Zubereitung mindestens eine CD8+ T-Zelle und/oder mindestens eine CD25+ T-Zelle und/oder mindestens eine CD8+/CD25+ T-Zelle und/oder mindestens eine CD4+/CD25+ T-Zelle und/oder mindestens einen zytotoxischen T-Lymphozyten (CTL) oder mindestens einen tumorinfiltrierenden Lymphozyten (TIL) und/oder mindestens eine trogozytotische T-Zelle umfasst.

8. Verfahren nach einem der Ansprüche 1-7, ferner umfassend das Abtrennen einzelner Klone aus dem aktivierten T-Zell-Zubereitung, wobei der Trennschritt klonale Expansion einzelner Zellen umfasst durch:

(i) Auftrennen der Zubereitung aktivierter T-Zellen in Einzelzellen und
(ii) Expandieren der Einzelzellen, um mindestens eine Zubereitung aktivierter T-Zellen zu erzeugen.

9. Verfahren nach einem der Ansprüche 1-8, wobei Schritt (a) und Schritt (b) das Bereitstellen einer Probe umfassen, die sowohl die Krebszelle als auch die T-Zelle umfasst.

10. Verfahren nach einem der Ansprüche 1-9, wobei die Probe (a) aus einem Gewebe mit einer Mikroumgebung stammt, wobei im Wesentlichen keine Komponenten aus der Probe entfernt oder isoliert wurden, ausgewählt aus: Vollblut, peripherem Blut, Knochenmark, Lymphknoten, einer Biopsie eines Primärtumors oder einer Biopsie einer Metastase oder Milz.

11. Verfahren nach einem der Ansprüche 1-10, wobei der Krebs der Probe (b) ein hämatologischer Krebs ist, ausgewählt aus: Hodgkin-Lymphom, Non-Hodgkin-Lymphom (B-Zell-Lymphom, diffuses großzelliges B-Zell-Lymphom, follikuläres Lymphom, chronische lymphatische Leukämie, Mantelzell-Lymphom, Randzone B-Zell-Lymphom, Burkitt-Lymphom, lymphoplasmazytisches Lymphom, Haarzell-Leukämie), akute myeloische Leukämie, chronische myeloische Leukämie, myelodysplastisches Syndrom, multiples Myelom oder akute lymphatische Leukämie.

12. Verfahren nach einem der Ansprüche 1-11, wobei der Krebs ein solider Krebs ist, ausgewählt aus: Eierstockkrebs, Rektumkarzinom, Magenkrebs, Hodenkrebs, Krebserkrankung der Afterregion, Gebärmutterkrebs, Darmkrebs, Nierenzellkarzinom, Leberkrebs, nicht-kleinzelligem Lungenkarzinom, Dünndarmkrebs, Speiseröhrenkrebs, Melanom, Kaposi-Sarkom, endokrine Tumore, Schilddrüsenkrebs, Nebenschilddrüsenkrebs, Nebennierenkrebs, Knochenkrebs, Bauchspeicheldrüsenkrebs, Hautkrebs, Kopf- oder Halskrebs, kutanem oder intraokulärem malignem Melanom, Glioblastom, Hypophysenadenom, Epidermoidkrebs, Plattenepithelkarzinom, Gebärmutterkrebs, Vaginalkarzinom, Weichteilsarkom, Harnröhrenkrebs, Vulvakarzinom, Peniskrebs, Blasenkrebs, Nieren- oder Harnleiterkrebs, Nierenbeckenkarzinom, spinaler Tumor, Neoplasma des zentralen Nervensystems (ZNS), primäres ZNS-Lymphom, Tumorangiogenese, metastatischen Läsionen dieser Krebserkrankungen und Kombinationen davon.

13. Verfahren nach einem der Ansprüche 1-12, wobei das Subjekt, das die Probe (a) und/oder die Probe (b) bereitstellt:

(i) keine vorherige Behandlung aufgrund des Krebses erhalten hat;
(ii) mindestens eine vorherige Behandlung für den Krebs erhalten hat; oder
(iii) eine minimale Resterkrankung (MRD) aufweist.

**14.** Verfahren nach einem der Ansprüche 1-13, ferner umfassend das Auswerten der krebsabtötenden Aktivität der aktivierten T-Zelle oder der aktivierten T-Zell-Zubereitung.

**15.** Verfahren nach Anspruch 14, wobei das Bewerten Folgendes umfasst:

(a) Bereitstellen einer aktivierten T-Zelle oder einer Zubereitung davon, die gemäß dem Verfahren der Ansprüche 1-14 erhältlich ist;
(b) Bereitstellen einer Krebszelle, wobei die Krebszelle von demselben Subjekt stammt;
(c) Inkontaktbringen der aktivierten T-Zelle oder dessen Zubereitung mit den Krebszellen für einen Zeitraum, der ausreicht, damit die aktivierte T-Zelle die Krebszellen abtöten kann;
(d) Bestimmen des Gehalts an Krebszellen nach Schritt (c) und optional Bestimmen des Gehalts an aktivierten T-Zellen nach Schritt (c); und optional
(e) Bestimmen des Verhältnisses von Krebszelle zu T-Zelle oder T-Zelle zu Krebszelle aus Schritt (d).

**16.** Verfahren nach Anspruch 15, wobei eine Abnahme des Gehalts oder der Menge von Krebszellen im Verhältnis zu einem Referenzgehalt auf eine erhöhte Abtötung von Krebszellen hinweist oder wobei eine verringerte oder keine wesentliche Änderung des Gehalts oder der Menge von Krebszellen im Verhältnis zu einem Referenzgehalt auf eine verringerte Abtötung von Krebszellen hinweist.

**17.** Verfahren nach Anspruch 15 oder 16, wobei ein effektives E:T-Verhältnis von 1:10 oder weniger anzeigt, dass die aktivierte T-Zelle oder die Zubereitung davon ein wirksamer Killer von Krebszellen ist, und wobei ein Verhältnis von 1:1, 1:3 oder 1:5 auf eine schlechte Abtötungsaktivität der T-Zelle hinweist.

**18.** Verfahren nach einem der Ansprüche 1 bis 17, wobei das effektive E:T-Verhältnis aus Werten berechnet wird, die aus der mathematischen Anpassung der experimentellen Werte einer Dosis-Wirkungskurve mehrerer Dosen des bispezifischen oder multispezifischen Antikörpers abgeleitet werden.

**19.** Verfahren nach einem der Ansprüche 1 bis 18, wobei in Schritt (c) dem Gemisch immunmodulatorische Mittel zugesetzt werden, wobei die immunmodulatorischen Mittel aus der Gruppe ausgewählt sind, bestehend aus Immuncheckpoint-Inhibitoren, Agonisten von T-Zellen und anderen immunmodulatorischen Arzneimitteln.

**20.** Verfahren nach Anspruch 19, wobei die Immuncheckpoint-Inhibitoren Immuncheckpoints hemmen, die aus der Gruppe ausgewählt sind, bestehend aus CTLA4, PD-1, PD-L1, PD-L2, TIM3, LAG3, CD160, 2B4, CD80, CD86, B7-H3 (CD276), B7-H4 (VTCN1), HVEM (TNFRSF14 oder CD270), BTLA, KIR, MHC-Klasse I, MHC-Klasse II, GAL9, VISTA, BTLA, TIGIT, LAIR1 und A2aR.

**21.** Zusammensetzung, umfassend aktivierte T-Zellen, die durch das Verfahren nach einem der Ansprüche 1-20 erhältlich sind, wobei die aktivierten T-Zellen ein effektives E:T-Verhältnis von 1:10 oder weniger aufweisen und wobei das effektive E:T-Verhältnis ein Verhältnis zwischen Effektorzellen (E) zu Zielzellen (T) ist, wobei die Effektorzellen (E) als die Anzahl der aktivierten T-Zellen (E) definiert sind, die durch einen bispezifischen oder multispezifischen Antikörper aktiviert werden, und die Zielzellen (T) als die Anzahl der Krebszellen definiert sind, die durch die aktivierten T-Zellen abgetötet werden.

**22.** Pharmazeutische Zusammensetzung, umfassend die Zusammensetzung aktivierter T-Zellen nach Anspruch 21 und einen pharmazeutisch zulässigen Träger.

**23.** Zusammensetzung aktivierter T-Zellen nach Anspruch 21 oder pharmazeutische Zusammensetzung nach Anspruch 22, wobei die Zusammensetzung oder pharmazeutische Zusammensetzung trogozytotisch aktivierte T-Zellen umfasst.

**24.** Zusammensetzung aktivierter T-Zellen nach Anspruch 21 oder 23 oder pharmazeutische Zusammensetzung nach Anspruch 22 oder 23, wobei die aktivierten T-Zellen mindestens 100 Kopien des Krebszelloberflächenmarkers und auch eine nachweisbare Menge eines bispezifischen oder multispezifischen Antikörpers umfassen.

25. Zusammensetzung aktivierter T-Zellen nach einem der Ansprüche 21, 23 oder 24 oder pharmazeutische Zusammensetzung nach einem der Ansprüche 22-24, wobei die aktivierten T-Zellen zytotoxische T-Lymphozyten oder Helfer-T-Zellen sind, ausgewählt aus CD8+ T-Zellen oder CD4+ T-Zellen.

26. Zusammensetzung aktivierter T-Zellen nach einem der Ansprüche 23-25 oder pharmazeutische Zusammensetzung nach einem der Ansprüche 23-25, umfassend trogozytotische T-Zellen in einer Konzentration von mindestens 30 % der Gesamtzahl der Zellen in der Zusammensetzung oder Zubereitung.

27. Zusammensetzung aktivierter T-Zellen nach einem der Ansprüche 21, 23-26 oder pharmazeutische Zusammensetzung nach einem der Ansprüche 22-26, umfassend immunmodulatorische Mittel, ausgewählt aus der Gruppe, bestehend aus Immuncheckpoint-Inhibitoren, Agonisten von T-Zellen und anderen immunmodulatorischen Arzneimitteln.

28. Zusammensetzung aktivierter T-Zellen nach Anspruch 27 oder pharmazeutische Zusammensetzung nach Anspruch 27, wobei Immuncheckpoint-Inhibitoren Immuncheckpoints hemmen, die aus der Gruppe ausgewählt sind, bestehend aus CTLA4, PD-1, PD-L1, PD-L2, TIM3, LAG3, CD160, 2B4, CD80, CD86, B7-H3 (CD276), B7-H4 (VTCN1), HVEM (TNFRSF14 oder CD270), BTLA, KIR, MHC-Klasse I, MHC-Klasse II, GAL9, VISTA, BTLA, TIGIT, LAIR1 und A2aR.

29. Pharmazeutische Zusammensetzung nach Anspruch 22 zur Verwendung in der adoptiven Krebstherapie zur Behandlung eines Subjektes, wobei das Subjekt dasselbe Subjekt wie in Schritt (a) ist und/oder wobei das Subjekt dasselbe Subjekt wie in Schritt (b) ist und/oder wobei das Subjekt sich von dem Subjekt des Schrittes (a) oder (b) unterscheidet.

30. Pharmazeutische Zusammensetzung zur Verwendung nach Anspruch 29 in der adoptiven Krebstherapie zur Behandlung eines Subjektes, das an einem hämatologischen Krebs leidet, ausgewählt aus chronischer lymphatischer Leukämie, akuter lymphatischer Leukämie, Non-Hodgkin-Lymphom, akuter myeloischer Leukämie, myelodysplastischem Syndrom oder multiplem Myelom.

31. Aktivierte T-Zelle, erhältlich durch das Verfahren nach einem der Ansprüche 1-20 oder die Zusammensetzung aktivierter T-Zellen nach einem der Ansprüche 21, 23-28 oder die pharmazeutische Zusammensetzung nach einem der Ansprüche 22-28 zur Verwendung in einem Verfahren zur Behandlung eines Subjektes, das Krebs hat, wobei das Verfahren die Verabreichung einer wirksamen Menge der aktivierten T-Zelle, der Zusammensetzung aktivierter T-Zellen oder der pharmazeutischen Zusammensetzung an das Subjekt umfasst, wobei die aktivierte T-Zelle, erhältlich durch das Verfahren nach einem der Ansprüche 1-20, ein effektives E:T-Verhältnis von 1:10 oder weniger aufweist und wobei das effektive E:T-Verhältnis ein Verhältnis zwischen Effektorzellen (E) zu Zielzellen (T) ist, wobei Effektorzellen (E) als die Anzahl der aktivierten T-Zellen (E) definiert sind, die durch einen bispezifischen oder multispezifischen Antikörper aktiviert werden, und Zielzellen (T) als die Anzahl der durch die aktivierten T-Zellen abgetöteten Krebszellen definiert sind.

32. Aktivierte T-Zelle, die Zusammensetzung aktivierter T-Zellen oder die pharmazeutische Zusammensetzung zur Verwendung in einem Verfahren zur Behandlung eines Subjektes, das Krebs hat, nach Anspruch 31, wobei das Verfahren umfasst:

    a) Bereitstellen einer Probe von dem Subjekt, wobei die Probe eine T-Zelle und eine Krebszelle umfasst;
    (b) Bilden eines *ex vivo* Reaktionsgemischs, das die Probe aus Schritt (a) und einen bispezifischen oder multispezifischen Antikörper umfasst, und Inkubieren des Gemischs unter Bedingungen und für einen Zeitraum, die ausreichen, um die T-Zelle zu aktivieren, wodurch mindestens eine aktivierte T-Zelle erzeugt wird;
    (c) Auswählen der aktivierten T-Zelle aus (c), wobei die Auswahl der aktivierten T-Zelle auf einer erhöhten Aktivität zur Abtötung von Krebszellen basiert, die durch Messen eines effektiven E:T-Verhältnisses bestimmt wird; und
    (d) Verabreichen einer wirksamen Menge der aktivierten T-Zellen an das Subjekt.

33. Aktivierte T-Zelle, Zusammensetzung aktivierter T-Zellen oder pharmazeutische Zusammensetzung zur Verwendung in einem Verfahren zur Behandlung eines Subjektes, das Krebs hat, nach Anspruch 32, wobei in Schritt (c) des Verfahrens das *ex vivo* Reaktionsgemisch unter Bedingungen und für einen Zeitraum inkubiert wird, die ausreichen, um es der mindestens einen T-Zelle zu ermöglichen, einen Zelloberflächenmarker von der mindestens einen Krebszelle zu erhalten.

34. Aktivierte T-Zelle, Zusammensetzung aktivierter T-Zellen oder pharmazeutische Zusammensetzung zur Verwendung in einem Verfahren zur Behandlung eines Subjektes, das Krebs hat, nach Anspruch 32 oder 33, wobei das Verfahren ferner einen, zwei oder alle der folgenden *in vitro* Schritte umfasst:

> (i) Expandieren der aktivierten T-Zellen aus (d);
> (ii) Anreichern der aktivierten T-Zelle aus (d); oder
> (iii) Reinigen der aktivierten T-Zelle aus (d).

35. Aktivierte T-Zelle, Zusammensetzung aktivierter T-Zellen oder pharmazeutische Zusammensetzung zur Verwendung in einem Verfahren zur Behandlung eines Subjektes, das Krebs hat, nach einem der Ansprüche 32-34, wobei das Verfahren ferner die Verabreichung eines zweiten therapeutischen Mittels oder Verfahrens umfasst.

36. Aktivierte T-Zelle, Zusammensetzung aktivierter T-Zellen oder pharmazeutische Zusammensetzung zur Verwendung in einem Verfahren zur Behandlung eines Subjektes, das Krebs hat, nach Anspruch 35, wobei in dem Verfahren das zweite therapeutische Mittel oder Verfahren aus mindestens einem von Folgendem ausgewählt ist: Chemotherapie, einer gezielten Krebstherapie, einem Onkolytikum, einem zytotoxischen Mittel, einer immunbasierten Therapie, einem Zytokin, einem chirurgischen Verfahren, einem Bestrahlungsverfahren, einem Agonisten von T-Zellen, einem Immuncheckpoint-Inhibitor, einem immunmodulatorischen Mittel oder einer Kombination mehrerer immunmodulatorischer Mittel, einem Impfstoff oder einer zellulären Immuntherapie.

37. Aktivierte T-Zelle, Zusammensetzung aktivierter T-Zellen oder pharmazeutische Zusammensetzung zur Verwendung nach Anspruch 36, wobei die immunmodulatorischen Mittel Immuncheckpoint-Inhibitoren sind.

38. Aktivierte T-Zelle, Zusammensetzung aktivierter T-Zellen oder pharmazeutische Zusammensetzung zur Verwendung nach Anspruch 37, wobei die Immuncheckpoint-Inhibitoren Immuncheckpoints hemmen, die aus der Gruppe ausgewählt sind, bestehend aus CTLA4, PD-1, PD-L1, PD-L2, TIM3, LAG3, CD160, 2B4, CD80, CD86, B7-H3 (CD276), B7-H4 (VTCN1), HVEM (TNFRSF14 oder CD270), BTLA, KIR, MHC-Klasse I, MHC-Klasse II, GAL9, VISTA, BTLA, TIGIT, LAIR1 und A2aR.

39. Ein *in vitro* Verfahren zur Bewertung der Ansprechbarkeit eines Subjektes auf eine Therapie, umfassend:

> (a) Bereitstellen einer Probe, die eine T-Zelle von einem Subjekt, das Krebs hat, umfasst;
> (b) Bereitstellen einer Probe, die eine Krebszelle von dem Subjekt umfasst;
> (c) Bilden eines *ex vivo* Reaktionsgemischs, das die T-Zelle, die Krebszelle, einen bispezifischen oder multispezifischen Antikörper mit einem ersten Element, das Affinität für die T-Zelle bereitstellt, und ein zweites Element, das Affinität für die Krebszelle aufweist, und die Therapie umfasst, und Inkubieren des Gemischs unter Bedingungen und für einen Zeitraum, die ausreichen, um die T-Zelle zu aktivieren, wodurch mindestens eine aktivierte T-Zelle erzeugt wird;
> (d) Auswählen der aktivierten T-Zelle aus (c), wobei die Auswahl der aktivierten T-Zelle auf einer erhöhten Aktivität zur Abtötung von Krebszellen basiert, die durch Messen eines effektiven E:T-Verhältnisses bestimmt wird, wobei das effektive E:T-Verhältnis ein Verhältnis zwischen Effektorzellen (E) zu Zielzellen (T) ist, wobei Effektorzellen (E) als aktivierte T-Zellen definiert sind und Zielzellen (T) als von den aktivierten T-Zellen abgetötete Krebszellen definiert sind, und wobei eine Zunahme der Zellabtötungsaktivität des Reaktionsgemischs in Gegenwart der Therapie relativ zu einem Referenzwert bei Abwesenheit der Therapie auf eine höhere Ansprechbarkeit auf die Therapie hinweist.

40. Verfahren nach Anspruch 39, wobei in Schritt (c) das *ex vivo* Reaktionsgemisch unter Bedingungen und für einen Zeitraum inkubiert wird, die ausreichen, um es der mindestens einen T-Zelle zu ermöglichen, einen Zelloberflächenmarker von der mindestens einen Krebszelle zu erhalten.

41. Verfahren nach Anspruch 39 oder 40, ferner umfassend einen, zwei oder alle der folgenden *in vitro* Schritte;

> (i) Expandieren der aktivierten T-Zellen aus (d);
> (ii) Anreichern der aktivierte T-Zelle aus (d); oder
> (iii) Reinigen der aktivierten T-Zelle aus (d).

42. Verfahren nach einem der Ansprüche 39-41, wobei Schritt (d) zusätzlich das Bestimmen des Gehalts an Krebszellen nach Schritt (c) und gegebenenfalls das Bestimmen des Gehalts an aktivierten T-Zellen nach Schritt (c) umfasst.

43. Verfahren nach einem der Ansprüche 39-42, wobei der bispezifische oder multispezifische Antikörper ausgewählt ist aus: Einem bispezifischen Immunglobulin (BsIgG), einem Immunglobulin, das operativ mit einem zusätzlichen Antigen-bindenden Molekül verbunden ist, einem bispezifischen Antikörper (BsAb)-Fragment, einem bispezifischen Fusionsprotein oder einem BsAk-Konjugat.

44. Verfahren nach einem der Ansprüche 39-43, wobei die Therapie ein immunmodulatorisches Mittel oder eine Kombination mehrerer immunmodulatorischer Mittel ist, ausgewählt aus: Einem Immuncheckpoint-Inhibitor oder einem Agonisten von T-Zellen oder einem Inhibitor von MDSCs und/oder Treg-Zellen oder Lenalidomid oder einem Impfstoff oder einem Zytokin oder einer autologen T-Zelle oder einem Modulator einer Komponente, die mit dem Aminosäurekatabolismus, der Signalisierung von tumorabgeleitetem extrazellulärem ATP, der Adenosin-Signalisierung, der Adenosin-Produktion, dem Chemokin und dem Chemokin-Rezeptor, der Erkennung von Fremdorganismen oder der Kinase-Signalisierungsaktivität oder einem Inhibitor von IDO, COX2, ARG1, ArG2, iNOS oder Phosphodiesterase; oder einem TLR-Agonisten oder einem Chemokin-Antagonisten assoziiert ist.

45. Verfahren nach Anspruch 44, wobei die immunmodulatorischen Mittel Immuncheckpoint-Inhibitoren sind.

46. Verfahren nach Anspruch 45, wobei die Immuncheckpoint-Inhibitoren Immuncheckpoints hemmen, die aus der Gruppe ausgewählt sind, bestehend aus CTLA4, PD-1, PD-L1, PD-L2, TIM3, LAG3, CD160, 2B4, CD80, CD86, B7-H3 (CD276), B7-H4 (VTCN1), HVEM (TNFRSF14 oder CD270), BTLA, KIR, MHC-Klasse I, MHC-Klasse II, GAL9, VISTA, BTLA, TIGIT, LAIR1 und A2aR.

47. Ein *in vitro* Verfahren zur Bewertung der Fähigkeit eines Subjektes, aktivierte T-Zellen zu erzeugen, umfassend:

(a) Bereitstellen einer Probe, die mindestens eine T-Zelle von einem Subjekt, das Krebs hat, umfasst;
(b) Bereitstellen einer Probe, die mindestens eine Krebszelle des Subjektes umfasst;
(c) Bilden eines *ex vivo* Reaktionsgemischs, das die mindestens eine T-Zelle, die mindestens eine Krebszelle und einen bispezifischen oder multispezifischen Antikörper mit einem ersten Element, das Affinität für die T-Zelle bereitstellt, und einem zweiten Element, das Affinität für die Krebszelle aufweist, umfasst, und Inkubieren des Gemischs unter Bedingungen und für einen Zeitraum, die ausreichen, um die T-Zelle zu aktivieren, wodurch mindestens eine aktivierte T-Zelle erzeugt wird;
(d) Auswählen der aktivierten T-Zelle aus (c), wobei die Auswahl der aktivierten T-Zelle auf einer erhöhten Aktivität zur Abtötung von Krebszellen basiert, die durch Messen eines effektiven E:T-Verhältnisses bestimmt wird, wobei das effektive E:T-Verhältnis ein Verhältnis zwischen Effektorzellen (E) zu Zielzellen (T) ist, wobei Effektorzellen (E) als aktivierte T-Zellen (E) definiert sind und Zielzellen (T) als von den aktivierten T-Zellen abgetötete Krebszellen (T) definiert sind; und
(e) Quantifizieren des Gehalts der aktivierten T-Zellen mit einem hohen effektiven E:T-Verhältnis in dem *ex vivo* Reaktionsgemisch, wobei ein erhöhter Gehalt aktivierter T-Zellen mit einem hohen effektiven E:T-Verhältnis in dem *ex vivo* Reaktionsgemisch im Vergleich zu einem Referenzgehalt aktivierter T-Zellen, der in einem *ex vivo* Reaktionsgemisch in Abwesenheit der T-Zelle, der Krebszelle oder des bispezifischen oder multispezifischen Antikörpers erzeugt wird, anzeigt, dass das Subjekt in der Lage ist, aktivierte T-Zellen mit einem hohen effektiven E:T-Verhältnis zu erzeugen, und als wahrscheinlich identifiziert wird, auf das Verfahren der Ansprüche 31-38 zu reagieren, und ein niedriger Gehalt aktivierter T-Zellen mit einem hohen effektiven E:T-Verhältnis in dem *ex vivo* Reaktionsgemisch anzeigt, dass das Subjekt weniger in der Lage ist, aktivierte T-Zellen zu erzeugen, und das Subjekt als weniger wahrscheinlich identifiziert wird, auf das Verfahren der Ansprüche 31-38 zu reagieren.

48. Verfahren nach Anspruch 47, wobei in Schritt (c) das *ex vivo* Reaktionsgemisch unter Bedingungen und für einen Zeitraum inkubiert wird, die ausreichen, um es der mindestens einen T-Zelle zu ermöglichen, einen Zelloberflächenmarker von der mindestens einen Krebszelle zu erhalten.

49. Verfahren nach Anspruch 47 oder 48, ferner umfassend einen, zwei oder alle der folgenden *in vitro* Schritte:

(i) Expandieren der aktivierten T-Zellen aus (d);
(ii) Anreichern der aktivierte T-Zelle aus (d); oder
(iii) Reinigen der aktivierten T-Zelle aus (d).

50. Verfahren nach einem der Ansprüche 47-49, wobei die Krebszelle eine Zelle ist, die aus einem hämatologischen Krebs, einem soliden Krebs oder einem metastatischen Krebs oder einer zirkulierenden Tumorzelle oder einer Kombination davon ausgewählt ist.

**51.** Verfahren nach einem der Ansprüche 47-50, wobei die T-Zelle eine Zelle ist, die ausgewählt ist aus: einer peripheren Blutprobe, einer Knochenmarkprobe, einer Lymphknotenprobe, einer Tumorprobe, die ein CTL und/oder ein TIL umfasst, oder einer Kombination davon.

**52.** Verfahren nach Anspruch 51, wobei die T-Zelle CD8 und/oder CD25 exprimiert und/oder die T-Zelle CD4 und/oder CD25 exprimiert.

**53.** Verfahren nach einem der Ansprüche 47-52, wobei das Vorhandensein eines Tumorantigens auf der aktivierten T-Zelle nachgewiesen wird.

**54.** Verfahren nach einem der Ansprüche 47-53, wobei ein erhöhter Gehalt an aktivierten T-Zellen in dem *ex vivo* Reaktionsgemisch anzeigt, dass das Subjekt in der Lage ist, aktivierte T-Zellen zu erzeugen.

**55.** Verfahren nach einem der Ansprüche 47-54, wobei die aktivierten T-Zellen trogozytotisch aktivierte T-Zellen umfassen.

**56.** Verfahren nach einem der Ansprüche 47-55, wobei ein erhöhtes Maß an Krebszellabtötung in Gegenwart des *ex vivo* Reaktionsgemischs auf die Aktivität der aktivierten T-Zellen in dem Reaktionsgemisch hinweist.

**57.** Ein *in vitro* Verfahren zum Identifizieren der Wirksamkeit einer aktivierten T-Zelle oder einer Zubereitung davon, die wahrscheinlich beim Abtöten einer Krebszelle *in vivo* wirksam ist, umfassend:

(a) Bereitstellen einer Population aktivierter T-Zellen unter Verwendung des Verfahrens nach einem der Ansprüche 1- 20; und
(b) ferner Bestimmen mindestens eines Parameters, der aus der Gruppe ausgewählt ist, bestehend aus: zytotoxischer Aktivität der aktivierten T-Zellen gegenüber der Krebszelle, Lebensfähigkeit der aktivierten T-Zellen, Proliferation der aktivierten T-Zellen, reduzierter Toxizität, reduziertem Off-Target-Effekt, Expression von Zelloberflächenmarkern auf den aktivierten T-Zellen, Niveau der trogozytotischen Zellen und pharmakodynamischen Parametern, die durch Anpassen von Dosis-Wirkungskurven von aktivierten T-Zellen und Krebszellen erhalten werden, die aus der Gruppe ausgewählt sind, bestehend aus: EC50, Emax, Basal E:T-Verhältnissen und kinetischen Parametern.

**58.** Ein *in vitro* Verfahren zur Bewertung der Wirksamkeit einer aktivierten T-Zelle oder einer Zubereitung davon, umfassend:

(a) Bereitstellen einer aktivierten T-Zelle oder einer Zubereitung davon unter Verwendung des Verfahrens nach einem der Ansprüche 1-20;
(b) Bereitstellen von Krebszellen, wobei die Krebszellen vom Subjekt stammen;
(c) Inkontaktbringen der aktivierten T-Zelle oder der Zubereitung davon mit den Krebszellen unter Bedingungen und für einen Zeitraum, die ausreichen, damit die aktivierte T-Zelle die Krebszellen abtöten kann; und
(d) Bestimmen des Gehalts an Krebszellen nach Schritt (c) und optional Bestimmen des Gehalts an aktivierten T-Zellen nach Schritt (c);

wobei eine Abnahme des Gehalts oder der Menge an Krebszellen auf eine erhöhte Abtötung von Krebszellen hinweist und wobei keine wesentliche Änderung des Gehalts oder der Menge an Krebszellen auf eine verminderte Abtötung von Krebszellen hinweist.

**59.** Verfahren nach Anspruch 58, wobei der Kontaktierungsschritt ferner die Zugabe eines bispezifischen oder multispezifischen Antikörpers bei steigenden Dosierungen umfasst, um eine Dosis-Wirkungskurve zu erzeugen.

**60.** Verfahren nach Anspruch 58 oder 59, wobei ein effektives E:T-Verhältnis von 1:10 oder weniger auf eine starke Aktivität zur Abtötung von Krebszellen hinweist.

**61.** Verfahren nach einem der Ansprüche 58-60, wobei, wenn die T-Zellen eine starke krebszellabtötende Aktivität aufweisen, das Subjekt als ein starker Responder auf den bispezifischen oder multispezifischen Antikörper identifiziert wird.

**Revendications**

1. Procédé de production *in vitro* d'une cellule T activée ou d'une préparation de cellules T activées, comprenant les étapes suivantes :

    (a) la fourniture d'un échantillon comprenant au moins une cellule T d'un sujet atteint d'un cancer ;
    (b) la fourniture d'un échantillon comprenant au moins une cellule cancéreuse d'un sujet, le type de cancer de la cellule cancéreuse étant le même que le type de cancer du sujet fournissant l'échantillon de l'étape (a) ;
    (c) la formation d'un mélange réactionnel *ex vivo* comprenant l'au moins une cellule T, l'au moins une cellule cancéreuse et un anticorps bispécifique ou multispécifique ayant un premier élément fournissant une affinité pour la cellule T et un second élément ayant une affinité pour la cellule cancéreuse, et l'incubation du mélange dans des conditions et pendant une période de temps suffisantes pour activer la cellule T, produisant ainsi au moins une cellule T activée ; et
    (d) la sélection de la cellule T activée de l'étape (c), la sélection de la cellule T activée étant basée sur son activité de destruction accrue des cellules cancéreuses déterminée en mesurant un rapport E:T effectif, ledit rapport E:T effectif étant un rapport entre les cellules effectrices (E) et les cellules cibles (T), les cellules effectrices (E) étant définies comme des cellules T activées et les cellules cibles (T) étant définies comme des cellules cancéreuses détruites par lesdites cellules T activées.

2. Procédé selon la revendication 1, dans lequel à l'étape (c), le mélange réactionnel ex *vivo* est incubé dans des conditions et pendant une période de temps suffisantes pour permettre à l'au moins une cellule T d'acquérir un marqueur de surface cellulaire à partir de l'au moins une cellule cancéreuse.

3. Procédé selon la revendication 1 ou 2, comprenant en outre une, deux ou toutes les étapes in vitro suivantes :

    (i) l'expansion des cellules T activées de l'étape (d) ;
    (ii) l'enrichissement des cellules T activées de l'étape (d) ; ou
    (iii) la purification des cellules T activées de l'étape (d).

4. Procédé selon l'une quelconque des revendications 1 à 3, dans lequel l'anticorps bispécifique ou multispécifique est choisi parmi : une immunoglobuline bispécifique (BsIgG), une immunoglobuline liée de manière opérationnelle à une molécule de liaison à l'antigène supplémentaire, un fragment d'anticorps bispécifique (BsAb) comprenant au moins deux fragments de scFv liés de manière opérationnelle par un lieur choisi parmi un fragment d'anticorps de reciblage à double affinité (DART) ou un engageur de cellules T bispécifique (BiTE), une protéine de fusion bispécifique ou un conjugué BsAb.

5. Procédé selon l'une quelconque des revendications 1 à 4, dans lequel l'anticorps bispécifique ou multispécifique est choisi dans la liste constituée de BsMAb CD123/CD3, BsMAb CD19/CD3 et EpCAM/CD3.

6. Procédé selon l'une quelconque des revendications 3 à 5, dans lequel l'étape de sélection (d) et/ou l'étape d'enrichissement (ii) comprennent l'utilisation d'un composé marqué par fluorescence qui se lie à (i) au moins un antigène cancéreux ou diffuse dans la membrane des cellules cancéreuses ou (ii) au moins un marqueur de cellules T activées, ou à la fois (i) et (ii) ; ou l'utilisation d'une bille revêtue d'un anticorps ou d'un fragment de celui-ci qui se lie à (i) au moins un antigène cancéreux ou (ii) au moins un marqueur de cellules T activées, ou à la fois (i) et (ii).

7. Procédé selon l'une quelconque des revendications 1 à 6, dans lequel la cellule T activée ou la préparation comprend au moins une cellule T CD8+ et/ou au moins une cellule T CD25+ et/ou au moins une cellule T CD8+/CD25+ et/ou au moins une cellule T CD4+/CD25+ et/ou au moins un lymphocyte T cytotoxique (CTL) ou au moins un lymphocyte infiltrant une tumeur (TIL) et/ou au moins une cellule T trogocytotique.

8. Procédé selon l'une quelconque des revendications 1 à 7, comprenant en outre la séparation des clones individuels de la préparation de cellules T activées, l'étape de séparation comprenant l'expansion clonale de cellules individuelles par :

    (i) séparation de la préparation de cellules T activées en cellules individuelles et
    (ii) expansion des cellules individuelles pour générer au moins une préparation de cellules T activées.

9. Procédé selon l'une quelconque des revendications 1 à 8, dans lequel l'étape (a) et l'étape (b) comprennent la fourniture d'un échantillon comprenant à la fois la cellule cancéreuse et la cellule T.

10. Procédé selon l'une quelconque des revendications 1 à 9, dans lequel l'échantillon (a) est dérivé d'un tissu avec un microenvironnement, sensiblement aucun composant n'ayant été retiré ou isolé de l'échantillon, choisi parmi : le sang total, le sang périphérique, la moelle osseuse, les ganglions lymphatiques, une biopsie d'une tumeur primaire ou une biopsie d'une métastase ou d'une rate.

11. Procédé selon l'une quelconque des revendications 1 à 10, dans lequel le cancer de l'échantillon (b) est un cancer hématologique choisi parmi : le lymphome de Hodgkin, le lymphome non hodgkinien (lymphome à cellules B, lymphome diffus à grandes cellules B, lymphome folliculaire, leucémie lymphoïde chronique, lymphome à cellules du manteau, lymphome à cellules B de la zone marginale, lymphome de Burkitt, lymphome lymphoplasmocytaire, leucémie à tricholeucocytes), la leucémie myéloïde aiguë, la leucémie myéloïde chronique, le syndrome myélodysplasique, le myélome multiple ou la leucémie aiguë lymphoblastique.

12. Procédé selon l'une des revendications 1 à 11, dans lequel le cancer est un cancer solide choisi parmi : le cancer de l'ovaire, le cancer du rectum, le cancer de l'estomac, le cancer du testicule, le cancer de l'anus, le cancer de l'utérus, le cancer du côlon, le carcinome à cellules rénales, le cancer du foie, le cancer bronchique non à petites cellules, le cancer de l'intestin grêle, le cancer de l'œsophage, le mélanome, le sarcome de Kaposi, le cancer du système endocrinien, le cancer de la glande thyroïde, le cancer de la parathyroïde, le cancer de la glande surrénale, le cancer des os, le cancer du pancréas, le cancer de la peau, le cancer de la tête ou du cou, le mélanome cutané ou intraoculaire, le gliome du tronc cérébral, l'adénome hypophysaire, le carcinome épidermoïde, le cancer du col de l'utérus, le carcinome cellulaire squameux, le cancer des trompes de Fallope, le carcinome endométrial, le cancer du vagin, le sarcome des tissus mous, le cancer de l'urètre, le cancer de la vulve, le cancer du pénis, le cancer de la vessie, le cancer du rein ou de l'urètre, le cancer du bassinet du rein, la tumeur de l'axis, la tumeur du système nerveux central (SNC), le lymphome primitif du SNC, l'angiogenèse tumorale, les lésions métastatiques desdits cancers, ou une combinaison de ces derniers.

13. Procédé selon l'une quelconque des revendications 1 à 12, dans lequel le sujet fournissant l'échantillon (a) et/ou l'échantillon (b) :

   (i) n'a pas reçu de traitement antérieur pour le cancer ;
   (ii) a reçu au moins un traitement antérieur pour le cancer ; ou
   (iii) a une maladie résiduelle minime (MRM).

14. Procédé selon l'une quelconque des revendications 1 à 13, comprenant en outre l'évaluation de l'activité de destruction du cancer de la cellule T activée ou de la préparation de cellules T activées.

15. Procédé selon la revendication 14, dans lequel l'évaluation comprend :

   (a) la fourniture d'une cellule T activée ou d'une préparation de celle-ci pouvant être obtenue selon le procédé des revendications 1 à 14 ;
   (b) la fourniture d'une cellule cancéreuse, la cellule cancéreuse provenant du même sujet ;
   (c) la mise en contact de la cellule T activée ou de sa préparation avec les cellules cancéreuses pendant une période de temps suffisante pour permettre à la cellule T activée de détruire les cellules cancéreuses ;
   (d) la détermination du niveau de cellules cancéreuses après l'étape (c), et éventuellement, la détermination du niveau de cellules T activées après l'étape (c) ; et éventuellement,
   (e) la détermination du rapport entre les cellules cancéreuses et les cellules T, ou entre les cellules T et les cellules cancéreuses, de l'étape (d).

16. Procédé selon la revendication 15, dans lequel une diminution du niveau ou de la quantité de cellules cancéreuses, par rapport à un niveau de référence, indique une augmentation de la destruction des cellules cancéreuses, ou dans lequel un changement mineur ou aucun changement substantiel du niveau ou de la quantité de cellules cancéreuses par rapport à un niveau de référence indique une diminution de la destruction des cellules cancéreuses

17. Procédé selon la revendication 15 ou 16, dans lequel un rapport E:T effectif de 1:10 ou moins indique que la cellule T activée ou sa préparation est un destructeur efficace de cellules cancéreuses, et dans lequel un rapport de 1:1, 1:3 ou 1:5 indique une faible activité de destruction de cellules T.

18. Procédé selon l'une quelconque des revendications 1 à 17, dans lequel le rapport E:T effectif est calculé à partir de valeurs dérivées de l'ajustement mathématique des valeurs expérimentales d'une courbe dose-réponse de doses multiples de l'anticorps bispécifique ou multispécifique.

19. Procédé selon l'une quelconque des revendications 1 à 18, dans lequel, dans l'étape (c), des agents immunomodulateurs sont ajoutés au mélange, lesdits agents immunomodulateurs étant choisis dans le groupe constitué d'inhibiteurs de points de contrôle immunitaire, d'agonistes de cellules T et d'autres médicaments immunomodulateurs.

20. Procédé selon la revendication 19, dans lequel les inhibiteurs de points de contrôle immunitaire inhibent les points de contrôle immunitaire choisis dans le groupe constitué de CTLA4, PD-1, PD-L1, PD-L2, TIM3, LAG3, CD160, 2B4, CD80, CD86, B7-H3 (CD276), B7-H4 (VTCN1), HVEM (TNFRSF14 ou CD270), BTLA, KIR, MHC de classe I, MHC de classe II, GAL9, VISTA, BTLA, TIGIT, LAIR1 et A2aR.

21. Composition comprenant des cellules T activées pouvant être obtenues par le procédé selon l'une quelconque des revendications 1 à 20, lesdites cellules T activées ayant un rapport E:T effectif de 1:10 ou moins et ledit rapport E:T effectif étant un rapport entre les cellules effectrices (E) et les cellules cibles (T), les cellules effectrices (E) étant définies comme le nombre de cellules T activées (E) activées par un anticorps bispécifique ou multispécifique et les cellules cibles (T) étant définies comme le nombre de cellules cancéreuses détruites par lesdites cellules T activées.

22. Composition pharmaceutique comprenant la composition de cellules T activées selon la revendication 21 et un support pharmaceutiquement acceptable.

23. Composition de cellules T activées selon la revendication 21, ou composition pharmaceutique selon la revendication 22, ladite composition ou composition pharmaceutique comprenant des cellules T activées trogocytotiques.

24. Composition de cellules T activées selon la revendication 21 ou 23, ou composition pharmaceutique selon la revendication 22 ou 23, les cellules T activées comprenant au moins 100 copies du marqueur de surface des cellules cancéreuses et comprenant également une quantité détectable d'anticorps bispécifique ou multispécifique.

25. Composition de cellules T activées selon l'une quelconque des revendications 21, 23 ou 24, ou composition pharmaceutique selon l'une quelconque des revendications 22 à 24, les cellules T activées étant des cellules T cytotoxiques ou des cellules T auxiliaires choisies parmi les cellules T CD8+ ou les cellules T CD4+.

26. Composition de cellules T activées selon l'une quelconque des revendications 23 à 25, ou composition pharmaceutique selon l'une quelconque des revendications 23 à 25, comprenant des cellules T trogocytotiques à une concentration d'au moins 30 % du nombre total de cellules dans la composition ou la préparation.

27. Composition de cellules T activées selon l'une quelconque des revendications 21, 23 à 26, ou composition pharmaceutique selon l'une quelconque des revendications 22 à 26, comprenant des agents immunomodulateurs choisis dans le groupe constitué d'inhibiteurs de points de contrôle immunitaire, d'agonistes de cellules T et d'autres médicaments immunomodulateurs.

28. Composition de cellules T activées selon la revendication 27 ou composition pharmaceutique selon la revendication 27, les inhibiteurs de points de contrôle immunitaire inhibant les points de contrôle immunitaire choisis dans le groupe constitué de CTLA4, PD-1, PD-L1, PD-L2, TIM3, LAG3, CD160, 2B4, CD80, CD86, B7-H3 (CD276), B7-H4 (VTCN1), HVEM (TNFRSF14 ou CD270), BTLA, KIR, MHC de classe I, MHC de classe II, GAL9, VISTA, BTLA, TIGIT, LAIR1 et A2aR.

29. Composition pharmaceutique selon la revendication 22 destinée à être utilisée en thérapie anticancéreuse adoptive pour traiter un sujet, le sujet étant le même sujet que celui de l'étape (a) et/ou le sujet étant le même sujet que celui de l'étape (b) et/ou le sujet étant différent du sujet de l'étape (a) ou (b).

30. Composition pharmaceutique destinée à être utilisée selon la revendication 29 en thérapie anticancéreuse adoptive pour traiter un sujet atteint d'un cancer hématologique choisi parmi la leucémie lymphoïde chronique, la leucémie aiguë lymphoblastique, le lymphome non hodgkinien, la la leucémie myéloïde aiguë, le syndrome myélodysplasique ou le myélome multiple.

**31.** Cellule T activée pouvant être obtenue par le procédé selon l'une quelconque des revendications 1 à 20 ou composition de cellules T activées selon l'une quelconque des revendications 21, 23 à 28 ou composition pharmaceutique selon l'une quelconque des revendications 22 à 28 destinée à être utilisée dans un procédé de traitement d'un sujet atteint d'un cancer, ledit procédé comprenant l'administration d'une quantité efficace de ladite cellule T activée, de la composition de cellules T activées ou de la composition pharmaceutique, au sujet, la cellule T activée pouvant être obtenue par le procédé selon l'une quelconque des revendications 1 à 20 ayant un rapport E:T effectif de 1:10 ou moins et ledit rapport E:T effectif étant un rapport entre les cellules effectrices (E) et les cellules cibles (T), les cellules effectrices (E) étant définies comme le nombre de cellules T activées (E) activées par un anticorps bispécifique ou multispécifique et les cellules cibles (T) étant définies comme le nombre de cellules cancéreuses détruites par lesdites cellules T activées.

**32.** Cellule T activée, composition de cellules T activées ou composition pharmaceutique, destinée à être utilisée dans un procédé de traitement d'un sujet atteint d'un cancer, selon la revendication 31, ledit procédé comprenant :

(a) la fourniture d'un échantillon du sujet, l'échantillon comprenant une cellule T et une cellule cancéreuse ;
(b) la formation d'un mélange réactionnel ex *vivo* comprenant l'échantillon de l'étape (a) et un anticorps bispécifique ou multispécifique et l'incubation du mélange dans des conditions et pendant une période de temps suffisantes pour activer la cellule T, produisant ainsi au moins une cellule T activée ;
(c) la sélection de la cellule T activée de l'étape (c), la sélection de la cellule T activée étant basée sur une activité de destruction accrue des cellules cancéreuses déterminée en mesurant un rapport E:T effectif ; et
(d) l'administration d'une quantité efficace des cellules T activées au sujet.

**33.** Cellule T activée, composition de cellules T activées ou composition pharmaceutique destinée à être utilisée dans un procédé de traitement d'un sujet atteint d'un cancer, selon la revendication 32, dans l'étape (c) dudit procédé, le mélange réactionnel ex *vivo* étant incubé dans des conditions et pendant une période de temps suffisantes pour permettre à l'au moins une cellule T d'acquérir un marqueur de surface cellulaire à partir de l'au moins une cellule cancéreuse.

**34.** Cellule T activée, composition de cellules T activées ou composition pharmaceutique destinée à être utilisée dans un procédé de traitement d'un sujet atteint d'un cancer, selon la revendication 32 ou 33, ledit procédé comprenant en outre une, deux ou toutes les étapes in vitro suivantes :

(i) l'expansion des cellules T activées de l'étape (d) ;
(ii) l'enrichissement des cellules T activées de l'étape (d) ; ou
(iii) la purification des cellules T activées de l'étape (d).

**35.** Cellule T activée, composition de cellules T activées ou composition pharmaceutique destinée à être utilisée dans un procédé de traitement d'un sujet atteint d'un cancer, selon l'une quelconque des revendications 32 à 34, ledit procédé comprenant en outre l'administration d'un second agent ou protocole thérapeutique.

**36.** Cellule T activée, composition de cellules T activées ou composition pharmaceutique destinée à être utilisée dans un procédé de traitement d'un sujet atteint d'un cancer, selon la revendication 35, dans ledit procédé, le second agent ou protocole thérapeutique étant choisi parmi au moins une chimiothérapie, une thérapie anticancéreuse ciblée, un médicament oncolytique, un agent cytotoxique, une immunothérapie, une cytokine, une intervention chirurgicale, une radiothérapie, un agoniste des cellules T, un inhibiteur de points de contrôle immunitaire, un agent immunomodulateur ou une combinaison de multiples agents immunomodulateurs, un vaccin ou une immunothérapie cellulaire.

**37.** Cellule T activée, composition de cellules T activées ou composition pharmaceutique destinée à être utilisée selon la revendication 36, lesdits agents immunomodulateurs étant des inhibiteurs de points de contrôle immunitaire.

**38.** Cellule T activée, composition de cellules T activées ou composition pharmaceutique destinée à être utilisée selon la revendication 37, les inhibiteurs de points de contrôle immunitaire inhibant les points de contrôle immunitaire choisis dans le groupe constitué de CTLA4, PD-1, PD-L1, PD-L2, TIM3, LAG3, CD160, 2B4, CD80, CD86, B7-H3 (CD276), B7-H4 (VTCN1), HVEM (TNFRSF14 ou CD270), BTLA, KIR, MHC de classe I, MHC de classe II, GAL9, VISTA, BTLA, TIGIT, LAIR1 et A2aR.

**39.** Procédé d'évaluation in vitro de la réponse d'un sujet à un traitement comprenant :

(a) la fourniture d'un échantillon comprenant une cellule T d'un sujet atteint d'un cancer ;
(b) la fourniture d'un échantillon comprenant une cellule cancéreuse du sujet ;
(c) la formation d'un mélange réactionnel ex *vivo* comprenant la cellule T, la cellule cancéreuse, un anticorps bispécifique ou multispécifique ayant un premier élément fournissant une affinité pour la cellule T et un second élément ayant une affinité pour la cellule cancéreuse, et le traitement, et l'incubation du mélange dans des conditions et pendant une période de temps suffisantes pour activer la cellule T, produisant ainsi au moins une cellule T activée ;
(d) la sélection de la cellule T activée de l'étape (c), la sélection de la cellule T activée étant basée sur une activité de destruction accrue des cellules cancéreuses déterminée en mesurant un rapport E:T effectif, ledit rapport E:T effectif étant un rapport entre les cellules effectrices (E) et les cellules cibles (T), les cellules effectrices (E) étant définies comme des cellules T activées et les cellules cibles (T) étant définies comme des cellules cancéreuses détruites par lesdites cellules T activées, et une augmentation de l'activité de destruction des cellules du mélange réactionnel avec traitement par rapport à une valeur de référence sans traitement indiquant une meilleure réponse au traitement.

40. Procédé selon la revendication 39, dans lequel à l'étape (c), le mélange réactionnel ex *vivo* est incubé dans des conditions et pendant une période de temps suffisantes pour permettre à l'au moins une cellule T d'acquérir un marqueur de surface cellulaire à partir de l'au moins une cellule cancéreuse.

41. Procédé selon la revendication 39 ou 40, comprenant en outre une, deux ou toutes les étapes in vitro suivantes :

(i) l'expansion des cellules T activées de l'étape (d) ;
(ii) l'enrichissement des cellules T activées de l'étape (d) ; ou
(iii) la purification des cellules T activées de l'étape (d).

42. Procédé selon l'une quelconque des revendications 39 à 41, dans lequel l'étape (d) comprend en outre la détermination du niveau de cellules cancéreuses après l'étape (c), et éventuellement, la détermination du niveau de cellules T activées après l'étape (c).

43. Procédé selon l'une quelconque des revendications 39 à 42, dans lequel l'anticorps bispécifique ou multispécifique est choisi parmi : une immunoglobuline bispécifique (BsIgG), une immunoglobuline liée de manière opérationnelle à une molécule de liaison à l'antigène supplémentaire, un fragment d'anticorps bispécifique (BsAb), une protéine de fusion bispécifique ou un conjugué BsAb.

44. Procédé selon l'une quelconque des revendications 39 à 43, dans lequel le traitement est un agent immunomodulateur ou une combinaison de plusieurs agents immunomodulateurs choisis parmi : un inhibiteur de points de contrôle immunitaire, ou un agoniste des cellules T, ou un inhibiteur des MDSC et/ou des cellules Treg, ou le lénalidomide, ou un vaccin, ou une cytokine, ou une cellule T autologue, ou un modulateur d'un composant associé au catabolisme des acides aminés, à la signalisation de l'ATP extracellulaire dérivé d'une tumeur, à la signalisation de l'adénosine, à la production d'adénosine, à la chimiokine et au récepteur de la chimiokine, à la reconnaissance d'organismes étrangers, ou à l'activité de signalisation des kinases, ou un inhibiteur de IDO, COX2, ARG1, ArG2, iNOS ou phosphodiestérase, ou un agoniste de TLR ou un antagoniste de chimiokine.

45. Procédé selon la revendication 44, dans lequel lesdits agents immunomodulateurs sont des inhibiteurs de points de contrôle immunitaire.

46. Procédé selon la revendication 45, dans lequel les inhibiteurs de points de contrôle immunitaire inhibent des points de contrôle immunitaire choisis dans le groupe constitué de CTLA4, PD-1, PD-L1, PD-L2, TIM3, LAG3, CD160, 2B4, CD80, CD86, B7-H3 (CD276), B7-H4 (VTCN1), HVEM (TNFRSF14 ou CD270), BTLA, KIR, MHC de classe I, MHC de classe II, GAL9, VISTA, BTLA, TIGIT, LAIR1 et A2aR.

47. Procédé d'évaluation *in vitro* de la capacité d'un sujet à générer des cellules T activées, comprenant :

(a) la fourniture d'un échantillon comprenant au moins une cellule T d'un sujet atteint d'un cancer ;
(b) la fourniture d'un échantillon comprenant au moins une cellule cancéreuse du sujet ;
(c) la formation d'un mélange réactionnel ex *vivo* comprenant l'au moins une cellule T, l'au moins une cellule cancéreuse et un anticorps bispécifique ou multispécifique ayant un premier élément fournissant une affinité pour la cellule T et un second élément ayant une affinité pour la cellule cancéreuse, et l'incubation du mélange dans des conditions et pendant une période de temps suffisantes pour activer la cellule T, produisant ainsi au moins une cellule T activée ;

(d) la sélection de la cellule T activée de l'étape (c), la sélection de la cellule T activée étant basée sur une activité de destruction accrue des cellules cancéreuses déterminée en mesurant un rapport E:T effectif, ledit rapport E:T effectif étant un rapport entre les cellules effectrices (E) et les cellules cibles (T), les cellules effectrices (E) étant définies comme des cellules T activées (E) et les cellules cibles (T) étant définies comme des cellules cancéreuses (T) détruites par lesdites cellules T activées ; et

(e) la quantification du niveau des cellules T activées ayant un rapport E:T effectif élevé dans le mélange réactionnel ex *vivo*, un niveau élevé de cellules T activées ayant un rapport E:T effectif élevé dans le mélange réactionnel ex *vivo* par rapport à un niveau de référence de cellules T activées, généré dans un mélange réactionnel ex *vivo* en l'absence de la cellule T, de la cellule cancéreuse ou de l'anticorps bispécifique ou multispécifique, indiquant que le sujet est capable de générer des cellules T activées ayant un rapport E:T effectif élevé et est identifié comme étant susceptible de répondre au procédé des revendications 31 à 38, et un faible niveau de cellules T activées ayant un rapport E:T effectif élevé dans le mélange réactionnel ex *vivo* indiquant que le sujet est moins capable de générer des cellules T activées et est identifié comme étant moins susceptible de répondre au procédé des revendications 31 à 38.

48. Procédé selon la revendication 47, dans lequel à l'étape (c), le mélange réactionnel ex *vivo* est incubé dans des conditions et pendant une période de temps suffisantes pour permettre à l'au moins une cellule T d'acquérir un marqueur de surface cellulaire à partir de l'au moins une cellule cancéreuse.

49. Procédé selon la revendication 47 ou 48, comprenant en outre une, deux ou toutes les étapes in vitro suivantes :

(i) l'expansion des cellules T activées de l'étape (d) ;
(ii) l'enrichissement des cellules T activées de l'étape (d) ; ou
(iii) la purification des cellules T activées de l'étape (d).

50. Procédé selon l'une quelconque des revendications 47 à 49, dans lequel la cellule cancéreuse est une cellule choisie parmi un cancer hématologique, un cancer solide ou un cancer métastatique, ou une cellule tumorale circulante ou une combinaison de celles-ci.

51. Procédé selon l'une quelconque des revendications 47 à 50, dans lequel la cellule T est une cellule choisie parmi : un échantillon de sang périphérique, un échantillon de moelle osseuse, un échantillon de ganglion lymphatique, un échantillon de tumeur comprenant un CTL et/ou un TIL, ou une combinaison de ceux-ci.

52. Procédé selon la revendication 51, dans lequel la cellule T exprime CD8 et/ou CD25 et/ou la cellule T exprime CD4 et/ou CD25.

53. Procédé selon l'une quelconque des revendications 47 à 52, dans lequel la présence d'un antigène tumoral sur la cellule T activée est détectée.

54. Procédé selon l'une quelconque des revendications 47 à 53, dans lequel un niveau élevé de cellules T activées dans le mélange réactionnel ex *vivo* indique que le sujet est capable de générer des cellules T activées.

55. Procédé selon l'une quelconque des revendications 47 à 54, dans lequel les cellules T activées comprennent des cellules T activées trogocytotiques.

56. Procédé selon l'une quelconque des revendications 47 à 55, dans lequel un niveau élevé de destruction des cellules cancéreuses en présence du mélange réactionnel ex *vivo* indique l'activité des cellules T activées dans le mélange réactionnel.

57. Procédé d'identification *in vitro* de l'efficacité d'une cellule T activée ou d'une préparation de celle-ci, susceptible d'être efficace pour détruire une cellule cancéreuse *in vivo,* comprenant :

(a) la fourniture d'une population de cellules T activées en utilisant le procédé selon l'une quelconque des revendications 1 à 20 ; et
(b) la détermination en outre d'au moins un paramètre choisi dans le groupe constitué de : l'activité cytotoxique des cellules T activées envers la cellule cancéreuse, la viabilité des cellules T activées, la prolifération des cellules T activées, la toxicité réduite, l'effet hors cible réduit, l'expression de marqueurs de surface cellulaire sur les cellules T activées, le niveau de cellules trogocytotiques et les paramètres pharmacodynamiques obtenus en ajustant les courbes dose-réponse des cellules T activées et des cellules cancéreuses choisies dans le groupe constitué de : CE50, Emax, rapports E:T basaux et paramètres cinétiques.

58. Procédé d'évaluation *in vitro* de la puissance d'une cellule T activée ou d'une préparation de celle-ci, comprenant :

(a) la fourniture d'une cellule T activée ou d'une préparation de celle-ci en utilisant le procédé selon l'une quelconque des revendications 1 à 20 ;
(b) la fourniture des cellules cancéreuses, les cellules cancéreuses provenant du sujet ;
(c) la mise en contact de la cellule T activée ou de sa préparation avec les cellules cancéreuses dans des conditions et pendant une période de temps suffisantes pour permettre à la cellule T activée de détruire les cellules cancéreuses ; et
(d) la détermination du niveau de cellules cancéreuses après l'étape (c), et éventuellement, la détermination du niveau de cellules T activées après l'étape (c) ;

une diminution du niveau ou de la quantité de cellules cancéreuses indiquant une augmentation de la destruction des cellules cancéreuses, et aucun changement substantiel du niveau ou de la quantité de cellules cancéreuses indiquant une diminution de la destruction des cellules cancéreuses.

59. Procédé selon la revendication 58, dans lequel l'étape de mise en contact comprend en outre l'addition d'un anticorps bispécifique ou multispécifique à des doses croissantes pour générer une courbe dose-réponse.

60. Procédé selon la revendication 58 ou 59, dans lequel un rapport E:T effectif de 1:10 ou moins indique une puissante activité de destruction des cellules cancéreuses.

61. Procédé selon l'une quelconque des revendications 58 à 60, dans lequel si les cellules T ont une puissante activité de destruction des cellules cancéreuses, le sujet est identifié comme répondant fortement à l'anticorps bispécifique ou multispécifique.

**FIGURE 1**

FIGURE 2

# Trogocytosis Measured by Cell Surface Labeling

**Antibody Labelling**

Fluorescently labelled antibody binds selectively target on cell surface

**SPECIFIC**

**Cell Tracker Dye**

Fluorescent Liphophilic dye distributes within lipid membrane bilayer

**NONSPECIFIC**

**FIGURE 3**

Cytotoxic T cell

Cancer cell

FIGURE 4

Trogocytosis Measured by Cell Surface Labeling

**Measurement of Trogocytosis by Labeling with Cell**

FIGURE 5

## FIGURE 6

| | T-Cells | Plasma cells | Basal E:T |
|---|---|---|---|
| Cells # at baseline | 2809 | 7679 | 1:2.7 |

| | T- Cells CD8+CD25+ | Live Tumor Cells | Effective E:T Ratio |
|---|---|---|---|
| Variation with drug conc. # | 265 | 1616 | 1:6.1 |

## FIGURE 7

| | T-Cells | CD19+ | Basal E:T |
|---|---|---|---|
| Cells # at baseline | 1101 | 33166 | 1:30.1 |

| | T- Cells CD8+CD25+ | Live Tumor Cells | Effective E:T Ratio |
|---|---|---|---|
| Variation with drug conc. # | 3799 | 21761 | 1:5.7 |

## FIGURE 8

### Sample 1

### Sample 2

## FIGURE 8 (Cont.)

### Sample 3

| | | T-cells CD8+CD25+ | Live Blast Cells | Effective Ratio E:T |
|---|---|---|---|---|
| **Variation with drug conc. #** | Sample 1 | 1234 | 983 | 1:0.8 |
| | Sample 2 | 1414 | 10057 | 1:7.1 |
| | Sample 3 | 245 | 489 | 1:2 |

FIGURE 9

FIGURE 9 (Cont.)

C

## FIGURE 10

**FIGURE 11**

## FIGURE 12

FIGURE 13

**FIGURE 13 (Cont.)**

C

(CD4+CD25+)&(CD8+CD25+) [1:1]

Tumor cells Survival % vs T-Cells #

FIGURE 14

| % CD5+CD25+ | Basal | 120h CD3-CD123 | After FACS |
|---|---|---|---|
| Sample 1 | 0.25% | 89.8% | 99.1% |
| Sample 2 | 0.3% | 75.2% | 99.6% |

FIGURE 15

**FIGURE 16**

FIGURE 17

**A**

**B**

FIGURE 18

# Before BsMAb

FIGURE 18 (Cont.)

## After BsMAb

**E**

**F**

**G**

**H**

FIGURE 19

A

B

C

D

[CD3/CD123]

[CD3/CD123]

FIGURE 20

A

B

FIGURE 21

A

B

C

FIGURE 22

Effective E:T ratio = ΔCTLs : Δ Blast (tumor) cells
= 60:6,000 = 1:100

## FIGURE 23

**A**

**B**

FIGURE 24

FIGURE 25

FIGURE 25 (Cont.)

# FIGURE 25 (Cont.)

**5**

# FIGURE 26

**FIGURE 27**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8034334 B
- US 20120244133 A
- WO 9856915 A
- WO 0164942 A
- WO 0034784 A
- WO 9945110 A
- WO 0060070 A
- US 20040009530 A
- US 7501121 B
- US 8008449 B
- WO 2006121168 A
- US 8354509 B
- WO 2009114335 A
- WO 2009101611 A
- US 8609089 B
- US 2010028330 A
- US 20120114649 A
- WO 2011066342 A
- WO 2010027827 A
- WO 2013079174 A
- WO 2010077634 A
- WO 2007005874 A
- US 7943743 B
- US 20120039906 A
- US 20110150892 A
- WO 2010019570 A
- WO 2014008218 A
- US 8552156 B
- WO 2011155607 A
- EP 2581113 A
- US 2014044728 A
- US 5811097 A
- US 8703491 B
- US 20130109101 A1
- US 20100298255 A1
- US 8313948 B
- US 8703491 B2
- US 8313948 B2

**Non-patent literature cited in the description**

- **WONG et al.** Blinatumomab induces autologous T-cell killing of chronic lymphocytic leukemia cells. *Haematologica*, 2013, vol. 98 (12), 1930-8
- **SPIESS et al.** *Mol. Immunol.*, 2015, vol. 67, 95-106
- *CHEMICAL ABSTRACTS*, 477202-00-9
- **BENNETT et al.** *Clin. Lymphoma, Myeloma, and Leukemia*, 2014, vol. 14 (4), 305-18
- **ADAMS JL et al.** Big opportunities for small molecules in immuno-oncology. *Nat Rev Drug Discov*, 2015, vol. 14 (9), 603-22
- **BENNETT TA et al.** Pharmacological profiles of acute myeloid leukemia treatments in patient samples by automated flow cytometry: a bridge to individualized medicine. *Clin. Lymphoma Myeloma Leuk.*, 2014, vol. 14 (4), 305-18
- **BUIL-BRUNA N et al.** Bringing Model-Based Prediction to Oncology Clinical Practice: A Review of Pharmacometrics Principles and Applications. *The Oncologist*, 2016, vol. 21 (2), 220-32
- **CHOTHIA C et al.** Canonical structures for the hypervariable regions of immunoglobulins. *J Mol Biol*, 1987, vol. 196 (4), 901-17
- **HAMID O et al.** Safety and tumor responses with lambrolizumab (anti-PD-1) in melanoma. *N Engl J Med*, 2013, vol. 369 (2), 134-44
- **KABAT EA et al.** Sequences of Proteins of Immunological Interest. US Department of Health and Human Services, 1991
- **LARBI A ; FULOP T.** From "truly naïve" to "exhausted senescent" T cells: when markers predict functionality. *Cytometry Part A*, 2014, vol. 85 (1), 25-35
- **MARTIN F et al.** The affinity-selection of a minibody polypeptide inhibitor of human interleukin-6. *EMBO J*, 1994, vol. 13 (22), 5303-9
- **MAUS MV et al.** Adoptive immunotherapy for cancer or viruses. *Annu Rev Immunol*, 2014, vol. 32, 189-225
- **MCCONNELL SJ et al.** Tendamistat as a scaffold for conformationally constrained peptide libraries. *J Mol. Biol*, 1995, vol. 250 (4), 460-70
- **MONTES M et al.** Optimum in vitro expansion of human antigen-specific CD8 T cells for adoptive transfer therapy. *Clin Exp Immunol*, 2005, vol. 142 (2), 292-302
- **MOULD DR et al.** Basic Concepts in Population Modeling, Simulation, and Model-Based Drug Development - Part 2: Introduction to Pharmacokinetics Modeling Methods. *CPT Pharmacometrics Syst Pharmacol*, 2013, vol. 2 (4), e38

- **PARDOLL DM**. The blockade of immune checkpoints in cancer immunotherapy. *Nat Rev Cancer*, 2012, vol. 12 (4), 252-64
- **ROSENBERG SA et al.** Use of tumor-infiltrating lymphocytes and interleukin-2 in the immunotherapy of patients with metastatic melanoma. *A preliminary report. N Eng J Med*, 1988, vol. 19 (25), 1676-80
- **SCOTT AM et al.** Monoclonal antibodies in cancer therapy. *Cancer Immun*, 2012, vol. 12, 14
- **SERAFINI P et al.** Myeloid-derived suppressor cells promote cross-tolerance in B-cell lymphoma by expanding regulatory T cells. *Cancer Res*, 2008, vol. 68 (13), 5439-49
- **SONG DG et al.** CD27 costimulation augments the survival and antitumor activity of redirected human T cells in vivo. *Blood*, 2012, vol. 119 (3), 696-706
- **SPIESS C et al.** Alternative molecular formats and therapeutic applications for bispecific antibodies. *Mol Immunol*, 2015, vol. 67, 95-106
- **TRAMONTANO A et al.** The making of the minibody: an engineered beta-protein for the display of conformationally constrained peptides. *J Mol. Recognit*, 1994, vol. 7 (1), 9-24
- **UPTON RN et al.** Basic Concepts in Population Modeling, Simulation, and Model-Based Drug Development: Part 3-Introduction to Pharmacodynamic Modeling Methods. *CPT Pharmacometrics Syst Pharmacol*, 2014, vol. 3 (1), e88
- Guidance for Industry Population Pharmacokinetics. US Department of Health and Human Services Food, 1999